(19) Europäisches Patentamt / European Patent Office / Office européen des brevets

(11) **EP 4 417 607 A1**

(12) **EUROPEAN PATENT APPLICATION**
published in accordance with Art. 153(4) EPC

(43) Date of publication:
**21.08.2024 Bulletin 2024/34**

(21) Application number: **22869415.4**

(22) Date of filing: **16.09.2022**

(51) International Patent Classification (IPC):
***C07D 471/04*** *(2006.01)* ***A61K 31/519*** *(2006.01)*
***A61P 35/00*** *(2006.01)*

(52) Cooperative Patent Classification (CPC):
**A61K 31/519; A61P 35/00; C07D 471/04**

(86) International application number:
**PCT/CN2022/119376**

(87) International publication number:
**WO 2023/041049 (23.03.2023 Gazette 2023/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:
**17.09.2021 CN 202111094943**
**22.10.2021 CN 202111236113**
**11.11.2021 CN 202111333282**
**29.11.2021 CN 202111431104**
**30.12.2021 CN 202111683730**
**11.02.2022 CN 202210185443**
**02.04.2022 CN 202210343652**

(71) Applicant: **Nanjing Zaiming Pharmaceutical Co., Ltd.**
**Nanjing, Jiangsu 210032 (CN)**

(72) Inventors:
- **ZHANG, Yan**
  **Nanjing, Jiangsu 210042 (CN)**
- **YANG, Shengwei**
  **Nanjing, Jiangsu 210042 (CN)**
- **YANG, Guimei**
  **Shanghai 201318 (CN)**
- **WANG, Feng**
  **Nanjing, Jiangsu 210042 (CN)**
- **TANG, Feng**
  **Shanghai 201318 (CN)**
- **PENG, Shaoping**
  **Nanjing, Jiangsu 210042 (CN)**
- **TANG, Renhong**
  **Shanghai 201318 (CN)**
- **REN, Jinsheng**
  **Nanjing, Jiangsu 210042 (CN)**

(74) Representative: **Richly & Ritschel Patentanwälte PartG mbB**
**Sattlerweg 20**
**51429 Bergisch Gladbach (DE)**

# (54) HETEROCYCLIC COMPOUND AS SOS1 INHIBITOR AND USES THEREOF

(57) Provided are a class of heterocyclic compounds as an SOS1 inhibitor, specifically disclosed are a compound represented by formula (I), a pharmaceutically acceptable salt thereof, a pharmaceutical composition containing the compound and the salt thereof, and a use of the compound and the salt thereof in the preparation of drugs for treating cancers.

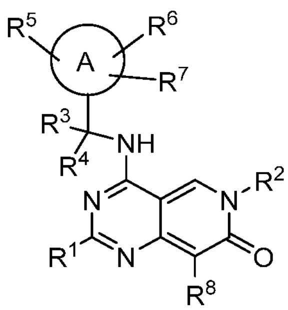

(I)

EP 4 417 607 A1

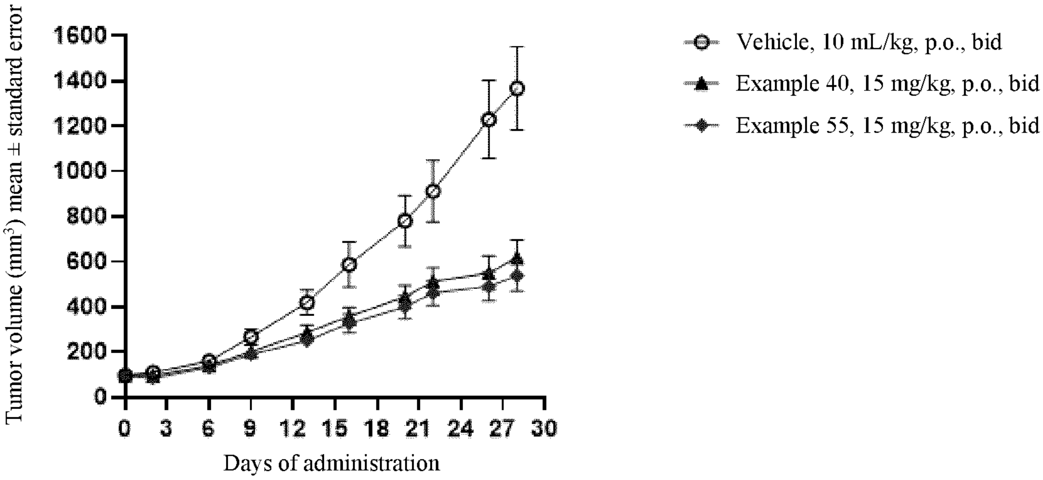
Tumor volume (mm3) mean ± standard error
1600
1400
1200
1000
800
600
400
200
0
0 3 6 9 12 15 18 21 24 27 30
Days of administration
Vehicle, 10 mL/kg, p.o., bid
Example 40, 15 mg/kg, p.o., bid
Example 55, 15 mg/kg, p.o., bid

FIG. 1

## Description

CROSS-REFERENCE TO RELATED APPLICATIONS

The present application claims the benefit and priority to the following 7 Chinese Patent Applications for Invention, the contents of which are incorporated herein by reference in their entirety:

Chinese Patent Application No. 202111094943.1 filed with China National Intellectual Property Administration on Sept. 17, 2021;
Chinese Patent Application No. 202111236113.8 filed with China National Intellectual Property Administration on Oct. 22, 2021;
Chinese Patent Application No. 202111333282.3 filed with China National Intellectual Property Administration on Nov. 11, 2021;
Chinese Patent Application No. 202111431104.4 filed with China National Intellectual Property Administration on Nov. 29, 2021;
Chinese Patent Application No. 202111683730.2 filed with China National Intellectual Property Administration on Dec. 30, 2021;
Chinese Patent Application No. 202210185443.7 filed with China National Intellectual Property Administration on Feb. 11, 2022; and
Chinese Patent Application No. 202210343652.X filed with China National Intellectual Property Administration on Apr. 02, 2022.

### TECHNICAL FIELD

The present invention belongs to the technical field of pharmaceuticals, and relates to an SOS1 inhibitor compound or an optical isomer thereof or a pharmaceutically acceptable salt thereof, a pharmaceutical composition comprising same, and use thereof as an SOS1 inhibitor.

### BACKGROUND

The proteins of the RAS family include KRAS (V-Ki-ras2 Kirsten rat sarcoma viral oncogene homolog), NRAS (neuroblastoma RAS viral oncogene homolog), HRAS (Harvey rat sarcoma viral oncogene), and the like, and are small GTPases present in cells in the GTP-bound state or the GDP-bound state (McCormick et al., J.Mol.Med. (Berl)., 2016, 94(3), 253-8; Nimnual et al., Sci. STKE., 2002, 2002(145): pe36).

The proteins of the RAS family play an important role in human cancers. Tumors caused by RAS protein mutations account for 20-30% of all tumors in humans, and RAS protein mutations are considered as tumorigenic drivers, particularly in lung cancer, colorectal cancer, and pancreatic cancer (Malumbres & Barbacid 2002 Nature Reviews Cancer*,* Pylayeva-Gupta et al., 2011 Nature Reviews Cancer*).*

SOS1 (son of sevenless homolog 1) protein is a regulatory protein widely expressed in cells, and plays an important role in the regulation in RAS and RAC signal transduction pathways in cells as a guanine nucleotide exchange factor of RAS and RAC proteins. SOS1 has two binding sites for the proteins of the RAS family: a catalytic site that binds to a GDP-bound RAS family protein to facilitate guanine nucleotide exchange; an allosteric site that binds to a GTP-bound RAS family protein, which results in a further increase in the catalytic GEF function of SOS1 (Freedman et al., Proc. Natl. Acad. Sci. USA., 2006, 103(45): 16692-7; Pierre et al., Biochem. Pharmacol., 2011, 82(9): 1049-56). Published data suggest that SOS1 is involved in mutant KRAS activation and oncogenic signaling in cancer (Jeng et al., Nat. Commun., 2012, 3: 1168). Consumption of the SOS1 level decreases the proliferation rate and survival of tumor cells carrying KRAS mutations, whereas no effect is observed in KRAS wild-type cell lines. The effect of SOS1 loss can not be compensated by introducing SOS1 with a mutation in the catalytic site, further demonstrating the important role of the SOS1 GEF activity in KRAS mutant cancer cells.

In recent decades, the RAS family protein-SOS1 protein interaction has been under increasing investigation. Small activating molecules have been identified that bind to the lipophilic pocket of SOS1 in close proximity to the RAS binding site (Burns et al., Proc. Natl. Acad. Sci. 2014, 111(9): 3401-6). However, the binding of these molecules appears to result in an increase in nucleotide exchange, thereby activating, rather than inactivating, RAS.

Although some SOS1 small-molecule inhibitors are reported in the existing documents (such as Patent Documents WO2018/115380A1 and WO2018/172250A1), a large number of patients still cannot obtain a satisfactory clinical treatment effect, so that the development of SOS1 inhibitors with better activity, selectivity, and safety is still needed.

## SUMMARY

The present invention provides a compound of formula (I) and a stereoisomer thereof or a pharmaceutically acceptable salt thereof. The compounds can inhibit the activity of SOS1, thereby affecting the biological function.

The present invention provides a compound of formula (I) or a stereoisomer thereof or a pharmaceutically acceptable salt thereof,

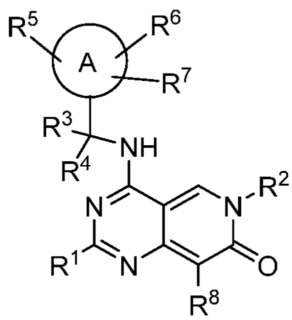

(I)

wherein

ring A is selected from $C_{6-10}$ aryl, 5-10 membered heterocyclyl, or 5-10 membered heteroaryl;
$R^1$ is selected from H, halogen, hydroxy, cyano, amino, $C_{1-6}$ alkyl, $C_{1-6}$ haloalkyl, $C_{1-6}$ deuterated alkyl, -O-$C_{1-6}$ alkyl, or $C_{3-6}$ cycloalkyl;
$R^2$ is selected from $C_{1-6}$ alkyl, $C_{2-6}$ alkenyl, $C_{2-6}$ alkynyl, $C_{3-10}$ cycloalkyl, $C_{4-10}$ cycloalkenyl, $C_{6-10}$ aryl, 3-10 membered heterocyclyl, or 5-10 membered heteroaryl, wherein the $C_{1-6}$ alkyl, $C_{2-6}$ alkenyl, $C_{2-6}$ alkynyl, $C_{3-10}$ cycloalkyl, $C_{4-10}$ cycloalkenyl, $C_{6-10}$ aryl, 3-10 membered heterocyclyl, or 5-10 membered heteroaryl is optionally substituted with $R^{2b}$ and/or $R^{2c}$;
$R^{2b}$ is independently selected from halogen, hydroxy, cyano, amino, -$OR^{2c}$, -$N(R^{2c})_2$, -$NHR^{2c}$, - $C(O)R^{2c}$ -$C(O)N(R^{2c})_2$, -$C(O)NHR^{2c}$, -$C(O)OR^{2c}$, -$S(O)_2R^{2c}$, -$S(O)_2N(R^{2c})_2$, -$S(O)_2NHR^{2c}$,-$NHC(O)R^{2c}$, -$C(S)R^{2c}$, or -$N(C_{1-4}$ alkyl)$C(O)R^{2c}$;
$R^{2c}$ is independently selected from $C_{1-6}$ alkyl, $C_{2-6}$ alkenyl, $C_{2-6}$ alkynyl, $C_{3-10}$ cycloalkyl, $C_{6-10}$ aryl, 3-10 membered heterocyclyl, or 5-10 membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3-10 membered heterocyclyl, or 5-10 membered heteroaryl is optionally substituted with $R^{2d}$ and/or $R^{2f}$;
$R^{2d}$ is independently selected from halogen, cyano, -$OR^{2f}$, -$N(R^{2f})_2$, -$C(O)R^{2f}$, -$C(O)N(R^{2f})_2$, - $C(O)OR^{2f}$, -$S(O)_2R^{2f}$, -$S(O)_2N(R^{2f})_2$, -$N(C_{1-4}$ alkyl)$R^{2f}$, -$NHC(O)R^{2f}$, or -$N(C_{1-4}$ alkyl)$C(O)R^{2f}$;
$R^{2f}$ is independently selected from H, $C_{1-6}$ alkyl, $C_{2-6}$ alkenyl, $C_{2-6}$ alkynyl, $C_{3-10}$ cycloalkyl, $C_{6-10}$ aryl, 3-10 membered heterocyclyl, or 5-10 membered heteroaryl;
$R^3$ and $R^4$ are independently selected from H, deuterium, $C_{1-3}$ deuterated alkyl, $C_{1-6}$ alkyl, or $C_{1-6}$ haloalkyl;
$R^5$, $R^6$, and $R^7$ are independently selected from H, nitro, halogen, hydroxy, cyano, amino, $C_{1-6}$ alkyl, $C_{3-6}$ cycloalkyl, -O-$C_{1-6}$ alkyl, -O-$C_{3-6}$ cycloalkyl, -0-(3-8 membered heterocyclyl), -$S(O)_2$-$C_{1-4}$ alkyl, -$P(O)(R^{x2})_2$, -$C(O)R^{x2}$, -$C(O)N(R^{x2})_2$, -$C(O)OR^{x2}$,

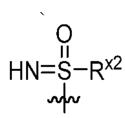

, -$SF_5$, 3-8 membered heterocyclyl, or 5-10 membered heteroaryl, wherein the $C_{1-6}$ alkyl, $C_{3-6}$ cycloalkyl, -$S(O)_2$-$C_{1-4}$ alkyl, 3-8 membered heterocyclyl, or 5-10 membered heteroaryl is optionally substituted with $R^{x1}$;
$R^{x1}$ is independently selected from halogen, hydroxy, cyano, amino, or -$S(O)_2$-$C_{1-4}$ alkyl;
$R^{x2}$ is independently selected from H or $C_{1-6}$ alkyl;
$R^8$ is selected from H, halogen, hydroxy, cyano, amino, nitro,

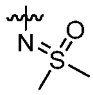, 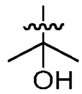,

-C(O)$R^{8a}$, -C(O)NH$R^{8a}$, -OC(O)$R^{8a}$, -NHC(O)$R^{8a}$, $C_{1-6}$ alkyl, $C_{2-6}$ alkenyl, $C_{2-6}$ alkynyl, $C_{1-6}$ haloalkyl, $C_{3-6}$ cycloalkyl, -$S(O)_2$-$C_{1-6}$ alkyl, -O-$C_{1-6}$ alkyl, -O-$C_{1-6}$ deuterated alkyl, -O-$C_{3-6}$ cycloalkyl, -O-(3-10 membered heterocyclyl), -O-(5-10 membered heteroaryl), -O-($C_{6-10}$ aryl), 3-10 membered heterocyclyl, 5-10 membered heteroaryl, or $C_{6-10}$ aryl, wherein the amino, $C_{1-6}$ alkyl, $C_{2-6}$ alkenyl, $C_{2-6}$ alkynyl, $C_{3-6}$ cycloalkyl, -$S(O)_2$-$C_{1-6}$ alkyl, -O-$C_{1-6}$ alkyl, -O-$C_{1-6}$ deuterated alkyl, -O-$C_{3-6}$ cycloalkyl, -O-(3-10 membered heterocyclyl), -O-(5-10 membered heteroaryl), -O-($C_{6-10}$ aryl), 3-10 membered heterocyclyl, 5-10 membered heteroaryl, or $C_{6-10}$ aryl is optionally substituted with $R^{8a}$;

$R^{8a}$ is independently selected from deuterium, halogen, hydroxy, cyano, amino, $C_{1-6}$ alkyl, $C_{1-6}$ deuterated alkyl, -O-$C_{1-6}$ alkyl, $C_{1-6}$ haloalkyl, -C(O)-$C_{1-6}$ alkyl, -$S(O)_2R^{8b}$, $C_{3-6}$ cycloalkyl, 3-8 membered heterocyclyl, or 5-6 membered heteroaryl, wherein the amino, -O-$C_{1-6}$ alkyl, $C_{1-6}$ alkyl, $C_{3-6}$ cycloalkyl, 3-8 membered heterocyclyl, or 5-6 membered heteroaryl is optionally substituted with $R^{8b}$; $R^{8b}$ is independently selected from halogen, hydroxy, cyano, amino, $C_{1-6}$ alkyl, 3-8 membered heterocyclyl, -C(O)-$C_{1-6}$ alkyl, or $C_{3-6}$ cycloalkyl, wherein the 3-8 membered heterocyclyl is optionally substituted with $C_{1-6}$ alkyl;

provided that when $R^8$ is selected from H, halogen, $C_{1-6}$ alkyl, or $C_{3-6}$ cycloalkyl, then $R^5$ is selected from nitro, hydroxy, cyano, -O-$C_{1-6}$ alkyl, -O-$C_{3-6}$ cycloalkyl, -O-(3-8 membered heterocyclyl), - P(O)$(R^{x2})_2$, -C(O)$R^{x2}$, -C(O)N$(R^{x2})_2$, -C(O)O$R^{x2}$,

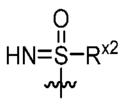, $-SF_5$, 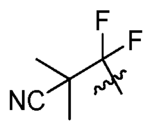, 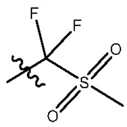,

or 5-10 membered heteroaryl, wherein the 5-10 membered heteroaryl is optionally substituted with $R^{x1}$.

In some embodiments, ring Ais selected from phenyl, 9-10 membered heterocyclyl, or 9-10 membered heteroaryl.

In some embodiments, ring A is selected from phenyl, 5-6 membered heterocyclyl, or 5-6 membered heteroaryl.

In some embodiments, ring A is selected from phenyl.

In some embodiments, $R^1$ is selected from H, halogen, $C_{1-6}$ alkyl, or $C_{1-6}$ deuterated alkyl.

In some embodiments, $R^1$ is selected from H, $C_{1-6}$ alkyl, or $C_{1-6}$ deuterated alkyl.

In some embodiments, $R^1$ is selected from H, $C_{1-3}$ alkyl, or $C_{1-3}$ deuterated alkyl.

In some embodiments, $R^1$ is selected from $C_{1-6}$ alkyl.

In some embodiments, $R^1$ is selected from H or $CH_3$. In some embodiments, $R^2$ is selected from $C_{3-10}$ cycloalkyl, $C_{4-10}$ cycloalkenyl, $C_{6-10}$ aryl, 3-10 membered heterocyclyl, or 5-10 membered heteroaryl, wherein the $C_{3-10}$ cycloalkyl, $C_{4-10}$ cycloalkenyl, $C_{6-10}$ aryl, 3-10 membered heterocyclyl, or 5-10 membered heteroaryl is optionally substituted with $R^{2b}$ and/or $R^{2c}$.

In some embodiments, $R^2$ is selected from $C_{3-10}$ cycloalkyl or 3-10 membered heterocyclyl, wherein the $C_{3-10}$ cycloalkyl or 3-10 membered heterocyclyl is optionally substituted with $R^{2b}$ and/or $R^{2c}$.

In some embodiments, $R^2$ is selected from $C_{3-6}$ cycloalkyl or 4-9 membered heterocyclyl, wherein the $C_{3-6}$ cycloalkyl or 4-9 membered heterocyclyl is optionally substituted with $R^{2b}$ and/or $R^{2c}$.

In some embodiments, $R^2$ is selected from $C_{3-6}$ cycloalkyl or 5-6 membered heterocyclyl, wherein the $C_{3-6}$ cycloalkyl or 5-6 membered heterocyclyl is optionally substituted with $R^{2b}$ and/or $R^{2c}$.

In some embodiments, $R^2$ is selected from cyclopropyl, tetrahydropyrrolyl, or morpholinyl, wherein the cyclopropyl, tetrahydropyrrolyl, or morpholinyl is optionally substituted with $R^{2b}$ and/or $R^{2c}$.

In some embodiments, $R^2$ is selected from cyclopropyl or tetrahydropyrrolyl, wherein the cyclopropyl or tetrahydropyrrolyl is optionally substituted with $R^{2b}$ and/or $R^{2c}$.

In some embodiments, $R^2$ is selected from tetrahydropyrrolyl, wherein the tetrahydropyrrolyl is optionally substituted with $R^{2b}$ and/or $R^{2c}$.

In some embodiments, $R^2$ is selected from cyclopropyl, wherein the cyclopropyl is optionally substituted with $R^{2b}$ and/or $R^{2c}$, and $R^8$ is not selected from

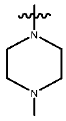

or $C(O)CH_3$.

In some embodiments, $R^2$ is selected from

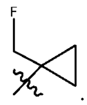.

In some embodiments, $R^2$ is selected from

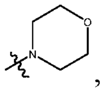,

and $R^8$ is selected from hydroxy, amino, $-CH=CH_2$, $-O-CD_3$, $CHF_2$, $CF_3$, $-O-CH_3$, $-O-CH_2CH_3$, $-O-CH_2CF_3$, $-O-CH_2CHF_2$,

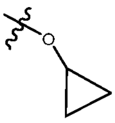, 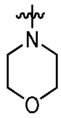, 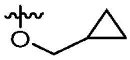,

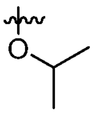, 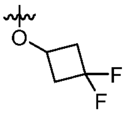, 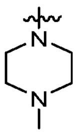, 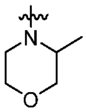, 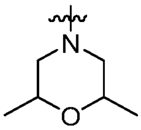, 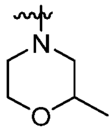, 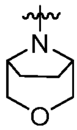, 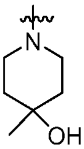, 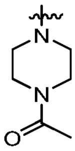, 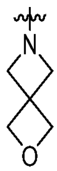,

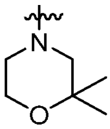, 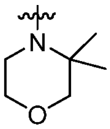, 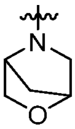, 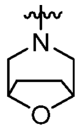, 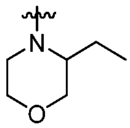, 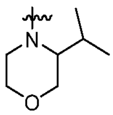, 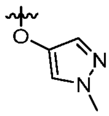, 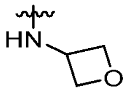, 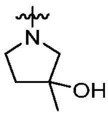,

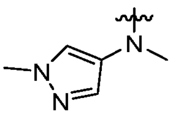 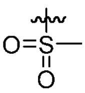, 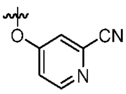, 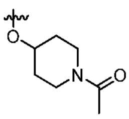, 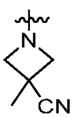, 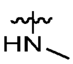, 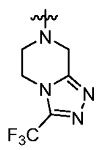, 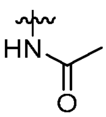, 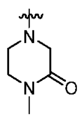, 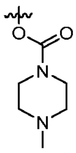,

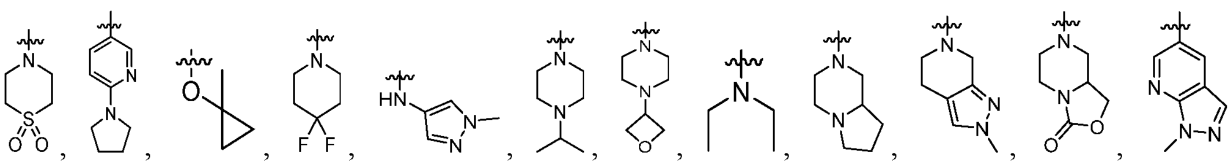,

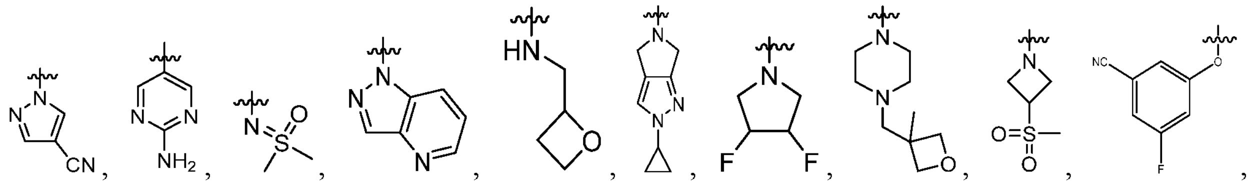

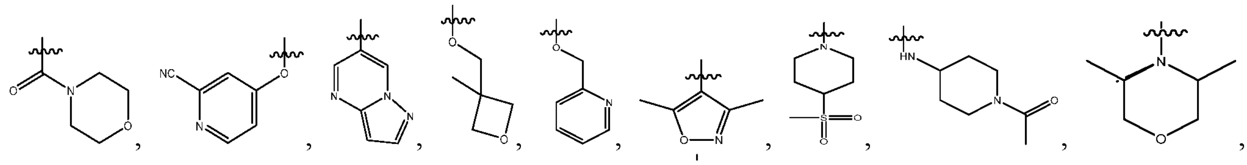

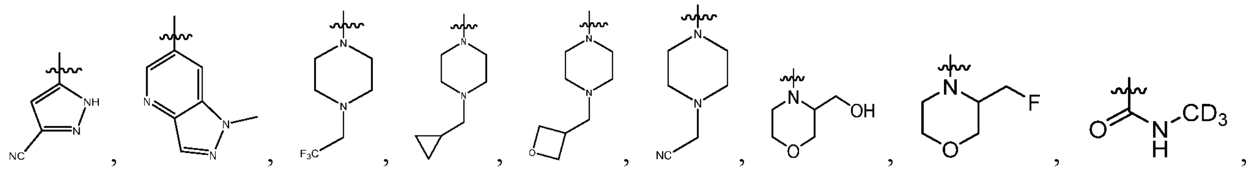

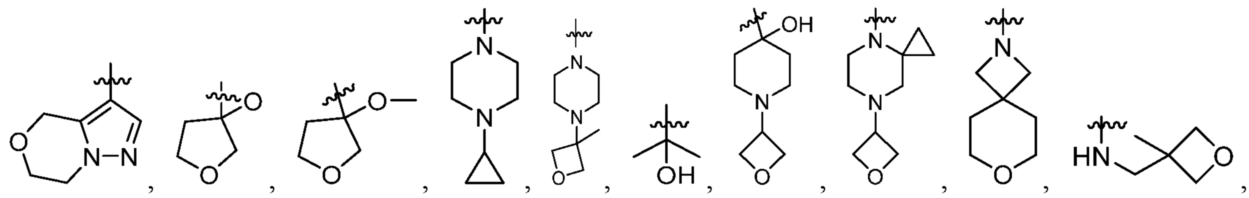

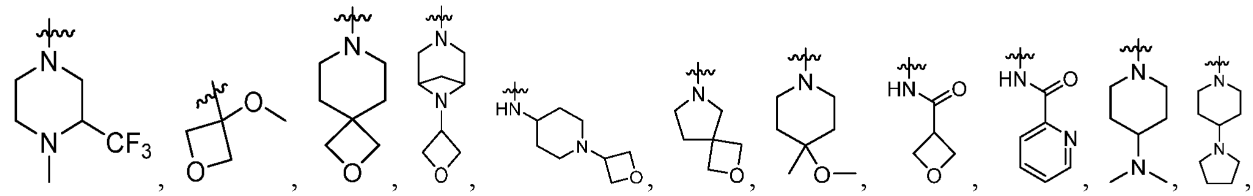

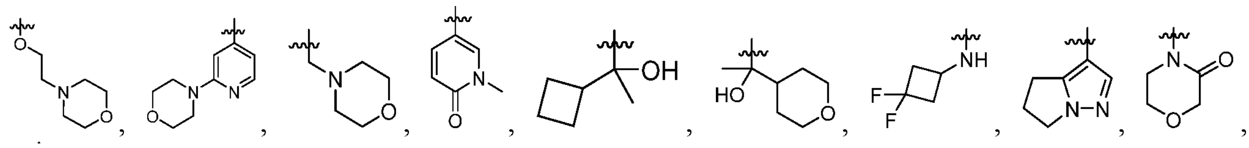

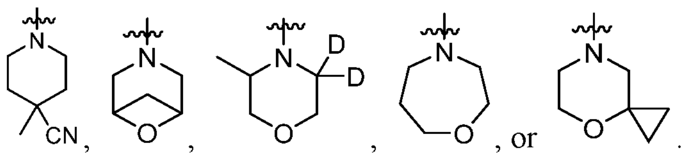

, or .

In some embodiments, $R^2$ is selected from

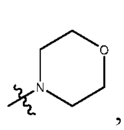

,

and $R^8$ is selected from $-O-CD_3$, $-O-CH_3$,

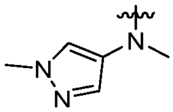, 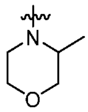, 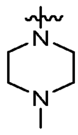, 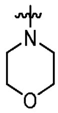, 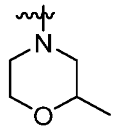, 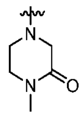, 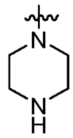, 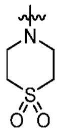, 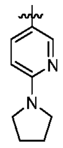, 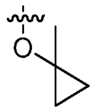, 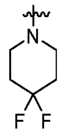, 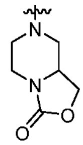,

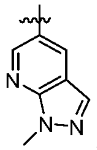, 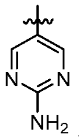, 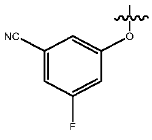, 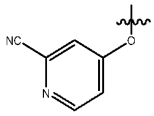, 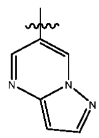, 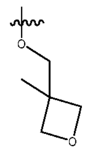, 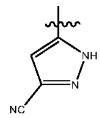, 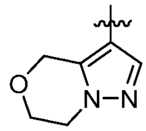, 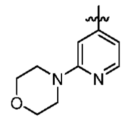, or

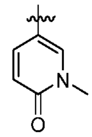.

In some embodiments, $R^{2b}$ is independently selected from $-OR^{2c}$, $-N(R2^{c})_2$, halogen, hydroxy, cyano, amino, $-C(O)R^{2c}$, or $-C(S)R^{2c}$.

In some embodiments, $R^{2b}$ is independently selected from $-OR^{2c}$, $-N(R^{2c})_2$, halogen, hydroxy, cyano, amino, or $-C(O)R^{2c}$.

In some embodiments, $R^{2b}$ is independently selected from cyano, $-C(O)R^{2c}$, or $-C(S)R^{2c}$.

In some embodiments, $R^{2b}$ is independently selected from cyano or $-C(O)R^{2c}$.

In some embodiments, $R^{2b}$ is independently selected from cyano, $-C(O)CH_3$, or $-C(S)CH_3$.

In some embodiments, $R^{2b}$ is independently selected from cyano or $-C(O)CH_3$.

In some embodiments, $R^{2c}$ is independently selected from $C_{1-6}$ alkyl, $C_{3-10}$ cycloalkyl, $C_{6-10}$ aryl, 3-10 membered heterocyclyl, or 5-10 membered heteroaryl, wherein the $C_{1-6}$ alkyl, $C_{3-10}$ cycloalkyl, $C_{6-10}$ aryl, 3-10 membered heterocyclyl, or 5-10 membered heteroaryl is optionally substituted with $R^{2d}$ and/or $R^{2f}$.

In some embodiments, $R^{2c}$ is independently selected from $C_{1-6}$ alkyl, 4-9 membered heterocyclyl, or 5-6 membered heteroaryl, wherein the $C_{1-6}$ alkyl, 4-9 membered heterocyclyl, or 5-6 membered heteroaryl is optionally substituted with $R^{2d}$ and/or $R^{2f}$.

In some embodiments, $R^{2c}$ is independently selected from $C_{1-6}$ alkyl, wherein the $C_{1-6}$ alkyl is optionally substituted with $R^{2d}$ and/or $R^{2f}$.

In some embodiments, $R^{2c}$ is $CH_3$, $CH_2F$, $CHF_2$, $CF_3$, or $CH_2CN$.

In some embodiments, $R^{2c}$ is $CH_3$, $CH_2F$, $CHF_2$, or $CF_3$.

In some embodiments, $R^{2d}$ is selected from halogen or cyano.

In some embodiments, $R^{2d}$ is selected from halogen.

In some embodiments, $R^{2d}$ is selected from F.

In some embodiments, $R^3$ and $R^4$ are independently selected from H, deuterium, $C_{1-3}$ deuterated alkyl, or $C_{1-6}$ alkyl.

In some embodiments, $R^3$ and $R^4$ are independently selected from H, deuterium, $CH_3$, or $CD_3$.

In some embodiments, $R^3$ is selected from H or deuterium, and $R^4$ is selected from $CH_3$ or $CD_3$.

In some embodiments, $R^3$ is selected from H, and $R^4$ is selected from $CH_3$.

In some embodiments, $R^5$, $R^6$, and R' are independently selected from H, nitro, halogen, cyano, hydroxy, amino, $C_{1-6}$ alkyl, $C_{3-6}$ cycloalkyl, $-O-C_{1-6}$ alkyl, $-S(O)_2-C_{1-4}$ alkyl, $-C(O)R^{x2}$, $-SF_5$, 4-7 membered heterocyclyl, or 5-6 membered heteroaryl, wherein the $C_{1-6}$ alkyl, $C_{3-6}$ cycloalkyl, $-S(O)_2-C_{1-4}$ alkyl, 4-7 membered heterocyclyl, or 5-6 membered heteroaryl is optionally substituted with $R^{x1}$. In some embodiments, $R^5$, $R^6$, and $R^7$ are independently selected from H, nitro, halogen, cyano, hydroxy, amino, $C_{1-6}$ alkyl, $C_{3-6}$ cycloalkyl, $-O-C_{1-6}$ alkyl, $-S(O)_2-C_{1-4}$ alkyl, $-C(O)R^{x2}$, 4-7 membered heterocyclyl, or 5-6 membered heteroaryl, wherein the $C_{1-6}$ alkyl, $C_{3-6}$ cycloalkyl, $-S(O)_2-C_{1-4}$ alkyl, 4-7 membered heterocyclyl, or 5-6 membered heteroaryl is optionally substituted with $R^{x1}$.

In some embodiments, $R^5$, $R^6$, and $R^7$ are independently selected from H, nitro, halogen, cyano, $-SF_5$, or $C_{1-6}$ alkyl, wherein the $C_{1-6}$ alkyl is optionally substituted with $R^{x1}$.

In some embodiments, $R^5$, $R^6$, and $R^7$ are independently selected from H, nitro, halogen, cyano, or $C_{1-6}$ alkyl, wherein the $C_{1-6}$ alkyl is optionally substituted with $R^{x1}$.

In some embodiments, $R^5$, $R^6$, and $R^7$ are independently selected from H, nitro, halogen, cyano, methyl, ethyl, butyl, or $-SF_5$, wherein the methyl, ethyl, or butyl is optionally substituted with $R^{x1}$.

In some embodiments, $R^5$, $R^6$, and $R^7$ are independently selected from H, nitro, halogen, cyano, methyl, ethyl, or butyl, wherein the methyl, ethyl, or butyl is optionally substituted with $R^{x1}$.

In some embodiments, $R^5$, $R^6$, and $R^7$ are independently selected from H, F, nitro, cyano, $CH_3$, $CHF_2$, $CF_2CH_3$,

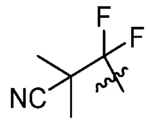,

or $-SF_5$.

In some embodiments, $R^5$, $R^6$, and $R^7$ are independently selected from H, F, nitro, cyano, $CH_3$, $CHF_2$, $CF_2CH_3$, or

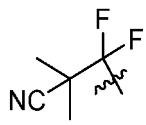.

In some embodiments, $R^5$ is selected from nitro, cyano, $CHF_2$, $CF_3$, $CF_2CH_3$, $-SF_5$, or

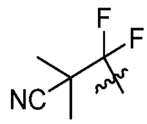.

In some embodiments, $R^6$ is selected from H, F, $CF_3$, or $CH_3$.

In some embodiments, $R^7$ is selected from H.

In some embodiments, when $R^8$ is selected from H, halogen, $C_{1-6}$ alkyl, or $C_{3-6}$ cycloalkyl, $R^5$ is selected from nitro, hydroxy, cyano, $-O-C_{1-6}$ alkyl, $-O-C_{3-6}$, -O-(3-8 membered heterocyclyl), - $C(O)R^{x2}$, $-C(O)N(R^{x2})_2$, $-C(O)OR^{x2}$, $-SF_5$,

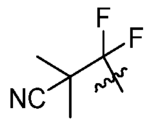, 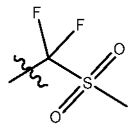,

or 5-10 membered heteroaryl, wherein the 5-10 membered heteroaryl is optionally substituted with $R^{x1}$.

In some embodiments, when $R^8$ is selected from H, $R^5$ is selected from nitro, hydroxy, cyano, $-O-C_{1-6}$ alkyl, $-O-C_{3-6}$ cycloalkyl, -0-(3-8 membered heterocyclyl), $-C(O)R^{x2}$, $-C(O)N(R^{x2})_2$, $-C(O)OR^{x2}$, $-SF_5$,

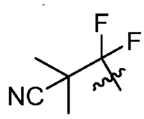, 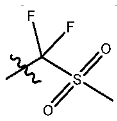,

or 5-10 membered heteroaryl, wherein the 5-10 membered heteroaryl is optionally substituted with $R^{x1}$.

In some embodiments, when $R^8$ is selected from H, halogen, $C_{1-6}$ alkyl, or $C_{3-6}$ cycloalkyl, $R^5$ is selected from nitro, cyano, $-O-C_{1-6}$ alkyl, $-O-C_{3-6}$ cycloalkyl, -O-(3-8 membered heterocyclyl), - $C(O)R^{x2}$, $-SF_5$,

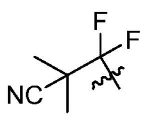, 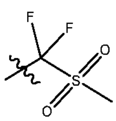,

or 5-10 membered heteroaryl, wherein the 5-10 membered heteroaryl is optionally substituted with $R^{x1}$.

In some embodiments, when $R^8$ is selected from H, $R^5$ is selected from nitro, cyano, -O-$C_{1-6}$ alkyl, - O-$C_{3-6}$ cycloalkyl, -0-(3-8 membered heterocyclyl), -C(O)$R^{x2}$, -$SF_5$,

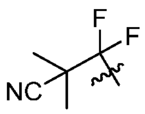 , 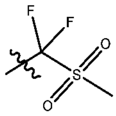 ,

or 5-10 membered heteroaryl, wherein the 5-10 membered heteroaryl is optionally substituted with $R^{x1}$.

In some embodiments, when $R^8$ is selected from H, halogen, $C_{1-6}$ alkyl, or $C_{3-6}$ cycloalkyl, $R^5$ is selected from nitro, cyano,

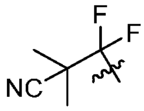 ,

or -$SF_5$.

In some embodiments, when $R^8$ is selected from H, $R^5$ is selected from nitro, cyano,

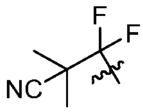 ,

or - $SF_5$.

In some embodiments, when $R^8$ is selected from H, halogen, $C_{1-6}$ alkyl, or $C_{3-6}$ cycloalkyl, $R^5$ is selected from nitro, cyano, or

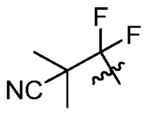 .

In some embodiments, $R^{x1}$ is independently selected from halogen or cyano.

In some embodiments, $R^8$ is selected from H halogen, hydroxy, cyano, amino, nitro,

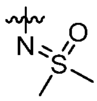 , -

OC(O)$R^{8a}$, $C_{1-6}$ alkyl, $C_{2-6}$ alkenyl, $C_{2-6}$ alkynyl, $C_{1-6}$ haloalkyl, $C_{3-6}$ cycloalkyl, -S(O)$_2$-$C_{1-6}$ alkyl, - O-$C_{1-6}$ alkyl, -O-$C_{1-6}$ deuterated alkyl, -O-$C_{3-6}$ cycloalkyl, -O-(3-10 membered heterocyclyl), -O-(5-10 membered heteroaryl), 3-10 membered heterocyclyl, 5-10 membered heteroaryl, or $C_{6-10}$ aryl, wherein the amino, $C_{2-6}$ alkenyl, $C_{2-6}$ alkynyl, -O-$C_{1-6}$ deuterated alkyl, $C_{3-6}$ cycloalkyl, -S(O)$_2$-$C_{1-6}$ alkyl, -O-$C_{1-6}$ alkyl, -O-$C_{3-6}$ cycloalkyl, -O-(3-10 membered heterocyclyl), -O-(5-10 membered heteroaryl), 3-10 membered heterocyclyl, 5-10 membered heteroaryl, or $C_{6-10}$ aryl is optionally substituted with $R^{8a}$. In some embodiments, $R^8$ is selected from H, halogen, hydroxy, cyano, amino, nitro, $C_{1-6}$ alkyl, $C_{2-6}$ alkenyl, $C_{2-6}$ alkynyl, $C_{1-6}$ haloalkyl, $C_{3-6}$ cycloalkyl, -S(O)$_2$-$C_{1-6}$ alkyl, -O-$C_{1-6}$ alkyl, -O-$C_{1-6}$ deuterated alkyl, -O-$C_{3-6}$ cycloalkyl, -O-(3-8 membered heterocyclyl), -O-(5-10 membered heteroaryl), 3-8 membered heterocyclyl, or 5-10 membered heteroaryl, wherein the amino, $C_{2-6}$ alkenyl, $C_{2-6}$ alkynyl, -O-$C_{1-6}$ deuterated alkyl, $C_{3-6}$ cycloalkyl, -S(O)$_2$-$C_{1-6}$ alkyl, -O-$C_{1-6}$ alkyl, -O-$C_{3-6}$ cycloalkyl, -O-(3-8 membered heterocyclyl), -O-(5-10 membered heteroaryl), 3-8 membered heterocyclyl, or 5-10 membered heteroaryl is optionally substituted with $R^{8a}$.

In some embodiments, $R^8$ is selected from H, halogen, hydroxy, cyano, amino, nitro, $C_{1-6}$ alkyl, $C_{2-6}$ alkenyl, $C_{2-6}$ alkynyl, $C_{1-6}$ haloalkyl, $C_{3-6}$ cycloalkyl, -O-$C_{1-6}$ alkyl, -O-$C_{1-6}$ deuterated alkyl, -O-$C_{3-6}$ cycloalkyl, -O-(3-8 membered heterocyclyl), 3-8 membered heterocyclyl, or 5-10 membered heteroaryl, wherein the $C_{2-6}$ alkenyl, $C_{2-6}$ alkynyl, -O-$C_{1-6}$ deuterated alkyl, $C_{3-6}$ cycloalkyl, -O-$C_{1-6}$ alkyl, -O-$C_{3-6}$ cycloalkyl, -O-(3-8 membered heterocyclyl), 3-8 membered heterocyclyl, or 5-10 membered heteroaryl is optionally substituted with $R^{8a}$.

In some embodiments, $R^8$ is selected from H, halogen, hydroxy, cyano, amino, nitro, $C_{1-6}$ alkyl, $C_{1-6}$ haloalkyl, $C_{3-6}$ cycloalkyl, -O-$C_{1-6}$ alkyl, -O-$C_{3-6}$ cycloalkyl, -O-(3-8 membered heterocyclyl), 3-8 membered heterocyclyl, or 5-10 membered heteroaryl, wherein the $C_{3-6}$ cycloalkyl, -O-$C_{1-6}$ alkyl, -O-$C_{3-6}$ cycloalkyl, -O-(3-8 membered heterocyclyl), 3-8 membered heterocyclyl, or 5-10 membered heteroaryl is optionally substituted with $R^{8a}$.

$R^8$ is selected from H, halogen, hydroxy, cyano, amino, nitro,

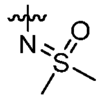 , 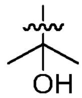 ,

-C(O)$R^{8a}$, - C(O)NH$R^{8a}$, -OC(O)$R^{8a}$, $C_{1-6}$ alkyl, $C_{2-6}$ alkenyl, $C_{2-6}$ alkynyl, $C_{1-6}$ haloalkyl, $C_{3-6}$ cycloalkyl, -S(O)$_2$-$C_{1-6}$ alkyl, -O-$C_{1-6}$ alkyl, -O-$C_{1-6}$ deuterated alkyl, -O-$C_{3-6}$ cycloalkyl, -O-(3-10 membered heterocyclyl), -O-(5-10 membered heteroaryl), -O-($C_{6-10}$ aryl), 3-10 membered heterocyclyl, 5-10 membered heteroaryl, or $C_{6-10}$ aryl, wherein the amino, $C_{2-6}$ alkenyl, $C_{2-6}$ alkynyl, $C_{3-6}$ cycloalkyl, - S(O)$_2$-$C_{1-6}$ alkyl, -O-$C_{1-6}$ alkyl, -O-$C_{1-6}$ deuterated alkyl, -O-$C_{3-6}$ cycloalkyl, -O-(3-10 membered heterocyclyl), -O-(5-10 membered heteroaryl), -O-($C_{6-10}$ aryl), 3-10 membered heterocyclyl, 5-10 membered heteroaryl, or $C_{6-10}$ aryl is optionally substituted with $R^{8a}$.

In some embodiments, $R^8$ is independently selected from H, hydroxy, cyano, amino,

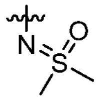 , 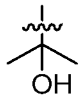 ,

-C(O)$R^{8a}$, -C(O)NH$R^{8a}$, -OC(O)$R^{8a}$, -NHC(O)$R^{8a}$, $C_{1-6}$ alkyl, $C_{2-6}$ alkenyl, -O-$C_{1-6}$ deuterated alkyl, $C_{1-6}$ haloalkyl, -S(O)$_2$-$C_{1-6}$ alkyl, -O-$C_{1-6}$ alkyl, -O-$C_{3-6}$ cycloalkyl, -O-(3-10 membered heterocyclyl), -O-(5-10 membered heteroaryl), -O-($C_{6-10}$ aryl), 3-10 membered heterocyclyl, 5-10 membered heteroaryl, or $C_{6-10}$ aryl, wherein the amino, $C_{1-6}$ alkyl, $C_{2-6}$ alkenyl, -O-$C_{1-6}$ deuterated alkyl, -S(O)$_2$-$C_{1-6}$ alkyl, -O-$C_{1-6}$ alkyl, -O-$C_{3-6}$ cycloalkyl, -O-(3-10 membered heterocyclyl), -O-(5-10 membered heteroaryl), -O-($C_{6-10}$ aryl), 3-10 membered heterocyclyl, 5-10 membered heteroaryl, or $C_{6-10}$ aryl is optionally substituted with $R^{8a}$.

In some embodiments, $R^8$ is independently selected from H, hydroxy, cyano, amino,

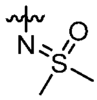 , 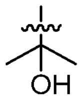 ,

-C(O)$R^{8a}$, -C(O)NH$R^{8a}$, -OC(O)$R^{8a}$, -NHC(O)$R^{8a}$, $C_{1-6}$ alkyl, $C_{2-6}$ alkenyl, $C_{2-6}$ alkynyl, -O-$C_{1-6}$ deuterated alkyl, $C_{1-6}$ haloalkyl, -S(O)$_2$-$C_{1-6}$ alkyl, -O-$C_{1-6}$ alkyl, -O-$C_{3-6}$ cycloalkyl, -O-(3-10 membered heterocyclyl), -O-(5-10 membered heteroaryl), -O-($C_{6-10}$ aryl), 3-10 membered heterocyclyl, 5-10 membered heteroaryl, or $C_{6-10}$ aryl, wherein the amino, $C_{1-6}$ alkyl, $C_{2-6}$ alkenyl, $C_{2-6}$ alkynyl, -O-$C_{1-6}$ deuterated alkyl, -S(O)$_2$-$C_{1-6}$ alkyl, -O-$C_{1-6}$ alkyl, -O-$C_{3-6}$ cycloalkyl, -O-(3-10 membered heterocyclyl), -O-(5-10 membered heteroaryl), -O-($C_{6-10}$ aryl), 3-10 membered heterocyclyl, 5-10 membered heteroaryl, or $C_{6-10}$ aryl is optionally substituted with $R^{8a}$.

In some embodiments, $R^8$ is selected from H, hydroxy, cyano, amino,

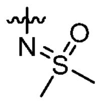 , 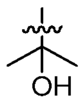 ,

-C(O)$R^{8a}$, - C(O)NH$R^{8a}$, -OC(O)$R^{8a}$, $C_{2-6}$ alkenyl, $C_{2-6}$ alkynyl, -O-$C_{1-6}$ deuterated alkyl, $C_{1-6}$ haloalkyl, -S(O)$_2$-$C_{1-6}$ alkyl, -O-$C_{1-6}$ alkyl, -O-$C_{3-6}$ cycloalkyl, -O-(3-10 membered heterocyclyl), -O-(5-10 membered heteroaryl), -O-($C_{6-10}$ aryl), 3-10 membered heterocyclyl, 5-10 membered heteroaryl, or $C_{6-10}$ aryl, wherein the amino, $C_{2-6}$ alkenyl, $C_{2-6}$ alkynyl, -O-$C_{1-6}$ deuterated alkyl, -S(O)$_2$-$C_{1-6}$ alkyl, -O-$C_{1-6}$ alkyl, -O-$C_{3-6}$ cycloalkyl, -O-(3-10 membered heterocyclyl), -O-(5-10 membered heteroaryl), -O-($C_{6-10}$ aryl), 3-10 membered heterocyclyl, 5-10 membered heteroaryl, or $C_{6-10}$ aryl is optionally substituted with $R^{8a}$.

In some embodiments, $R^8$ is selected from H, hydroxy, cyano, amino,

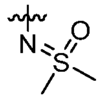,

-OC(O)$R^{8a}$, $C_{2-6}$ alkenyl, $C_{2-6}$ alkynyl, -O-$C_{1-6}$ deuterated alkyl, $C_{1-6}$ haloalkyl, -S(O)$_2$-$C_{1-6}$ alkyl, -O-$C_{1-6}$ alkyl, -O-$C_{3-6}$ cycloalkyl, -O-(3-10 membered heterocyclyl), -O-(5-10 membered heteroaryl), 3-10 membered heterocyclyl, 5-10 membered heteroaryl, or $C_{6-10}$ aryl, wherein the amino, $C_{2-6}$ alkenyl, $C_{2-6}$ alkynyl, -O-$C_{1-6}$ deuterated alkyl, -S(O)$_2$-$C_{1-6}$ alkyl, -O-$C_{1-6}$ alkyl, -O-$C_{3-6}$ cycloalkyl, -O-(3-10 membered heterocyclyl), -O-(5-10 membered heteroaryl), 3-10 membered heterocyclyl, 5-10 membered heteroaryl, or $C_{6-10}$ aryl is optionally substituted with $R^{8a}$.

In some embodiments, $R^8$ is selected from H, hydroxy, cyano, amino, $C_{2-6}$ alkenyl, $C_{2-6}$ alkynyl, -O-$C_{1-6}$ deuterated alkyl, $C_{1-6}$ haloalkyl, -S(O)$_2$-$C_{1-6}$ alkyl, -O-$C_{1-6}$ alkyl, -O-$C_{3-6}$ cycloalkyl, -O-(3-8 membered heterocyclyl), -O-(5-10 membered heteroaryl), 3-8 membered heterocyclyl, or 5-10 membered heteroaryl, wherein the amino, $C_{2-6}$ alkenyl, $C_{2-6}$ alkynyl, -O-$C_{1-6}$ deuterated alkyl, -S(O)$_2$-$C_{1-6}$ alkyl, -O-$C_{1-6}$ alkyl, -O-$C_{3-6}$ cycloalkyl, -O-(3-8 membered heterocyclyl), -O-(5-10 membered heteroaryl), 3-8 membered heterocyclyl, or 5-10 membered heteroaryl is optionally substituted with $R^{8a}$.

In some embodiments, $R^8$ is selected from H, hydroxy, cyano, $C_{2-6}$ alkenyl, $C_{2-6}$ alkynyl, -O-$C_{1-6}$ deuterated alkyl, $C_{1-6}$ haloalkyl, -O-$C_{1-6}$ alkyl, -O-$C_{3-6}$ cycloalkyl, -O-(3-8 membered heterocyclyl), 3-8 membered heterocyclyl, or 5-10 membered heteroaryl, wherein the $C_{2-6}$ alkenyl, $C_{2-6}$ alkynyl, -O-$C_{1-6}$ deuterated alkyl, -O-$C_{1-6}$ alkyl, -O-$C_{3-6}$ cycloalkyl, -O-(3-8 membered heterocyclyl), 3-8 membered heterocyclyl, or 5-10 membered heteroaryl is optionally substituted with $R^{8a}$.

In some embodiments, $R^8$ is selected from H, cyano, $C_{2-6}$ alkenyl, $C_{2-6}$ alkynyl, -O-$C_{1-6}$ deuterated alkyl, $C_{1-6}$ haloalkyl, -O-$C_{1-6}$ alkyl, -O-$C_{3-6}$ cycloalkyl, -O-(3-8 membered heterocyclyl), 3-8 membered heterocyclyl, or 5-10 membered heteroaryl, wherein the $C_{2-6}$ alkenyl, $C_{2-6}$ alkynyl, -O-$C_{1-6}$ deuterated alkyl, -O-$C_{1-6}$ alkyl, -O-$C_{3-6}$ cycloalkyl, -O-(3-8 membered heterocyclyl), 3-8 membered heterocyclyl, or 5-10 membered heteroaryl is optionally substituted with $R^{8a}$.

In some embodiments, $R^8$ is selected from H, $C_{1-6}$ haloalkyl, -O-$C_{1-6}$ alkyl, -O-$C_{3-6}$ cycloalkyl, -O-(3-8 membered heterocyclyl), 3-8 membered heterocyclyl, or 5-10 membered heteroaryl, wherein the - O-$C_{1-6}$ alkyl, -O-$C_{3-6}$ cycloalkyl, -O-(3-8 membered heterocyclyl), 3-8 membered heterocyclyl, or 5-10 membered heteroaryl is optionally substituted with $R^{8a}$.

In some embodiments, $R^8$ is selected from H, hydroxy, cyano, amino,

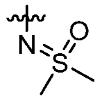, 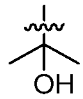,

-C(O)$R^{8a}$, - C(O)NH$R^{8a}$, -OC(O)$R^{8a}$, -NHC(O)$R^{8a}$, $C_{1-3}$ alkyl, $C_{2-3}$ alkenyl, $C_{2-3}$ alkynyl, -O-$C_{1-3}$ deuterated alkyl, $C_{1-3}$ haloalkyl, -S(O)$_2$-$C_{1-3}$ alkyl, -O-$C_{1-3}$ alkyl, -O-$C_{3-6}$ cycloalkyl, -O-(5-6 membered heteroaryl), - O-(4-7 membered heterocyclyl), -O-($C_{6-10}$ aryl), 4-9 membered heterocyclyl, 5-9 membered heteroaryl, or $C_{6-10}$ aryl, wherein the amino, $C_{1-3}$ alkyl, $C_{2-3}$ alkenyl, $C_{2-3}$ alkynyl, -O-$C_{1-3}$ deuterated alkyl, -S(O)$_2$-$C_{1-3}$ alkyl, -O-$C_{1-3}$ alkyl, -O-$C_{3-6}$ cycloalkyl, -O-(5-6 membered heteroaryl), -O-(4-7 membered heterocyclyl), -O-($C_{6-10}$ aryl), 4-9 membered heterocyclyl, 5-9 membered heteroaryl, or $C_{6-10}$ aryl is optionally substituted with $R^{8a}$.

In some embodiments, $R^8$ is selected from H, hydroxy, cyano, amino,

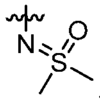, 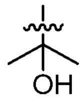,

-C(O)$R^{8a}$, - C(O)NH$R^{8a}$, -OC(O)$R^{8a}$, $C_{2-3}$ alkenyl, $C_{2-3}$ alkynyl, -O-$C_{1-3}$ deuterated alkyl, $C_{1-3}$ haloalkyl, -S(O)$_2$-$C_{1-3}$ alkyl, -O-$C_{1-3}$ alkyl, -O-$C_{3-6}$ cycloalkyl, -O-(5-6 membered heteroaryl), -O-(4-7 membered heterocyclyl), -O-($C_{6-10}$ aryl), 4-9 membered heterocyclyl, 5-9 membered heteroaryl, or $C_{6-10}$ aryl, wherein the amino, $C_{2-3}$ alkenyl, $C_{2-3}$ alkynyl, -O-$C_{1-3}$ deuterated alkyl, -S(O)$_2$-$C_{1-3}$ alkyl, -O-$C_{1-3}$ alkyl, -O-$C_{3-6}$ cycloalkyl, -O-(5-6 membered heteroaryl), -O-(4-7 membered heterocyclyl), -O-($C_{6-10}$ aryl), 4-9 membered heterocyclyl, 5-9 membered heteroaryl, or $C_{6-10}$ aryl is optionally substituted with $R^{8a}$.

In some embodiments, $R^8$ is selected from H, hydroxy, cyano, amino,

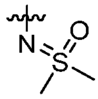,

$-OC(O)R^{8a}$, $C_{2-3}$ alkenyl, $C_{2-3}$ alkynyl, -O-$C_{1-3}$ deuterated alkyl, $C_{1-3}$ haloalkyl, -$S(O)_2$-$C_{1-3}$ alkyl, -O-$C_{1-3}$ alkyl, -O-$C_{3-6}$ cycloalkyl, -O-(5-6 membered heteroaryl), -O-(4-7 membered heterocyclyl), 4-9 membered heterocyclyl, 5-9 membered heteroaryl, or $C_{6-10}$ aryl, wherein the amino, $C_{2-3}$ alkenyl, $C_{2-3}$ alkynyl, - O-$C_{1-3}$ deuterated alkyl, -$S(O)_2$-$C_{1-3}$ alkyl, -O-$C_{1-3}$ alkyl, -O-$C_{3-6}$ cycloalkyl, -O-(5-6 membered heteroaryl), -O-(4-7 membered heterocyclyl), 4-9 membered heterocyclyl, 5-9 membered heteroaryl, or $C_{6-10}$ aryl is optionally substituted with $R^{8a}$.

In some embodiments, $R^8$ is selected from H, hydroxy, cyano, amino, $C_{2-3}$ alkenyl, $C_{2-3}$ alkynyl, -O-$C_{1-3}$ deuterated alkyl, $C_{1-3}$ haloalkyl, -$S(O)_2$-$C_{1-3}$ alkyl, -O-$C_{1-3}$ alkyl, -O-$C_{3-6}$ cycloalkyl, -O-(5-6 membered heteroaryl), -O-(4-7 membered heterocyclyl), 5-8 membered heterocyclyl, or 5-6 membered heteroaryl, wherein the amino, $C_{2-3}$ alkenyl, $C_{2-3}$ alkynyl, -O-$C_{1-3}$ deuterated alkyl, -$S(O)_2$-$C_{1-3}$ alkyl, -O-$C_{1-3}$ alkyl, -O-$C_{3-6}$ cycloalkyl, -O-(5-6 membered heteroaryl), -O-(4-7 membered heterocyclyl), 5-8 membered heterocyclyl, or 5-6 membered heteroaryl is optionally substituted with $R^{8a}$.

In some embodiments, $R^8$ is selected from H, hydroxy, cyano, $C_{2-3}$ alkenyl, $C_{2-3}$ alkynyl, -O-$C_{1-3}$ deuterated alkyl, $C_{1-3}$ haloalkyl, -O-$C_{1-3}$ alkyl, -O-$C_{3-6}$ cycloalkyl, -O-(4-7 membered heterocyclyl), 4-7 membered heterocyclyl, or 5-6 membered heteroaryl, wherein the $C_{2-3}$ alkenyl, $C_{2-3}$ alkynyl, -O-$C_{1-3}$ deuterated alkyl, -O-$C_{1-3}$ alkyl, -O-$C_{3-6}$ cycloalkyl, -O-(4-7 membered heterocyclyl), 4-7 membered heterocyclyl, or 5-6 membered heteroaryl is optionally substituted with $R^{8a}$.

In some embodiments, $R^8$ is selected from H, cyano, $C_{2-3}$ alkenyl, $C_{2-3}$ alkynyl, -O-$C_{1-3}$ deuterated alkyl, $C_{1-3}$ haloalkyl, -O-$C_{1-3}$ alkyl, -O-$C_{3-6}$ cycloalkyl, -O-(4-7 membered heterocyclyl), 4-7 membered heterocyclyl, or 5-6 membered heteroaryl, wherein the $C_{2-3}$ alkenyl, $C_{2-3}$ alkynyl, -O-$C_{1-3}$ deuterated alkyl, -O-$C_{1-3}$ alkyl, -O-$C_{3-6}$ cycloalkyl, -O-(4-7 membered heterocyclyl), 4-7 membered heterocyclyl, or 5-6 membered heteroaryl is optionally substituted with $R^{8a}$.

In some embodiments, $R^8$ is selected from H, $C_{1-3}$ haloalkyl, -O-$C_{1-3}$ alkyl, -O-$C_{3-6}$ cycloalkyl, -O-(4-7 membered heterocyclyl), 4-7 membered heterocyclyl, or 5-6 membered heteroaryl, wherein the -O-$C_{1-3}$ alkyl, -O-$C_{3-6}$ cycloalkyl, -O-(4-7 membered heterocyclyl), 4-7 membered heterocyclyl, or 5-6 membered heteroaryl is optionally substituted with $R^{8a}$.

In some embodiments, $R^8$ is selected from H, hydroxy, cyano, amino,

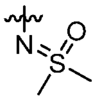, 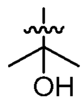,

$-C(O)R^{8a}$, - $C(O)NHR^{8a}$, $-OC(O)R^{8a}$, $-NHC(O)R^{8a}$, $C_{1-3}$ alkyl, $C_{2-3}$ alkenyl, -O-$C_{1-3}$ deuterated alkyl, $C_{1-3}$ haloalkyl, -$S(O)_2$-$C_{1-3}$ alkyl, -O-$C_{1-3}$ alkyl, -O-$C_{3-6}$ cycloalkyl, -O-(5-6 membered heteroaryl), -O-(4-6 membered heterocyclyl), -O-phenyl, 4-9 membered heterocyclyl, 5-9 membered heteroaryl, or $C_{6-10}$ aryl, wherein the amino, $C_{1-3}$ alkyl, $C_{2-3}$ alkenyl, -O-$C_{1-3}$ deuterated alkyl, -$S(O)_2$-$C_{1-3}$ alkyl, -O-$C_{1-3}$ alkyl, -O-$C_{3-6}$ cycloalkyl, -O-(5-6 membered heteroaryl), -O-(4-6 membered heterocyclyl), -O-phenyl, 4-9 membered heterocyclyl, 5-9 membered heteroaryl, or $C_{6-10}$ aryl is optionally substituted with $R^{8a}$.

In some embodiments, $R^8$ is selected from H, hydroxy, cyano, amino,

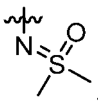, 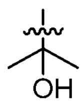,

$-C(O)R^{8a}$, - $C(O)NHR^{8a}$, $-OC(O)R^{8a}$, $-NHC(O)R^{8a}$, $C_{1-3}$ alkyl, $C_{2-3}$ alkenyl, -O-$C_{1-3}$ deuterated alkyl, $C_{1-3}$ haloalkyl, -$S(O)_2$-$C_{1-3}$ alkyl, -O-$C_{1-3}$ alkyl, -O-$C_{3-6}$ cycloalkyl, -O-(5-6 membered heteroaryl), -O-(4-6 membered heterocyclyl), -O-phenyl, 4-9 membered heterocyclyl, 5-9 membered heteroaryl, or $C_{6-10}$ aryl, wherein the amino, $C_{1-3}$ alkyl, $C_{2-3}$ alkenyl, -O-$C_{1-3}$ deuterated alkyl, -$S(O)_2$-$C_{1-3}$ alkyl, -O-$C_{1-3}$ alkyl, -O-(5-6 membered heteroaryl), -O-(4-6 membered heterocyclyl), -O-phenyl, 4-9 membered heterocyclyl, 5-9 membered heteroaryl, or $C_{6-10}$ aryl is optionally substituted with $R^{8a}$.

In some embodiments, $R^8$ is selected from H, hydroxy, cyano, amino,

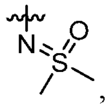, 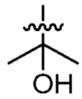,

-C(O)$R^{8a}$, - C(O)$NHR^{8a}$, -OC(O)$R^{8a}$, $C_{2-3}$ alkenyl, -O-$C_{1-3}$ deuterated alkyl, $C_{1-3}$ haloalkyl, -S(O)$_2$-$C_{1-3}$ alkyl, -O-$C_{1-3}$ alkyl, -O-$C_{3-6}$ cycloalkyl, -O-(5-6 membered heteroaryl), -O-(4-6 membered heterocyclyl), -O-phenyl, 4-9 membered heterocyclyl, 5-9 membered heteroaryl, or $C_{6-10}$ aryl, wherein the amino, $C_{2-3}$ alkenyl, -O-$C_{1-3}$ deuterated alkyl, -S(O)$_2$-$C_{1-3}$ alkyl, -O-$C_{1-3}$ alkyl, -O-(5-6 membered heteroaryl), -O-(4-6 membered heterocyclyl), -O-phenyl, 4-9 membered heterocyclyl, 5-9 membered heteroaryl, or $C_{6-10}$ aryl is optionally substituted with $R^{8a}$.

In some embodiments, $R^8$ is selected from H hydroxy, cyano, amino,

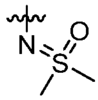,

-OC(O)$R^{8a}$, $C_{2-3}$ alkenyl, -O-$C_{1-3}$ deuterated alkyl, $C_{1-3}$ haloalkyl, -S(O)$_2$-$C_{1-3}$ alkyl, -O-$C_{1-3}$ alkyl, -O-$C_{3-6}$ cycloalkyl, -O-(5-6 membered heteroaryl), -O-(4-6 membered heterocyclyl), 4-9 membered heterocyclyl, 5-9 membered heteroaryl, or $C_{6-10}$ aryl, wherein the amino, $C_{2-3}$ alkenyl, -O-$C_{1-3}$ deuterated alkyl, -S(O)$_2$-$C_{1-3}$ alkyl, -O-$C_{1-3}$ alkyl, -O-(5-6 membered heteroaryl), -O-(4-6 membered heterocyclyl), 4-9 membered heterocyclyl, 5-9 membered heteroaryl, or $C_{6-10}$ aryl is optionally substituted with $R^{8a}$.

In some embodiments, $R^8$ is selected from H, hydroxy, cyano, amino, $C_{2-3}$ alkenyl, -O-$C_{1-3}$ deuterated alkyl, $C_{1-3}$ haloalkyl, -S(O)$_2$-$C_{1-3}$ alkyl, -O-$C_{1-3}$ alkyl, -O-$C_{3-6}$ cycloalkyl, -O-(5-6 membered heteroaryl), 5-8 membered heterocyclyl, or 5-6 membered heteroaryl, wherein the amino, $C_{2-3}$ alkenyl, -O-$C_{1-3}$ deuterated alkyl, -S(O)$_2$-$C_{1-3}$ alkyl, -O-$C_{1-3}$ alkyl, -O-(5-6 membered heteroaryl), 5-8 membered heterocyclyl, or 5-6 membered heteroaryl is optionally substituted with $R^{8a}$.

In some embodiments, $R^8$ is selected from H, hydroxy, cyano, $C_{2-3}$ alkenyl, -O-$C_{1-3}$ deuterated alkyl, $C_{1-3}$ haloalkyl, -O-$C_{1-3}$ alkyl, -O-$C_{3-6}$ cycloalkyl, 4-7 membered heterocyclyl, or 5-6 membered heteroaryl, wherein the $C_{2-3}$ alkenyl, -O-$C_{1-3}$ deuterated alkyl, -O-$C_{1-3}$ alkyl, 5-8 membered heterocyclyl, or 5-6 membered heteroaryl is optionally substituted with $R^{8a}$.

In some embodiments, $R^8$ is selected from H, cyano, $C_{2-3}$ alkenyl, -O-$C_{1-3}$ deuterated alkyl, $C_{1-3}$ haloalkyl, -O-$C_{1-3}$ alkyl, -O-$C_{3-6}$ cycloalkyl, or 5-6-membered heteroaryl, wherein the $C_{2-3}$ alkenyl, - O-$C_{1-3}$ deuterated alkyl, -O-$C_{1-3}$ alkyl, or 5-6-membered heteroaryl is optionally substituted with $R^{8a}$. In some embodiments, $R^8$ is selected from H, $C_{1-3}$ haloalkyl, -O-$C_{1-3}$ alkyl, -O-$C_{3-6}$ cycloalkyl, or 5-6 membered heteroaryl, wherein the -O-$C_{1-3}$ alkyl or 5-6 membered heteroaryl is optionally substituted with $R^{8a}$.

In some embodiments, $R^8$ is selected from H, hydroxy, cyano, amino,

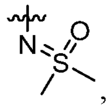, 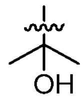,

-C(O)$R^{8a}$, - C(O)$NHR^{8a}$, -OC(O)$R^{8a}$, -NHC(O)$R^{8a}$, $CH_3$, $CH_2CH_3$, -CH=$CH_2$, -O-$CD_3$, $C_{1-3}$ haloalkyl, -S(O)$_2$-$CH_3$, -O-$CH_3$, -O-$CH_2CH_3$, -O-CH($CH_3$)$_2$, -O-cyclobutyl, -O-pyrazolyl, -O-cyclopropyl, -O-pyridinyl, -O-piperidinyl, -O-phenyl, piperidinyl, tetrahydrofuranyl, 3,6-diazabicyclo[3.1.1]heptanyl, phenyl, azetidinyl, pyrimidinyl, pyrazolyl, pyridinyl, morpholinyl, piperazinyl, tetrahydropyrrolyl, 3-oxa-8-azabicyclo[3.2. 1]octanyl, piperidinyl, 2-oxa-6-aza-spiro[3,3]heptanyl, 2-oxa-5-azabicyclo[2.2.1] heptanyl, 8-oxa-3-azabicyclo[3.2.1]octanyl,

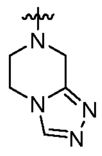, 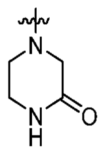, 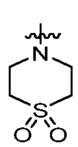, 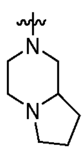, 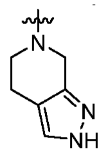, 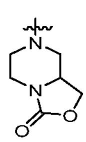,

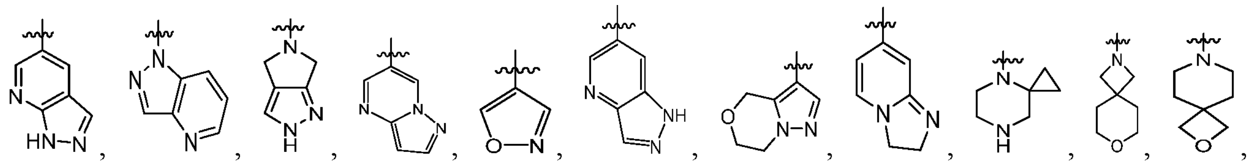

, , , , , , , , , , ,

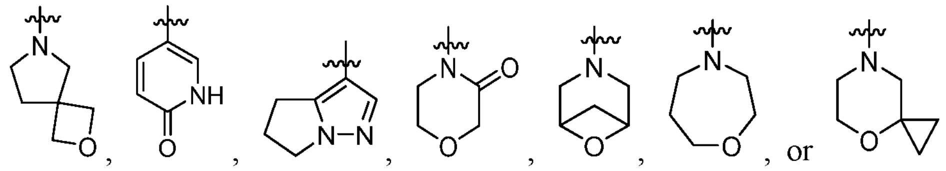

, , , , , , or ,

wherein the amino, $CH_3$, $CH_2CH_3$, $-S(O)_2-CH_3$, $-O-CH_3$, $-O-CH_2CH_3$, $-O-(CH_3)_2$, -O-cyclobutyl, -O-pyrazolyl, -O-cyclopropyl, -O-pyridinyl, -O-piperidinyl, -O-phenyl, piperidinyl, tetrahydrofuranyl, 3,6-diazabicyclo[3.1.1]heptanyl, phenyl, azetidinyl, pyrimidinyl, pyrazolyl, pyridinyl, morpholinyl, piperazinyl, tetrahydropyrrolyl, 3-oxa-8-azabicyclor[3.2.1]octanyl, piperidinyl, 2-oxa-6-aza-spiro[3,3]heptanyl, 2-oxa-5-azabicyclo[2.2.1]heptanyl, 8-oxa-3-azabicyclo[3.2.1]octanyl,

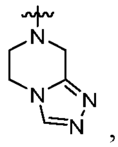

,

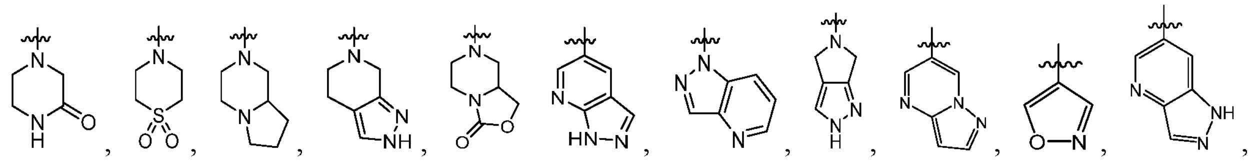

, , , , , , , , , , ,

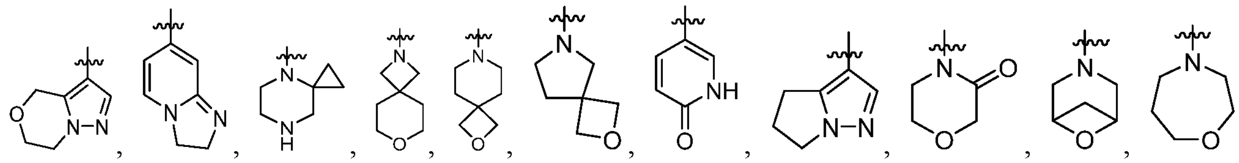

, , , , , , , , , , , ,

or

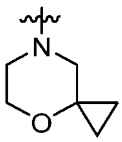

is optionally substituted with $R^{8a}$.

In some embodiments, $R^8$ is selected from H, hydroxy, cyano, amino,

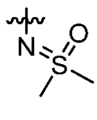

,

$-OC(O)R^{8a}$, $-CH=CH_2$, $-O-CD_3$, $C_{1-3}$ haloalkyl, $-S(O)_2-CH_3$, $-O-CH_3$, $-O-CH_2CH_3$, $-O-CH(CH_3)_2$, -O-cyclobutyl, -O-pyrazolyl, -O-cyclopropyl, -O-pyridinyl, -O-piperidinyl, phenyl, azetidinyl, pyrimidinyl, pyrazolyl, pyridinyl, morpholinyl, piperazinyl, tetrahydropyrrolyl, 3-oxa-8-azabicyclo[3.2.1]octanyl, piperidinyl, 2-oxa-6-aza-spiro[3,3]heptanyl, 2-oxa-5-azabicyclo[2.2.1]heptanyl, 8-oxa-3-azabicyclo[3.2.1]octanyl,

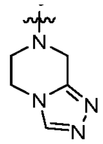, 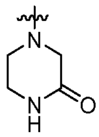, 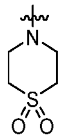, 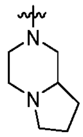, 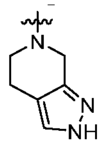, 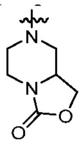, 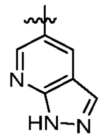, 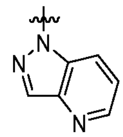,

or

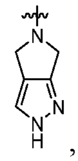,

wherein the amino, $-S(O)_2-CH_3$, $-O-CH_3$, $-O-CH_2CH_3$, $-O-(CH_3)_2$, -O-cyclobutyl, -O-pyrazolyl, -O-cyclopropyl, -O-pyridinyl, -O-piperidinyl, phenyl, azetidinyl, pyrimidinyl, pyrazolyl, pyridinyl, morpholinyl, piperazinyl, tetrahydropyrrolyl, 3-oxa-8-azabicyclo[3.2.1]octanyl, piperidinyl, 2-oxa-6-aza-spiro[3,3]heptanyl, 2-oxa-5-azabicyclo[2.2.1]heptanyl, 8-oxa-3-azabicyclo[3.2.1]octanyl,

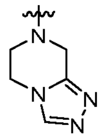, 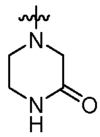, 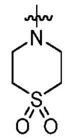, 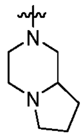, 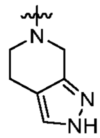, 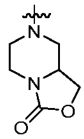, 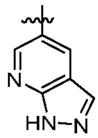, 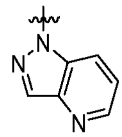, or 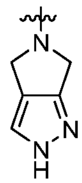

is optionally substituted with $R^{8a}$.

In some embodiments, $R^8$ is selected from H, hydroxy, cyano, amino, $-CH=CH_2$, $-O-CD_3$, $C_{1-3}$ haloalkyl, $-S(O)_2-CH_3$, $-O-CH_3$, $-O-CH_2CH_3$, $-O-CH(CH_3)_2$, -O-cyclobutyl, -O-pyrazolyl, -O-cyclopropyl, pyrazolyl, pyridinyl, morpholinyl, piperazinyl, tetrahydropyrrolyl, 3-oxa-8-azabicyclo[3.2.1]octanyl, piperidinyl, 2-oxa-6-aza-spiro[3,3]heptanyl, 2-oxa-5-azabicyclo[2.2.1]heptanyl, or 8-oxa-3-azabicyclo[3.2.1]octanyl, wherein the amino, $-S(O)_2-CH_3$, $-O-CH_3$, $-O-CH_2CH_3$, $-O-(CH_3)_2$, -O-cyclobutyl, -O-pyrazolyl, -O-cyclopropyl, pyrazolyl, pyridinyl, morpholinyl, piperazinyl, tetrahydropyrrolyl, 3-oxa-8-azabicyclo[3.2.1]octanyl, piperidinyl, 2-oxa-6-aza-spiro[3,3]heptanyl, 2-oxa-5-azabicyclo[2.2.1]heptanyl, or 8-oxa-3-azabicyclo[3.2.1]octanyl is optionally substituted with $R^{8a}$.

In some embodiments, $R^8$ is selected from H, hydroxy, cyano, $-CH=CH_2$, $-O-CD_3$, $C_{1-3}$ haloalkyl, $-O-CH_3$, $-O-CH_2CH_3$, -O-cyclopropyl, pyrazolyl, pyridinyl, or morpholinyl, wherein the $-O-CH_3$, $-O-CH_2CH_3$, -O-cyclopropyl, pyrazolyl, pyridinyl, or morpholinyl is optionally substituted with $R^{8a}$.

In some embodiments, $R^8$ is selected from H, cyano, $-CH=CH_2$, $-O-CD_3$, $C_{1-3}$ haloalkyl, $-O-CH_3$, $-O-CH_2CH_3$, -O-cyclopropyl, pyrazolyl, or pyridinyl, wherein the $-O-CH_3$, $-O-CH_2CH_3$, -O-cyclopropyl, pyrazolyl, or pyridinyl is optionally substituted with $R^{8a}$.

In some embodiments, $R^8$ is selected from H, $C_{1-3}$ haloalkyl, $-O-CH_3$, $-O-CH_2CH_3$, -O-cyclopropyl, or pyrazolyl, wherein the $-O-CH_3$, $-O-CH_2CH_3$, -O-cyclopropyl, or pyrazolyl is optionally substituted with $R^{8a}$.

In some embodiments, $R^8$ is selected from H hydroxy, cyano, amino, $-CH=CH_2$, $-O-CD_3$ $CHF_2$, $CF_3$, $-O-CH_3$, $-O-CH_2CH_3$, $-O-CH_2CF_3$, $-O-CH_2CHF_2$,

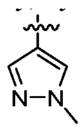, 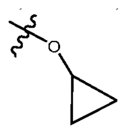, 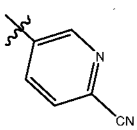, 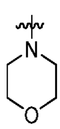, 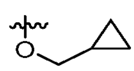,

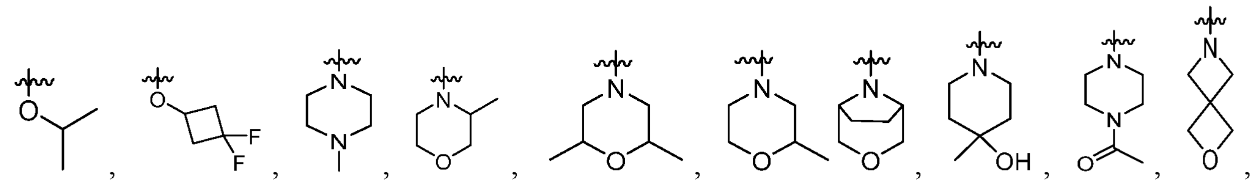

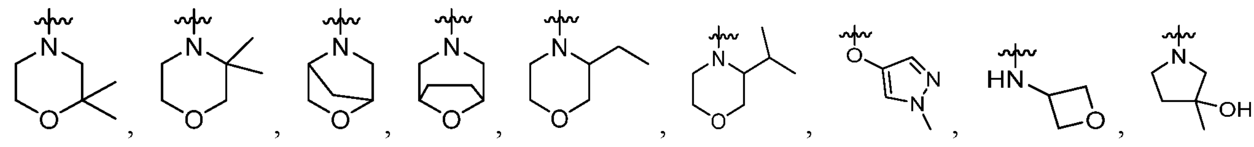

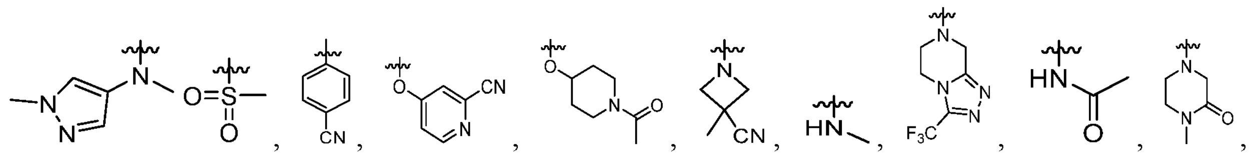

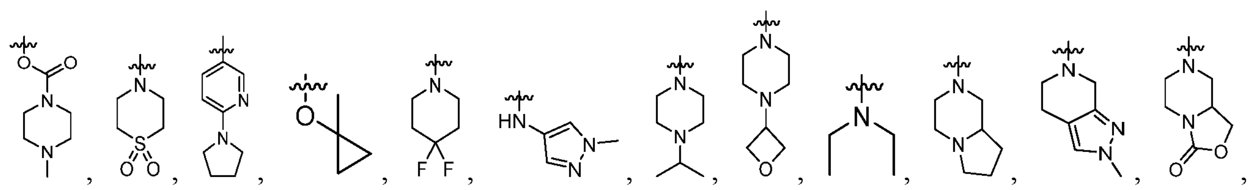

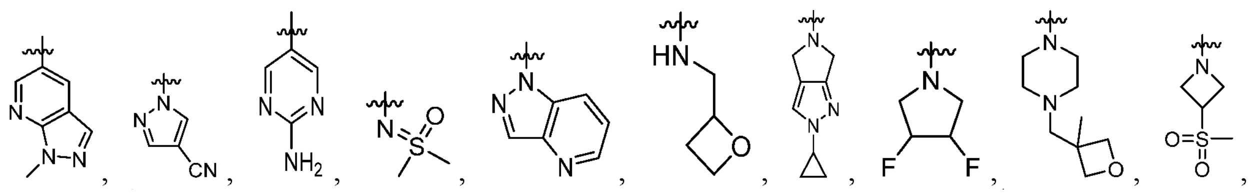

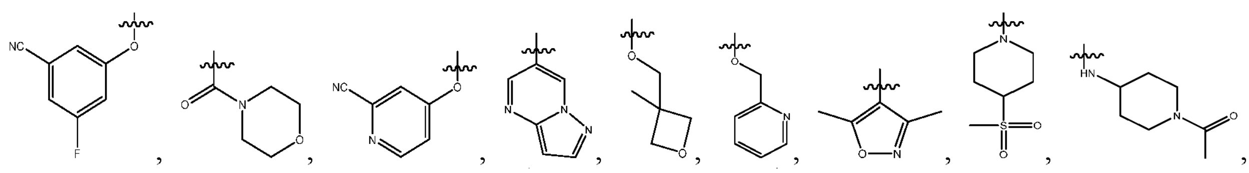

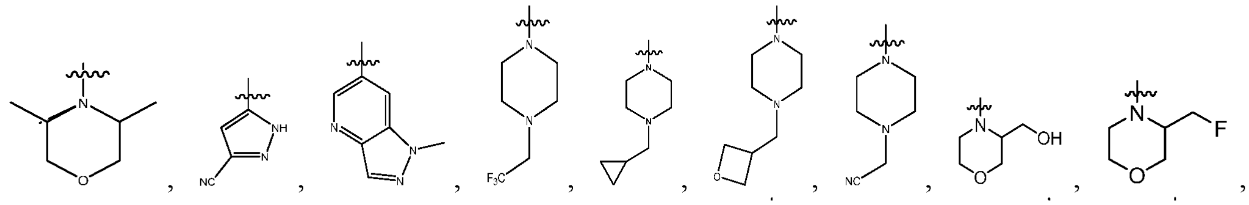

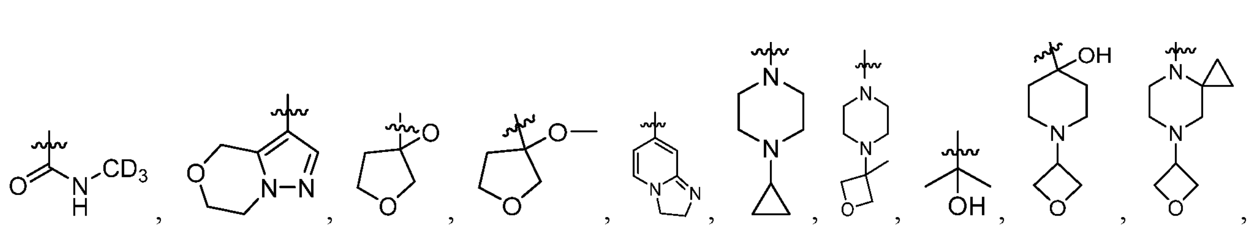

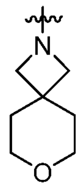, 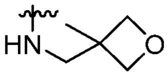, 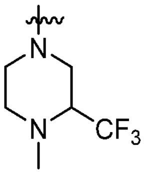, 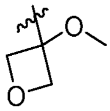, 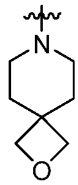, 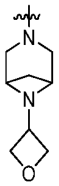, 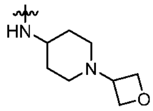, 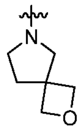, 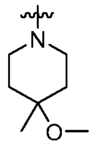,

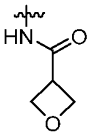, 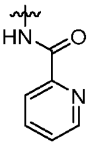, 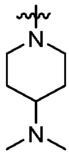, 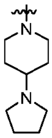, 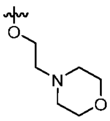, 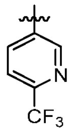, 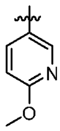, 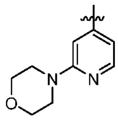, 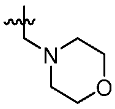, 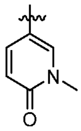, 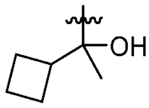,

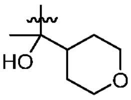, 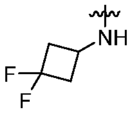, 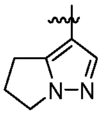, 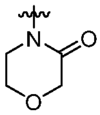, 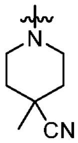, 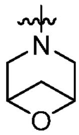, 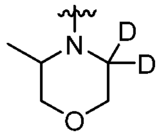, 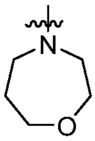, or 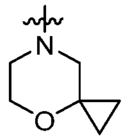.

In some embodiments, $R^8$ is selected from H, hydroxy, cyano, amino, $-CH=CH_2$, $-O-CD_3$, $CHF_2$, $CF_3$, $-O-CH_3$, $-O-CH_2CH_3$, $-O-CH_2CF_3$, $-O-CH_2CHF_2$,

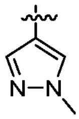, 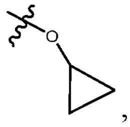, 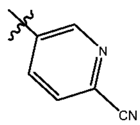, 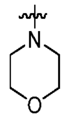, 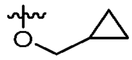,

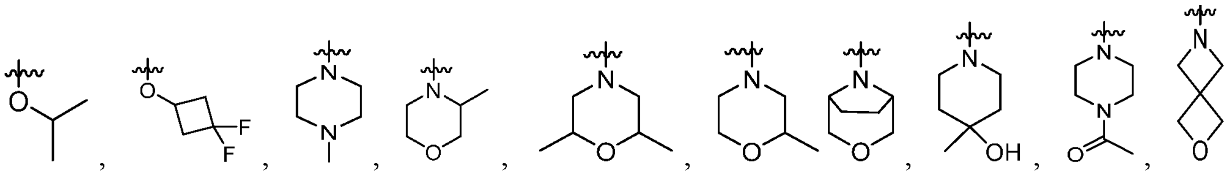, , , , , , , , , ,

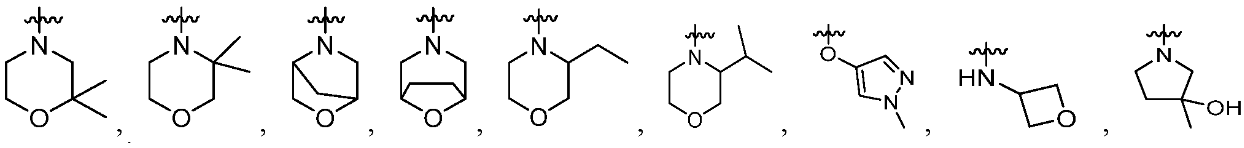, , , , , , , , ,

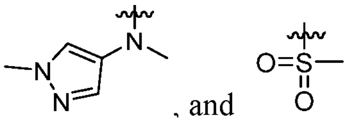, and .

In some embodiments $R^8$ is selected from H hydroxy, cyano, $-CH=CH_2$, $-O-CD_3$, $CHF_2$, $CF_3$, $-O-CH_3$, $-O-CH_2CH_3$, $-O-CH_2CF_3$, $-O-CH_2CHF_2$,

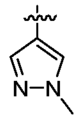, 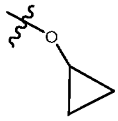, or 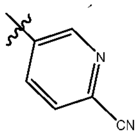.

In some embodiments, $R^8$ is selected from H, cyano, $-CH=CH_2$, $-O-CD_3$, $CHF_2$, $CF_3$, $-O-CH_3$, $-O-CH_2CH_3$, -O-

$CH_2CF_3$ -O-$CH_2CHF_2$,

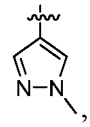, 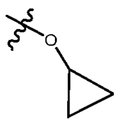, or 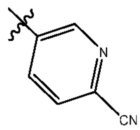.

In some embodiments, $R^8$ is selected from H, $CF_3$, -O-$CH_3$, -O-$CH_2CH_3$, -O-$CH_2CF_3$, -O-$CH_2CHF_2$, or

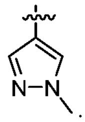.

In some embodiments, $R^{8a}$ is independently selected from halogen, hydroxy, cyano, amino, $C_{1-6}$ alkyl, $C_{1-6}$ deuterated alkyl, -O-$C_{1-6}$ alkyl, $C_{1-6}$ haloalkyl, -C(O)-$C_{1-6}$ alkyl, -S(O)$_2$$R^{8b}$, $C_{3-6}$ cycloalkyl, 3-8 membered heterocyclyl, or 5-6 membered heteroaryl, wherein the -O-$C_{1-6}$ alkyl, $C_{1-6}$ alkyl, $C_{3-6}$ cycloalkyl, 3-8 membered heterocyclyl, or 5-6 membered heteroaryl is optionally substituted with $R^{8b}$. In some embodiments, $R^{8a}$ is independently selected from halogen, hydroxy, cyano, amino, $C_{1-6}$ alkyl, $C_{1-6}$ haloalkyl, -C(O)-$C_{1-6}$ alkyl, -S(O)$_2$$R^{8b}$, $C_{3-6}$ cycloalkyl, 3-8 membered heterocyclyl, or 5-6 membered heteroaryl, wherein the $C_{3-6}$ cycloalkyl, 3-8 membered heterocyclyl, or 5-6 membered heteroaryl is optionally substituted with $R^{8b}$.

In some embodiments, $R^{8a}$ is independently selected from halogen, hydroxy, cyano, amino, $C_{1-6}$ alkyl, $C_{1-6}$ haloalkyl, -C(O)-$C_{1-6}$ alkyl, $C_{3-6}$ cycloalkyl, 3-8 membered heterocyclyl, or 5-6 membered heteroaryl, wherein the $C_{3-6}$ cycloalkyl, 3-8 membered heterocyclyl, or 5-6 membered heteroaryl is optionally substituted with $R^{8b}$.

In some embodiments, $R^{8a}$ is independently selected from halogen, hydroxy, cyano, amino, $C_{1-6}$ alkyl, or $C_{1-6}$ haloalkyl.

In some embodiments, $R^{8a}$ is independently selected from deuterium, halogen, hydroxy, cyano, amino, $C_{1-6}$ alkyl, $C_{1-3}$ deuterated alkyl, -O-$C_{1-3}$ alkyl, -C(O)-$C_{1-3}$ alkyl, -S(O)$_2$$R^{8b}$, $C_{3-6}$ cycloalkyl, 4-7 membered heterocyclyl, or 5-6 membered heteroaryl, wherein the amino, -O-$C_{1-3}$ alkyl, $C_{1-6}$ alkyl, $C_{3-6}$ cycloalkyl, 4-7 membered heterocyclyl, or 5-6 membered heteroaryl is optionally substituted with $R^{8b}$.

In some embodiments, $R^{8a}$ is independently selected from halogen, hydroxy, cyano, amino, $C_{1-6}$ alkyl, $C_{1-3}$ deuterated alkyl, -O-$C_{1-3}$ alkyl, -C(O)-$C_{1-3}$ alkyl, -S(O)$_2$$R^{8b}$, $C_{3-6}$ cycloalkyl, 4-7 membered heterocyclyl, or 5-6 membered heteroaryl, wherein the -O-$C_{1-3}$ alkyl, $C_{1-6}$ alkyl, $C_{3-6}$ cycloalkyl, 4-7 membered heterocyclyl, or 5-6 membered heteroaryl is optionally substituted with $R^{8b}$.

In some embodiments, $R^{8a}$ is independently selected from halogen, hydroxy, cyano, amino, $C_{1-6}$ alkyl, -C(O)-$C_{1-3}$ alkyl, -S(O)$_2$$R^{8b}$, $C_{3-6}$ cycloalkyl, 4-7 membered heterocyclyl, or 5-6 membered heteroaryl, wherein the $C_{3-6}$ cycloalkyl, 4-7 membered heterocyclyl, or 5-6 membered heteroaryl is optionally substituted with $R^{8b}$.

In some embodiments, $R^{8a}$ is independently selected from halogen, hydroxy, cyano, $C_{1-6}$ alkyl, -C(O)-$C_{1-3}$ alkyl, $C_{3-6}$ cycloalkyl, 4-7 membered heterocyclyl, or 5-6 membered heteroaryl, wherein the $C_{3-6}$ cycloalkyl, 4-7 membered heterocyclyl, or 5-6 membered heteroaryl is optionally substituted with $R^{8b}$. In some embodiments, $R^{8a}$ is independently selected from deuterium, halogen, hydroxy, cyano, amino, -$CH_3$, -$CH_2CH_3$, -$CD_3$, -O-$CH_3$, -CH($CH_3$)$_2$, -C(O)-$CH_3$, -S(O)$_2$$CH_3$, cyclopropyl, oxetanyl, pyrazolyl, piperazinyl, tetrahydropyrrolyl, morpholinyl, pyridinyl, or piperidinyl, wherein the amino, -$CH_3$, - $CH_2CH_3$, -O-$CH_3$, cyclopropyl, oxetanyl, pyrazolyl, piperazinyl, tetrahydropyrrolyl, morpholinyl, pyridinyl, or piperidinyl is optionally substituted with $R^{8b}$.

In some embodiments, $R^{8a}$ is independently selected from halogen, hydroxy, cyano, amino, -$CH_3$, - $CH_2CH_3$, -$CD_3$, -O-$CH_3$, -CH($CH_3$)$_2$, -C(O)-$CH_3$, -S(O)$_2$$CH_3$, cyclopropyl, oxetanyl, pyrazolyl, piperazinyl, tetrahydropyrrolyl, morpholinyl, pyridinyl, or piperidinyl, wherein the -$CH_3$, -$CH_2CH_3$, - O-$CH_3$, cyclopropyl, oxetanyl, pyrazolyl, piperazinyl, tetrahydropyrrolyl, morpholinyl, pyridinyl, or piperidinyl is optionally substituted with $R^{8b}$.

In some embodiments, $R^{8a}$ is independently selected from halogen, hydroxy, cyano, amino, -$CH_3$, - $CH_2CH_3$, -CH($CH_3$)$_2$, -C(O)-$CH_3$, -S(O)$_2$$CH_3$, cyclopropyl, oxetanyl, pyrazolyl, piperazinyl, or tetrahydropyrrolyl.

In some embodiments, $R^{8a}$ is independently selected from halogen, hydroxy, cyano, -$CH_3$, -$CH_2CH_3$, -CH($CH_3$)$_2$, -C(O)-$CH_3$, cyclopropyl, oxetanyl, or pyrazolyl.

In some embodiments, $R^{8a}$ is independently selected from halogen, cyano, or $C_{1-6}$ alkyl.

In some embodiments, $R^{8a}$ is independently selected from halogen or $C_{1-6}$ alkyl.

In some embodiments, $R^{8a}$ is independently selected from F or $CH_3$.

In some embodiments, $R^{8b}$ is independently selected from halogen, hydroxy, cyano, amino, $C_{1-6}$ alkyl, or 3-8 membered heterocyclyl.

In some embodiments, $R^{8b}$ is independently selected from halogen, hydroxy, cyano, amino, or $C_{1-6}$ alkyl.

In some embodiments, $R^{8b}$ is independently selected from halogen, $C_{1-6}$ alkyl, or 4-6 membered heterocyclyl.

In some embodiments, $R^{8b}$ is independently selected from halogen, hydroxy, cyano, $C_{1-6}$ alkyl, 3-8 membered heterocyclyl, -C(O)-$C_{1-6}$ alkyl, or $C_{3-6}$ cycloalkyl, wherein the 3-8 membered heterocyclyl is optionally substituted with $C_{1-6}$ alkyl.

In some embodiments, $R^{8b}$ is independently selected from halogen, hydroxy, cyano, $C_{1-6}$ alkyl, 4-6 membered heterocyclyl, -C(O)-$C_{1-3}$ alkyl, or $C_{3-6}$ cycloalkyl, wherein the 4-6 membered heterocyclyl is optionally substituted with $C_{1-3}$ alkyl.

In some embodiments, $R^{8b}$ is independently selected from F, $CH_3$, oxetanyl, hydroxy, cyano, -C(O)-$CH_3$, cyclopropyl, or 3-methyloxetane.

In some embodiments, $R^{8b}$ is independently selected from F, $CH_3$, or oxetanyl.

In some embodiments, $R^{8b}$ is independently selected from $C_{1-6}$ alkyl.

In some embodiments, the compound of formula (I) or the stereoisomer thereof or the pharmaceutically acceptable salt thereof is selected from a compound of formula (II) or a stereoisomer thereof or a pharmaceutically acceptable salt thereof,

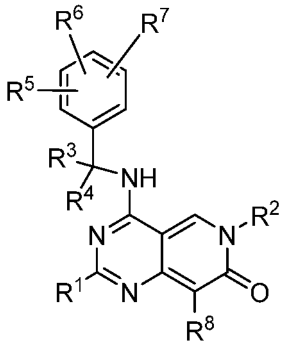

(II)

wherein $R^1$, $R^2$, $R^3$, $R^4$, $R^5$, $R^6$, $R^7$, and $R^8$ are as defined above.

It should be understood that in claim 12 referring to formula (II), when claim 12 refers to the preceding claim x, $R^1$, $R^2$, $R^3$, $R^4$, $R^5$, $R^6$, $R^7$, and $R^8$ in formula (II) are as defined in claim x. For example, when claim 12 refers to the preceding claim 1, $R^1$, $R^2$, $R^3$, $R^4$, $R^5$, $R^6$, $R^7$, and $R^8$ in formula (II) are as defined in claim 1; when claim 12 refers to the preceding claim 2, $R^1$, $R^2$, $R^3$, $R^4$, $R^5$, $R^6$, $R^7$, and $R^8$ in formula (II) are as defined in claim 2, and so on.

In some embodiments, the compound of formula (I) or the stereoisomer thereof or the pharmaceutically acceptable salt thereof is selected from a compound of formula (III) or a stereoisomer thereof or a pharmaceutically acceptable salt thereof,

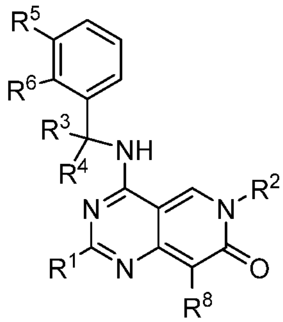

(III)

wherein R, $R^2$, $R^3$, $R^4$, $R^5$, $R^6$, and $R^8$ are as defined above.

It should be understood that in claim 13 referring to formula (III), when claim 13 refers to the preceding claim x, $R^1$, $R^2$, $R^3$, $R^4$, $R^5$, $R^6$, and $R^8$ in formula (III) are as defined in claim x. For example, when claim 13 refers to the preceding claim 1, $R^1$, $R^2$, $R^3$, $R^4$, $R^5$, $R^6$, and $R^8$ in formula (III) are as defined in claim 1; when claim 13 refers to the preceding claim 2, $R^1$, $R^2$, $R^3$, $R^4$, $R^5$, $R^6$, and $R^8$ in formula (III) are as defined in claim 2, and so on.

Without conflict, it should be understood that the embodiments described above may be combined arbitrarily,

forming a technical solution that includes the features of the combined embodiments. Such combined technical solutions are within the scope of the present invention.

In some embodiments, the compound of formula (I) or the stereoisomer thereof or the pharmaceutically acceptable salt thereof is selected from the following compounds or stereoisomers thereof or pharmaceutically acceptable salts thereof:

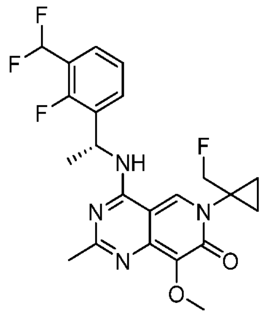
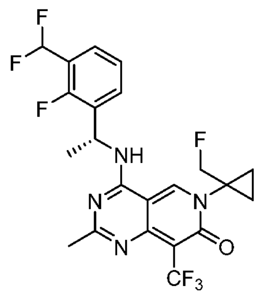
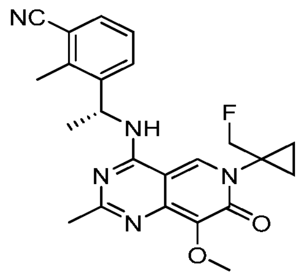
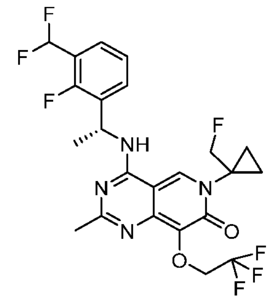
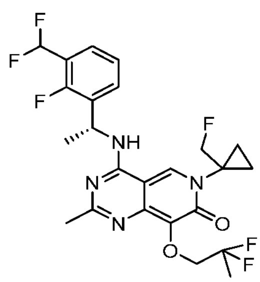
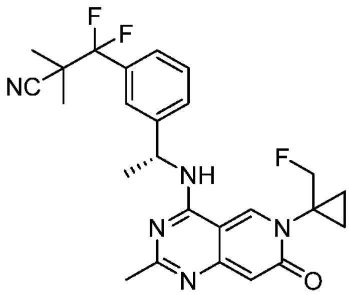
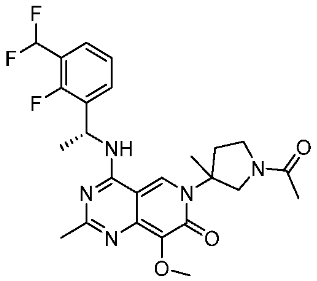
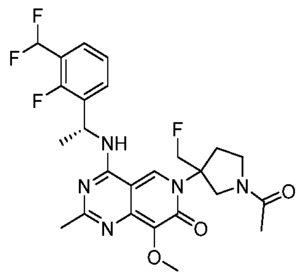
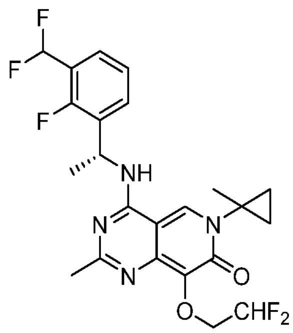
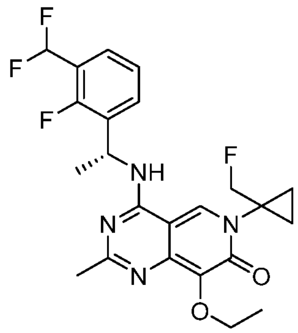
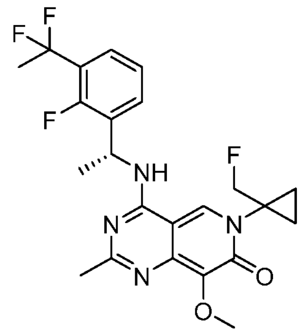
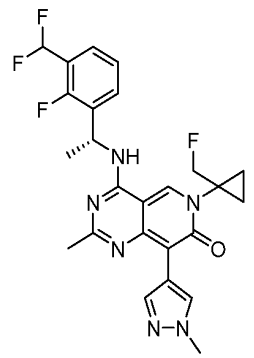
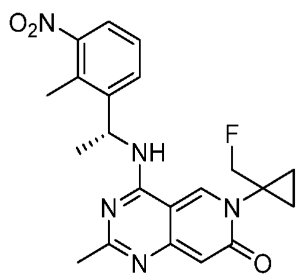
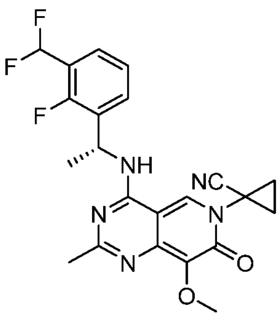
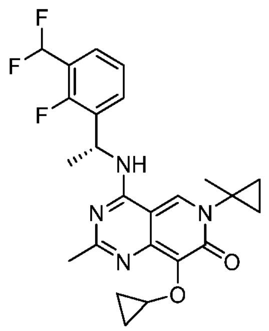
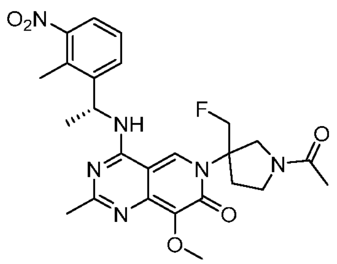

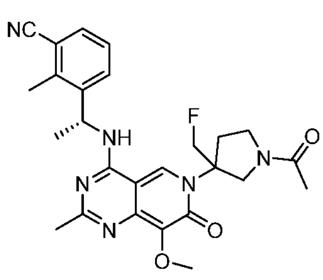
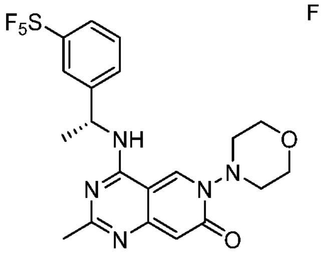
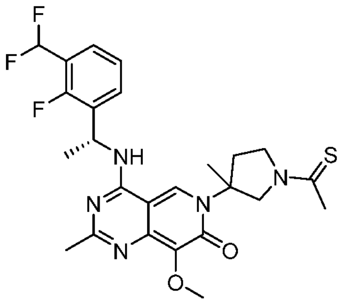
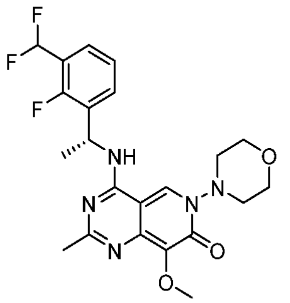

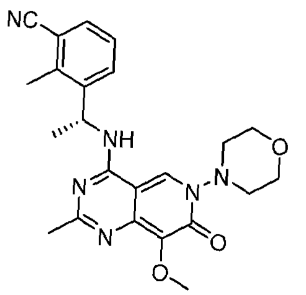
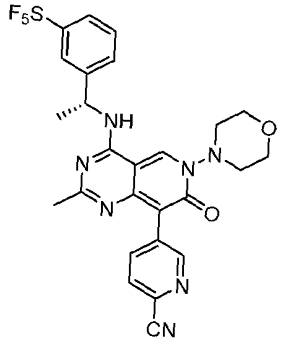
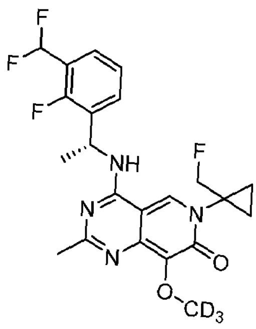
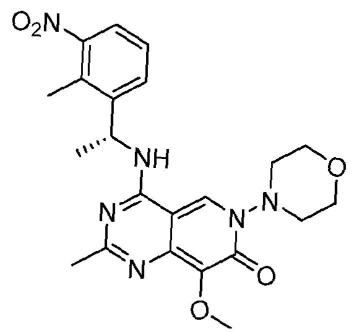

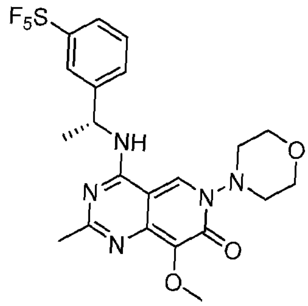
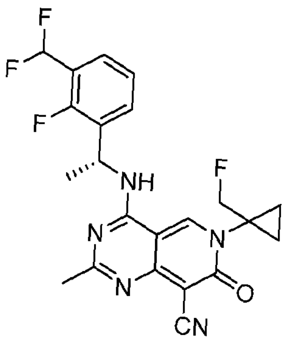
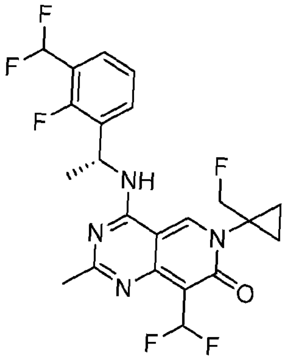
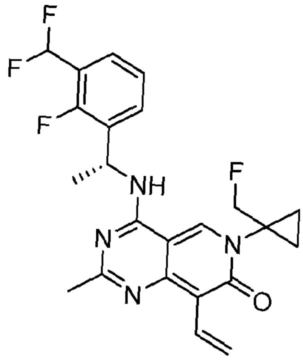
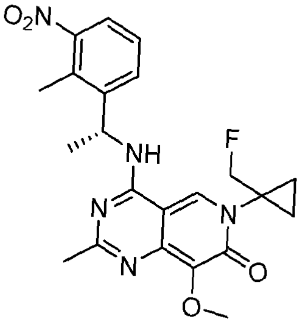
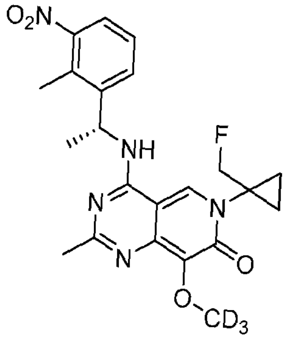
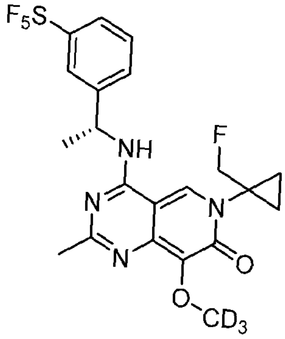
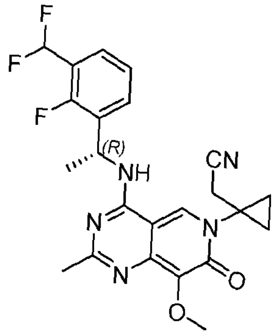
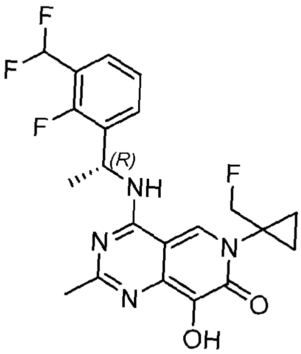
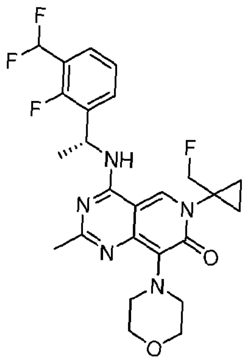
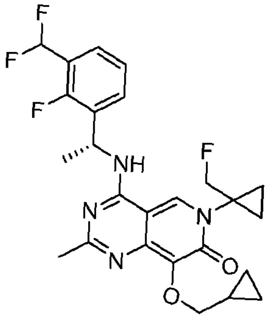
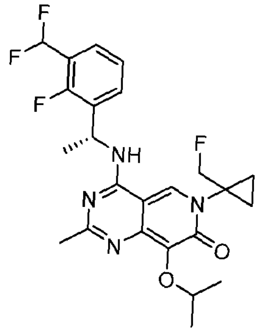
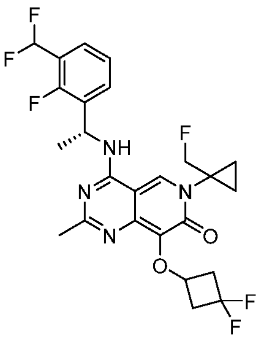
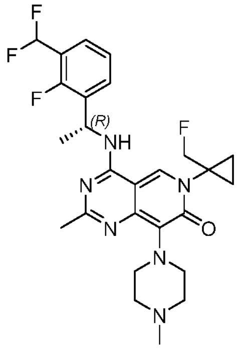
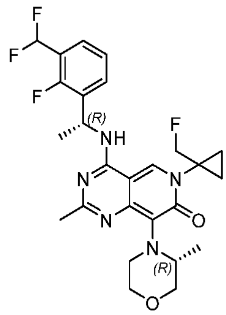
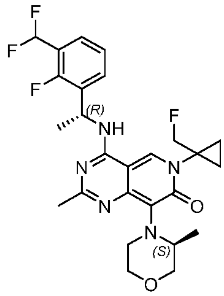

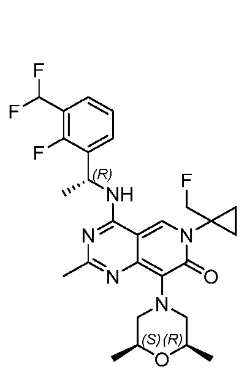

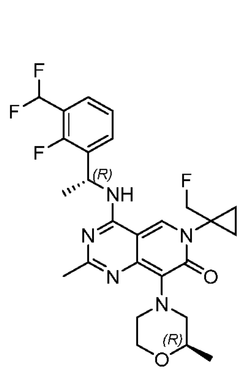

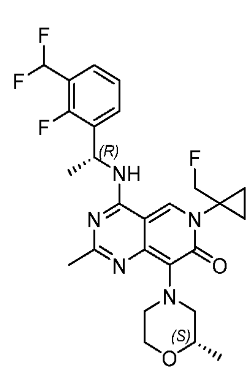

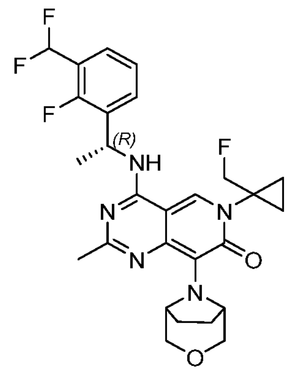

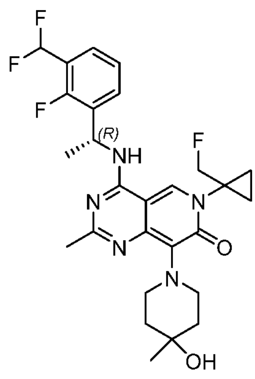

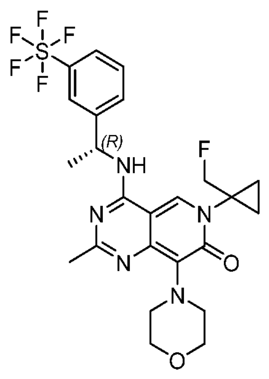

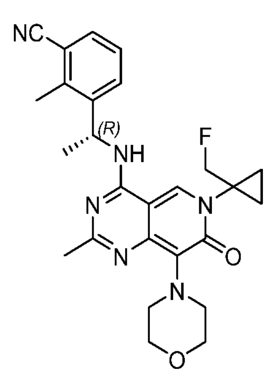

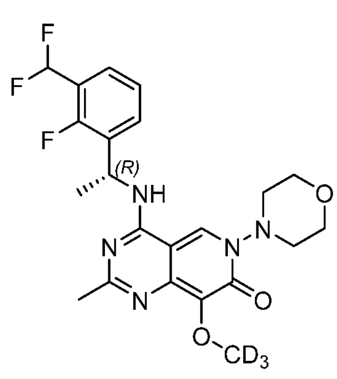

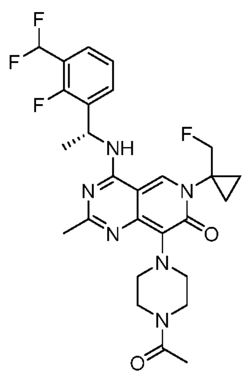

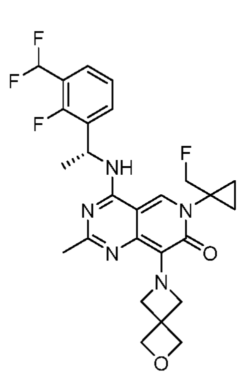

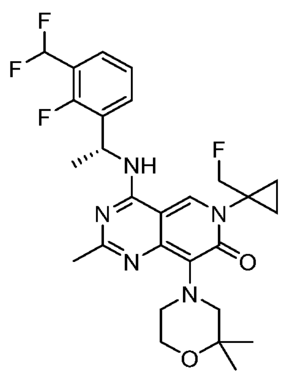

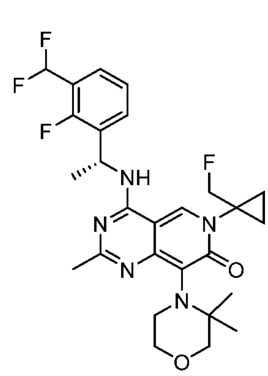

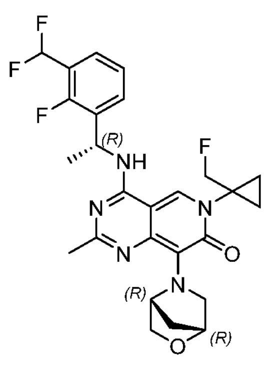

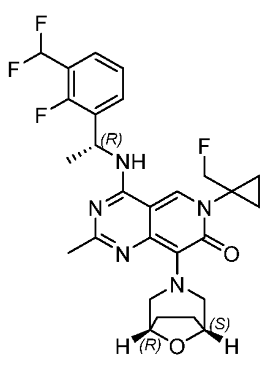

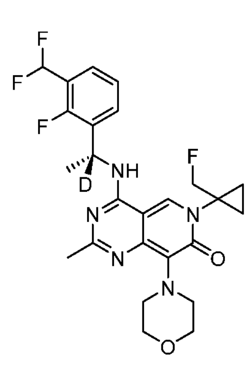

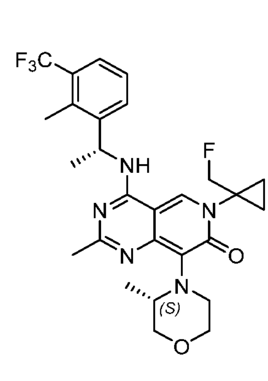

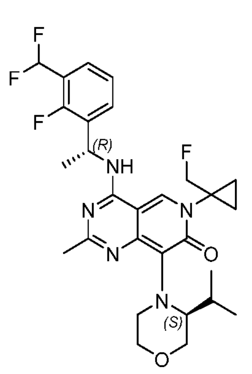
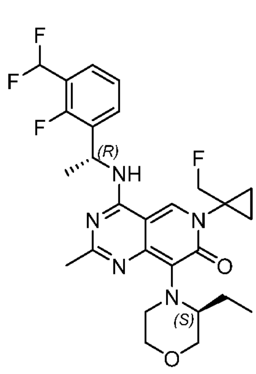
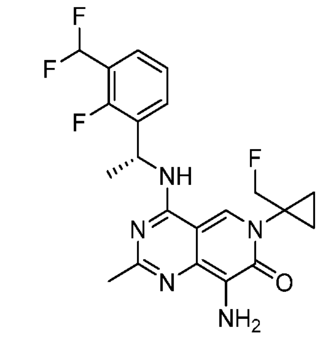
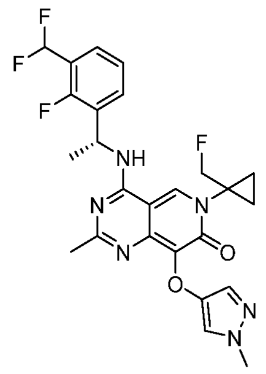

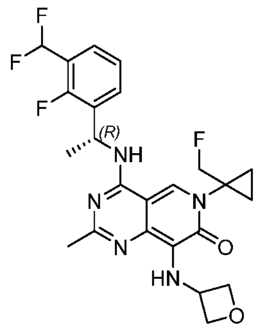
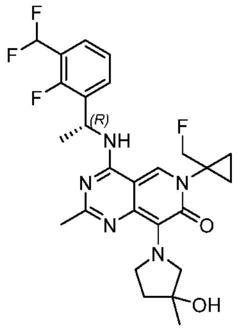
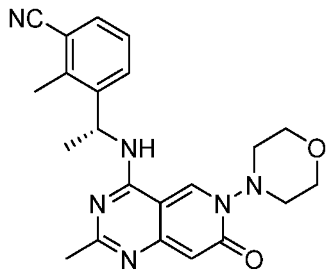
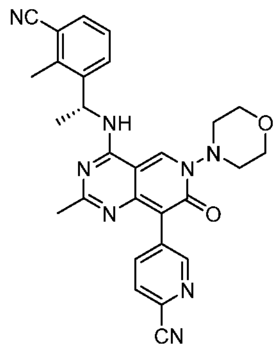

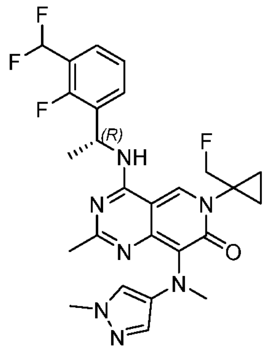
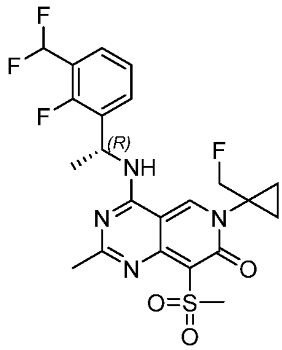
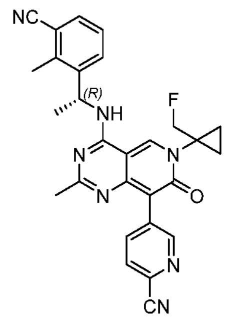
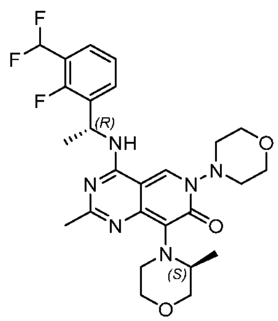

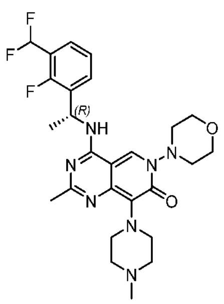
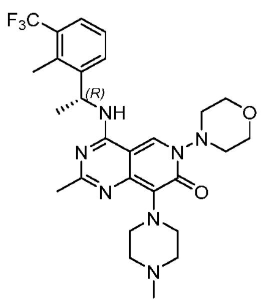
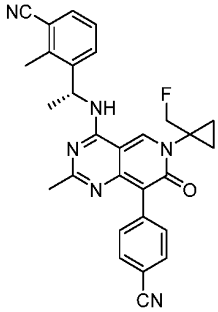
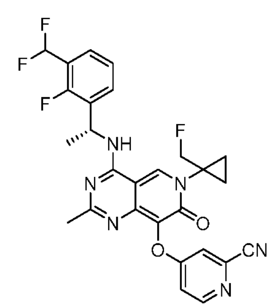

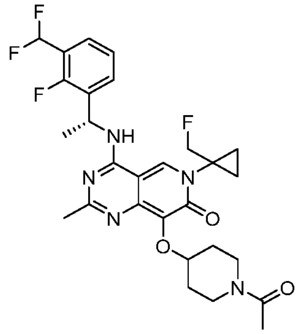
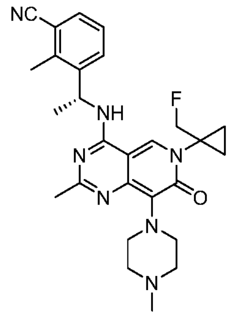
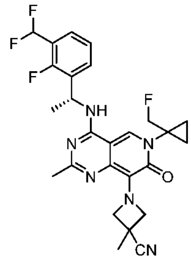
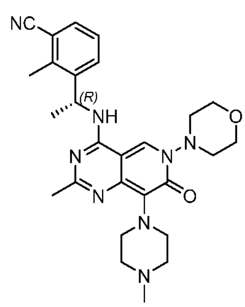

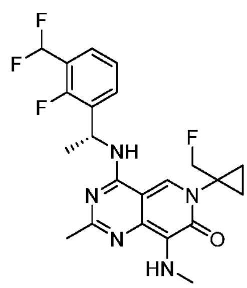
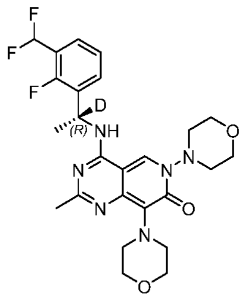
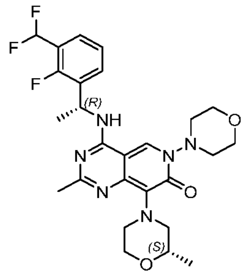
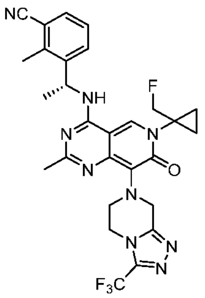

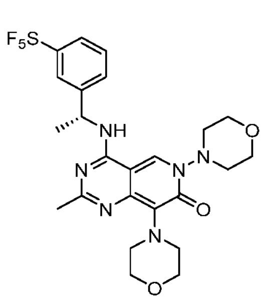
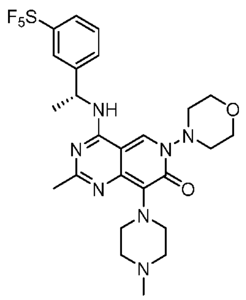
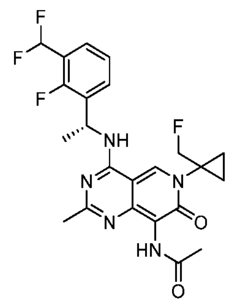
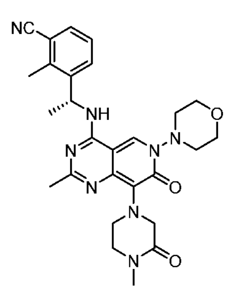

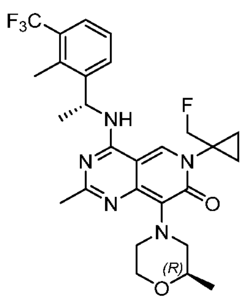
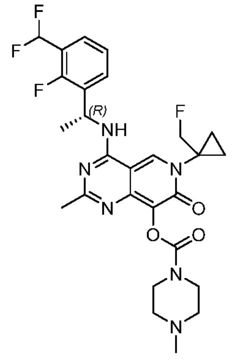
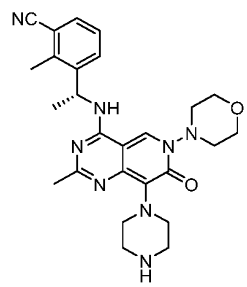
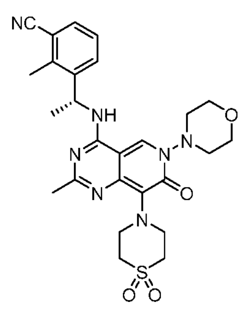

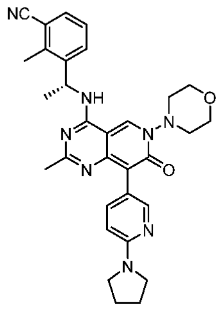
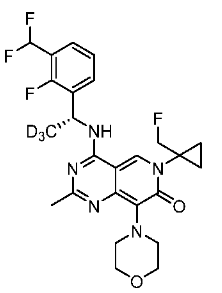
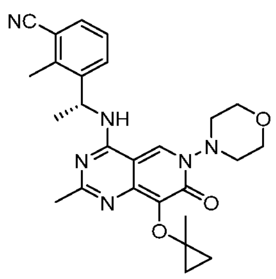
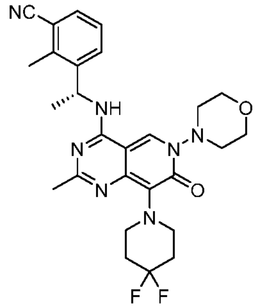

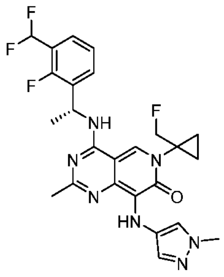
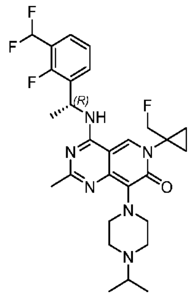
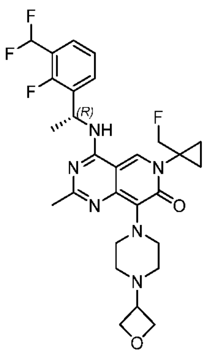
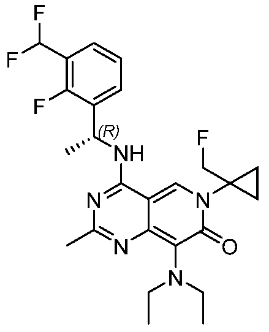
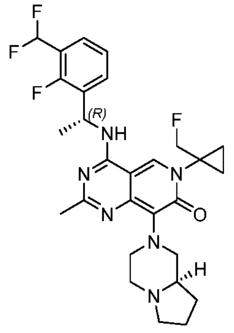

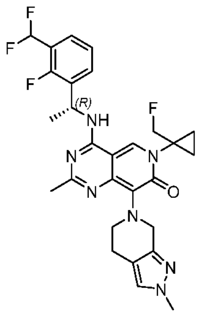
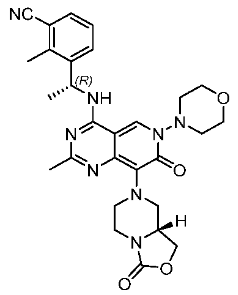
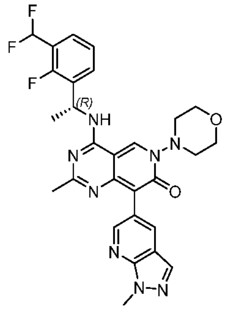
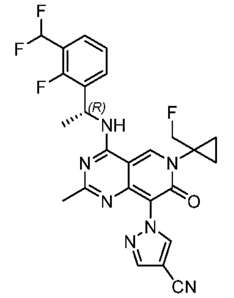

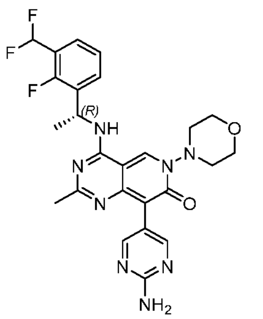
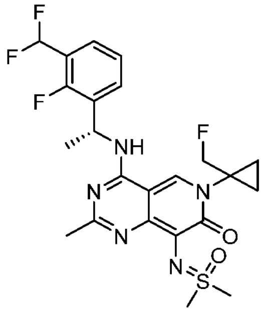
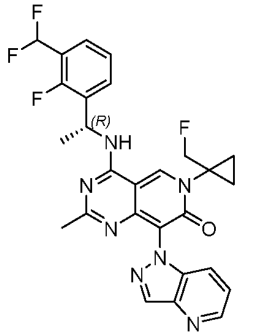
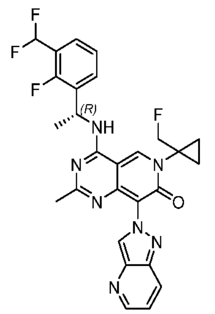

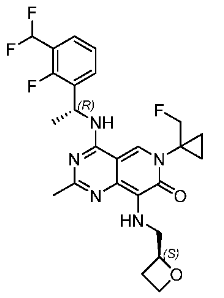
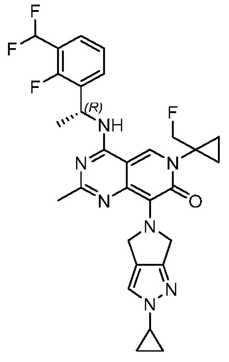
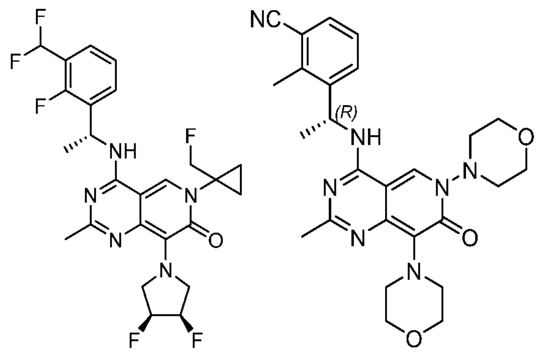

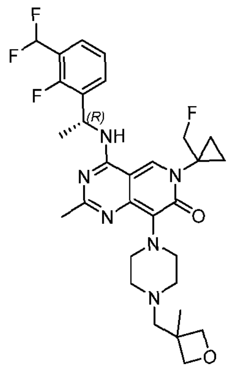
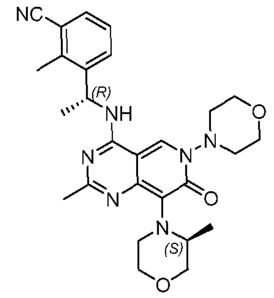
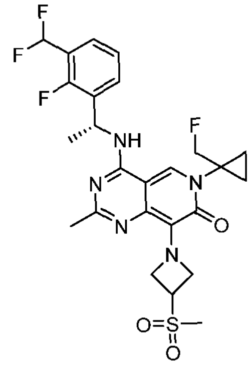
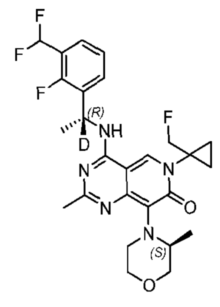
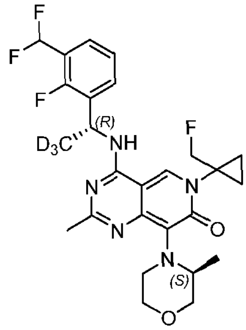
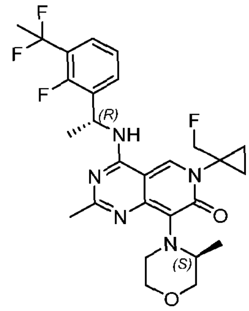
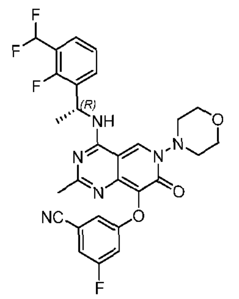
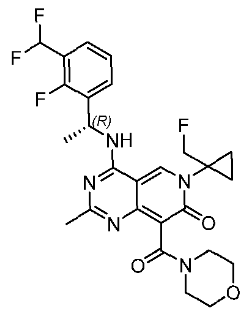
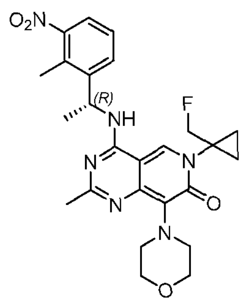
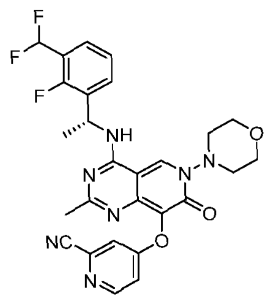
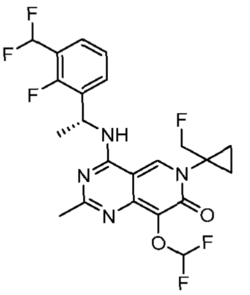
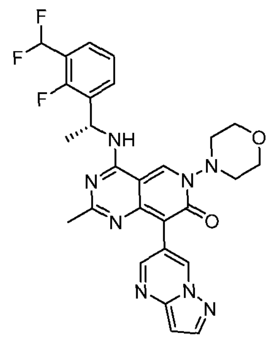
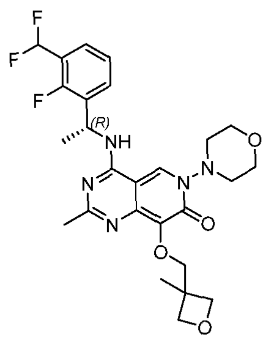
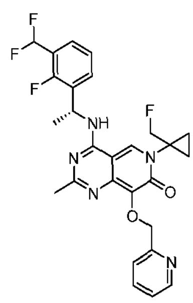
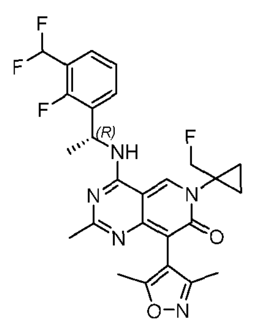
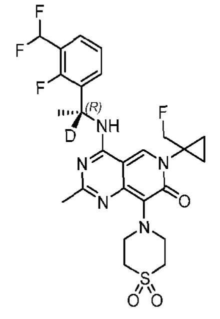
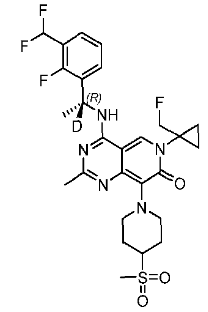

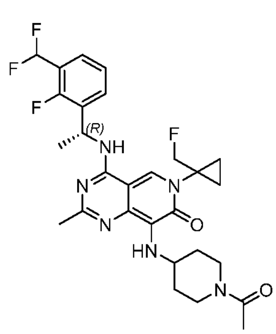
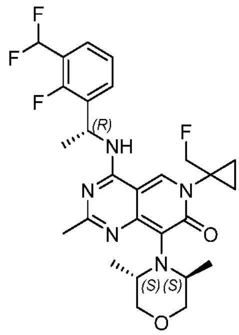
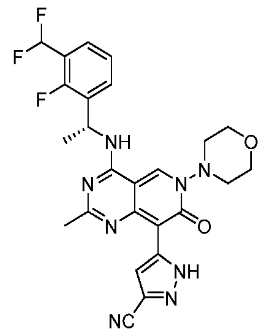
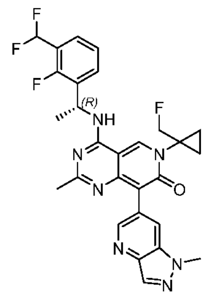

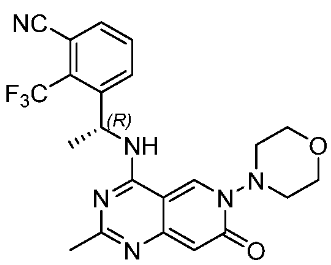
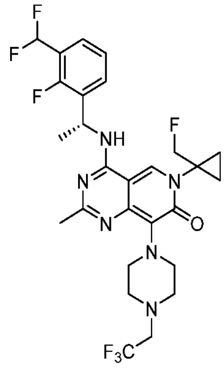
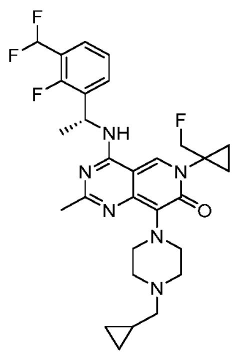
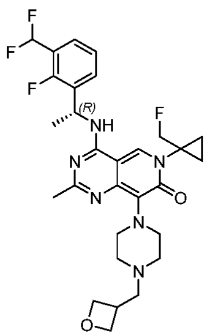

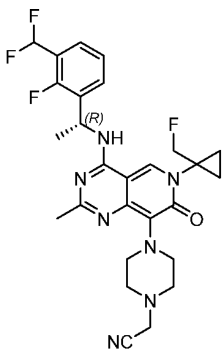
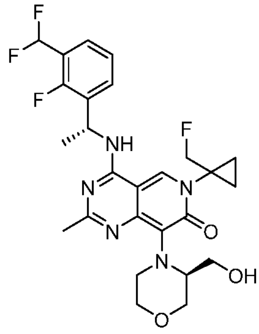
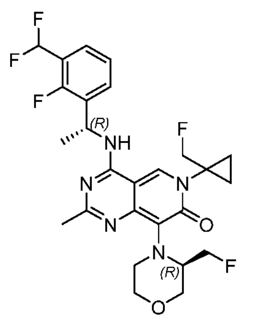
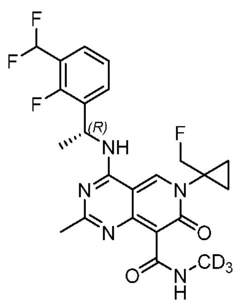

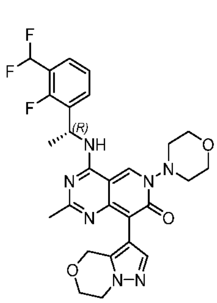
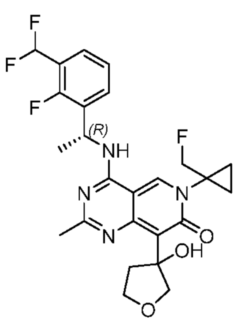
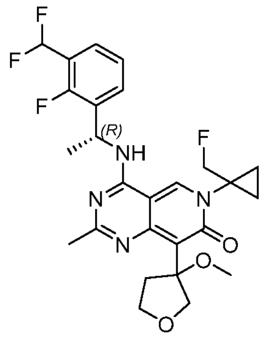
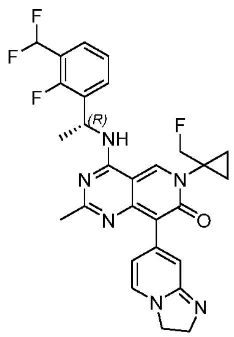
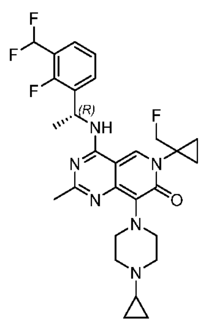

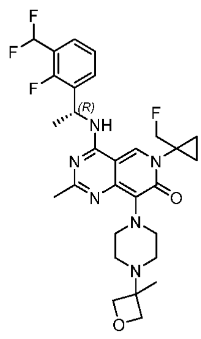
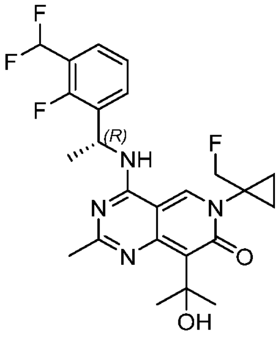
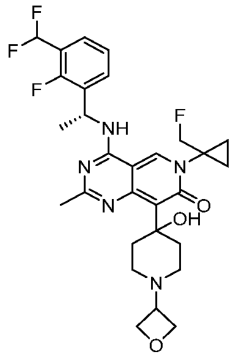
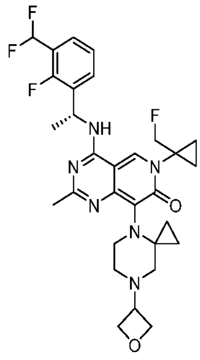
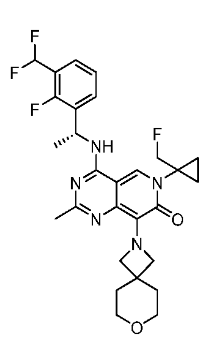

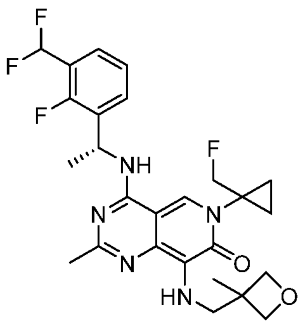
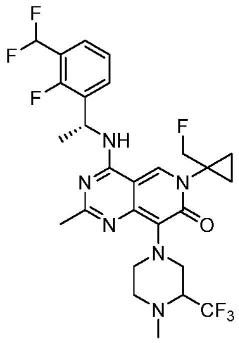
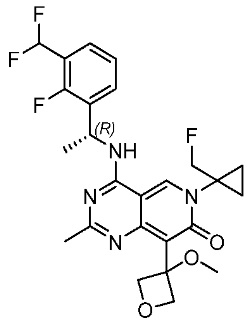
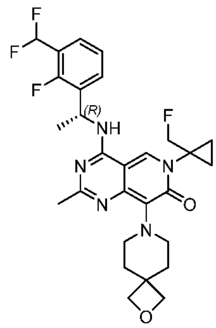

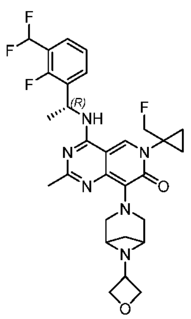
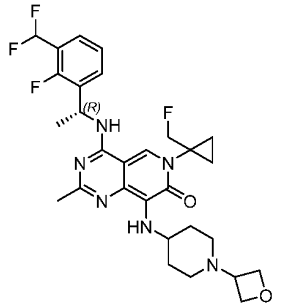
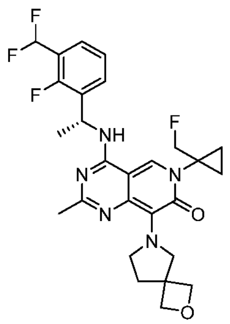
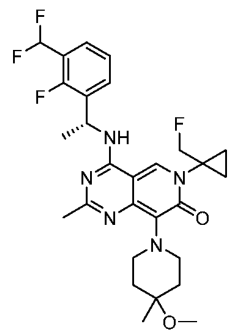

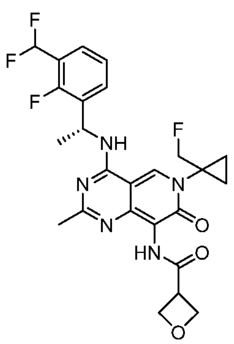
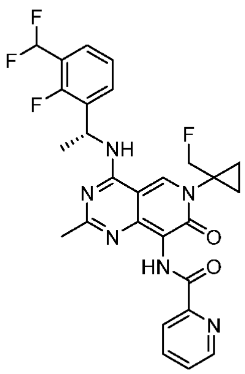
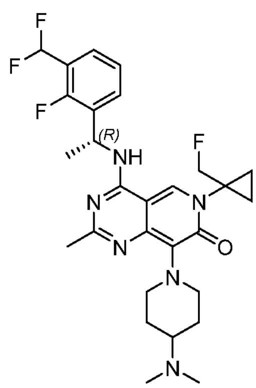
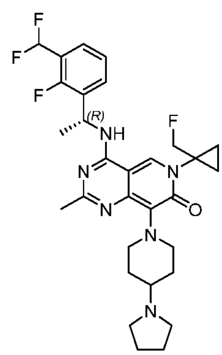

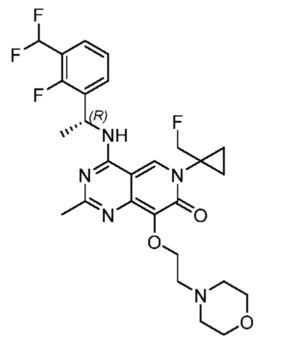
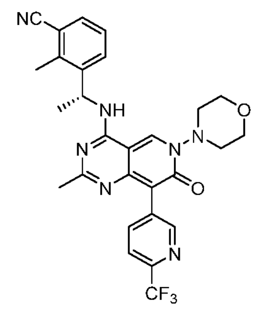
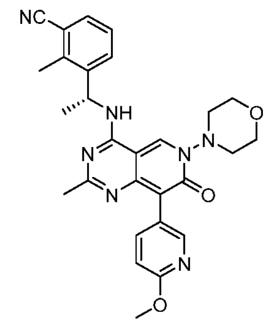
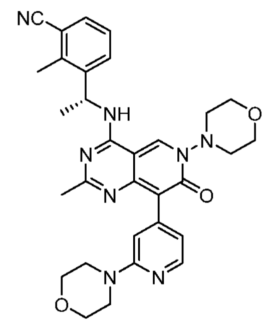

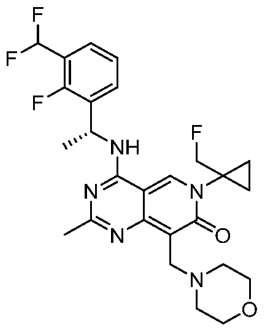
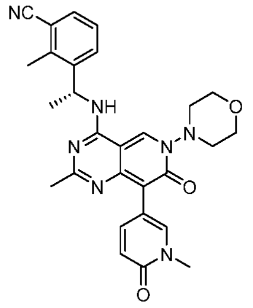
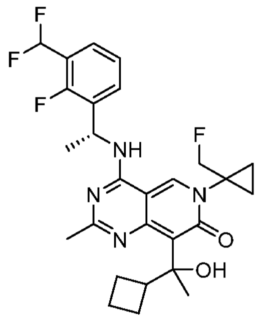
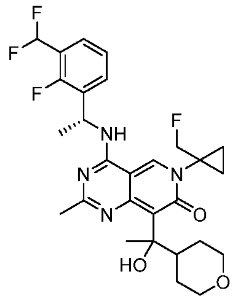

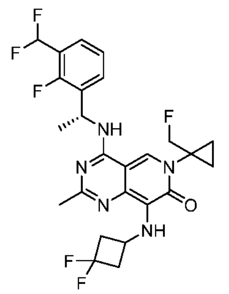
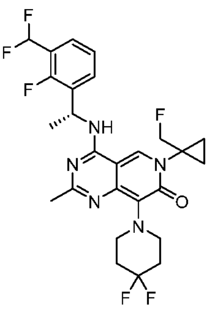
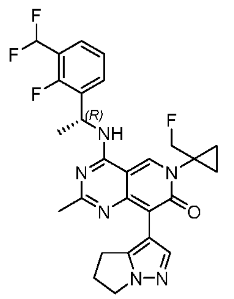
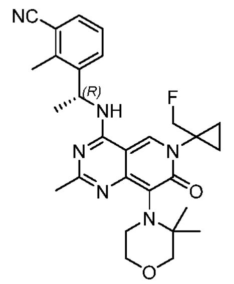
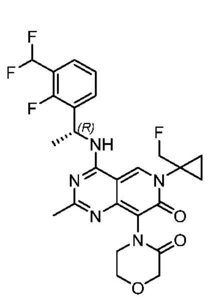

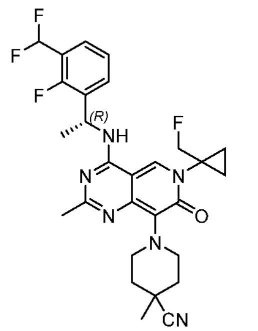
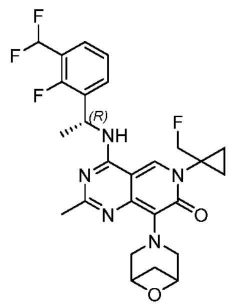
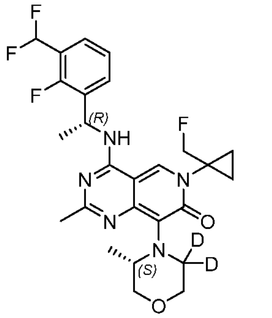
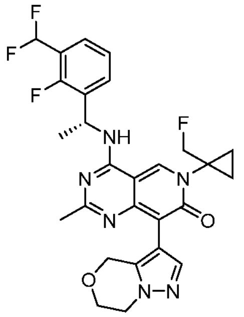

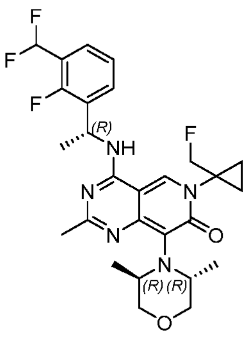 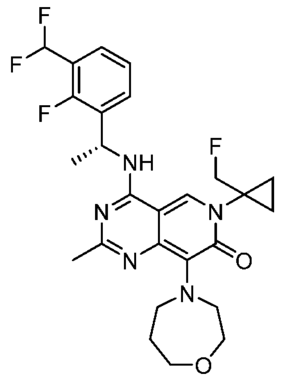 or 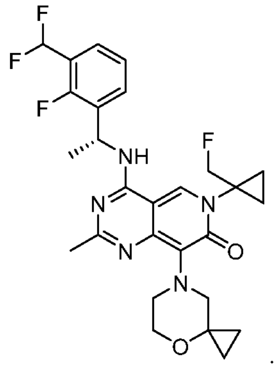 .

Further, the present invention provides a pharmaceutical composition, comprising the compound of formula (I) or the stereoisomer thereof or the pharmaceutically acceptable salt thereof and a pharmaceutically acceptable excipient.

Further, the present invention relates to use of the compound of formula (I) or the stereoisomer thereof or the pharmaceutically acceptable salt thereof or the pharmaceutical composition thereof in preparing a medicament for use in preventing or treating an SOS 1-related disease.

Further, the present invention relates to use of the compound of formula (I) or the pharmaceutically acceptable salt thereof or the pharmaceutical composition thereof in preventing or treating an SOS1-related disease.

Further, the present invention relates to the compound of formula (I) or the stereoisomer thereof or the pharmaceutically acceptable salt thereof or the pharmaceutical composition thereof for use in preventing or treating an SOS1-related disease.

The present invention further relates to a method for treating an SOS1-related disease, comprising administering to a subject (e.g., a mammal, preferably a human) in need of such treatment a therapeutically effective amount of the compound of formula (I) or the stereoisomer thereof or the pharmaceutically acceptable salt thereof or the pharmaceutical composition thereof described herein. Further, the SOS1-related disease is selected from cancer.

In another aspect, the present invention provides use of the compound of formula (I) or the stereoisomer thereof or the pharmaceutically acceptable salt thereof or the pharmaceutical composition thereof in preparing a medicament for use in preventing or treating cancer.

In another aspect, the present invention provides use of the compound of formula (I) or the stereoisomer thereof or the pharmaceutically acceptable salt thereof or the pharmaceutical composition thereof in preventing or treating cancer.

In another aspect, the present invention provides the compound of formula (I) or the stereoisomer thereof or the pharmaceutically acceptable salt thereof or the pharmaceutical composition thereof for use in preventing or treating cancer.

In another aspect, the present invention provides a method for treating cancer in a subject, comprising administering to a subject in need of such treatment a therapeutically effective amount of the compound of formula (I) or the stereoisomer thereof or the pharmaceutically acceptable salt thereof or the pharmaceutical composition thereof.

TERMINOLOGY AND DEFINITIONS

Unless otherwise stated, the terms used in the present application have the following meanings, and the definitions of groups and terms described in the present application, including definitions thereof as examples, exemplary definitions, preferred definitions, definitions documented in tables, definitions of specific compounds in the examples, and the like, may be arbitrarily combined and incorporated with each other. A certain term, unless otherwise specifically defined, should not be considered uncertain or unclear, but construed according to its common meaning in the field. When referring to a trade name, it is intended to refer to its corresponding commercial product or its active ingredient.

Herein,

“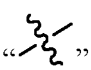”

represents a connection site.

The illustration of the pure compounds of the racemate or the enantiomer herein is from Maehr, J. Chem. Ed. 1985, 62: 114-120. Unless otherwise stated, the absolute configuration of a stereogenic center is represented by a

wedged bond and a wedged dashed bond ( 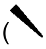 and 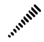 ), and the relative configuration of a stereogenic center (e.g., *cis*- or *trans*-configuration of alicyclic compounds) is represented by a black solid bond and a dashed bond ( 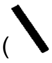 and 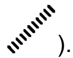 ).

The term "tautomer" refers to functional isomers resulting from the rapid movement of an atom in a molecule between two positions. The compounds disclosed herein may exhibit the tautomerism. Tautomeric compounds may exist in two or more interconvertible forms. Tautomers generally exist in an equilibrium form. Trying to separate a single tautomer usually leads to a mixture, the physicochemical properties of which are consistent with the mixture of the compound. The position of the equilibrium depends on the chemical properties of the molecule. For example, in many aliphatic aldehydes and ketones such as acetaldehyde, the keto form predominates; whereas in phenol, the enol form predominates. The present application comprises all tautomeric forms of the compound.

The term "stereoisomer" refers to isomers resulting from different spatial arrangements of atoms in a molecule, including *cis-trans* isomers, enantiomers, and diastereoisomers.

The compound of the present application may have an asymmetric atom such as a carbon atom, a sulfur atom, a nitrogen atom, a phosphorus atom, or an asymmetric double bond, and thus the compound of the present application may exist in the form of a particular geometric isomer or stereoisomer. The form of a particular geometric isomer or stereoisomer may be *cis* and *trans* isomers, E and Z geometric isomers, (-)- and (+)- enantiomers, (R)- and (S)- enantiomers, diastereoisomers, (D)-isomers, (L)-isomers, and racemic mixtures or other mixtures thereof, such as an enantiomer or diastereoisomer enriched mixture, and all of the above isomers as well as mixtures thereof are encompassed within the definition scope of the compound of the present application. An additional asymmetric carbon atom, asymmetric sulfur atom, asymmetric nitrogen atom, or asymmetric phosphorus atom may be present in substituents such as alkyl. All of these isomers and mixtures thereof referred to in the substituents are also encompassed within the definition scope of the compound of the present application. The compound with asymmetric atoms disclosed herein can be separated in an optically active pure form or in a racemic form. The optically active pure form can be isolated from a racemic mixture or synthesized by using chiral starting materials or chiral reagents.

The term "substituted" means that any one or more (e.g., 1, 2, 3, 4, 5, or 6) hydrogen atoms on a specific atom are substituted with substituents, wherein the substituents may be deuterium and hydrogen variants, as long as the valence of the specific atom is normal and the substituted compound is stable. When the substituent is oxo (namely =O), it means that two hydrogen atoms are substituted, and oxo is not available on an aromatic group.

The term "optional" or "optionally" means that the subsequently described event or circumstance may, but not necessarily, occur. The description includes instances where the event or circumstance occurs and instances where the event or circumstance does not. For example, an ethyl "optionally" substituted with halogen, means that the ethyl may be unsubstituted ($CH_2CH_3$), monosubstituted (e.g., $CH_2CH_2F$, $CH_2CH_2Cl$, or the like), polysubstituted (e.g., $CHFCH_2F$, $CH_2CHF_2$, $CHFCH_2Cl$, $CH_2CHCl_2$, or the like), or fully substituted ($CF_2CF_3$, $CF_2CCl_3$, $CCl_2CCl_3$, or the like). It will be understood by those skilled in the art that for any group comprising one or more substituents, no substitution or substituting pattern that is spatially impossible and/or cannot be synthesized will be introduced.

When any variable (e.g., $R^a$ or $R^b$) occurs at least once in the constitution or structure of a compound, the variable is independently defined in each case. For example, if a group is substituted with 2 $R^b$, the definition of each $R^b$ is independent.

When the linking direction of the linking group referred to herein is not specified, the linking direction is arbitrary. For example, when $L^1$ in the structural unit

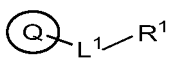

is selected from "$C_{1-3}$ alkylene-O", then $L^1$ may link ring Q and $R^1$ in a direction same as left-to-right reading order to constitute "ring Q-$C_{1-3}$ alkylene-O-$R^1$", or link ring Q and $R^1$ in a direction opposite to the left-to-right reading order to constitute "ring Q-O-$C_{1-3}$ alkylene-$R^1$".

$C_{m-n}$ used herein means that the portion has an integer number of carbon atoms in the range of m-n. For example, "$C_{1-10}$" means that the group may have 1 carbon atom, 2 carbon atoms, 3 carbon atoms, 4 carbon atoms, 5 carbon atoms, 6 carbon atoms, 7 carbon atoms, 8 carbon atoms, 9 carbon atoms, or 10 carbon atoms.

The term "alkyl" refers to hydrocarbyl with a general formula of $C_nH_{2n+1}$. The alkyl may be linear or branched. The term "$C_{1-10}$ alkyl" should be understood to represent a linear or branched saturated monovalent hydrocarbyl group having 1, 2, 3, 4, 5, 6, 7, 8, 9, or 10 carbon atoms. The alkyl includes, but is not limited to, methyl, ethyl, propyl, butyl, pentyl, hexyl, isopropyl, isobutyl, *sec*-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, and the like. The term "$C_{1-6}$ alkyl" refers to alkyl containing 1 to 6 carbon atoms (e.g., methyl, ethyl, *n*-propyl,

isopropyl, *n*-butyl, isobutyl, *sec*-butyl, *tert*-butyl, *n*-pentyl, 1-methylbutyl, 2-methylbutyl, 3-methylbutyl, neopentyl, hexyl, 2-methylpentyl, and the like). The "$C_{1}$-$_{10}$ alkyl" described herein may include "$C_{1}$-$_{6}$ alkyl", "$C_{1}$-$_{3}$ alkyl" or the like, and the "$C_{1}$-$_{6}$ alkyl" may further include "$C_{1}$-$_{3}$ alkyl".

The term "haloalkyl" is meant to include monohaloalkyl and polyhaloalkyl. For example, the term "$C_{1-6}$ haloalkyl" refers to $C_{1-6}$ alkyl as defined above substituted with one or more halogens, including but not limited to trifluoromethyl, 2,2,2-trifluoroethyl, 4-chlorobutyl, 3-bromopropyl, trichloromethyl, pentafluoroethyl, pentachloroethyl, and the like.

The term "deuterated alkyl" is meant to include monodeuterated alkyl and polydeuterated alkyl. For example, the term "$C_{1}$-$_{6}$ deuterated alkyl" refers to $C_{1-6}$ alkyl as defined above substituted with one or more deuterium, including but not limited to $CD_3$, $CH_2CD_3$, and the like.

The term "alkenyl" refers to a linear or branched unsaturated aliphatic hydrocarbon group consisting of carbon atoms and hydrogen atoms and having at least one double bond. For example, the term "$C_{2}$-$_{10}$ alkenyl" should be understood to preferably represent a linear or branched monovalent hydrocarbyl group comprising one or more double bonds and having 2, 3, 4, 5, 6, 7, 8, 9, or 10 carbon atoms, and "$C_2$-$C_{10}$ alkenyl" is preferably "$C_{2}$-$_{6}$ alkenyl", further preferably "$C_{2}$-$_{4}$ alkenyl", and still further preferably $C_2$ or $C_3$ alkenyl. It should be understood that in the case that the alkenyl comprises more than one double bond, the double bonds can be separated from one another or conjugated. The alkenyl includes, but is not limited to, vinyl, allyl, (*E*)-2-methylvinyl, (*Z*)-2-methylvinyl, (*E*)-but-2-enyl, (*Z*)-but-2-enyl, (*E*)-but-1-enyl, (*Z*)-but-1-enyl, isopropenyl, 2-methylprop-2-enyl, 1-methylprop-2-enyl, 2-methylprop-1-enyl, (*E*)-1-methylprop-1-enyl, and (*Z*)-1-methylprop-1-enyl.

The term "alkynyl" refers to a linear or branched unsaturated aliphatic hydrocarbon group consisting of carbon atoms and hydrogen atoms and having at least one triple bond. For example, the term "$C_{2}$-$_{10}$ alkynyl" should be understood to preferably represent a linear or branched monovalent hydrocarbyl group comprising one or more triple bonds and having 2, 3, 4, 5, 6, 7, 8, 9, or 10 carbon atoms. Examples of "$C_{2}$-$_{10}$ alkynyl" include, but are not limited to, ethynyl (-C=CH), prop-1-ynyl (1-propynyl, -C≡$CCH_3$), prop-2-ynyl (propargyl), but-1-ynyl, but-2-ynyl, or but-3-ynyl. "$C_{2}$-$_{10}$ alkynyl" may include "$C_{2}$-$_{3}$ alkynyl", and examples of "$C_{2}$-$_{3}$ alkynyl" include ethynyl (-C≡CH), prop-1-ynyl (1-propynyl, -C≡$CCH_3$), and prop-2-ynyl (propargyl).

The term "cycloalkyl" refers to a fully saturated carbon ring that exists in the form of a monocyclic ring, a fused ring, a bridged ring, a spiro ring, or the like. Unless otherwise specified, the carbon ring is generally a carbon ring formed by 3 to 10 carbon atoms. For example, the term "$C_{3}$-$_{10}$ cycloalkyl" should be understood as a saturated monovalent monocyclic, fused, spiro, or bridged ring having 3, 4, 5, 6, 7, 8, 9, or 10 carbon atoms. Specific examples of cycloalkyl include, but are not limited to, cyclopropyl, cyclobutyl, cyclopentyl, cyclohexyl, cycloheptyl, cyclooctyl, cyclononyl, cyclodecyl, norbomyl (bicyclo[2.2.1]heptyl), bicyclo[2.2.2]octyl, adamantyl, spiro[4.5]decyl, and the like. Spirocycloalkyl refers to cycloalkyl that exist as a spiro ring. The term "$C_{3}$-$_{10}$ cycloalkyl" may include "$C_{3}$-$_{6}$ cycloalkyl", and "$C_{3}$-$_{6}$ cycloalkyl" should be understood to represent a saturated monovalent monocyclic or bicyclic hydrocarbon ring having 3, 4, 5, or 6 carbon atoms. Specific examples include, but are not limited to, cyclopropyl, cyclobutyl, cyclopentyl, cyclohexyl, or the like.

The term "cycloalkenyl" refers to a non-aromatic carbon ring that is not fully saturated and exists in the form of a monocyclic ring, a fused ring, a bridged ring, a spiro ring, or the like. Unless otherwise specified, the carbon ring is generally a 4-10 membered ring. Specific examples of cycloalkenyl include, but are not limited to, cyclopentenyl, cyclopentadienyl, cyclohexenyl, cyclohexadienyl, cycloheptenyl, cycloheptadienyl, and the like.

The term "heterocyclyl" refers to a fully saturated or partially saturated (non-aromatic heteroaromatic group on the whole) monovalent monocyclic, fused cyclic, spiro cyclic, or bridged cyclic group, and ring atoms of the group include 1-5 heteroatoms or heteroatom groups (i.e., heteroatom-containing atom groups). The "heteroatom or heteroatom group" includes, but is not limited to, a nitrogen atom (N), an oxygen atom (O), a sulfur atom (S), a phosphorus atom (P), a boron atom (B), =O, =S, -O-N=, -C(=O)O-, -C(=O)-, -C(=S)-, -$S(=O)_2$-, and -S(=O)-, and optionally substituted -NH-, -S(=O)(=NH)-, -C(=O)NH-, -C(=NH)-, -$S(=O)_2$NH-, -S(=O)NH-, -NHC(=O)NH-, and the like. The term "3-10 membered heterocyclyl" refers to a heterocyclyl group having a ring atom number of 3, 4, 5, 6, 7, 8, 9, or 10, and ring atoms include 1-5 heteroatoms or heteroatom groups independently selected from those described above. Preferably, "3-10 membered heterocyclyl" includes "4-7 membered heterocyclyl", wherein examples of 4-membered heterocyclyl include, but are not limited to, azetidinyl or oxetanyl; examples of 5-membered heterocyclyl include, but are not limited to, tetrahydrofuranyl, dioxolyl, pyrrolidinyl, imidazolidinyl, pyrazolidinyl, pyrrolinyl, 4,5-dihydrooxazolyl, or 2,5-dihydro-1*H*-pyrrolyl; examples of 6-membered heterocyclyl include, but are not limited to, tetrahydropyranyl, piperidinyl, morpholinyl, dithianyl, thiomorpholinyl, piperazinyl, trithianyl, tetrahydropyridinyl, 4*H*-[1,3,4]thiadiazinyl,

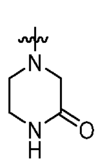, 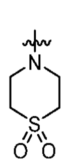, 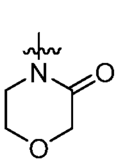, or 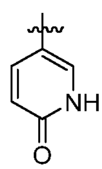;

examples of 7-membered heterocyclyl include, but are not limited to, diazepanyl. The heterocyclyl may also be a bicyclic group, wherein examples of 5,5-membered bicyclic groups include, but are not limited to, hexahydrocyclopenta[*c*]pyrrol-2(1*H*)-yl,

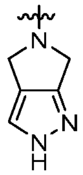, or 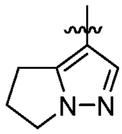;

examples of 5,6-membered bicyclic groups include, but are not limited to, hexahydropyrrolo[1,2-*a*]pyrazin-2(1*H*)-yl, 5,6,7,8-tetrahydro-[1,2,4]triazolo[4,3-*a*]pyrazinyl, 5,6,7,8-tetrahydroimidazo[1,5-*a*]pyrazinyl,

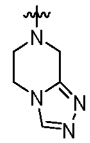, 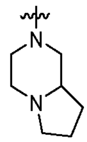, 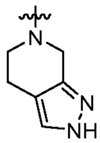,

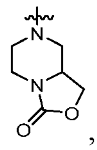, 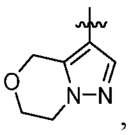, or 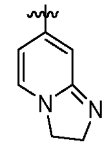.

Optionally, the heterocyclyl may be a benzo-fused ring group of the 4-7 membered heterocyclyl described above. Examples include, but are not limited to, dihydroisoquinolinyl and the like. Preferably, "3-10 membered heterocyclyl" may include "5-10 membered heterocyclyl", "4-7 membered heterocyclyl", "5-6 membered heterocyclyl", "6-8 membered heterocyclyl", "4-10 membered heterocycloalkyl", "5-10 membered heterocycloalkyl", "4-7 membered heterocycloalkyl", "5-6 membered heterocycloalkyl", "6-8 membered heterocycloalkyl", and the like, and "4-7 membered heterocyclyl" may further include "4-6 membered heterocyclyl", "5-6 membered heterocyclyl", "4-7 membered heterocycloalkyl", "4-6 membered heterocycloalkyl", "5-6 membered heterocycloalkyl", and the like. Although some bicyclic heterocyclyl herein comprise, in part, a benzene ring or a heteroaromatic ring, the heterocyclyl is still non-aromatic on the whole. The term "aryl" refers to an aromatic monocyclic or fused polycyclic group of carbon atoms with the conjugated $\pi$-electron system. For example, aryl may have 6-20 carbon atoms, 6-14 carbon atoms, or 6-12 carbon atoms. The term "$C_{6-20}$ aryl" should be understood as an aromatic or partially aromatic monovalent monocyclic, bicyclic, or tricyclic hydrocarbon ring having 6-20 carbon atoms. In particular, a ring having 6 carbon atoms ("$C_6$ aryl"), such as phenyl, or a ring having 9 carbon atoms ("$C_9$ aryl"), such as indanyl or indenyl, or a ring having 10 carbon atoms ("$C_{10}$ aryl"), such as tetrahydronaphthyl, dihydronaphthyl, or naphthyl, or a ring having 13 carbon atoms ("$C_{13}$ aryl"), such as fluorenyl, or a ring having 14 carbon atoms ("$C_{14}$ aryl"), such as anthryl. The term "$C_{6-10}$ aryl" should be understood as an aromatic or partially aromatic monovalent all-carbon monocyclic or bicyclic group having 6-10 carbon atoms. In particular, a ring having 6 carbon atoms ("$C_6$ aryl"), such as phenyl, a ring having 9 carbon atoms ("$C_9$ aryl"), such as indanyl or indenyl, or a ring having 10 carbon atoms ("$C_{10}$ aryl"), such as tetrahydronaphthyl, dihydronaphthyl, or naphthyl.

The term "heteroaryl" refers to an aromatic cyclic group having an aromatic monocyclic or fused polycyclic system in which at least one ring atom selected from N, O, and S is comprised, while the remaining ring atoms are C. The term "5-10 membered heteroaryl" should be understood as a monovalent aromatic monocyclic or bicyclic ring system which has 5, 6, 7, 8, 9, or 10 ring atoms, in particular 5, 6, 9, or 10 ring atoms, comprises 1-5, preferably 1-3, heteroatoms independently selected from N, O, and S. In particular, the heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, or the like, and benzo derivatives thereof, such as benzofuranyl, benzothienyl, benzothiazolyl, benzoxazolyl, benzoisoxazolyl, benzimidazolyl, benzotriazolyl, indazolyl, indolyl, isoindolyl, or the like; or pyridinyl, pyridazinyl, pyrimidinyl, pyrazinyl, triazinyl, or the like, and benzo derivatives thereof, such as quinolyl, quinazolinyl, isoquinolyl, or the like; or azocinyl, indolizinyl, purinyl, or the like, and benzo derivatives thereof; or cinnolinyl, phthalazinyl, quinazolinyl, quinoxalinyl, naphthyridinyl, pteridinyl, carbazolyl, acridinyl, phenazinyl, phenothiazinyl, phenoxazinyl, or the like. The term "5-6 membered heteroaryl" refers to an aromatic ring system which has 5 or 6 ring atoms, and comprises 1-3, preferably 1-2, heteroatoms independently selected from N, O, and S.

The term "halo-" or "halogen" refers to fluorine, chlorine, bromine and iodine.

The term "hydroxy" refers to -OH group.
The term "cyano" refers to -CN group.
The term "amino" refers to $-NH_2$ group.
The term "nitro" refers to $-NO_2$ group.
The term "therapeutically effective amount" refers to an amount of the compound of the present application for (i) treating a specific disease, condition or disorder; (ii) alleviating, ameliorating or eliminating one or more symptoms of a specific disease, condition or disorder, or (iii) delaying onset of one or more symptoms of a specific disease, condition or disorder described herein. The amount of the compound disclosed herein constituting the "therapeutically effective amount" varies dependently on the compound, the disease state and its severity, the administration regimen, and the age of the mammal to be treated, but can be determined routinely by those skilled in the art in accordance with their knowledge and the present disclosure.
The term "prevent" or "prevention" means administering the compound or formulation described herein to prevent a disease or one or more symptoms associated with the disease, and includes: preventing the occurrence of the disease or disease state in an individual (e.g., a mammal), particularly when such an individual (e.g., a mammal) is predisposed to the disease state but has not yet been diagnosed with it.
The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.
The term "pharmaceutically acceptable salt" refers to salts of pharmaceutically acceptable acids or bases, including salts formed from the compound and an inorganic or organic acid, and salts formed from the compound and an inorganic or organic base.
The term "pharmaceutical composition" refers to a mixture consisting of one or more of the compounds or the salts thereof of the present application and a pharmaceutically acceptable excipient. The pharmaceutical composition is intended to facilitate the administration of the compound of the present application to an organism.
The term "pharmaceutically acceptable excipients" refers to those which do not have a significant irritating effect on an organism and do not impair the biological activity and properties of the active compound. Suitable excipients are well known to those skilled in the art, such as carbohydrate, wax, water-soluble and/or water-swellable polymers, hydrophilic or hydrophobic materials, gelatin, oil, solvent, water, and the like.
The term "individual" includes mammals and non-mammals. Examples of mammals include, but are not limited to, any member of the class Mammalia: humans, non-human primates (e.g., chimpanzees and other apes and monkeys); livestock animals, such as cattle, horses, sheep, goats, and pigs; domestic animals, such as rabbits, dogs, and cats; laboratory animals, including rodents, such as rats, mice, guinea pigs, and the like. Examples of non-human mammals include, but are not limited to, birds, fish, and the like. In one embodiment associated with the methods and compositions provided herein, the mammal is a human.
The word "comprise" and variations thereof such as "comprises" or "comprising" should be understood in an open, non-exclusive sense, i.e., "including but not limited to".
The present application also comprises isotopically-labeled compounds which are identical to those documented herein but have one or more atoms replaced by an atom having an atomic mass or mass number different from the atomic mass or mass number usually found in nature. Examples of isotopes that can be incorporated into the compounds of the present application include isotopes of hydrogen, carbon, nitrogen, oxygen, phosphorus, sulfur, fluorine, iodine and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$ and $^{36}Cl$, respectively, and the like.
Certain isotopically-labeled compounds disclosed herein (e.g., those labeled with $^{3}H$ and $^{14}C$) can be used to analyze compounds and/or substrate tissue distribution. Tritiated (i.e., $^{3}H$) and carbon-14 (i.e., $^{14}C$) isotopes are particularly preferred for their ease of preparation and detectability. Positron emitting isotopes, such as $^{15}O$, $^{13}N$, $^{11}C$ and $^{18}F$ can be used in positron emission tomography (PET) studies to determine substrate occupancy. Isotopically-labeled compounds disclosed herein can generally be prepared by following procedures analogous to those disclosed in the schemes and/or examples below while substituting a non-isotopically labeled reagent with an isotopically-labeled reagent.
The pharmaceutical composition disclosed herein can be prepared by combining the compound disclosed herein with a suitable pharmaceutically acceptable excipient, and can be formulated, for example, into a solid, semisolid, liquid, or gaseous formulation such as tablet, pill, capsule, powder, granule, ointment, emulsion, suspension, suppository, injection, inhalant, gel, microsphere, aerosol, and the like.
Typical routes of administration of the compound or the stereoisomer thereof or the pharmaceutically acceptable salt thereof or the pharmaceutical composition thereof disclosed herein include, but are not limited to, oral, rectal, local, inhalation, parenteral, sublingual, intravaginal, intranasal, intraocular, intraperitoneal, intramuscular, subcutaneous and intravenous administration.
The pharmaceutical composition disclosed herein can be manufactured by methods well known in the art, such

as by conventional mixing, dissolving, granulating, emulsifying, lyophilizing, and the like.

In some embodiments, the pharmaceutical composition is in an oral form. For oral administration, the pharmaceutical composition can be formulated by mixing the active compounds with pharmaceutically acceptable excipients well known in the art. These excipients enable the compounds disclosed herein to be formulated into tablets, pills, pastilles, dragees, capsules, liquids, gels, slurries, suspensions and the like for oral administration to a patient.

A solid oral composition can be prepared by conventional mixing, filling or tableting. For example, it can be obtained by the following method: mixing the active compounds with solid excipients, optionally grinding the resulting mixture, adding additional suitable excipients if desired, and processing the mixture into granules to get the core parts of tablets or dragees. Suitable excipients include, but are not limited to: binders, diluents, disintegrants, lubricants, glidants, sweeteners or flavoring agents, and the like.

The pharmaceutical compositions may also be suitable for parenteral administration, such as sterile solutions, suspensions or lyophilized products in suitable unit dosage forms.

In all of the administration methods of the compound of general formula I described herein, the daily dose administered is from 0.01 mg/kg to 200 mg/kg of body weight, given in individual or separated doses.

The compounds disclosed herein can be prepared by a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples disclosed herein.

The chemical reactions of the specific embodiments disclosed herein are conducted in a suitable solvent that must be suitable for the chemical changes in the present invention and the reagents and materials required. In order to obtain the compounds disclosed herein, it is sometimes necessary for those skilled in the art to modify or select a synthesis procedure or a reaction process based on the existing embodiments.

## BRIEF DESCRIPTION OF THE DRAWING

FIG. 1 shows the experiment results of the compound of the present invention in inhibiting the growth of the DLD-1 subcutaneous tumor model.

## DETAILED DESCRIPTION

The following examples illustrate the technical schemes of the present invention in detail, but the protection scope of the present invention includes but is not limited thereto.

The structures of the compounds are determined by nuclear magnetic resonance (NMR) and/or mass spectrometry (MS). NMR shifts are given in $10^{-6}$ (ppm). Solvents for NMR determination are deuterated dimethyl sulfoxide, deuterated chloroform, deuterated methanol or the like, and internal standard is tetramethylsilane (TMS); "IC50" refers to the half maximal inhibitory concentration, which is the concentration at which half of the maximal inhibitory effect is achieved.

Abbreviation:

1,2-EDB: 1,2-dibromoethane; $(BPin)_2$: bis(pinacolato)diboron; $Pd(dppf)Cl_2$: [1,1'-bis(diphenylphosphino)ferrocene]palladium dichloride; KOAc: potassium acetate; dioxane: dioxane; $Na_2CO_3$: sodium carbonate; THF: tetrahydrofuran; $SmI_2$: samarium diiodide; STAB: sodium triacetoxyborohydride; DCM: dichloromethane; DCE: dichloroethane; DMF: *N,N*-dimethylformamide; Togni: 1-(trifluoromethyl)-1,2-benziodoxol-3(1*H*)-one; DBU: 1,8-diazabicyclo[5.4.0]-7-undecene; PyAOP: (3*H*-1,2,3-triazolo[4,5-*b*]pyridin-3-yloxy)tri-1-pyrrolidinylhexafluorophosphate; DAST: diethylaminosulfur trifluoride, DMSO: dimethyl sulfoxide; HATU: 2-(7-azabenzotriazol-1-yl)-*N,N,N',N'*-tetramethyluronium hexafluorophosphate; DIEA: *N*,*N*-diisopropylethylamine; iPrOH: isopropanol; NBS: *N*-bromosuccinimide; ACN or MeCN: acetonitrile; NaOtBu: sodium *tert*-butoxide; $Pd(PPh_3)_2Cl_2$: bis(triphenylphosphine)palladium dichloride; TEA: triethylamine; toluene: toluene; $Ac_2O$: acetic anhydride; Lawesson's Reagent or Lawesson reagent: 2,4-bis(4-methoxyphenyl)-1,3-dithio-2,4-diphosphetane-2,4-disulfide; NCS: *N*-chlorosuccinimide; TBHP: *tert*-butyl hydroperoxide; $NaBD_4$: sodium borodeuteride; $NHBn_2$: dibenzylamine; $NaBH(OAc)_3$: sodium triacetoxyborohydride.

### Example 1: (*R*)-4-((1-(3-(difluoromethyl)-2-fluorophenyl)ethyl)amino)-6-(1-(fluoromethyl) cyclopropyl)-8-methoxy-2-methylpyrido[4,3-*d*]pyrimidine-7(6*H*)-one

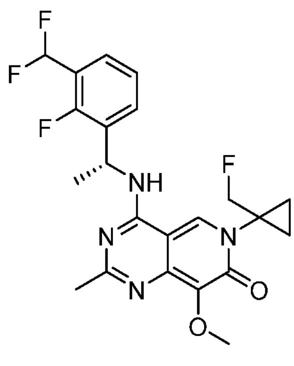

**Step 1: synthesis of (*R*)-8-bromo-4-((1-(3-(difluoromethyl)-2-fluorophenyl)ethyl)amino)-6-(1-(fluoromethyl)cyclopropyl)-2-methylpyrido[4,3-*d*]pyrimidine-7(6*H*)-one**

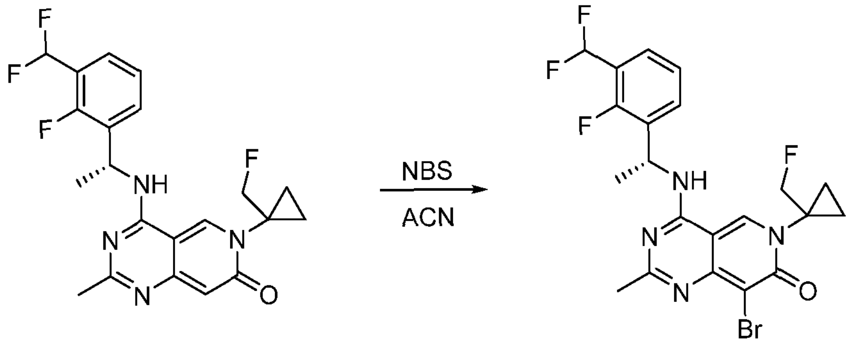


(*R*)-4-((1-(3-(Difluoromethyl)-2-fluorophenyl)ethyl)amino)-6-(1-(fluoromethyl)cyclopropyl)-2-methylpyrido[4,3-*d*]pyrimidine-7(6*H*)-one (700 mg) and *N*-bromosuccinimide (NBS) (311 mg) were placed in acetonitrile (5 mL), and the system was purged with nitrogen. The reaction was completed after the mixture was stirred for reaction at room temperature for 1 h. The reaction mixture was concentrated and purified by column chromatography (dichloromethane/methanol gradient elution) to give the title compound.

**Step 2: synthesis of (*R*)-4-((1-(3-(difluoromethyl)-2-fluorophenyl)ethyl)amino)-6-(1-(fluoromethyl) cyclopropyl)-8-methoxy-2-methylpyrido[4,3-*d*]pyrimidine-7(6*H*)-one**

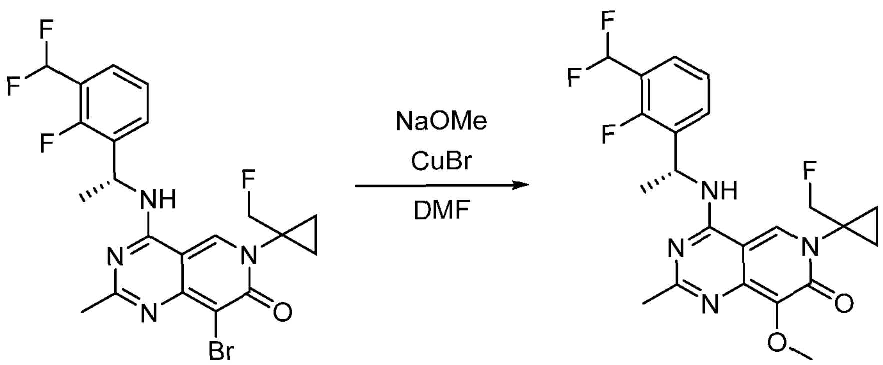


(*R*)-8-Bromo-4-((1-(3-(difluoromethyl)-2-fluorophenyl)ethyl)amino)-6-(1-(fluoromethyl)cyclopropyl)-2-methylpyrido[4,3-*d*]pyrimidine-7(6*H*)-one (50.0 mg) was dissolved in 0.5 mL of *N*,*N*-dimethylformamide. 0.5 mL of a 5.4 M solution of sodium methoxide in methanol was added, and then cuprous bromide (3.00 mg) was added. The reaction was completed after the mixture was heated to 60 °C and stirred for 4 h. The reaction mixture was cooled to room

temperature, poured into a saturated ammonium chloride solution, extracted with ethyl acetate, dried over anhydrous sodium sulfate, filtered, concentrated, and purified by preparative HPLC (mobile phase: acetonitrile/water) to give the title compound (20.0 mg).

$^1$H NMR (400 MHz, DMSO-*d*$_6$) δ 8.96 (s, 1H), 8.82 (d, *J* = 7.0 Hz, 1H), 7.64 (t, *J* = 7.4 Hz, 1H), 7.52 (t, *J* = 7.0 Hz, 1H), 7.32 (t, *J* = 7.7 Hz, 1H), 7.24 (t, *J* = 54.4 Hz, 1H), 5.82-5.70 (m, 1H), 4.69-4.59 (m, 2H), 3.77 (s, 3H), 2.21 (s, 3H), 1.58 (d, *J* = 7.1 Hz, 3H), 1.33-1.23 (m, 4H). LC/MS(m/z, MH$^+$): 451.2.

**Example 2: (*R*)-4-((1-(3-(difluoromethyl)-2-fluorophenyl)ethyl)amino)-6-(1-(fluoromethyl) cyclopropyl)-2-methyl-8-(trifluoromethyl)pyrido[4,3-*d*]pyrimidine-7(6*H*)-one**

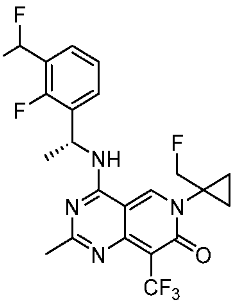

**Step 1: synthesis of (*R*)-4-((1-(3-(difluoromethyl)-2-fluorophenyl)ethyl)amino)-6-(1-(fluoromethyl)cyclopropyl)-2-methyl-8-(trifluoromethyl)pyrido[4,3-*d*]pyrimidine-7(6*H*)-one**

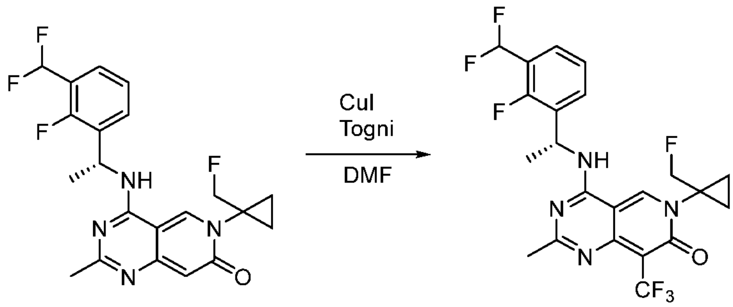


(*R*)-4-((1-(3-(difluoromethyl)-2-fluorophenyl)ethyl)amino)-6-(1-(fluoromethyl)cyclopropyl)-2-methylpyrido[4,3-*d*]pyrimidine-7(6*H*)-one (100 mg), cuprous iodide (4.53 mg), and 1-(trifluoromethyl)-1,2-benziodoxol-3(1*H*)-one (0.0902 mg) were dissolved in 1 mL of *N,N*-dimethylformamide, and the mixture was heated to 80 °C and stirred overnight. After the reaction was completed, the reaction mixture was cooled to room temperature, poured into a saturated ammonium chloride solution, extracted with ethyl acetate, dried over anhydrous sodium sulfate, filtered, concentrated, and purified by preparative HPLC (mobile phase: acetonitrile/water) to give the title compound.

$^1$H NMR (400 MHz, DMSO-*d*$_6$) δ 9.44 (s, 1H), 9.10 (d, *J* = 7.0 Hz, 1H), 7.66 (t, *J* = 7.1 Hz, 1H), 7.53 (t, *J* = 6.8 Hz, 1H), 7.33 (t, *J* = 7.7 Hz, 1H), 7.23 (t, *J* = 54.4 Hz, 1H), 5.85-5.68 (m, 1H), 4.80-4.48 (m, 2H), 2.24 (s, 3H), 1.60 (d, *J* = 7.1 Hz, 3H), 1.34-1.24 (m, 4H). LC/MS(m/z, MH$^+$): 489.2.

**Example 3: (*R*)-3-(1-((6-(1-(fluoromethyl)cyclopropyl)-8-methoxy-2-methyl-7-oxo-6,7-dihydropyrido[4,3-*d*]pyrimidin-4-yl)amino)ethyl)-2-methylbenzonitrile**

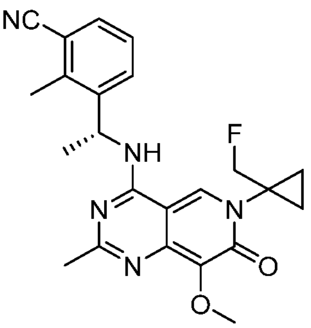

**Step 1: (*R*)-3-(1-((6-(1-(fluoromethyl)cyclopropyl)-2-methyl-7-oxo-6,7-dihydropyrido[4,3-*d*]pyrimidin-4-yl)amino)ethyl)-2-methylbenzonitrile**

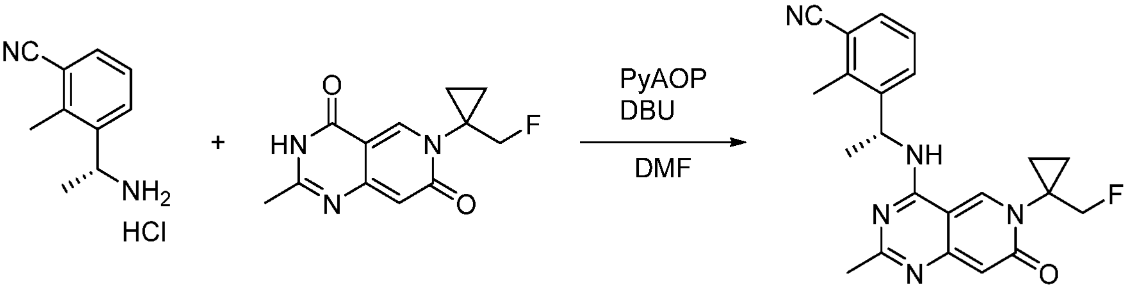


6-(1-(Fluoromethyl)cyclopropyl)-2-methylpyrido[4,3-*d*]pyrimidine-4,7(3*H*,6*H*)-dione was prepared in the same manner as in the preparation method of intermediate E-7a on page 128 in patent CN111372932A except for replacing C-1a in the preparation process of E-4a with 1-(fluoromethyl)cyclopropylamine hydrochloride.

6-(1-(Fluoromethyl)cyclopropyl)-2-methylpyrido[4,3-*d*]pyrimidine-4,7(3*H*,6*H*)-dione (250 mg) and (*R*)-3-(1-aminoethyl)-2-tolunitrile hydrochloride (217 mg) was dissolved in 5 mL of *N*,*N*-dimethylformamide. 1,8-Diazabicyclo[5.4.0]-7-undecene (449 μL) was added, and the mixture was stirred in an ice bath for 30 min. Then (3*H*-1,2,3-triazolo[4,5-*b*]pyridin-3-yloxy)tri-1-pyrrolidinylhexafluorophosphate (627 mg) was added, and the mixture was reacted at room temperature overnight. After the reaction was completed, the mixture was diluted with ethyl acetate, washed with water, and washed with saturated brine. The organic phase was dried over anhydrous sodium sulfate, filtered, concentrated, and purified by column chromatography (petroleum ether/ethyl acetate gradient elution) to give the title compound.

**Step 2: (*R*)-3-(1-((8-bromo-6-(1-(fluoromethyl)cyclopropyl)-2-methyl-7-oxo-6,7-dihydropyrido [4,3-*d*]pyrimidin-4-yl)amino)ethyl)-2-methylbenzonitrile**

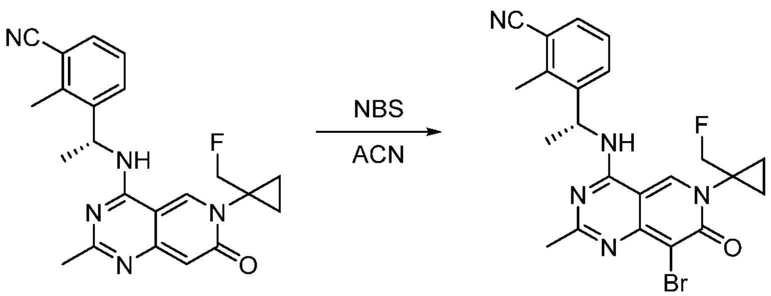


(*R*)-3-(1-((6-(1-(Fluoromethyl)cyclopropyl)-2-methyl-7-oxo-6,7-dihydropyrido[4,3-*d*]pyrimidin-4-yl)amino)ethyl)-2-methylbenzonitrile (200 mg) was dissolved in 3 mL of acetonitrile, and *N*-bromosuccinimide (90.9 mg) was dissolved in 1 mL of acetonitrile and added dropwise to the reaction mixture. The reaction was completed after the

mixture was stirred at room temperature for 1 h. The reaction mixture was concentrated and purified by column chromatography (petroleum ether/ethyl acetate gradient elution) to give the title compound.

**Step 3: (*R*)-3-(1-((6-(1-(fluoromethyl)cyclopropyl)-8-methoxy-2-methyl-7-oxo-6,7-dihydropyrido[4,3-*d*]pyrimidin-4-yl)amino)ethyl)-2-methylbenzonitrile**

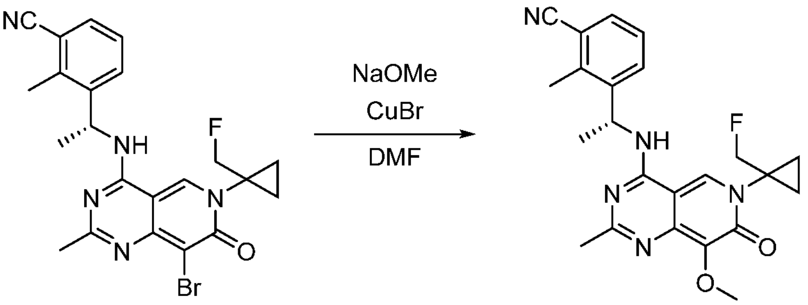


(*R*)-3-(1-((8-Bromo-6-(1-(fluoromethyl)cyclopropyl)-2-methyl-7-oxo-6,7-dihydropyrido[4,3-*d*]pyrimidin-4-yl)amino)ethyl)-2-methylbenzonitrile (103 mg) was dissolved in 1.5 mL of *N,N*-dimethylformamide. 0.8 mL of a 5.4 M solution of sodium methoxide in methanol was added, and then cuprous bromide (6.28 mg) was added. The mixture was heated to 70 °C and stirred overnight. After the reaction was completed, the reaction mixture was cooled to room temperature, poured into a saturated ammonium chloride solution, extracted with ethyl acetate, dried over anhydrous sodium sulfate, filtered, concentrated, and purified by preparative HPLC (acetonitrile/water) to give the title compound.

$^1$H NMR (400 MHz, DMSO-$d_6$) δ 8.92 (s, 1H), 8.87 (d, *J* = 6.8 Hz, 1H), 7.72 (d, *J* = 7.9 Hz, 1H), 7.66 (d, *J* = 7.7 Hz, 1H), 7.40 (t, *J* = 7.8 Hz, 1H), 5.65-5.55 (m, 1H), 4.74-4.53 (m, 2H), 3.76 (s, 3H), 2.67 (s, 3H), 2.21 (s, 3H), 1.52 (d, *J* = 7.0 Hz, 3H), 1.33-1.23 (m, 4H).

LC/MS(m/z, $MH^+$): 422.2.

**Example 4: (*R*)-4-((1-(3-(difluoromethyl)-2-fluorophenyl)ethyl)amino)-6-(1-(fluoromethyl) cyclopropyl)-2-methyl-8-(2,2,2-trifluoroethoxy)pyrido[4,3-*d*]pyrimidine-7(6*H*)-one**

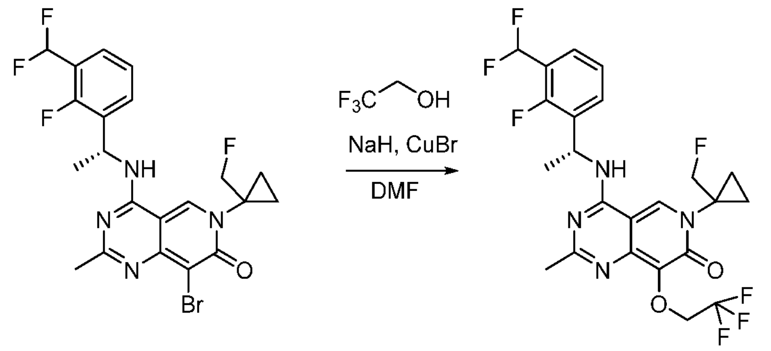


2,2,2-Trifluoroethanol (1 mL) and DMF (1 mL) were placed in a reaction flask, and the system was purged with nitrogen. NaH (48.07 mg) was added in an ice bath, and after the mixture was stirred for half an hour, 8-bromo-4-[[(1*R*)-1-[3-(difluoromethyl)-2-fluoro-phenyl]ethyl]amino]-6-[1-(fluoromethyl)cyclopropyl]-2-methyl-pyrido[4,3-*d*]pyrimidine-7-one (50 mg) and cuprous bromide (4.31 mg) were added in an ice bath. The mixture was heated to 70 °C and stirred overnight. After the reaction was completed, the reaction mixture was quenched by pouring it into 10 mL of water and extracted with EA. The organic phases were combined, concentrated, and purified by preparative HPLC (mobile phase: acetonitrile/water) to give the title compound.

$^1$H NMR (400 MHz, $CD_3OD$) δ9.06 (s, 1H), 7.63-7.58 (m, 1H), 7.55-7.49 (m, 1H), 7.28 (t, *J* = 7.7 Hz, 1H), 7.02 (t, *J* = 54.9 Hz, 1H), 5.88-5.80 (m, 1H), 4.70-4.63 (m, 2H), 4.59 (s, 1H), 2.34 (s, 3H), 1.66 (d, *J* = 7.1 Hz, 1H), 1.32-1.28 (m, 4H). LC/MS(m/z, $MH^+$): 518.9.

**Example 5: (*R*)-4-((1-(3-(difluoromethyl)-2-fluorophenyl)ethyl)amino)-8-(2,2-difluoropropoxy)-6-(1-(fluoromethyl)cyclopropyl)-2-methylpyrido[4,3-*d*]pyrimidine-7(6*H*)-one**

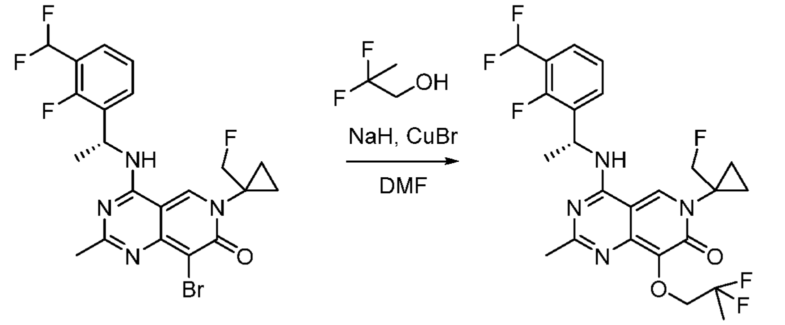


2,2-Difluoropropanol (1 mL) and *N,N*-dimethylformamide (1 mL) were placed in a reaction flask, and the system was purged with nitrogen. Sodium hydride (48.07 mg) was added in an ice bath, and after the mixture was stirred for half an hour, 8-bromo-4-[[(1*R*)-1-[3-(difluoromethyl)-2-fluorophenyl]ethyl]amino]-6-[1-(fluoromethyl)cyclopropyl]-2-methyl-pyrido[4,3-*d*]pyrimidine-7-one (50 mg) and cuprous bromide (4.31 mg) were added in an ice bath. The mixture was heated to 70 °C and stirred overnight. After the reaction was completed, the reaction mixture was quenched by pouring it into 10 mL of water and extracted with ethyl acetate. The organic phases were combined, concentrated, and purified by preparative HPLC (mobile phase: acetonitrile/water) to give the title compound.

$^1$H NMR (400 MHz, DMSO-$d_6$) δ 9.26 (s, 1H), 9.11 (d, *J* = 7.1 Hz, 1H), 7.67 (t, *J* = 7.5 Hz, 1H), 7.53 (t, *J* = 7.8 Hz, 1H), 7.33 (t, *J* = 7.7 Hz, 1H), 7.24 (t, *J* = 54.3 Hz, 1H), 5.79-5.76 (m, 1H), 5.24-5.19 (m, 1H), 4.82-4.40 (m, 2H), 2.23 (s, 3H), 1.67-1.45 (m, 6H), 1.34-1.23 (m, 4H). LC/MS(m/z, $MH^+$): 515.2.

**Example 6: (*R*)-3,3-difluoro-3-(3-(1-((6-(1-(fluoromethyl)cyclopropyl)-2-methyl-7-oxo-6,7-dihydropyrido[4,3-*d*]pyrimidin-4-yl)amino)ethyl)phenyl)-2,2-dimethylpropanenitrile**

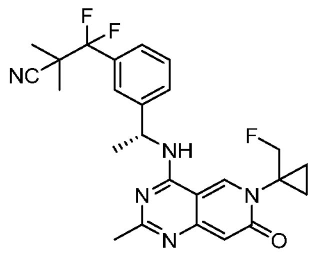

**Step 1: synthesis of 3-(3-bromophenyl)-2,2-dimethyl-3-oxopropanenitrile**

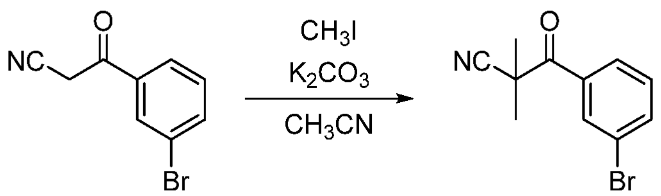


3-(3-Bromophenyl)-3-oxopropanenitrile (2.00 g) was dissolved in 20 mL of acetonitrile. Iodomethane (3.17 g) and potassium carbonate (3.70 g) were added, and after the mixture was heated to 50 °C and reacted for 2 h, the reaction mixture was diluted with ethyl acetate and filtered. The filtrate was concentrated and separated by column chromatography (petroleum ether/ethyl acetate gradient elution) to give the title compound.

**Step 2: synthesis of 3-(3-bromophenyl)-3,3-difluoro-2,2-dimethylpropanenitrile**

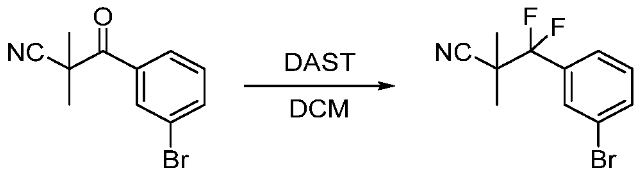


3-(3-Bromophenyl)-2,2-dimethyl-3-oxopropanenitrile (2.30 g) was dissolved in 20 mL of dichloromethane. The mixture was cooled to 0 °C, and diethylaminosulfur trifluoride (14.7 g) was added dropwise thereto. After the dropwise addition, the mixture was reacted at room temperature for another 16 h. The reaction mixture was slowly added dropwise into an ice saturated sodium bicarbonate solution, followed by liquid separation. After the aqueous phase was extracted with ethyl acetate, the organic phases were combined, dried over anhydrous sodium sulfate, filtered, concentrated, and separated by column chromatography (petroleum ether/ethyl acetate gradient elution) to give the title compound.

**Step 3: synthesis of 3-(3-acetylphenyl)-3,3-difluoro-2,2-dimethylpropanenitrile**

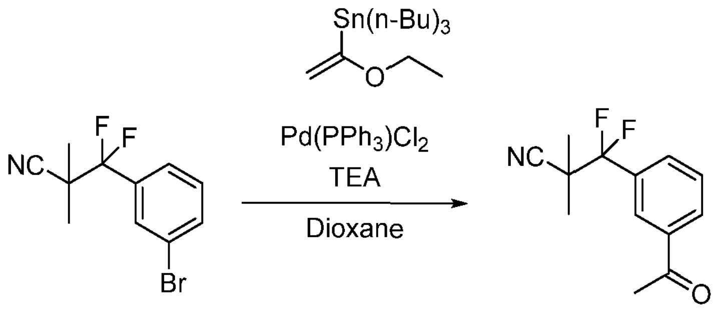


3-(3-Bromophenyl)-3,3-difluoro-2,2-dimethylpropanenitrile (2.00 g) was dissolved in 10 mL of extradry 1,4-dioxane. Then tributyl(1-ethoxyethylene)tin (3.95 g) and bis(triphenylphosphine)palladium dichloride (512 mg) were added. Finally, triethylamine (1.48 g) was added. After the mixture was heated 100 °C and reacted for 6 h under argon atmosphere, the reaction mixture was cooled to room temperature, 3 M diluted hydrochloric acid was added to adjust the pH to 1, and the mixture was reacted for another 3 h. The reaction mixture was diluted with ethyl acetate, followed by liquid separation. The aqueous phase was extracted with ethyl acetate, and the organic phases were combined, dried over anhydrous sodium sulfate, filtered, concentrated, and separated by column chromatography (petroleum ether/ethyl acetate gradient elution) to give the title compound.

**Step 4: synthesis of (*R*,*Z*)-*N*-(1-(3-(2-cyano-1,1-difluoro-2-methylpropyl)phenyl)ethylidene)-2-methylpropane-2-sulfinamide**

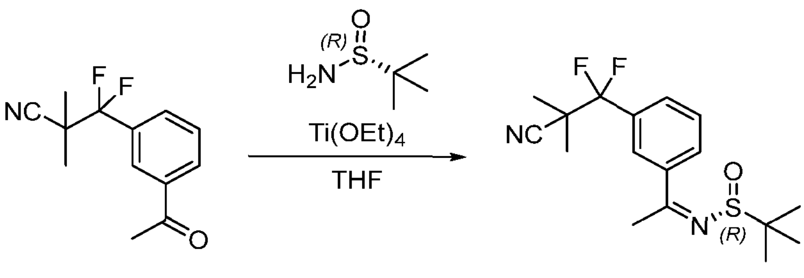


3-(3-Acetylphenyl)-3,3-difluoro-2,2-dimethylpropanenitrile (1.15 g) was dissolved in 10 mL of tetrahydrofuran.

(A)-2-Methylpropane-2-sulfinamide (1.17 g) and tetraethyl titanate (2.21 g) were added, and the mixture was heated to 60 °C and reacted overnight. The reaction mixture was cooled to room temperature, poured into ice water, stirred for 30 min, diluted with dichloromethane, and filtered. After the filter cake was rinsed with a small amount of dichloromethane, the filtrate was separated. After the aqueous phase was extracted with dichloromethane, the organic phases were combined, dried over anhydrous sodium sulfate, filtered, concentrated, and separated by column chromatography (petroleum ether/ethyl acetate gradient elution) to give the title compound.

**Step 5: synthesis of (*R*)-*N*-((*R*)-1-(3-(2-cyano-1,1-difluoro-2-methylpropyl)phenyl)ethyl)-2-methylpropane-2-sulfinamide**

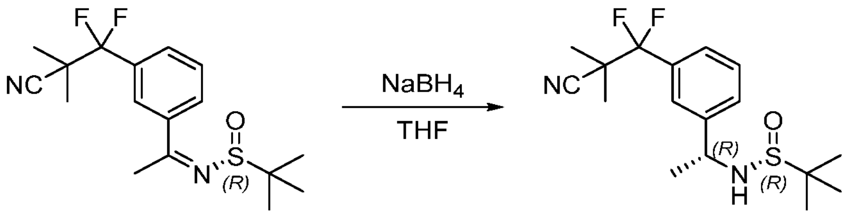


(*R*,*Z*)-*N*-(1-(3-(2-Cyano-1,1-difluoro-2-methylpropyl)phenyl)ethylidene)-2-methylpropane-2-sulfinamide (700 mg) was dissolved in 7 mL of tetrahydrofuran. After the mixture was cooled to 0 °C, sodium borohydride (93.3 mg) was slowly added thereto in batches, and then the mixture was brought to room temperature and reacted for 4 h. The reaction was quenched with a saturated aqueous ammonium chloride solution, and the mixture was separated. After the aqueous phase was extracted with ethyl acetate, the organic phases were combined, dried, concentrated, and separated by column chromatography (petroleum ether/ethyl acetate gradient elution) to give the title compound.

**Step 6: synthesis of (*R*)-3-(3-(1-aminoethyl)phenyl)-3,3-difluoro-2,2-dimethylpropanenitrile hydrochloride**

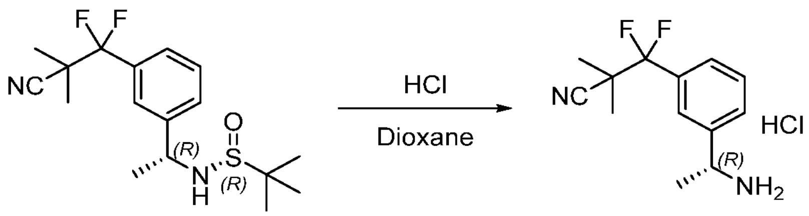


(*R*)-*N*-((*R*)-1-(3-(2-Cyano-1,1-difluoro-2-methylpropyl)phenyl)ethyl)-2-methylpropane-2-sulfinamide (480 mg) was dissolved in 8 mL of 1,4-dioxane. After the mixture was cooled to 10 °C or less, a solution of hydrochloric acid in dioxane (4 M, 1.75 mL) was added dropwise thereto. After the dropwise addition, the mixture was brought to room temperature and reacted for another 30 min, and a large number of solids precipitated. The mixture was filtered, and the filter cake was rinsed with a small amount of methyl *tert*-butyl ether and filtered. The filter cake was dried to give the title compound.

**Step 7: synthesis of (*R*)-3,3-difluoro-3-(3-(1-((6-(1-(fluoromethyl)cyclopropyl)-2-methyl-7-oxo-6,7-dihydropyrido[4,3-*d*]pyrimidin-4-yl)amino)ethyl)phenyl)-2,2-dimethylpropanenitrile**

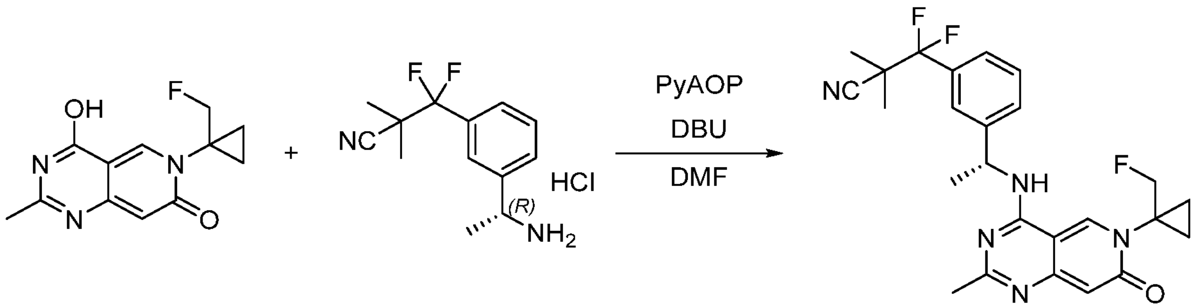


6-(1-(Fluoromethyl)cyclopropyl)-2-methylpyrido[4,3-*d*]pyrimidine-4,7(3*H*,6*H*)-dione (50.0 mg) was dissolved in 2 mL of *N,N*-dimethylformamide. (*R*)-3-(3-(1-aminoethyl)phenyl)-3,3-difluoro-2,2-dimethylpropanenitrile hydrochloride (82.6 mg) and 1,8-diazabicyclo[5.4.0]-7-undecene (91.6 mg) were added. Finally, (7-azabenzotriazole-1-oxy)tripyrrolephosphonium hexafluorophosphate (156 mg) was added, and the mixture was reacted at room temperature overnight. The reaction mixture was diluted with ethyl acetate and washed with saturated brine, and the organic phase was dried over anhydrous sodium sulfate, filtered, concentrated, and separated and purified by preparative HPLC (mobile phase: acetonitrile/water) to give the title compound.

$^1$H NMR (400 MHz, DMSO-$d_6$) δ 9.12 (s, 1H), 8.85 (d, *J* = 7.5 Hz, 1H), 7.63 (d, *J* = 7.8 Hz, 1H), 7.59 (s, 1H), 7.53 (t, *J* = 7.7 Hz, 1H), 7.41 (d, *J* = 7.8 Hz, 1H), 6.07 (s, 1H), 5.69-5.52 (m, 1H), 4.80-4.50 (m, 2H), 2.20 (s, 3H), 1.59 (d, *J* = 7.1 Hz, 3H), 1.44-1.18 (m, 10H). LC/MS(m/z, MH$^+$): 470.2.

**Example 7: 6-(1-acetyl-3-methylpyrrolidin-3-yl)-4-(((*R*)-1-(3-(difluoromethyl)-2-fluorophenyl) ethyl)amino)-8-methoxy-2-methylpyrido[4,3-*d*]pyrimidine-7(6*H*)-one**

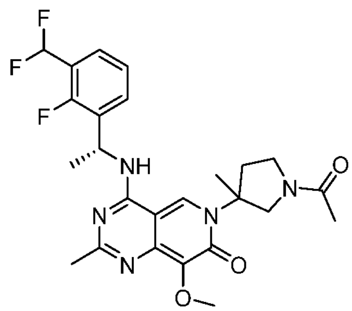

**Step 1: synthesis of *tert*-butyl 3-(2-(6-(((*R*)-1-(3-(difluoromethyl)-2-fluorophenyl)ethyl)amino)-5-(1,3-dioxolan-2-yl)-2-methylpyrimidin-4-yl)acetamido)-3-methylpyrrolidine-1-carboxylate**

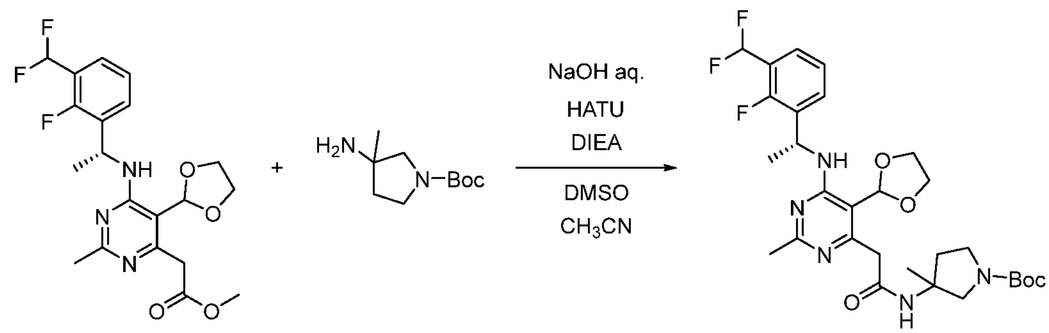


Methyl (*R*)-2-(6-((1-(3-(difluoromethyl)-2-fluorophenyl)ethyl)amino)-5-(1,3-dioxolan-2-yl)-2-methylpyrimidine-4-acetate (190 mg) was dissolved in a mixed solvent of 2 mL of dimethyl sulfoxide and 1 mL of acetonitrile. 0.36 mL of a 20% aqueous sodium hydroxide solution was added, and after the mixture was reacted at room temperature for 2 h,

*N,N*-diisopropylethylamine (115.45 mg), *tert*-butyl 3-amino-3-methylpyrrolidine-1-carboxylate (116.29 mg), and 2-(7-azabenzotriazol-1-yl)-*N,N,N',N'*-tetramethyluronium hexafluorophosphate (505 mg) were added thereto. After the addition was completed, the mixture was reacted at room temperature overnight. The reaction mixture was diluted with ethyl acetate and washed with saturated brine, and the organic phase was dried over anhydrous sodium sulfate, filtered, concentrated, and separated by column chromatography (dichloromethane/methanol gradient elution) to give the title compound.

**Step 2: synthesis of 4-(((*R*)-1-(3-(difluoromethyl)-2-fluorophenyl)ethyl)amino)-2-methyl-6-(3-methylpyrrolidin-3-yl)pyrido[4,3-*d*]pyrimidine-7(6*H*)-one**

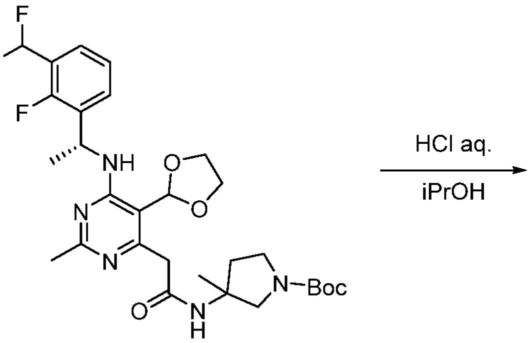


*tert*-Butyl 3-(2-(6-(((*R*)-1-(3-(difluoromethyl)-2-fluorophenyl)ethyl)amino)-5-(1,3-dioxolan-2-yl)-2-methylpyrimidin-4-yl)acetamido)-3-methylpyrrolidine-1-carboxylate (200 mg) was dissolved in 2 mL of isopropanol. 0.34 mL of 5 M diluted hydrochloric acid was added thereto, and after the mixture was heated to 80 °C and reacted overnight, the reaction mixture was concentrated to give the title compound (crude product), which was directly used in the next step.

**Step 3: synthesis of 6-(1-acetyl-3-methylpyrrolidin-3-yl)-4-(((*R*)-1-(3-(difluoromethyl)-2-fluorophenyl)ethyl)amino)-2-methylpyrido[4,3-*d*]pyrimidine-7(6*H*)-one**

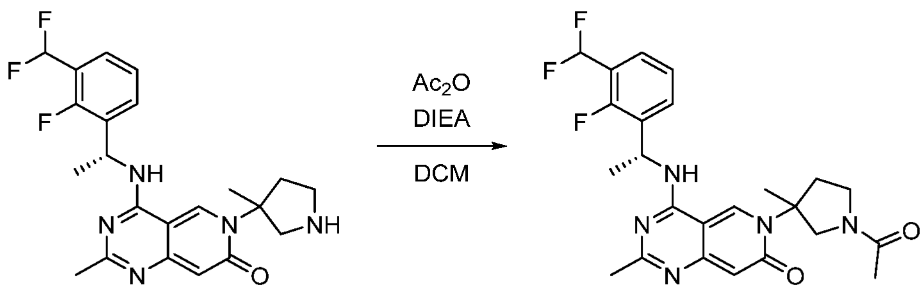


4-(((*R*)-1-(3-(Difluoromethyl)-2-fluorophenyl)ethyl)amino)-2-methyl-6-(3-methylpyrrolidin-3 - yl)pyrido[4,3-*d*]pyrimidine-7(6*H*)-one (172 mg) was dissolved in 5 mL of dichloromethane. *N,N*-Diisopropylethylamine (155 mg) and acetic anhydride (49 mg) were added, and then after the mixture was reacted at room temperature for another 6 h, the reaction mixture was concentrated and purified by column chromatography (petroleum ether/ethyl acetate gradient elution) to give the title compound.

**Step 4: synthesis of 6-(1-acetyl-3-methylpyrrolidin-3-yl)-8-bromo-4-(((*R*)-1-(3-(difluoromethyl)-2-fluorophenyl)ethyl)amino)-2-methylpyrido[4,3-*d*]pyrimidine-7(6*H*)-one**

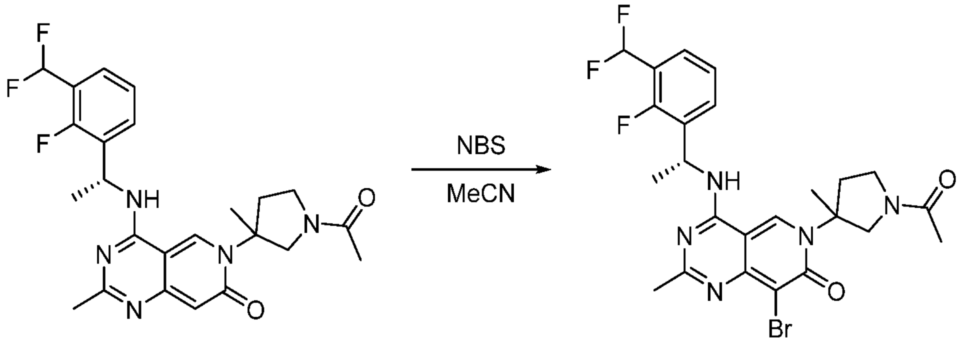


6-(1-Acetyl-3-methylpyrrolidin-3-yl)-4-(((*R*)-1-(3-(difluoromethyl)-2-fluorophenyl)ethyl)amino)-2-methylpyrido[4,3-*d*]pyrimidine-7(6*H*)-one (100 mg) was dissolved in 3 mL of acetonitrile, and *N*-bromosuccinimide (50.9 mg) was dissolved in 1 mL of acetonitrile and added dropwise to the reaction mixture. The reaction was completed after the mixture was stirred at 0 °C for 1 h. The reaction mixture was concentrated and purified by column chromatography (petroleum ether/ethyl acetate gradient elution) to give the title compound.

**Step 5: synthesis of 6-(1-acetyl-3-methylpyrrolidin-3-yl)-4-(((*R*)-1-(3-(difluoromethyl)-2-fluorophenyl)ethyl)amino)-8-methoxy-2-methylpyrido[4,3-*d*]pyrimidine-7(6*H*)-one**

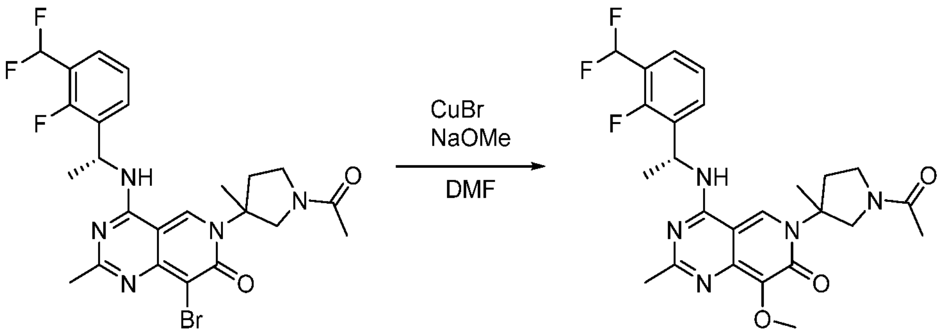


6-(1-Acetyl-3-methylpyrrolidin-3-yl)-8-bromo-4-(((*R*)-1-(3-(difluoromethyl)-2-fluorophenyl)ethyl)amino)-2-methylpyrido[4,3-*d*]pyrimidine-7(6*H*)-one (103 mg) was dissolved in 1.5 mL of *N*,*N*-dimethylformamide. 0.8 mL of a 5.4 M solution of sodium methoxide in methanol was added, and then cuprous bromide (6.28 mg) was added. The mixture was heated to 70 °C and stirred overnight. After the reaction was completed, the reaction mixture was cooled to room temperature, poured into a saturated ammonium chloride solution, extracted with ethyl acetate, dried over anhydrous sodium sulfate, filtered, concentrated, and purified by preparative HPLC (acetonitrile/water) to give the title compound.

$^1$H NMR (400 MHz, DMSO-$d_6$) $\delta$ 8.95-8.85 (m, 1H), 8.65-8.62 (m, 1H), 7.70-7.64 (m, 1H), 7.55-7.45 (m, 1H), 7.31 (t, *J* = 7.7 Hz, 1H), 7.24 (t, *J* = 54.3 Hz,1H), 5.84-5.72 (m, 1H), 4.55-4.45 (m, 1H), 3.76-3.75 (m, 3H), 3.73-3.55 (m, 2H), 3.45-3.30 (m, 1H), 2.85-2.70 (m, 1H), 2.70-2.50 (m, 1H), 2.21 (s, 3H), 1.98-1.96 (m, 3H), 1.59-1.56 (m, 6H). LC/MS(m/z, $MH^+$): 504.2.

**Example 8: 6-(1-acetyl-3-(fluoromethyl)pyrrolidin-3-yl)-4-(((*R*)-1-(3-(difluoromethyl)-2-fluorophenyl)ethyl)amino)-8-methoxy-2-methylpyrido[4,3-*d*]pyrimidine-7(6*H*)-one**

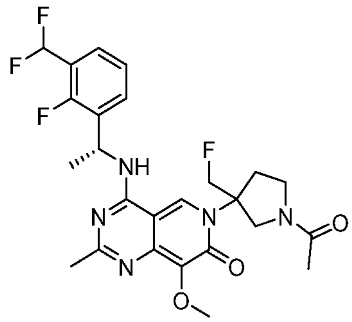

The title compound was prepared in the same manner as in Example 7 except for replacing *tert*-butyl 3-amino-3-methylpyrrolidine-1-carboxylate in step **1** with *tert*-butyl 3-amino-3-(fluoromethyl)pyrrolidine-1-carboxylate.

$^1$H NMR (400 MHz, DMSO-$d_6$) δ 9.00-8.93 (m, 1H), 8.60-8.55 (m, 1H), 7.66-7.61 (m, 1H), 7.54-7.50 (m, 1H), 7.35-7.29 (m, 1H), 7.23 (t, *J* = 54.4 Hz, 1H), 5.84-5.75 (m, 1H), 5.05-4.60 (m, 2H), 4.53-4.42 (m, 1H), 3.84-3.47 (m, 6H), 3.05-2.50 (m, 2H), 2.22 (s, 3H), 2.02-1.94 (m, 3H), 1.60 (d, *J* = 7.0 Hz, 3H). LC/MS(m/z, MH$^+$): 522.2.

**Example 9: (*R*)-8-(2,2-difluoroethoxy)-4-((1-(3-(difluoromethyl)-2-fluorophenyl)ethyl)amino)-2-methyl-6-(1-methylcyclopropyl)pyridinyl[4,3-*d*]pyrimidine-7(6*H*)-one**

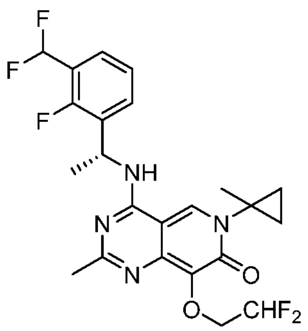

**Step 1: 2-bromo-2-(6-((*R*)-1-(3-(difluoromethyl)-2-fluorophenyl)ethyl)amino)-5-(1,3-dioxolan-2-yl)-2-methylpyrimidin-4-yl)-*N*-(1-methylcyclopropyl)acetamide**

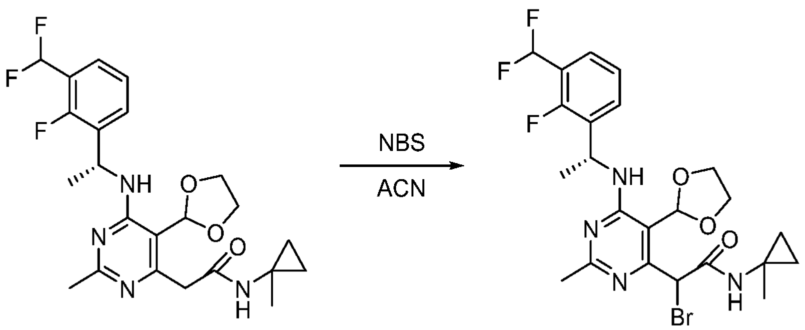


(*R*)-2-(6-((1-(3-(Difluoromethyl)-2-fluorophenyl)ethyl)amino)-5-(1,3-dioxolan-2-yl)-2-methylpyrimidin-4-yl)-*N*-(1-methylcyclopropyl)acetamide (180 mg) was dissolved in 4 mL of acetonitrile. *N*-Bromosuccinimide (68.9 mg) was added, and the mixture was stirred at room temperature for 30 min. After the reaction was completed, dichloromethane was added, and the mixture was washed with water and washed with saturated brine. The organic phase was dried over anhydrous sodium sulfate, filtered, concentrated, and purified by column chromatography (petroleum ether/ethyl acetate gradient elution) to give the title compound.

**Step 2: 2-(2,2-difluoroethoxy)-2-(6-((*R*)-1-(3-(difluoromethyl)-2-fluorophenyl)ethyl)amino)-5-(1,3-dioxolan-2-yl)-2-methylpyrimidin-4-yl)-*N*-(1-methylcyclopropyl)acetamide**

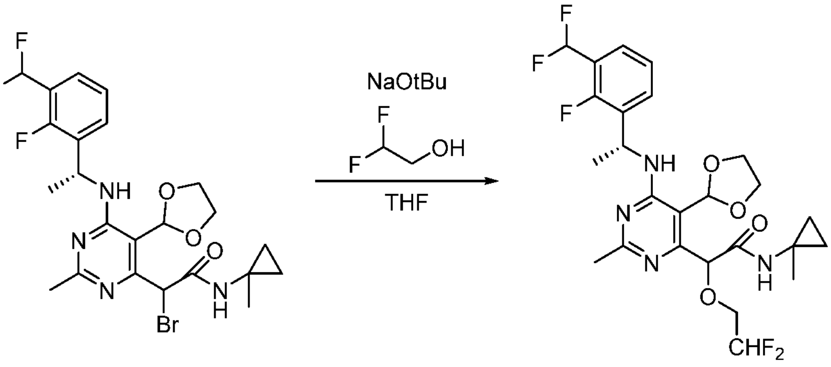


2-Bromo-2-(6-((*R*)-1-(3-(difluoromethyl)-2-fluorophenyl)ethyl)amino)-5-(1,3-dioxolan-2-yl)-2-methylpyrimidin-4-yl)-*N*-(1-methylcyclopropyl)acetamide (60.0 mg) and 2,2-difluoroethanol (90.6 mg) were dissolved in 1 mL of tetrahydrofuran. Sodium *tert*-butoxide (31.8 mg) was added to the reaction mixture. The reaction was completed after the mixture was stirred at room temperature for 1 h. The reaction mixture was poured into a saturated ammonium chloride solution, extracted with dichloromethane, dried, concentrated, and purified by column chromatography (petroleum ether/ethyl acetate gradient elution) to give the target compound.

**Step 3: (R*)*-8-(2,2-difluoroethoxy)-4-((1-(3-(difluoromethyl)-2-fluorophenyl)ethyl)amino)-2-methyl-6-(1-methylcyclopropyl)pyrido[4,3-*d*]pyrimidine-7(6*H*)-one**

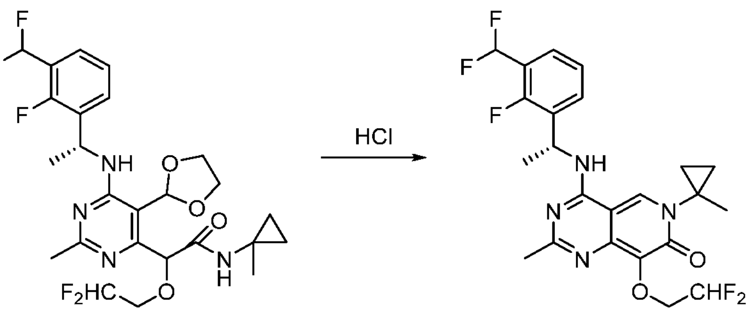


2-(2,2-Difluoroethoxy)-2-(6-((*R*)-1-(3-(difluoromethyl)-2-fluorophenyl)ethyl)amino)-5-(1,3-dioxolan-2-yl)-2-methylpyrimidin-4-yl)-*N*-(1-methylcyclopropyl)acetamide (54.0 mg) was dissolved in 1 mL of tetrahydrofuran. 0.5 mL of 2 M hydrochloric acid solution was added, and the mixture was reacted at 50 °C for 3 h. After the reaction was completed, the reaction mixture was cooled to room temperature, neutralized with a saturated sodium bicarbonate solution, extracted with ethyl acetate, dried, concentrated, and purified by preparative HPLC (mobile phase: acetonitrile/water) to give the title compound.

$^1$H NMR (400 MHz, DMSO-$d_6$) δ 9.04 (s, 1H), 8.76 (d, *J* = 7.0 Hz, 1H), 7.67 (t, *J* = 7.5 Hz, 1H), 7.52 (t, *J* = 7.1 Hz, 1H), 7.32 (t, *J* = 7.7 Hz, 1H), 7.23 (t, *J* = 54.4 Hz, 1H), 6.33 (m, 1H), 5.81-5.70 (m, 1H), 4.25 (m, 2H), 2.22 (s, 3H), 1.58 (d, *J* = 7.1 Hz, 3H), 1.52 (s, 3H), 1.18-1.02 (m, 4H). LC/MS(m/z, MH$^+$): 483.2.

**Example 10: (*R*)-4-((1-(3-(difluoromethyl)-2-fluorophenyl)ethyl)amino)-8-ethoxy-6-(1-(fluoromethyl)cyclopropyl)-2-methylpyrido[4,3-*d*]pyrimidine-7(6*H*)-one**

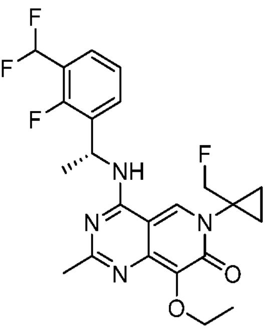

The title compound was prepared in the same manner as in Example 1 except for replacing the solution of sodium methoxide in methanol in step **2** with a solution of sodium ethoxide in ethanol.

$^{1}$H NMR (400 MHz, DMSO-*d*$_6$) δ 8.96 (s, 1H), 8.90 (bs, 1H), 7.66 (t, *J* = 7.2 Hz, 1H), 7.52 (t, *J* = 6.9 Hz, 1H), 7.33 (t, *J* = 7.7 Hz, 1H), 7.23 (t, *J* = 54.4 Hz, 1H), 5.84-5.70 (m, 1H), 4.80-4.50 (m, 2H), 4.08 (q, *J* = 7.0 Hz, 2H), 2.23 (s, 3H), 1.59 (d, *J* = 7.1 Hz, 3H), 1.45-1.15 (m, 7H). LC/MS(m/z, MH$^+$): 483.2.

**Example 11: (*R*)-4-((1-(3-(1,1-difluoroethyl)-2-fluorophenyl)ethyl)amino)-6-(1-(fluoromethyl) cyclopropyl)-8-methoxy-2-methylpyrido[4,3-*d*]pyrimidine-7(6*H*)-one**

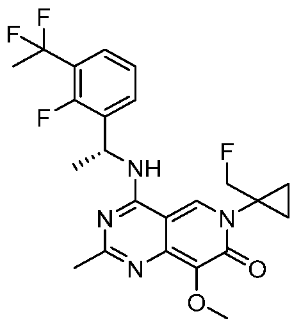

**Step 1: synthesis of (*R*)-8-bromo-4-((1-(3-(1,1-difluoroethyl)-2-fluorophenyl)ethyl)amino)-6-(1-(fluoromethyl)cyclopropyl-2-methylpyrido[4,3-*d*]pyrimidine-7(6*H*)-one**

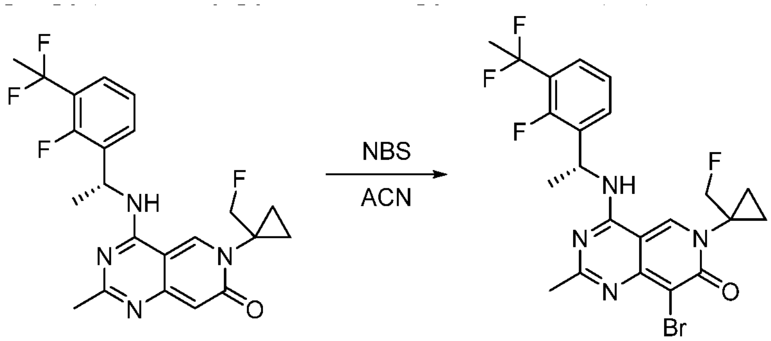


(*R*)-4-((1-(3-(1,1-Difluoroethyl)-2-fluorophenyl)ethyl)amino)-6-(1-(fluoromethyl)cyclopropyl)-2-methylpyrido[4,3-*d*]pyrimidine-7(6*H*)-one (700 mg) and *N*-bromosuccinimide (311 mg) were placed in acetonitrile (5 mL), and the system was purged with nitrogen. The reaction was completed after the mixture was stirred for reaction at room temperature for 1 h. The reaction mixture was concentrated and purified by column chromatography (dichloromethane/methanol gradient elution) to give the title compound.

**Step 2: synthesis of (*R*)-4-((1-(3-(1,1-difluoroethyl)-2-fluorophenyl)ethyl)amino)-6-(1-(fluoromethyl)cyclopropyl)-8-methoxy-2-methylpyrido[4,3-*d*]pyrimidine-7(6*H*)-one**

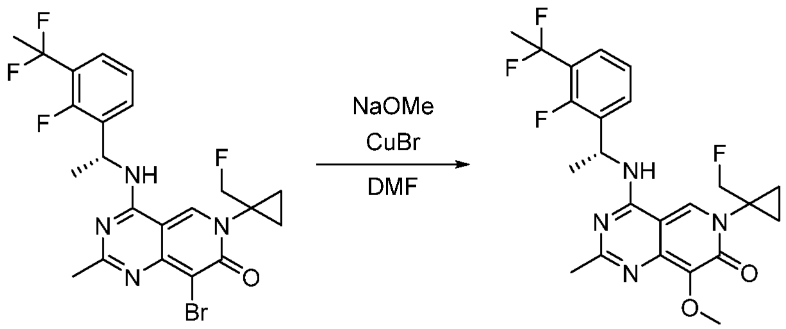


(*R*)-8-Bromo-4-((1-(3-(1,1-difluoroethyl)-2-fluorophenyl)ethyl)amino)-6-(1-(fluoromethyl)cyclopropyl)-2-methylpyrido[4,3-*d*]pyrimidine-7(6*H*)-one (50.0 mg) was dissolved in 0.5 mL of *N*,*N*-dimethylformamide. 0.5 mL of a 5.4 M solution of sodium methoxide in methanol was added, and then cuprous bromide (3.00 mg) was added. The reaction was completed after the mixture was heated to 60 °C and stirred for 4 h. The reaction mixture was cooled to room temperature, poured into a saturated ammonium chloride solution, extracted with ethyl acetate, dried over anhydrous sodium sulfate, filtered, concentrated, and purified by preparative HPLC (mobile phase: acetonitrile/water) to give the title compound.

$^1$H NMR (400 MHz, DMSO-$d_6$) δ 8.95 (s, 1H), 8.80 (d, *J* = 6.8 Hz, 1H), 7.59 (t, *J* = 6.9 Hz, 1H), 7.45 (t, *J* = 7.2 Hz, 1H), 7.27 (t, *J* = 7.8 Hz, 1H), 5.80-5.70 (m, 1H), 4.80-4.50 (m, 2H), 3.77 (s, 3H), 2.21 (s, 3H), 2.02 (t, *J* = 19.1 Hz, 3H), 1.58 (d, *J* = 7.1 Hz, 3H), 1.33-1.23 (m, 4H). LC/MS(m/z, MH$^+$): 465.2.

**Example 12: (*R*)-4-((1-(3-(difluoromethyl)-2-fluorophenyl)ethyl)amino)-6-(1-(fluoromethyl) cyclopropyl)-2-methyl-8-(1-methyl-1*H*-pyrazol-4-yl)pyrido[4,3-*d*]pyrimidine-7(6*H*)-one**

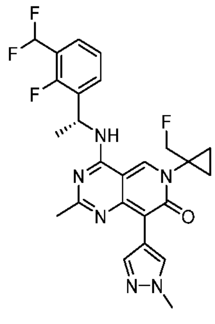

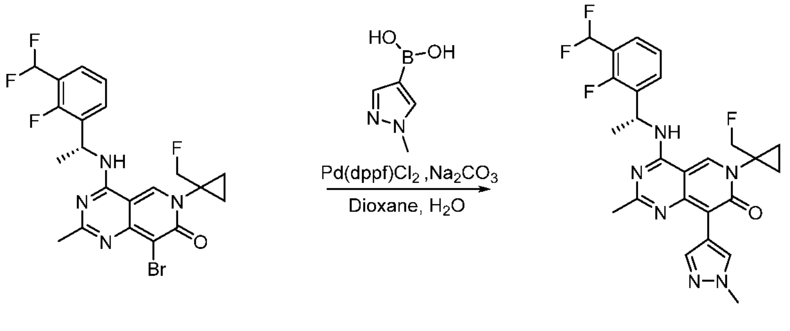


8-Bromo-4-[[(1*R*)-1-[3-(difluoromethyl)-2-fluoro-phenyl]ethyl]amino]-6-[1-(fluoromethyl)cyclopropyl]-2-methyl-pyrido[4,3-*d*]pyrimidine-7-one (50.0 mg), sodium carbonate (21.2 mg), and [1,1'-bis(diphenylphosphino)ferrocene]palladium dichloride (7.33 mg) were placed in a reaction flask. (1-Methyl-1*H*-pyrazol-4-yl)boronic acid (16.4 mg), dioxane

(1 mL), and water (0.2 mL) were added under nitrogen atmosphere. The reaction was completed after the mixture was heated to 100 °C and stirred for reaction for 3 h. The organic phase was concentrated and purified by column chromatography (dichloromethane/methanol gradient elution) to give the title compound.

$^1$H NMR (400 MHz, DMSO-$d_6$) δ 9.07 (s, 1H), 8.85 (d, *J* = 7.1 Hz, 1H), 8.71 (s, 1H), 8.60 (s, 1H), 7.67 (t, *J* = 7.2 Hz, 1H), 7.53 (t, *J* = 7.1 Hz, 1H), 7.33 (t, *J* = 7.7 Hz, 1H), 7.24 (t, *J* = 54.4 Hz, 1H), 5.84-5.76 (m, 1H), 4.87-4.53 (m, 2H), 3.86 (s, 3H), 2.31 (s, 3H), 1.60 (d, *J* = 7.1 Hz, 3H), 1.43-1.17 (m, 4H). LC/MS(m/z, MH$^+$): 501.2.

**Example 13: 6-[1-(fluoromethyl)cyclopropyl]-2-methyl-4-[(1*R*)-1-(2-methyl-3-nitrophenyl) ethyl]amino]pyrido[4,3-*d*]pyrimidine-7-one**

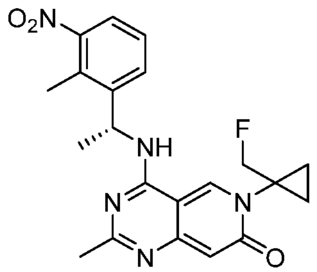

**Step 1: synthesis of 1-(2-methyl-3-nitrophenyl)ethanone**

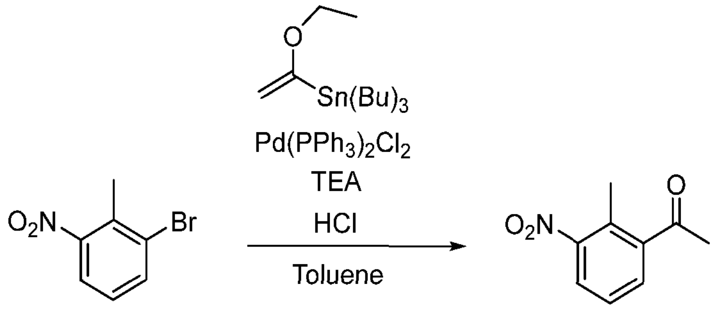


2-Bromo-6-nitrotoluene (5.00 g) was added to 30 mL of toluene. Triethylamine (5.86 g), tributyl(1-ethoxyethylene)tin (10.0 g), and bis(triphenylphosphine)palladium dichloride (649 mg) were added thereto. The system was purged with argon for three times. The reaction was completed after the mixture was reacted at 100 °C for 16 h. The reaction mixture was cooled to room temperature and filtered. The filtrate was distilled under reduced pressure to give a crude product, which was dissolved in tetrahydrofuran. Diluted hydrochloric acid (1 M, 23.1 mL) was added at 0 °C, and the reaction mixture was returned to room temperature and stirred for 15 min. The pH was adjusted with a saturated sodium bicarbonate solution to be slightly basic. Then potassium fluoride (1.61 g) was added. The mixture was stirred for 2 h and filtered through celite, and the filtrate was extracted with ethyl acetate three times. The organic phases were combined. The organic layer was concentrated and purified by column chromatography (petroleum ether/ethyl acetate gradient elution) to give the title compound.

Step 2: **(*R*)-2-methyl-*N*-(1-(2-methyl-3-nitrophenyl)ethylidene)propane-2-sulfinamide**

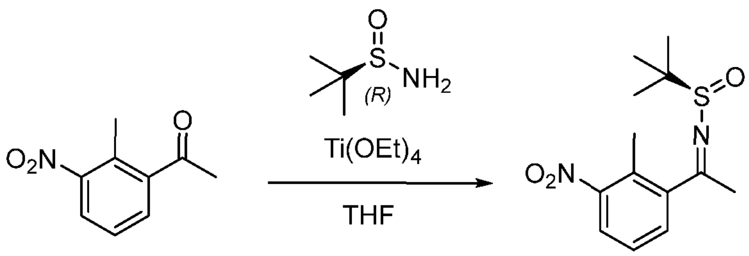


1-(2-Methyl-3-nitrophenyl)ethanone (3.35 g) was added to 90 mL of tetrahydrofuran. (*R*)-(+)-*tert*-butyl sulfinamide (3.40 g) and tetraethyl titanate (10.6 g) were added at room temperature. The system was purged with argon for three times. The reaction was completed after the mixture was reacted at 70 °C for 3 h. 50 mL of ice water was added, and the mixture was diluted with ethyl acetate and filtered through celite. The filtrate was separated to give an organic layer, which was dried over anhydrous sodium sulfate, filtered, concentrated and purified by column chromatography (petroleum ether/ethyl acetate gradient elution) to give the title compound.

**Step 3: synthesis of (*R*)-2-methyl-*N*-((*R*)-1-(2-methyl-3-nitrophenyl)ethyl)propane-2-sulfoximide**

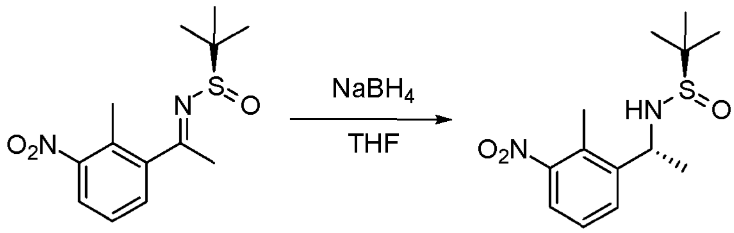


(*R*)-2-Methyl-*N*-(1-(2-methyl-3-nitrophenyl)ethylidene)propane-2-sulfinamide (4.00 g) was added to 30 mL of tetrahydrofuran. Sodium borohydride (520 mg) was added in batches under an ice bath. The reaction was completed after the mixture was reacted at room temperature for 12 h. The mixture was quenched with a saturated aqueous ammonium chloride solution, diluted with ethyl acetate, and extracted with saturated brine. The organic phase was dried over anhydrous sodium sulfate and filtered. The organic layer was concentrated and purified by column chromatography (petroleum ether/ethyl acetate gradient elution) to give the title compound.

**Step 4: synthesis of (1*R*)-1-(2-methyl-3-nitrophenyl)ethylamine hydrochloride**

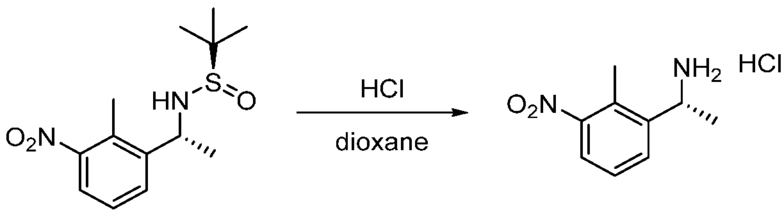


(*R*)-2-Methyl-*N*-((*R*)-1-(2-methyl-3-nitrophenyl)ethyl)propane-2-sulfoximide (1.77 g) was added to 20 mL of dioxane. A solution of hydrochloric acid in dioxane (4 M, 4.67 mL) was added under an ice bath. The reaction was completed after the mixture was reacted at room temperature for 10 min. The reaction mixture was subjected to suction filtration. The filter cake was washed with methyl *tert*-butyl ether and dried to give the title compound.

**Step 5: synthesis** of **6-[1-(fluoromethyl)cyclopropyl]-2-methyl-4-[(1*R*)-1-(2-methyl-3-nitrophenyl)ethyl]amino]pyrido[4,3-*d*]pyrimidine-7-one**

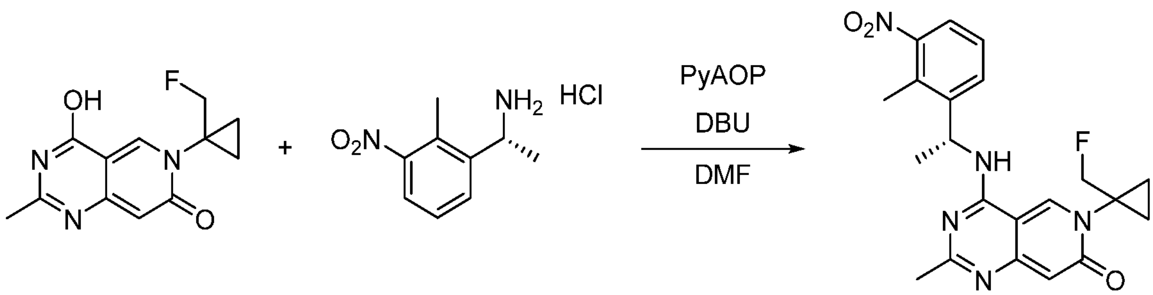


(1*R*)-1-(2-Methyl-3-nitrophenyl)ethylamine hydrochloride (130 mg) and 6-(1-(fluoromethyl)cyclopropyl)-4-hydroxy-2-methylpyrido[4,3-*d*]pyrimidine-7(6*H*)-one (100 mg) were added to 1 mL of *N*,*N*-dimethylformamide. 1,8-Diazabicyclo[5.4.0]undec-7-ene (183 mg) and (7-azabenzotriazole-1-propoxy)tripyrrolidinophosphonium hexafluorophosphate (313 mg) were added, and the reaction was completed after the mixture was reacted at room temperature for 12 h. The reaction mixture was diluted with ethyl acetate and extracted with saturated brine. The organic phase was dried over anhydrous sodium sulfate, filtered, concentrated, and purified by preparative HPLC (mobile phase: acetonitrile/water) to give the title compound.

$^1$H NMR (400 MHz, DMSO-$d_6$) δ 9.15 (s, 1H), 8.95 (d, *J* = 6.8 Hz, 1H), 7.72 (d, *J* = 7.8 Hz, 1H), 7.68 (d, *J* = 8.0 Hz, 1H), 7.43 (t, *J* = 7.9 Hz, 1H), 6.07 (s, 1H), 5.65 (m, 1H), 4.88-4.39 (m, 2H), 2.50 (s, 3H), 2.18 (s, 3H), 1.55 (d, *J* = 7.0 Hz, 3H), 1.37-1.19 (m, 4H). LC/MS(m/z, MH$^+$): 412.2.

**Example 14: (*R*)-1-(4-((1-(3-(difluoromethyl)-2-fluorophenyl)ethyl)amino)-8-methoxy-2-methyl-7-oxopyrido[4,3-*d*]pyrimidine-6(7*H*)-yl)cyclopropane-1-carbonitrile**

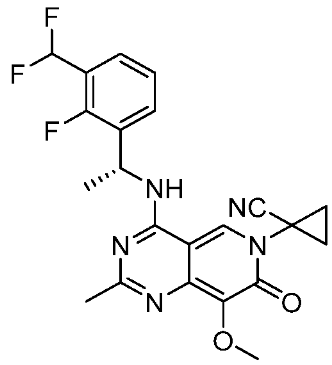

**Step 1: synthesis of (*R*)-*N*(1-cyanocyclopropyl)-2-(6-((1-(3-(difluoromethyl)-2-fluorophenyl)ethyl)amino)-5-(1,3-dioxolan-2-yl)-2-methylpyrimidin-4-yl)acetamide**

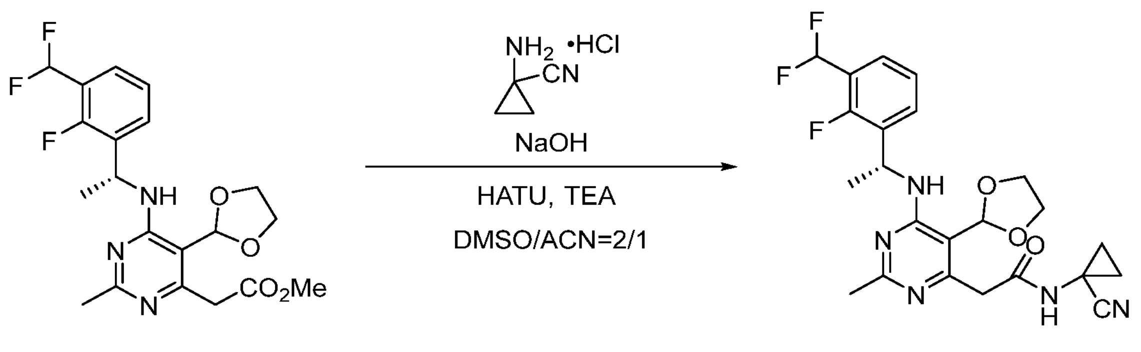


Methyl (*R*)-2-(6-((1-(3-(difluoromethyl)-2-fluorophenyl)ethyl)amino)-5-(1,3-dioxolan-2-yl)-2-methylpyrimidine-4-acetate (200 mg) was dissolved in dimethyl sulfoxide (2 mL) and acetonitrile (1 mL). An aqueous solution of sodium hydroxide (75.2 mg) having a concentration of 20% was added. After the mixture was reacted at room temperature for 30 min, triethylamine (95.2 mg) and 1-aminocyclopropanecarbonitrile hydrochloride (72.5 mg) and 2-(7-azabenzotriazol-1-yl)-*N*,*N*,*N*,*N*-tetramethyluronium hexafluorophosphate (266 mg) were added. The reaction was completed after the mixture was reacted at room temperature for 1 h. 10 mL of water was added, and the mixture was extracted with dichloromethane. The organic phases were combined, washed with a saturated sodium chloride solution, dried over anhydrous sodium sulfate, filtered, concentrated, and purified by column chromatography (dichloromethane/methanol gradient elution) to give the title compound.

**Step 2: synthesis of 2-bromo-*N*-(1-cyanocyclopropyl)-2-(6-(((*R*)-1-(3-(difluoromethyl)-2-fluorophenyl)ethyl)amino)-5-(1,3-dioxolan-2-yl)-2-methylpyrimidin-4-yl)acetamide**

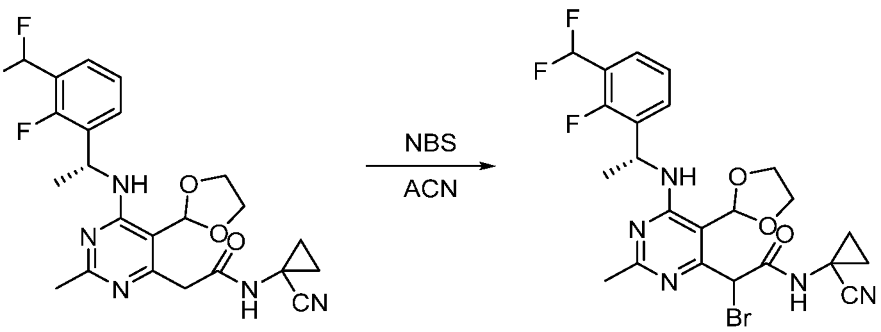


(*R*)-*N*-(1-Cyanocyclopropyl)-2-(6-((1-(3-(difluoromethyl)-2-fluorophenyl)ethyl)amino)-5-(1,3-dioxolan-2-yl)-2-methylpyrimidin-4-yl)acetamide (170 mg) was dissolved in 3 mL of acetonitrile. *N*-Bromosuccinimide (70.0 mg) was added at room temperature, and the reaction was completed after the mixture was reacted at room temperature for 30 min under argon atmosphere. The mixture was diluted with 20 mL of dichloromethane and extracted with 10 mL of water. The organic phases were combined, washed with a saturated sodium chloride solution, dried over anhydrous sodium sulfate, filtered, concentrated, and purified by column chromatography (dichloromethane/methanol gradient elution) to give the title compound.

**Step 3: synthesis of *N*-(1-cyanocyclopropyl)-2-(6-(((*R*)-1-(3-(difluoromethyl)-2-fluorophenyl) ethyl)amino)-5-(1,3-dioxolan-2-yl)-2-methylpyrimidin-4-yl)-2-methoxyacetamide**

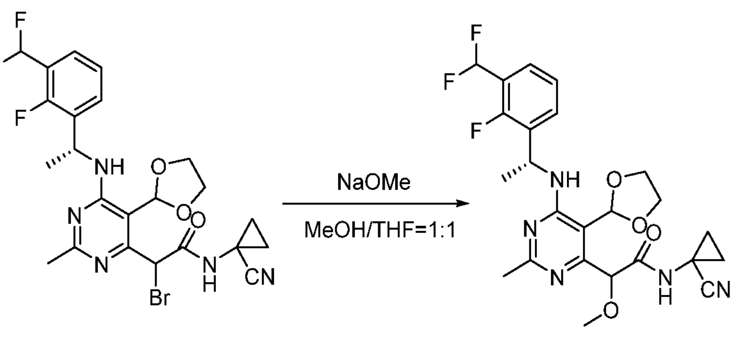


2-Bromo-*N*-(1-cyanocyclopropyl)-2-(6-(((R)-1-(3-(difluoromethyl)-2-fluorophenyl)ethyl)amino)-5-(1,3-dioxolan-2-yl)-2-methylpyrimidin-4-yl)acetamide (120 mg) was weighed. 2 mL of tetrahydrofuran and 2 mL of anhydrous methanol were added, and a sodium methoxide solution (5.4 M, 200 μL) was added. The reaction was completed after the mixture was reacted at room temperature for 1 h. The mixture was quenched with a saturated ammonium chloride solution and extracted with dichloromethane. The organic phases were combined, washed with a saturated sodium chloride solution, dried over anhydrous sodium sulfate, filtered, concentrated, and purified by column chromatography (dichloromethane/methanol gradient elution) to give the title compound.

**Step 4: synthesis of (*R*)-1-(4-((1-(3-(difluoromethyl)-2-fluorophenyl)ethyl)amino)-8-methoxy-2-methyl-7-oxopyrido[4,3-*d*]pyrimidine-6(7*H*)-yl)cyclopropane-1-carbonitrile**

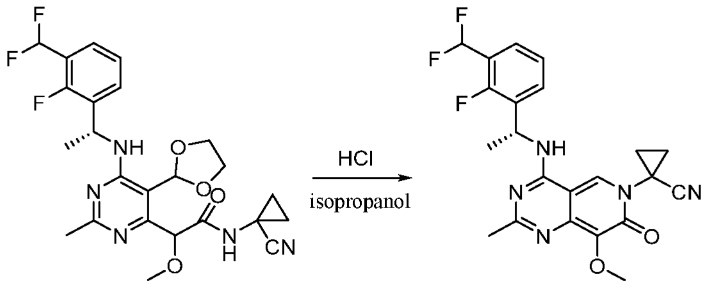


*N*-(1-Cyanocyclopropyl)-2-(6-(((*R*)-1-(3-(difluoromethyl)-2-fluorophenyl)ethyl)amino)-5-(1,3-dioxolan-2-yl)-2-methylpyrimidin-4-yl)-2-methoxyacetamide (120 mg) was dissolved in 4 mL of isopropanol. Diluted hydrochloric acid (2 M, 593 μL) was added at room temperature, and the reaction was completed after the mixture was reacted at 50 °C for 1 h. The pH was adjusted to 8-9 with ammonia water. 10 mL of water was added, and the mixture was extracted with ethyl acetate. The organic phases were combined, washed with saturated brine, dried over anhydrous sodium sulfate, filtered, concentrated, and purified by preparative HPLC (mobile phase: acetonitrile/water) to give the title compound.

$^1$H NMR (400 MHz, DMSO-$d_6$) δ 8.97 (s, 1H), 8.74 (d, *J* = 7.0 Hz, 1H), 7.68 (t, *J* = 7.1 Hz, 1H), 7.53 (t, *J* = 7.2 Hz, 1H), 7.32 (t, *J* = 7.7 Hz, 1H), 7.24 (t, *J* = 54.4 Hz, 1H), 5.80-5.69 (m, 1H), 3.80 (s, 3H), 2.22 (s, 3H), 2.05-1.93 (m, 2H), 1.80-1.76 (m, 2H), 1.58 (d, *J* = 7.1 Hz, 3H). LC/MS(m/z, MH$^+$): 443.2.

**Example 15: (*R*)-8-cyclopropoxy-4-((1-(3-(difluoromethyl)-2-fluorophenyl)ethyl)amino)-2-methyl-6-(1-methylcyclopropyl)pyrido[4,3-*d*]pyrimidine-7(6*H*)-one**

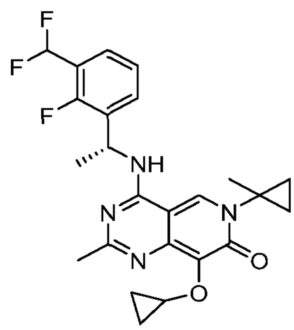

The title compound was prepared in the same manner as in Example **9** except for replacing 2,2-difluoroethanol in step **2** with cyclopropanol.

$^1$H NMR (400 MHz, DMSO-$d_6$) δ 9.01 (s, 1H), 8.69 (d, *J* = 7.1 Hz, 1H), 7.67 (t, *J* = 7.4 Hz, 1H), 7.52 (t, *J* = 6.9 Hz, 1H), 7.31 (t, *J* = 7.7 Hz, 1H), 7.23 (t, *J* = 54.4 Hz, 1H), 5.75 (m, 1H), 4.38-4.32 (m, 1H), 2.19 (s, 3H), 1.58 (d, *J* = 7.1 Hz, 3H), 1.52 (s, 3H), 1.25-0.99 (m, 4H), 0.80-0.69 (m, 2H), 0.41-0.31 (m, 2H). LC/MS(m/z, MH$^+$): 459.2.

**Example 16: 6-(1-acetyl-3-(fluoromethyl)pyrrolidin-3-yl)-8-methoxy-2-methyl-4-(((*R*)-1-(2-methyl-3-nitrophenyl)ethyl)amino)pyrido[4,3-*d*]pyrimidine-7(6*H*)-one**

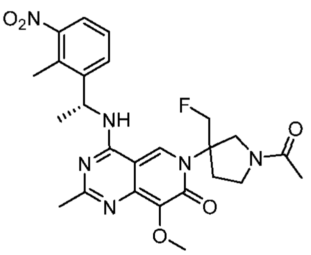

**Step 1: synthesis of methyl (*R*)-2-(5-(1,3-dioxolan-2-yl)-2-methyl-6-((1-(2-methyl-3-nitrophenyl)ethyl)amino)pyrimidin-4-yl)acetate**

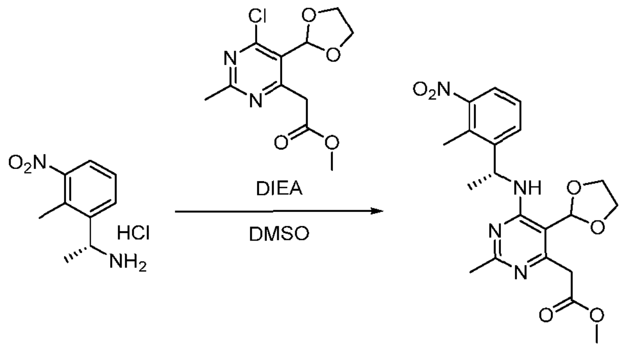


(*R*)-1-(2-Methyl-3-nitrophenyl)ethan-1-amine hydrochloride (250 mg) and methyl 2-(6-chloro-5-(1,3-dioxolan-2-yl)-2-methylpyrimidin-4-yl)acetate (471 mg) were dissolved in 3 mL of dimethyl sulfoxide. Then *N*,*N*-diisopropylethylamine (298 mg) was added. The mixture was heated to 80 °C, reacted for 8 h, cooled to room temperature, diluted with ethyl acetate, and washed with saturated brine. The organic phase was dried over anhydrous sodium sulfate, filtered, concentrated, and purified by column chromatography (petroleum ether/ethyl acetate gradient elution) to give the title compound.

**Step 2: synthesis of *tert*-butyl 3-(2-(5-(1,3-dioxolan-2-yl)-2-methyl-6-(((*R*)-1-(2-methyl-3-nitrophenyl)ethyl)amino))pyrimidin-4-yl)acetamido)-3-(fluoromethyl)pyrrolidine-1-carboxylate**

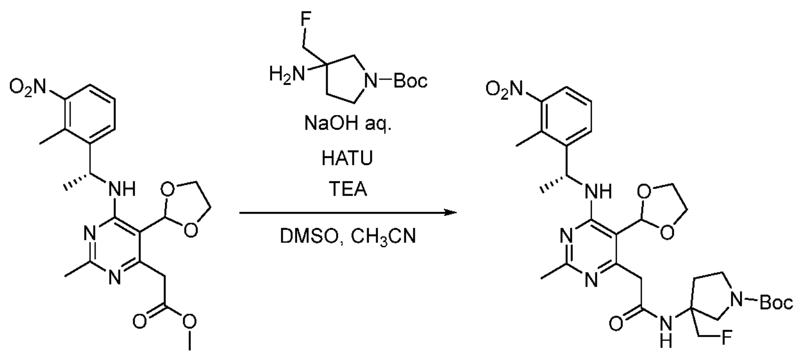


Methyl (*R*)-2-(5-(1,3-dioxolan-2-yl)-2-methyl-6-((1-(2-methyl-3-nitrophenyl)ethyl)amino)pyrimidin-4-yl)acetate (260 mg) was dissolved in a mixed solvent of 1 mL of dimethyl sulfoxide and 1 mL of acetonitrile. 0.5 mL of a 20% aqueous sodium hydroxide solution was added thereto. After the mixture was reacted at room temperature for 1 h, *tert*-butyl 3-amino-3-(fluoromethyl)pyrrolidine-1-carboxylate (204 mg) and triethylamine (315.89 mg, 3.12 mmol) were added thereto. Finally 2-(7-azabenzotriazol-1-yl)-*N*,*N*,*N'*,*N'*-tetramethyluronium hexafluorophosphate (1.18 g, 3.12 mmol) was added. After the mixture was reacted at room temperature for 30 min, the reaction mixture was diluted with

ethyl acetate, washed with a saturated sodium chloride solution, and the organic phase was dried over anhydrous sodium sulfate, filtered, concentrated, and purified by column chromatography (petroleum ether/ethyl acetate gradient elution) to give the title compound.

**Step 3: synthesis of *tert*-butyl 3-(2-(5-(1,3-dioxolan-2-yl)-2-methyl-6-(((*R*)-1-(2-methyl-3-nitrophenyl)ethyl)amino)pyrimidin-4-yl)-2-bromoacetamido)-3-(fluoromethyl)pyrrolidine-1-carboxylate**

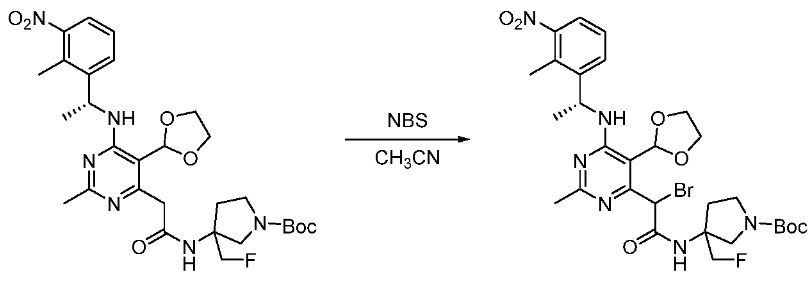


*tert*-Butyl 3-(2-(5-(1,3-dioxolan-2-yl)-2-methyl-6-(((*R*)-1-(2-methyl-3-nitrophenyl)ethyl)amino)) pyrimidin-4-yl)acetamido)-3-(fluoromethyl)pyrrolidine-1-carboxylate (245 mg) was dissolved in 5 mL of acetonitrile. *N*-Bromosuccinimide (72.4 mg) was added in batches thereto. The reaction was completed after the mixture was reacted at room temperature for 30 min, and the resulting mixture was concentrated and directly used in the next step.

**Step 4: synthesis of *tert*-butyl 3-(2-(5-(1,3-dioxolan-2-yl)-2-methyl-6-(((*R*)-1-(2-methyl-3-nitrophenyl)ethyl)amino)pyrimidin-4-yl)-2-methoxyacetamide)-3-(fluoromethyl)pyrrolidine-1-carboxylate**

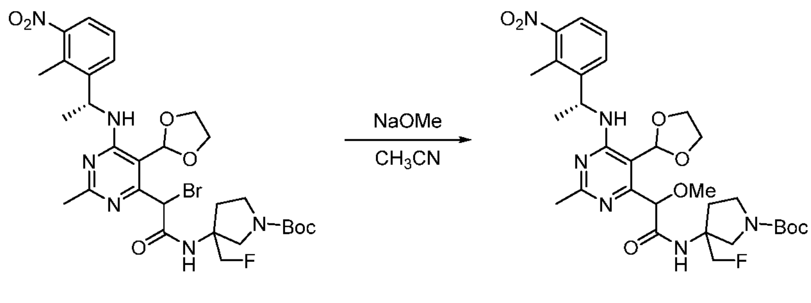


Sodium methoxide (99.1 mg) was added in batches to a solution of *tert*-butyl 3-(2-(5-(1,3-dioxolan-2-yl)-2-methyl-6-(((*R*)-1-(2-methyl-3-nitrophenyl)ethyl)amino)pyrimidin-4-yl)-2-bromoacetamido)-3-(fluoromethyl)pyrrolidine-1-carboxylate (250 mg) in acetonitrile. The mixture was reacted at room temperature for 1 h. After the reaction mixture was diluted with ethyl acetate and washed with saturated brine, the organic phase was dried over anhydrous sodium sulfate, filtered, concentrated, and purified by column chromatography (petroleum ether/ethyl acetate gradient elution) to give the title compound.

**Step 5: synthesis of 6-(3-(fluoromethyl)pyrrolidin-3-yl)-8-methoxy-2-methyl-4-(((*R*)-1-(2-methyl-3-nitrophenyl)ethyl)amino)pyrido[4,3-*d*]pyrimidine-7(6*H*)-one**

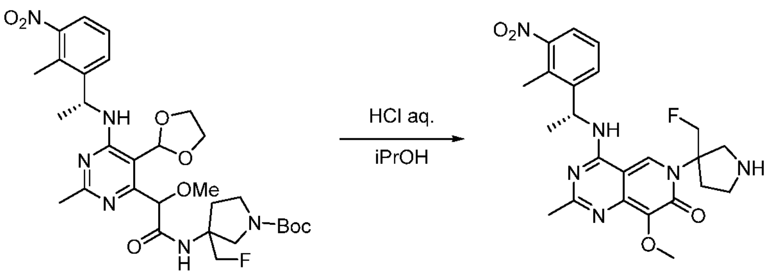


*tert*-Butyl 3-(2-(5-(1,3-dioxolan-2-yl)-2-methyl-6-(((*R*)-1-(2-methyl-3-nitrophenyl)ethyl)amino) pyrimidin-4-yl)-2-methoxyacetamido)-3-(fluoromethyl)pyrrolidine-1-carboxylate (170 mg) was dissolved in 5 mL of isopropanol. 0.25 mL of 5 M diluted hydrochloric acid was added thereto. After the mixture was heated to 80 °C and reacted for 4 h, the reaction mixture was concentrated to dryness, and the crude product was directly used in the next step.

**Step 6: synthesis of 6-(1-acetyl-3-(fluoromethyl)pyrrolidin-3-yl)-8-methoxy-2-methyl-4-(((*R*)-1-(2-methyl-3-nitrophenyl)ethyl)amino)pyrido[4,3-*d*]pyrimidine-7(6*H*)-one**

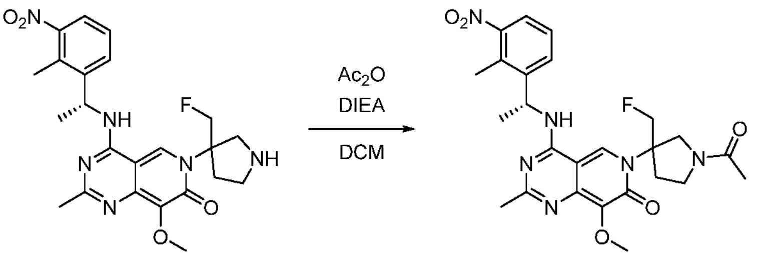


6-(3-(Fluoromethyl)pyrrolidin-3-yl)-8-methoxy-2-methyl-4-(((*R*)-1-(2-methyl-3-nitrophenyl)ethyl)amino)pyrido[4,3-*d*]pyrimidine-7(6*H*)-one (80.0 mg) was dissolved in 5 mL of dichloromethane. After *N*,*N*-diisopropylethylamine (109 mg) was added thereto, acetic anhydride (69.4 mg) was added dropwise. After the dropwise addition, the mixture was reacted at room temperature for 30 min, and the reaction mixture was concentrated and purified by preparative HPLC (mobile phase: acetonitrile/water) to give the title compound.

$^1H$ NMR (400 MHz, DMSO-$d_6$) δ 9.08-8.95 (m, 1H), 8.62-8.48 (m, 1H), 7.76-7.62 (m, 2H), 7.47-7.37 (m, 1H), 5.75-5.61 (m, 1H), 5.09-4.61 (m, 2H), 4.55-4.38 (m, 1H), 4.06-3.35 (m, 6H), 3.06-2.81 (m, 1H), 2.68-2.52 (m, 1H), 2.51-2.49 (m, 3H), 2.23 (s, 3H), 2.02-1.88 (m, 3H), 1.57 (d, *J* = 6.4 Hz, 3H). LC/MS(m/z, $MH^+$): 513.2.

**Example 17: 3-((1*R*)-1-((6-(1-acetyl-3-(fluoromethyl)pyrrolidin-3-yl)-8-methoxy-2-methyl-7-oxo-6,7-dihydropyrido[4,3-*d*]pyrimidin-4-yl)amino)ethyl)-2-methylbenzonitrile**

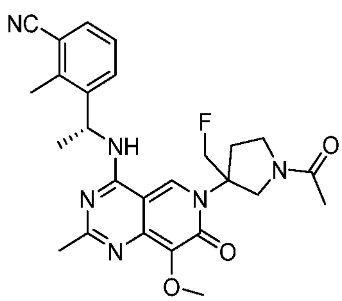

The title compound was prepared in the same manner as in Example 16 except for replacing (*R*)-1-(2-methyl-3-nitrophenyl)ethan-1-amine hydrochloride in step **1** with (*R*)-3-(1-aminoethyl)-2-methylbenzonitrile hydrochloride.

$^1H$ NMR (400 MHz, DMSO-$d_6$) δ 9.06-8.95 (m, 1H), 8.61-8.50 (m, 1H), 7.75-7.62 (m, 2H), 7.44-7.35 (m, 1H),

5.69-5.59 (m, 1H), 5.08-4.61 (m, 2H), 4.53-4.39 (m, 1H), 3.85-3.58 (m, 5H), 3.56-3.39 (m, 1H), 3.06-2.80 (m, 1H), 2.66 (s, 3H), 2.62-2.51 (m, 1H), 2.23 (s, 3H), 2.02-1.91 (m, 3H), 1.54 (d, *J* = 6.9 Hz, 3H). LC/MS(m/z, $MH^+$): 493.2.

**Example 18: (*R*)-2-methyl-6-morpholine-4-((1-(3-(pentafluoro-λ$^6$-sulfanyl)phenyl)ethyl)amino) pyrido[4,3-*d*]pyrimidine-7(6*H*)-one**

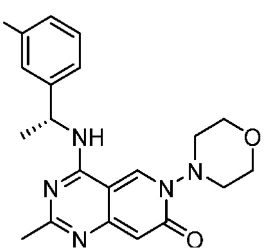

**Step 1: synthesis of methyl (*R*)-2-(5-(1,3-dioxolan-2-yl)-2-methyl-6-((1-(3-(pentafluoro-)λ$^6$-sulfanyl)phenyl)ethyl)amino)pyrimidin-4-yl)acetate**

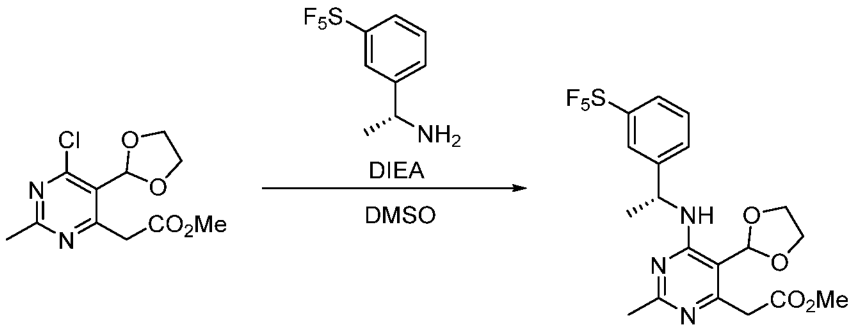


Methyl 2-[6-chloro-5-(1,3-dioxolan-2-yl)-2-methylpyrimidin-4-yl]acetate (238 mg), (*R*)-1-(3-(pentafluoro-λ$^6$-sulfanyl)phenyl)ethane-1-amine (215 mg), and *N*,*N*-diisopropylethylamine (169 mg) were placed in a reaction flask. Dimethyl sulfoxide (1 mL) was added when the system was purged with nitrogen, and the reaction was completed after the mixture was heated to 80 °C and stirred for reaction for 2 h. The reaction mixture was cooled to room temperature, quenched with water, and extracted with ethyl acetate. The organic layer was concentrated and purified by column chromatography (petroleum ether/ethyl acetate gradient elution) to give the title compound.

**Step 2: synthesis of (*R*)-2-(5-(1,3-dioxolan-2-yl)-2-methyl-6-((1-(3-(pentafluoro-λ$^6$-sulfanyl) phenyl)ethyl)amino)pyrimidin-4-yl)-*N*-morpholinoacetamide**

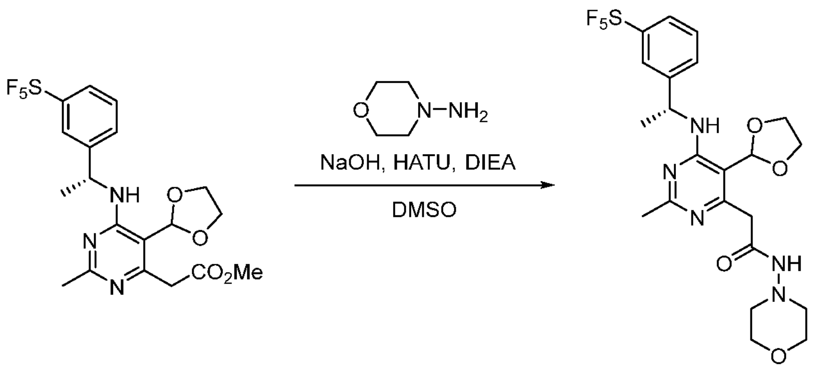

Methyl (*R*)-2-(5-(1,3-dioxolan-2-yl)-2-methyl-6-((1-(3-(pentafluoro-$\lambda^6$-sulfanyl)phenyl)ethyl)amino) pyrimidin-4-yl)acetate (210 mg) was dissolved in dimethyl sulfoxide (1 mL). A 4 N aqueous sodium hydroxide (69.5 mg) solution was added. After the mixture was stirred at room temperature for half an hour, *N,N*-diisopropylethylamine (87.9 mg) and *N*-aminomorpholine (57.7 mg) were added. After the mixture was stirred for 3 min, 2-(7-azabenzotriazol-1-yl)-*N,N,N',N'*-tetramethyluronium hexafluorophosphate (819 mg) was added. The reaction was completed after the mixture was reacted at room temperature for 2 h. The mixture was quenched with water and extracted with ethyl acetate. The organic layer was concentrated and purified by column chromatography (dichloromethane/methanol gradient elution) to give the title compound.

**Step 3: synthesis of (*R*)-2-methyl-6-morpholine-4-((1-(3-(pentafluoro-$\lambda^6$-sulfanyl)phenyl) ethyl)amino)pyrido[4,3-*d*]pyrimidine-7(6*H*)-one**

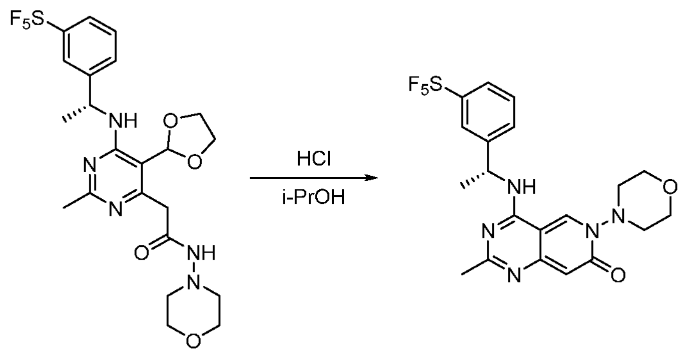


(*R*)-2-(5-(1,3-dioxolan-2-yl)-2-methyl-6-((1-(3-(pentafluoro-$\lambda^6$-sulfanyl)phenyl)ethyl)amino)pyrimidin-4-yl)-*N*-morpholinoacetamide (200 mg) was dissolved in 3 mL of isopropanol, 4 M diluted hydrochloric acid (13.1 mg) was added. The reaction was completed after the mixture was heated to 55 °C and stirred for reaction for 1 h. A saturated sodium bicarbonate solution was added to adjust the pH to 7-8, and the mixture was extracted with ethyl acetate. The organic phase was washed with water, dried over anhydrous sodium sulfate, filtered, concentrated, and purified by preparative HPLC (mobile phase: acetonitrile/water) to give the title compound.

$^1$H NMR (400 MHz, DMSO-$d_6$) $\delta$ 9.16 (s, 1H), 8.83 (d, *J* = 7.3 Hz, 1H), 7.97 (s, 1H), 7.78 (dd, *J* = 8.2, 1.6 Hz, 1H), 7.72 (d, *J* = 7.7 Hz, 1H), 7.59 (t, *J* = 7.9 Hz, 1H), 6.10 (s, 1H), 5.66-5.49 (m, 1H), 3.91-3.36 (m, 8H), 2.22 (s, 3H), 1.58 (d, *J* = 7.1 Hz, 3H). LC/MS(m/z, $MH^+$): 492.2.

**Example 19: 4-((*R*)-1-(3-(difluoromethyl)-2-fluorophenyl)ethyl)amino)-6-(1-thioacetyl-3-methylpyrrolidin-3-yl)-8-methoxy-2-methylpyrido[4,3-*d*]pyrimidine-7(6*H*)-one**

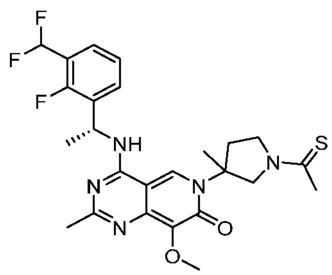

**Step 1: synthesis of methyl (*R*)-2-(6-(1-(3-(difluoromethyl)-2-fluorophenyl)ethyl)amino)-5-(1,3-dioxolan-2-yl)-2-methylpyrimidin-4-yl)-acetate**

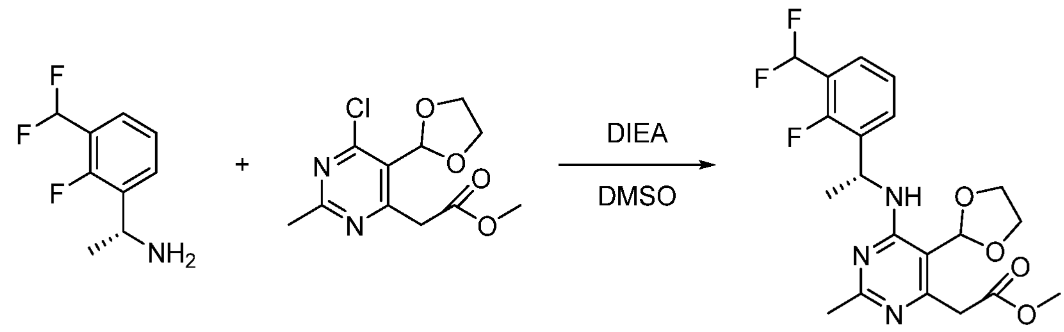


(*R*)-1-(3-(Difluoromethyl)-2-fluorophenyl)ethane-1-amine (3.05 g) and methyl 2-(6-chloro-5-(1,3-dioxolan-2-yl)-2-methylpyrimidin-4-yl)acetate (4.00 g) were dissolved in 20 mL of dimethyl sulfoxide. *N,N*-Diisopropylethylamine (2.27 g) was added, and the reaction was completed after the mixture was stirred at 80 °C for 10 h. Ethyl acetate was added, and the mixture was washed with water and washed with saturated brine. The organic phase was dried over anhydrous sodium sulfate, filtered, concentrated, and purified by column chromatography (petroleum ether/ethyl acetate gradient elution) to give the title compound.

**Step 2: synthesis of *tert*-butyl 3-(2-(6-((*R*)-1-(3-(difluoromethyl)-2-fluorophenyl)ethyl)amino)-5-(1,3-dioxolan-2-yl)-2-methylpyrimidin-4-yl)acetamido)-3-methylpyrrolidine-1-carboxylate**

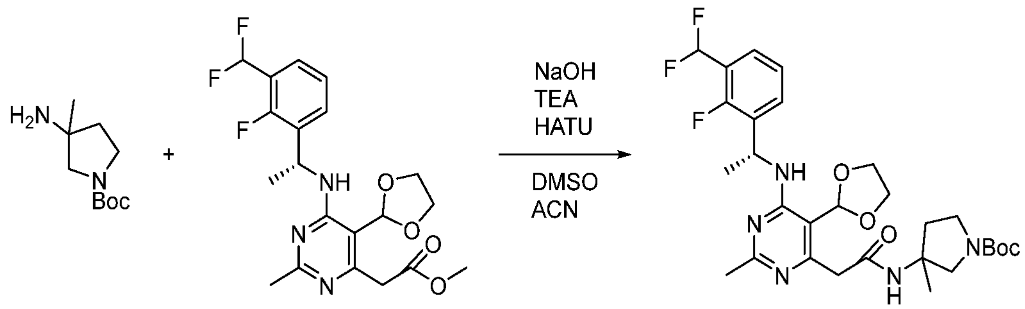


Methyl (*R*)-2-(6-(1-(3-(difluoromethyl)-2-fluorophenyl)ethyl)amino)-5-(1,3-dioxolan-2-yl)-2-methylpyrimidin-4-yl)-acetate (500 mg) was dissolved in a mixed solution of 5 mL of dimethyl sulfoxide and 2.5 mL of acetonitrile. A 20% aqueous sodium hydroxide solution (775 μL) was added, and the mixture was stirred at room temperature for 30 min. Then triethylamine (327 μL), *tert*-butyl 3-amino-3-methylpyrrolidine-1-carboxylate (235 mg), and 2-(7-azabenzotriazol-1-yl)-*N*,*N*,*N'*,*N'*-tetramethyluronium hexafluorophosphate (665 mg) were added. The reaction was completed after the mixture was stirred at room temperature for 30 min. Dichloromethane was added, and the mixture was washed with water and washed with saturated brine. The organic phase was dried over anhydrous sodium sulfate, filtered, concentrated, and purified by column chromatography (petroleum ether/ethyl acetate gradient elution) to give the title compound.

**Step 3: *tert*-butyl 3-(2-bromo-2-(6-((*R*)-1-(3-(difluoromethyl)-2-fluorophenyl)ethyl)amino)-5-(1,3-dioxolan-2-yl)-2-methylpyrimidin-4-yl)acetamido)-3-methylpyrrolidine-1-carboxylate**

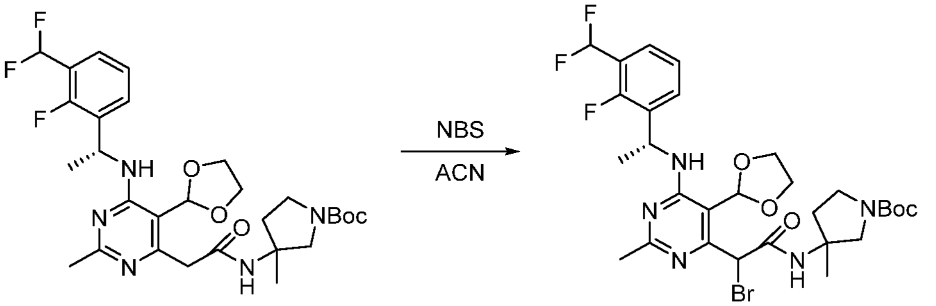


*tert*-Butyl 3-(2-(6-((*R*)-1-(3-(difluoromethyl)-2-fluorophenyl)ethyl)amino)-5-(1,3-dioxolan-2-yl)-2-methylpyrimidin-4-yl)acetamido)-3-methylpyrrolidine-1-carboxylate (410 mg, 0.690 mmol) was dissolved in 8 mL of acetonitrile. *N*-Bromosuccinimide (122 mg, 0.690 mmol) was added, and the mixture was stirred at room temperature for 30 min. After the reaction was completed, the mixture was concentrated and purified by column chromatography (petroleum ether/ethyl acetate gradient elution) to give the title compound.

**Step 4: *tert*-butyl 3-(2-(6-((*R*)-1-(3-(difluoromethyl)-2-fluorophenyl)ethyl)amino)-5-(1,3-dioxolan-2-yl)-2-methylpyrimidin-4-yl)-2-methoxyacetamido)-3-methylpyrrolidine-1-carboxylate**

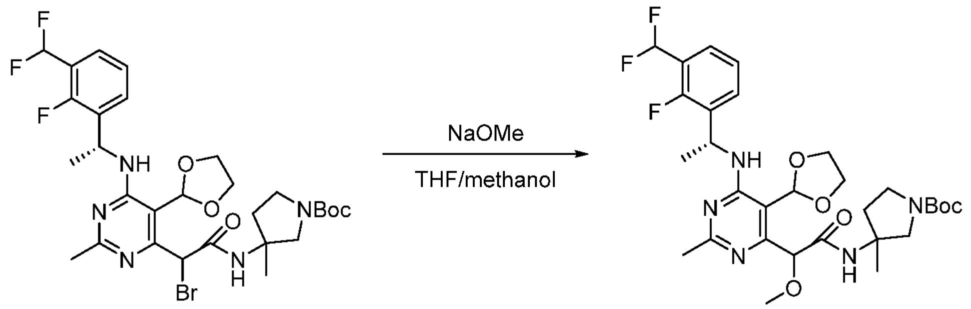


*tert*-Butyl 3-(2-Bromo-2-(6-((*R*)-1-(3-(difluoromethyl)-2-fluorophenyl)ethyl)amino)-5-(1,3-dioxolan-2-yl)-2-methylpyrimidin-4-yl)acetamido)-3-methylpyrrolidine-1-carboxylate (220 mg) was dissolved in a mixture of 2 mL of tetrahydrofuran and 2 mL of methanol. A 5.4 M solution (302 μL) of sodium methoxide in methanol was added. The reaction was completed after the mixture was stirred at room temperature for 30 min. The reaction mixture was poured into a saturated ammonium chloride solution and extracted with dichloromethane. The organic phase was dried over anhydrous sodium sulfate, filtered, concentrated, and purified by column chromatography (petroleum ether/ethyl acetate gradient elution) to give the title compound.

**Step 5: 4-((*R*)-1-(3-(difluoromethyl)-2-fluorophenyl)ethyl)amino)-8-methoxy-2-methyl-6-(3-methylpyrrolidin-3-yl)pyrido[4,3-*d*]pyrimidine-7(6*H*)-one**

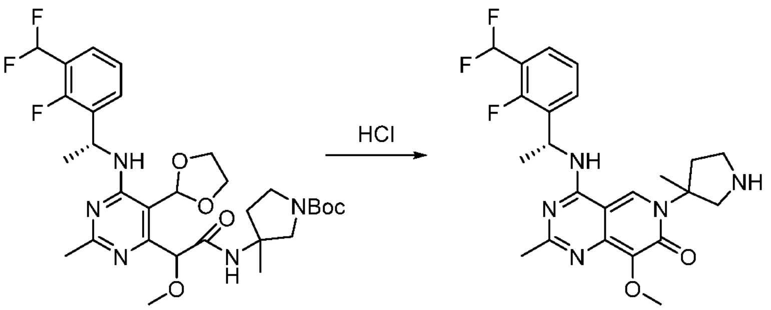


*tert*-Butyl 3-(2-(6-((*R*)-1-(3-(difluoromethyl)-2-fluorophenyl)ethyl)amino)-5-(1,3-dioxolan-2-yl)-2-methylpyrimidin-4-yl)-2-methoxyacetamido)-3-methylpyrrolidine-1-carboxylate (153 mg) was dissolved in 1 mL of isopropanol. 0.5 mL of a 5 M hydrochloric acid solution was added, and the reaction was completed after the mixture was reacted at 50 °C for 1 h. A2 N sodium hydroxide solution was added to adjust the pH to 8-9, and the mixture was extracted with dichloromethane. The organic phase was dried over anhydrous sodium sulfate, filtered, concentrated, and purified by column chromatography (dichloromethane/methanol gradient elution) to give the title compound.

**Step 6: 6-(1-acetyl-3-methylpyrrolidin-3-yl)-4-((*R*)-1-(3-(difluoromethyl)-2-fluorophenyl)ethyl) amino)-8-methoxy-2-methylpyrido[4,3-*d*]pyrimidine-7(6*H*)-one**

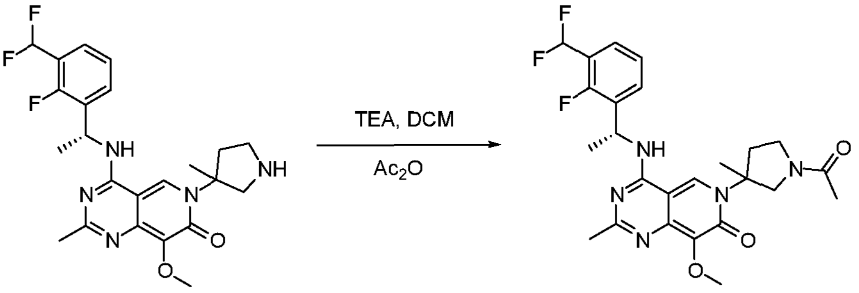


4-((*R*)-1-(3-(Difluoromethyl)-2-fluorophenyl)ethyl)amino)-8-methoxy-2-methyl-6-(3-methylpyrrolidin-3-yl)pyrido[4,3-*d*]pyrimidine-7(6*H*)-one (43.0 mg) and triethylamine (14.1 mg) were dissolved in 2 mL of dichloromethane. Acetic anhydride (11.4 mg) was added. The reaction was completed after the mixture was reacted at room temperature for 1 h. The reaction mixture was concentrated to give the title compound.

**Step 7: 4-((*R*)-1-(3-(difluoromethyl)-2-fluorophenyl)ethyl)amino)-6-(1-thioacetyl-3-methylpyrrolidin-3-yl)-8-methoxy-2-methylpyrido[4,3-*d*]pyrimidine-7(6*H*)-one**

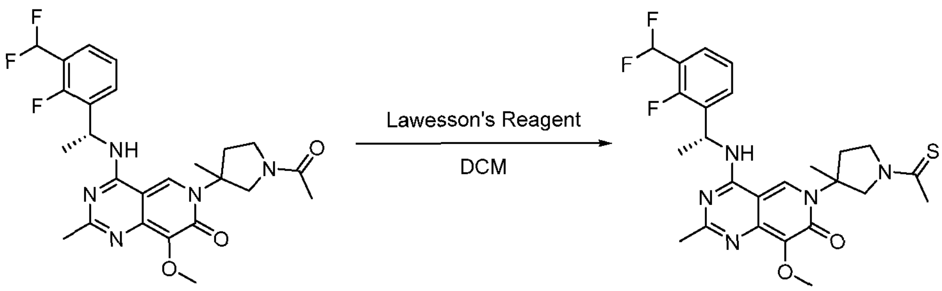


6-(1-Acetyl-3-methylpyrrolidin-3-yl)-4-((*R*)-1-(3-(difluoromethyl)-2-fluorophenyl)ethyl)amino)-8-methoxy-2-methylpyrido[4,3-*d*]pyrimidine-7(6*H*)-one (40.0 mg) was dissolved in 1 mL of dichloromethane. Lawesson reagent (38.5 mg) was added, and the mixture was reacted at 50 °C overnight. After the reaction was completed, the mixture was added to water, extracted with dichloromethane, dried over anhydrous sodium sulfate, filtered, concentrated, and purified by preparative HPLC (mobile phase: acetonitrile/water) to give the title compound.

$^1$H NMR (400 MHz, DMSO-$d_6$) δ 8.85 (s, 1H), 8.65 (d, *J* = 6.5 Hz, 1H), 7.67 (s, 1H), 7.53 (t, *J* = 6.9 Hz, 1H), 7.32 (t, *J* = 7.6 Hz, 1H), 7.24 (t, *J* = 54.8 Hz, 1H), 5.85-5.73 (m, 1H), 4.90-4.73 (m, 1H), 4.15-3.61 (m, 6H), 2.79-2.57 (m, 2H),2.55-2.52 (m, 3H), 2.23 (s, 3H), 1.63-1.57 (m, 6H). LC/MS(m/z, MH$^+$): 520.2.

**Example 20: (R)-4-((1-(3-(difluoromethyl)-2-fluorophenyl)ethyl)amino)-8-methoxy-2-methyl-6-morpholinepyrido[4,3-*d*]pyrimidine-7(6*H*)-one**

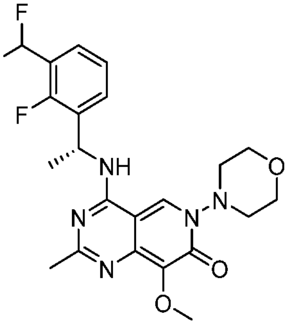

**Step 1: methyl (*R*)-2-(6-((1-(3-(difluoromethyl)-2-fluorophenyl)ethyl)amino)-5-(1,3-dioxolan-2-**yl)-2-methylpyrimidin-4-yl)acetat**e**

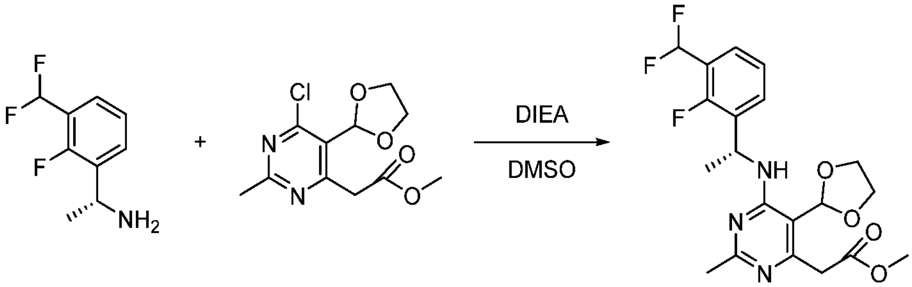


(*R*)-1-(3-(Difluoromethyl)-2-fluorophenyl)ethane-1-amine (3.05 g) and methyl 2-(6-chloro-5-(1,3-dioxolan-2-yl)-2-methylpyrimidin-4-yl)acetate (4.00 g) were dissolved in 20 mL of dimethyl sulfoxide. *N,N*-Diisopropylethylamine (2.27 g) was added, and the reaction was completed after the mixture was stirred at 80 °C for 10 h. Ethyl acetate was added, and the mixture was washed with water and washed with saturated brine. The organic phase was dried over anhydrous sodium sulfate, filtered, concentrated, and purified by column chromatography (petroleum ether/ethyl acetate gradient elution) to give the title compound.

**Step 2: (*R*)-2-(6-((1-(3-(difluoromethyl)-2-fluorophenyl)ethyl)amino)-5-(1,3-dioxolan-2-yl)-2-methylpyrimidin-4-yl)-N morpholinoacetamide**

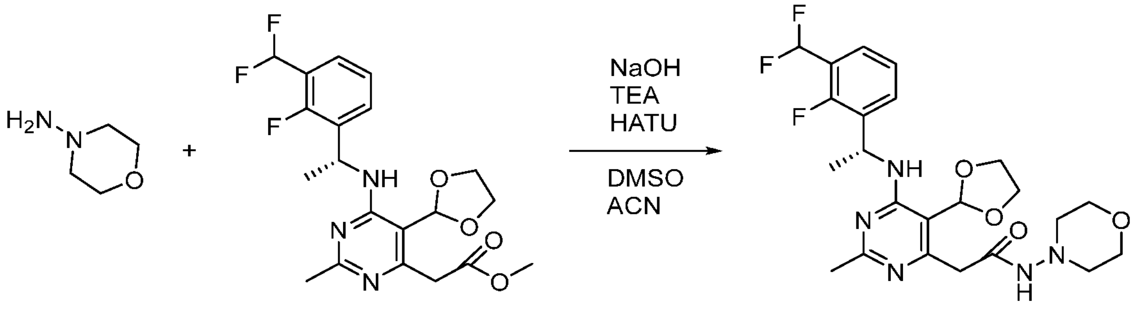


Methyl (*R*)-2-(6-((1-(3-(difluoromethyl)-2-fluorophenyl)ethyl)amino)-5-(1,3-dioxolan-2-yl)-2-methylpyrimidin-4-yl)-acetate (300 mg) was dissolved in 3 mL of dimethyl sulfoxide and 1.5 mL of acetonitrile. A 20% sodium hydroxide (465 μL) was added, and the mixture was stirred at room temperature for 30 min. Then triethylamine (197 μL), *N*-aminomorpholine (86.4 mg), and 2-(7-azabenzotriazol-1-yl)-*N*,*N*,*N*',*N*'-tetramethyluronium hexafluorophosphate (399 mg) were added. The reaction was completed after the mixture was stirred at room temperature for 30 min. Dichloromethane was added, and the mixture was washed with water and washed with saturated brine. The organic phase was dried over anhydrous sodium sulfate, filtered, concentrated, and purified by column chromatography (petroleum ether/ethyl acetate

gradient elution) to give the title compound.

**Step 3: 2-chloro-2-(6-((*R*)-1-(3-(difluoromethyl)-2-fluorophenyl)ethyl)amino)-5-(1,3-dioxolan-2-yl)-2-methylpyrimidin-4-yl)-*N*-morpholinoacetamide**

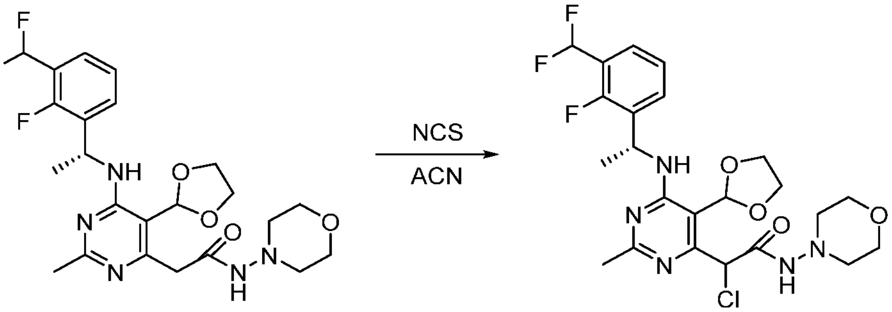


(*R*)-2-(6-((1-(3-(Difluoromethyl)-2-fluorophenyl)ethyl)amino)-5-(1,3-dioxolan-2-yl)-2-methylpyrimidin-4-yl)-N-morpholinoacetamide (100 mg) was dissolved in 2 mL of acetonitrile. *N*-Chlorosuccinimide (28.3 mg) was added, and the reaction was completed after the mixture was stirred at room temperature for 30 min. The mixture was concentrated and purified by column chromatography (petroleum ether/ethyl acetate gradient elution) to give the title compound.

**Step 4: 2-(6-((*R*)-1-(3-(difluoromethyl)-2-fluorophenyl)ethyl)amino)-5-(1,3-dioxolan-2-yl)-2-methylpyrimidin-4-yl)-2-methoxy-*N*-morpholineacetamide**

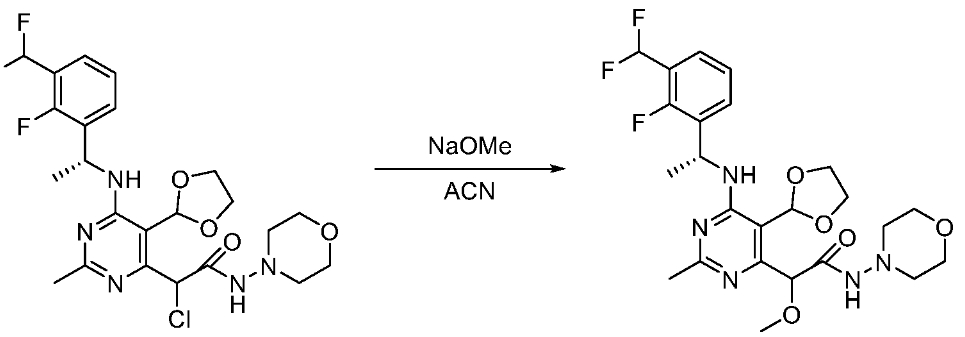


2-Chloro-2-(6-((*R*)-1-(3-(difluoromethyl)-2-fluorophenyl)ethyl)amino)-5-(1,3-dioxolan-2-yl)-2-methylpyrimidin-4-yl)-*N*-morpholinoacetamide (90.0 mg) was dissolved in 2 mL of acetonitrile. A 5.4 M solution (157 μL) of sodium methoxide in methanol was added. The reaction was completed after the mixture was stirred at room temperature for 30 min. The reaction mixture was poured into a saturated ammonium chloride solution and extracted with dichloromethane. The organic phase was dried over anhydrous sodium sulfate, filtered, concentrated, and purified by column chromatography (dichloromethane/methanol gradient elution) to give the title compound.

**Step 5: (*R*)-4-((1-(3-(difluoromethyl)-2-fluorophenyl)ethyl)amino)-8-methoxy-2-methyl-6-morpholinepyrido[4,3-*d*]pyrimidine-7(6*H*)-one**

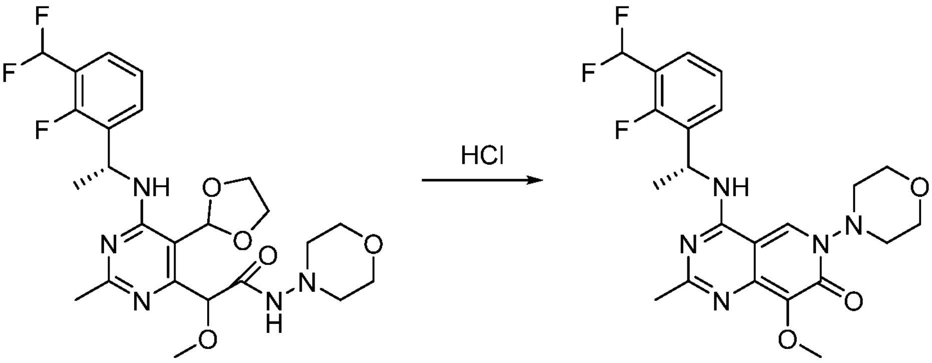


2-(6-((*R*)-1-(3-(Difluoromethyl)-2-fluorophenyl)ethyl)amino)-5-(1,3-dioxolan-2-yl)-2-methylpyrimidin-4-y)-2-methoxy-*N*-morpholineacetamide (45.0 mg) was dissolved in 0.3 mL of isopropanol. 0.15 mL of a 5 M hydrochloric acid solution was added, and the reaction was completed after the mixture was reacted at 50 °C for 1 h. The mixture was extracted with dichloromethane, and the organic phase was dried over anhydrous sodium sulfate, filtered, concentrated, and purified by preparative HPLC (mobile phase: acetonitrile/water) to give the title compound.

$^1$H NMR (400 MHz, DMSO-$d_6$) δ 9.02 (s, 1H), 8.78 (d, *J* = 7.3 Hz, 1H), 7.67 (t, *J* = 7.2 Hz, 1H), 7.52 (t, *J* = 6.9 Hz, 1H), 7.32 (t, *J* = 7.7 Hz, 1H), 7.16 (d, *J* = 54.3 Hz, 1H), 5.76 (m, 1H), 4.02-3.36 (m, 8H), 2.23 (s, 3H), 1.56 (d, *J* = 7.1 Hz, 3H).

LC/MS(m/z, MH$^+$): 464.2.

**Example 21: (*R*)-4-((1-(2-methyl-3-cyanophenyl)ethyl)amino)-8-methoxy-2-methyl-6-morpholinepyrido[4,3-*d*]pyrimidine-7(6*H*)-one**

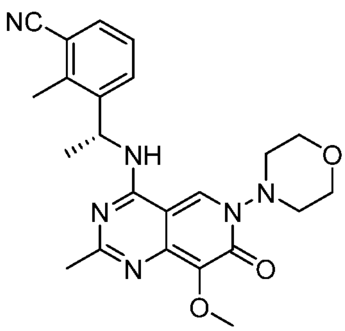

The title compound was prepared in the same manner as in Example **20** except for replacing (*R*)-1-(3-(difluoromethyl)-2-fluorophenyl)ethane-1-amine in step 1 with (R)-3-(1-aminoethyl)-2-tolunitrile. $^1$H NMR (400 MHz, DMSO-$d_6$) δ 8.99 (s, 1H), 8.84 (d, *J* = 6.9 Hz, 1H), 7.76 (d, *J* = 7.5 Hz, 1H), 7.66 (d, *J* = 7.6 Hz, 1H), 7.39 (t, *J* = 7.8 Hz, 1H), 5.67-5.56 (m, 1H), 3.86 -3.30 (m, 11H), 2.67 (s, 3H), 2.24 (s, 3H), 1.50 (d, *J* = 7.0 Hz, 3H). LC/MS(m/z, MH$^+$): 435.2.

**Example 22: (*R*)-5-(2-methyl-6-morpholinyl-7-oxo-4-((1-(3-(pentafluoro-λ$^6$-sulfanyl)phenyl) ethyl)amino)-6,7-dihydropyrido[4,3-*d*]pyrimidin-8-yl)pyridinecarbonitrile**

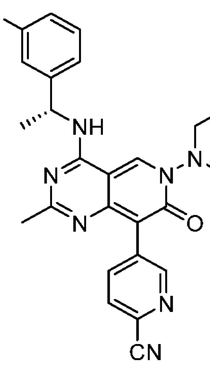

**Step 1: synthesis of (*R*)-8-bromo-2-methyl-6-morpholine-4-((1-(3-(pentafluoro-λ⁶-sulfanyl) phenyl)ethyl)amino)pyrido[4,3-*d*]pyrimidine-7(6*H*)-one**

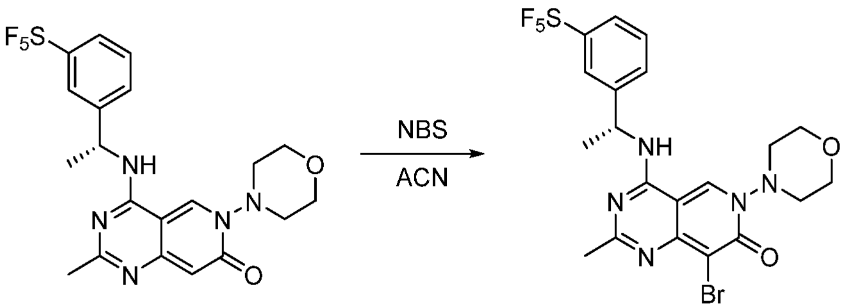


(*R*)-2-Methyl-6-morpholine-4-((1-(3-(pentafluoro-$\lambda^6$-sulfanyl)phenyl)ethyl)amino)pyrido[4,3-*d*]pyrimidine-7(6*H*)-one (120 mg) was dissolved in acetonitrile (5 mL). N-Bromosuccinimide (43.5 mg) was added, and the reaction was completed after the mixture was stirred at room temperature for reaction for half an hour. The mixture was quenched with water and extracted with ethyl acetate. The organic phase was dried over anhydrous sodium sulfate, filtered, concentrated, and purified by column chromatography (dichloromethane/methanol gradient elution) to give the title compound.

**Step 2: synthesis of (*R*)-5-(2-methyl-6-morpholinyl-7-oxo-4-((1-(3-(pentafluoro-$\lambda^6$-sulfanyl) phenyl)ethyl)amino)-6,7-dihydropyrido[4,3-*d*]pyrimidin-8-yl)pyridinecarbonitrile**

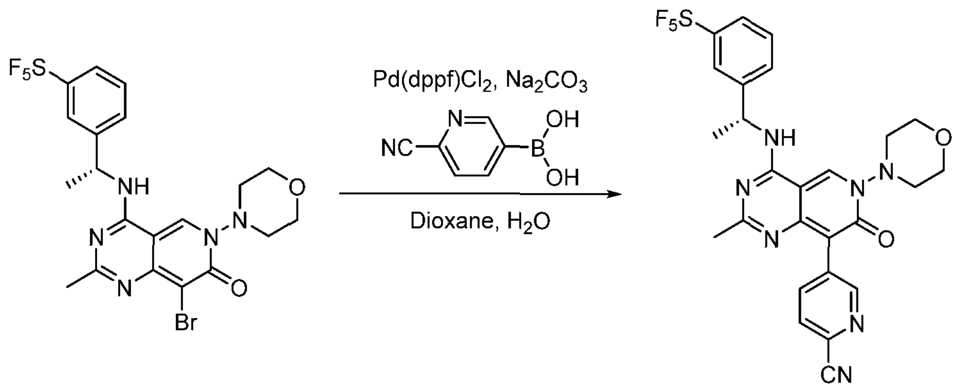


(*R*)-8-Bromo-2-methyl-6-morpholine-4-((1-(3-(pentafluoro-$\lambda^6$-sulfanyl)phenyl)ethyl)amino)pyrido[4,3-*d*]pyrimidine-7(6*H*)-one (60.0 mg), cyanopyridine boronic acid (20.2 mg), sodium carbonate (22.3 mg), and [1,1'-bis(diphenylphosphino)ferrocene]palladium dichloride (7.70 mg) were all placed in a reaction flask. Oxygen and water were removed,

and the system was purged with nitrogen. Dioxane (3 mL) and water (0.5 mL) were added, and the reaction was completed after the mixture was heated to 100 °C and stirred for reaction for 3 h. The reaction mixture was concentrated and purified by column chromatography (dichloromethane/methanol gradient elution) to give the title compound.

$^1H$ NMR (400 MHz, DMSO-$d_6$) δ 9.31 (s, 1H), 9.01 (d, *J* = 7.3 Hz, 1H), 8.84 (d, *J* = 2.0 Hz, 1H), 8.15 (dd, *J* = 8.1, 2.1 Hz, 1H), 8.04-7.96 (m, 2H), 7.84-7.71 (m, 2H), 7.60 (t, *J* = 8.0 Hz, 1H), 5.66-5.56 (m, 1H), 3.89-3.36 (m, 8H), 2.18 (s, 3H), 1.61 (d, *J* = 7.1 Hz, 3H). LC/MS(m/z, $MH^+$): 594.1.

**Example 23: (*R*)-4-((1-(3-(difluoromethyl)-2-fluorophenyl)ethyl)amino)-6-(1-(fluoromethyl) cyclopropyl)-8-(methoxy-$d_3$)-2-methylpyrido[4,3-*d*]pyrimidine-7(6*H*)-one**

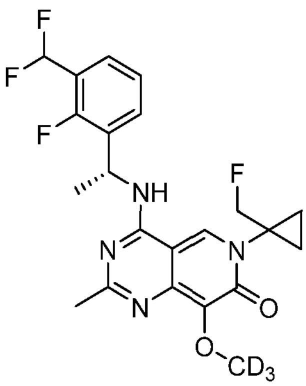

**Step 1: synthesis of methyl (*R*)-2-(6-((1-(3-(difluoromethyl)-2-fluorophenyl)ethyl)amino)-5-(1,3-dioxolan-2-yl)-2-methylpyrimidin-4-yl)acetate**

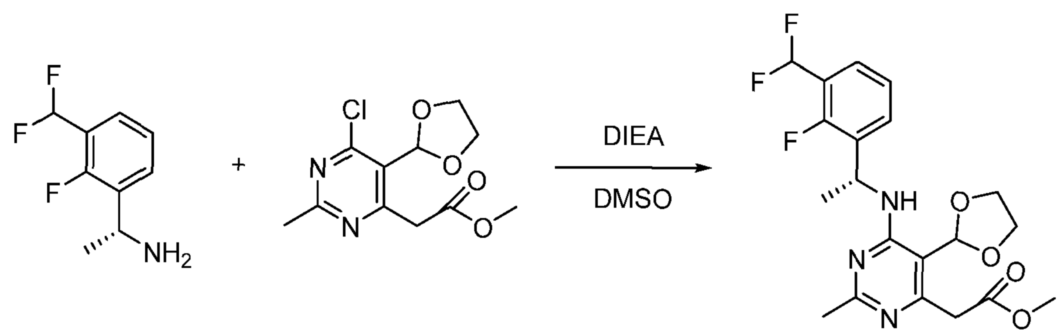


(*R*)-1-(3-(Difluoromethyl)-2-fluorophenyl)ethane-1-amine (3.05 g) and methyl 2-(6-chloro-5-(1,3-dioxolan-2-yl)-2-methylpyrimidin-4-yl)acetate (4.00 g) were dissolved in 20 mL of dimethyl sulfoxide. *N,N*-Diisopropylethylamine (2.27 g) was added, and the reaction was completed after the mixture was stirred at 80 °C for 10 h. Ethyl acetate was added, and the mixture was washed with water and washed with saturated brine. The organic phase was dried over anhydrous sodium sulfate, filtered, concentrated, and purified by column chromatography (petroleum ether/ethyl acetate gradient elution) to give the title compound.

**Step 2: synthesis of (*R*)-2-(6-((1-(3-(difluoromethyl)-2-fluorophenyl)ethyl)amino)-5-(1,3-dioxolan-2-yl)-2-methylpyrimidin-4-yl)-*N*-(1-(fluoromethyl)cyclopropyl)acetamide**

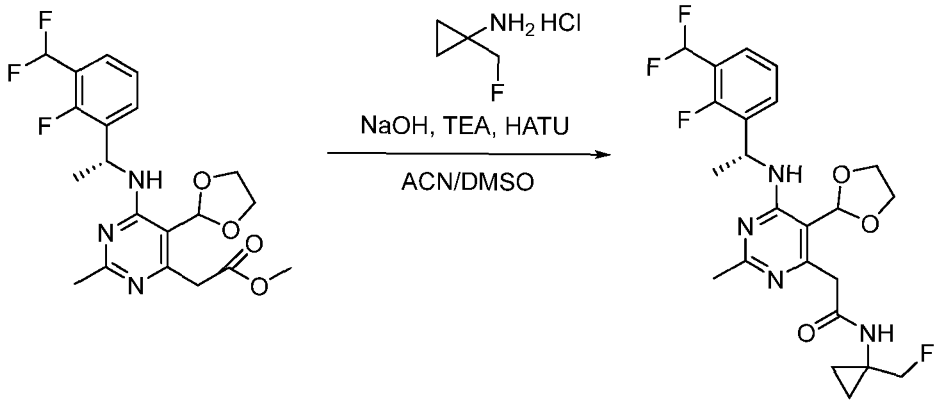


Methyl (R)-2-(6-((1-(3-(difluoromethyl)-2-fluorophenyl)ethyl)amino)-5-(1,3-dioxolan-2-yl)-2-methylpyrimidin-4-yl)-acetate (300 mg) was dissolved in 3 mL of dimethyl sulfoxide and 1.5 mL of acetonitrile. A 20% aqueous sodium hydroxide solution (465 μL) was added, and the mixture was stirred at room temperature for 30 min. Then triethylamine (390 μL), 1-(fluoromethyl)cyclopropane-1-amine hydrochloride (100 mg), and 2-(7-azabenzotriazol-1-yl)-*N,N,N',N'*-tetramethyluronium hexafluorophosphate (399 mg) were added. The reaction was completed after the mixture was stirred at room temperature for 30 min. Dichloromethane was added, and the mixture was washed with water and washed with saturated brine. The organic phase was dried over anhydrous sodium sulfate, filtered, concentrated, and purified by column chromatography (petroleum ether/ethyl acetate gradient elution) to give the title compound.

**Step 3: synthesis of 2-bromo-2-(6-((*R*)-1-(3-(difluoromethyl)-2-fluorophenyl)ethyl)amino)-5-(1,3-dioxolan-2-yl)-2-methylpyrimidin-4-yl)-*N*-(1-(fluoromethyl)cyclopropyl)acetamide**

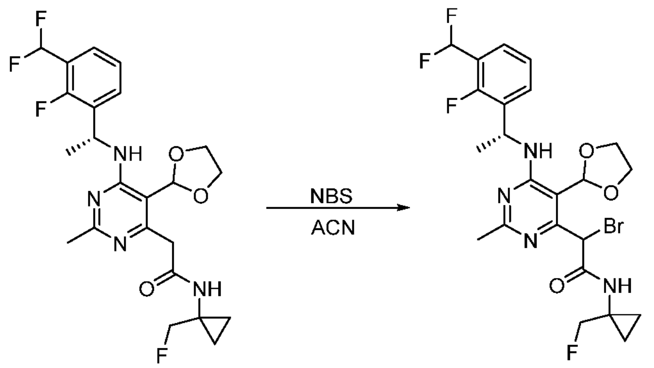


(*R*)-2-(6-((1-(3-(Difluoromethyl)-2-fluorophenyl)ethyl)amino)-5-(1,3-dioxolan-2-yl)-2-methylpyrimidin-4-yl)-*N*-(1-(fluoromethyl)cyclopropyl)acetamide (160 mg) was dissolved in acetonitrile (3 mL). A solution (1 mL) of *N*-bromosuccinimide (62.0 mg) in acetonitrile was slowly added dropwise, and the mixture was reacted at room temperature for 30 min. The reaction mixture was concentrated and purified by column chromatography (petroleum ether/ethyl acetate gradient elution) to give the title compound.

**Step 4: synthesis of 2-(6-((*R*)-1-(3-(difluoromethyl)-2-fluorophenyl)ethyl)amino)-5-(1,3-dioxolan-2-yl)-2-methylpyrimidin-4-yl)-*N*-(1-(fluoromethyl)cyclopropyl)-2-(methoxy-$d_3$)acetamide**

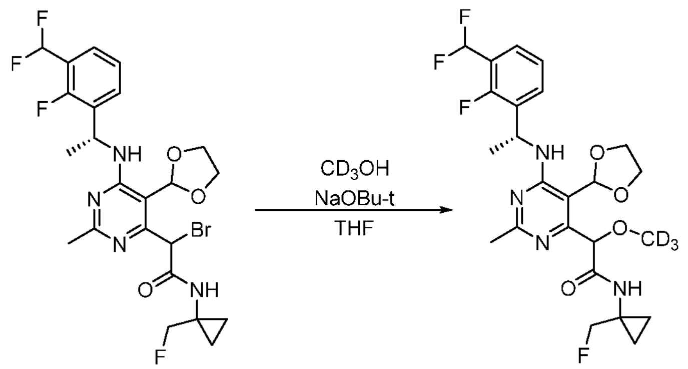


2-Bromo-2-(6-((*R*)-1-(3-(difluoromethyl)-2-fluorophenyl)ethyl)amino)-5-(1,3-dioxolan-2-yl)-2-methylpyrimidin-4-yl)-*N*-(1-(fluoromethyl)cyclopropyl)acetamide (85.0 mg) was added to a reaction flask and dissolved in anhydrous tetrahydrofuran (1.5 mL). Methanol-$d_3$ ($CD_3OH$) (54.6 mg) and sodium *tert*-butoxide (72.8 mg) were added. The reaction was completed after the mixture was stirred at room temperature for reaction for 1 h under argon atmosphere. The reaction mixture was quenched with water and extracted with ethyl acetate. The organic phases were combined, washed with saturated brine, dried over anhydrous sodium sulfate, and filtered. The filtrate was concentrated under reduced pressure to give the title compound, which was directly used in the next step.

**Step 5: synthesis of (*R*)-4-((1-(3-(difluoromethyl)-2-fluorophenyl)ethyl)amino)-6-(1-(fluoromethyl)cyclopropyl)-8-(methoxy-$d_3$)-2-methylpyrido[4,3-*d*]pyrimidine-7(6*H*)-one**

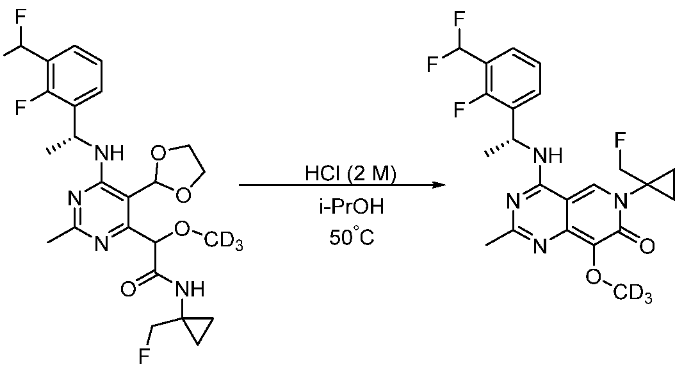


2-(6-((R)-1-(3-(Difluoromethyl)-2-fluorophenyl)ethyl)amino)-5-(1,3-dioxolan-2-yl)-2-methylpyrimidin-4-yl)-*N*-(1-(fluoromethyl)cyclopropyl)-2-(methoxy-$d_3$)acetamide (80.0 mg) was added to a reaction flask. Isopropanol (1.4 mL) was added, and then diluted hydrochloric acid (2 M, 0.2 mL) was added. The mixture was heated to 50 °C and reacted overnight. The reaction mixture was concentrated and purified by preparative HPLC (mobile phase: acetonitrile/water gradient elution) to give the title compound.

$^1$H NMR (400 MHz, DMSO-$d_6$) δ 8.96 (s, 1H), 8.83 (d, *J* = 7.1 Hz, 1H), 7.64 (t, *J* = 7.3 Hz, 1H), 7.52 (t, *J* = 6.9 Hz, 1H), 7.32 (t, *J* = 7.7 Hz, 1H), 7.24 (t, *J* = 54.4 Hz, 1H), 5.79-5.71 (m, 1H), 4.80-4.46 (m, 2H), 2.21 (s, 3H), 1.58 (d, *J* = 7.1 Hz, 3H), 1.37-1.20 (m, 4H). LC/MS(m/z, $MH^+$): 454.1.

**Example 24: (*R*)-4-((1-(2-methyl-3-nitrophenyl)ethyl)amino)-8-methoxy-2-methyl-6-morpholinepyrido[4,3-*d*]pyrimidine-7(6*H*)-one**

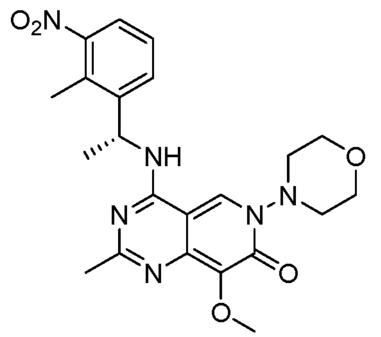

The title compound was prepared in the same manner as in Example **20** except for replacing (*R*)-1-(3-(difluoromethyl)-2-fluorophenyl)ethane-1-amine in step 1 with (*R*)-1-(2-methyl-3-nitrophenyl)ethane-1-amine.

$^1$H NMR (400 MHz, DMSO-$d_6$) δ 9.01 (s, 1H), 8.91 (d, *J* = 6.9 Hz, 1H), 7.75 (d, *J* = 7.7 Hz, 1H), 7.68 (d, *J* = 7.9 Hz, 1H), 7.43 (t, *J* = 7.9 Hz, 1H), 5.70-5.61 (m, 1H), 4.14-3.45 (m, 11H), 2.50 (s, 3H), 2.24 (s, 3H), 1.52 (d, *J* = 6.9 Hz, 3H). LC/MS(m/z, MH$^+$): 455.2.

**Example 25: (*R*)-8-methoxy-2-methyl-6-morpholine-4-((1-(3-(pentafluoro-)λ$^6$-sulfanyl)phenyl)) ethyl)amino)pyrido[4,3-*d*]pyrimidine-7(6*H*)-one**

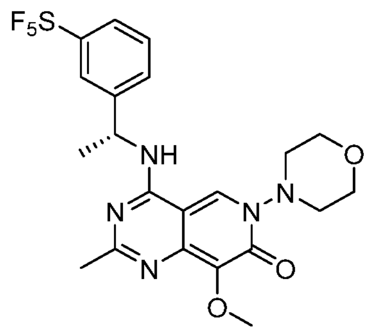

The title compound was prepared in the same manner as in Example **20** except for replacing (*R*)-1-(3-(difluoromethyl)-2-fluorophenyl)ethane-1-amine in step **1** with (*R*)-1-(3-(pentafluoro-λ$^6$-sulfanyl)phenyl)ethane-1 -amine.

$^1$H NMR (400 MHz, DMSO-$d_6$) δ 8.95 (s, 1H), 8.80 (d, *J* = 7.4 Hz, 1H), 7.97 (s, 1H), 7.78 (dd, *J* = 8.3, 1.6 Hz, 1H), 7.72 (d, *J* = 7.8 Hz, 1H), 7.59 (t, *J* = 7.9 Hz, 1H), 5.62-5.53 (m, 1H), 4.11-3.45 (m, 11H), 2.26 (s, 3H), 1.58 (d, *J* = 7.1 Hz, 3H). LC/MS(m/z, MH$^+$): 522.2.

**Example 26 (*R*)-4-((1-(3-(difluoromethyl)-2-fluorophenyl)ethyl)amino)-6-(1-(fluoromethyl) cyclopropyl)-2-methyl-7-oxo-6,7-dihydropyrido[4,3-*d*]pyrimidine-8-carbonitrile**

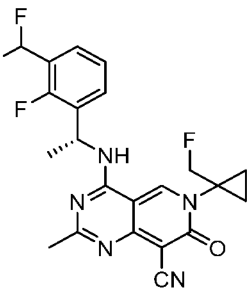

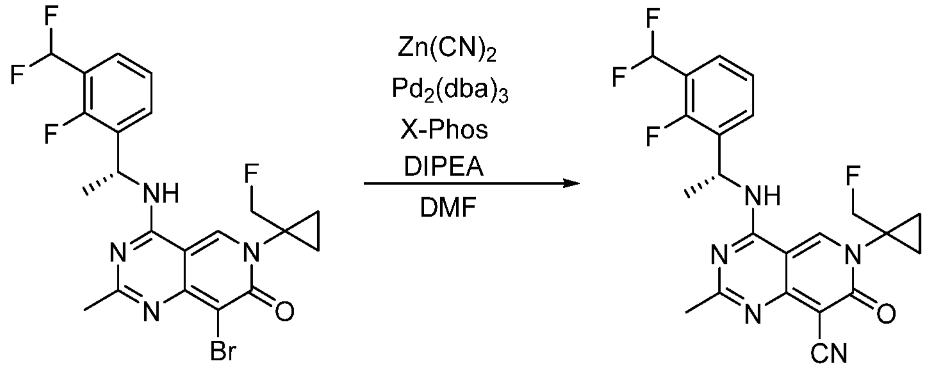


(*R*)-8-Bromo-4-((1-(3-(difluoromethyl)-2-fluorophenyl)ethyl)amino)-6-(1-(fluoromethyl)cyclopropyl)-2-methylpyrido[4,3-*d*]pyrimidine-7(6*H*)-one (40.0 mg), zinc cyanide (18.8 mg), tris(dibenzylideneacetone)dipalladium(0) (4.61 mg), 2-dicyclohexylphosphonium-2,4,6-triisopropylbiphenyl (7.64 mg), and *N*,*N*-diisopropylethylamine (20.7 mg) were added to 1 mL of *N,N*-dimethylformamide. The reaction was completed after the mixture was heated to 100 °C and reacted for 4 h under argon atmosphere. The reaction mixture was poured into water and extracted with ethyl acetate. The organic layers were combined, dried over anhydrous sodium sulfate, filtered, concentrated, and purified by column chromatography (dichloromethane/methanol gradient elution) to give the title compound.

$^1$H NMR (400 MHz, $CDCl_3$) δ 9.13 (s, 1H), 8.02 (d, *J* = 6.9 Hz, 1H), 7.60 (t, *J* = 7.4 Hz, 1H), 7.44 (t, *J* = 7.2 Hz, 1H), 7.13 (t, *J* = 7.7 Hz, 1H), 6.88 (t, *J* = 55.0 Hz, 1H), 5.90-5.81 (m, 1H), 4.69-4.26 (m, 2H), 2.40 (s, 3H), 1.68 (d, *J* = 7.0 Hz, 3H), 1.45-1.24 (m, 4H). LC/MS(m/z, $MH^+$): 446.2.

**Example 27 (*R*)-8-(difluoromethyl)-4-((1-(3-(difluoromethyl)-2-fluorophenyl)ethyl)amino)-6-(1-(fluoromethyl)cyclopropyl)-2-methylpyrido[4,3-*d*]pyrimidine-7(6*H*)-one**

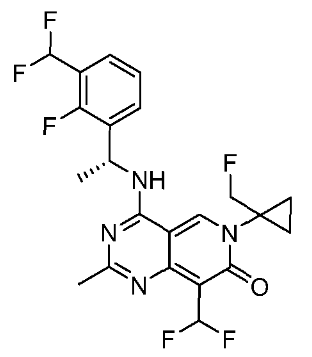

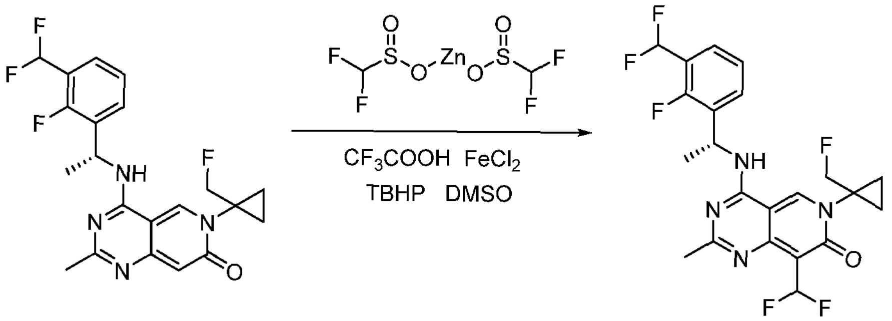


(*R*)-4-((1-(3-(Difluoromethyl)-2-fluorophenyl)ethyl)amino)-6-(1-(fluoromethyl)cyclopropyl)-2-methylpyrido[4,3-*d*]pyrimidine-7(6*H*)-one (80.0 mg) was added to 1 mL of dimethyl sulfoxide. Trifluoroacetic acid (21.7 mg) was added. Then ferrous chloride (12.06 mg) and bis((difluoromethyl)sulfinyl)oxy)zinc (140.6 mg) were added. Finally *tert*-butyl hydroperoxide (23.71 mg) was added. The reaction was completed after the mixture was reacted at room temperature for 3 h. The reaction mixture was poured into water and extracted with dichloromethane. The organic layers were combined, dried over anhydrous sodium sulfate, filtered, concentrated, and purified by column chromatography (dichloromethane/methanol gradient elution) to give the title compound.

$^1H$ NMR (400 MHz, DMSO-$d_6$) δ 9.37 (s, 1H), 9.08 (d, *J* = 7.0 Hz, 1H), 7.66 (t, *J* = 7.5 Hz, 1H), 7.53 (t, *J* = 7.1 Hz, 1H), 7.44 (t, *J* = 54.4 Hz, 1H), 7.33 (t, *J* = 7.9 Hz, 1H), 7.24 (t, *J* = 54.3 Hz, 1H), 5.81-5.71 (m, 1H), 4.81-4.47 (m, 2H), 2.23 (s, 3H), 1.59 (d, *J* = 7.0 Hz, 3H), 1.41 - 1.21 (m, 4H). LC/MS(m/z, $MH^+$): 471.2.

**Example 28 (*R*)-4-((1-(3-(difluoromethyl)-2-fluorophenyl)ethyl)amino)-6-(1-(fluoromethyl) cyclopropyl)-2-methyl-8-vinylpyrido[4,3-*d*]pyrimidine-7(6*H*)-one**

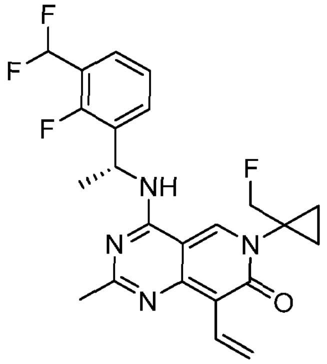

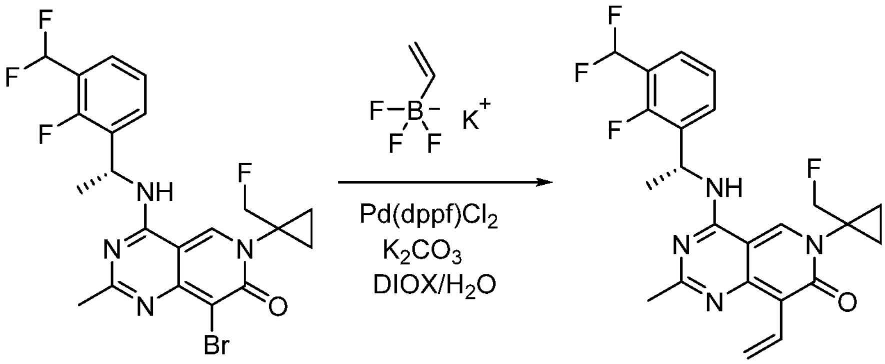


(*R*)-4-((1-(3-(Difluoromethyl)-2-fluorophenyl)ethyl)amino)-6-(1-(fluoromethyl)cyclopropyl)-8-bromo-2-methylpyrido[4,3-*d*]pyrimidine-7(6*H*)-one (70.0 mg), potassium vinyltrifluoroborate (25.8 mg), 1,1'-bis(diphenylphosphino)ferrocene dichloropalladium (9.38 mg), and potassium carbonate (35.4 mg) were added to 1 mL of dioxane and 0.2 mL of water. The reaction was completed after the mixture was heated to 100 °C and reacted for 2 h under argon atmosphere. The reaction mixture was directly concentrated and purified by column chromatography (dichloromethane/methanol gradient elution) to give the title compound.

$^1$H NMR (400 MHz, DMSO-*d$_6$*) δ 9.22 (s, 1H), 8.97 (d, *J* = 6.9 Hz, 1H), 7.73 (t, *J* = 7.0 Hz, 1H), 7.62-7.57 (m, 2H), 7.39 (t, *J* = 7.7 Hz, 1H), 7.31 (t, *J* = 54.4 Hz, 1H), 6.71 (dd, *J* = 17.8, 3.5 Hz, 1H), 5.88-5.80 (m, 1H), 5.35 (dd, *J* = 12.1, 3.5 Hz, 1H), 4.90-4.52 (m, 2H), 2.31 (s, 3H), 1.66 (d, *J* = 6.9 Hz, 3H), 1.46-1.28 (m, 4H). LC/MS(m/z, MH$^+$): 447.2.

**Example 29 (*R*)-6-(1-(fluoromethyl)cyclopropyl)-8-methoxy-2-methyl-4-((1-(2-methyl-3-nitrophenyl)ethyl)amino)pyrido[4,3-*d*]pyrimidine-7(6*H*)-one**

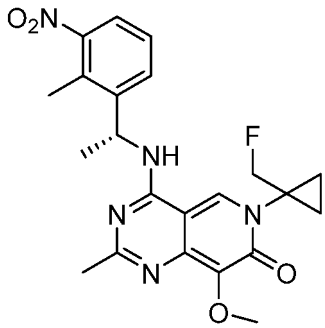

The title compound was prepared in the same manner as in Example **3** except for replacing (*R*)-3-(1-aminoethyl)-2-tolunitrile hydrochloride in step 1 with (1*R*)-1-(2-methyl-3-nitrophenyl)ethylamine hydrochloride.

$^1$H NMR (400 MHz, DMSO-*d$_6$*) δ 8.94 (s, 1H), 8.91 (d, *J*= 6.4 Hz, 1H), 7.72 (d, *J*= 7.9 Hz, 1H), 7.68 (d, *J*= 7.9 Hz, 1H), 7.43 (t, *J*= 7.9 Hz, 1H), 5.70-5.61 (m, 1H), 4.81-4.46 (m, 2H), 3.76 (s, 3H), 2.50 (s, 3H), 2.22 (s, 3H), 1.55 (d, *J*= 7.0 Hz, 3H), 1.37-1.24 (m, 4H). LC/MS(m/z, MH$^+$): 442.2.

**Example 30 (*R*)-6-(1-(fluoromethyl)cyclopropyl)-8-(methoxy-d3)-2-methyl-4-((1-(2-methyl-3-nitrophenyl)ethyl)amino)pyrido[4,3-*d*]pyrimidine-7(6*H*)-one**

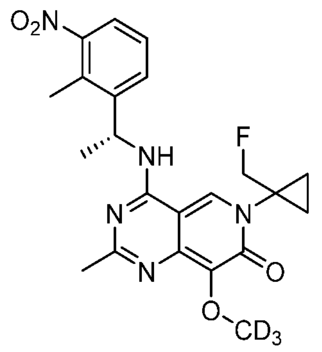

The title compound was prepared in the same manner as in Example **23** except for replacing methyl (*R*)-2-(6-((1-(3-(difluoromethyl)-2-fluorophenyl)ethyl)amino)-5-(1,3-dioxolan-2-yl)-2-methylpyrimidin-4-yl)-acetate in step **2** with methyl (*R*)-2-(5-(1,3-dioxolan-2-yl)-2-methyl-6-((1-(2-methyl-3-nitrophenyl)ethyl)amino)pyrimidin-4-yl)acetate.

$^1$H NMR (400 MHz, DMSO-$d_6$) δ 8.94 (s, 1H), 8.91 (d, *J* = 6.4 Hz, 1H), 7.72 (d, *J* = 7.9 Hz, 1H), 7.68 (d, *J* = 7.9 Hz, 1H), 7.43 (t, *J* = 7.9 Hz, 1H), 5.70-5.61 (m, 1H), 4.81-4.46 (m, 2H), 2.50 (s, 3H), 2.22 (s, 3H), 1.55 (d, *J* = 7.0 Hz, 3H), 1.37-1.24 (m, 4H). LC/MS(m/z, MH$^+$): 445.2.

**Example 31 (*R*)-6-(1-(fluoromethyl)cyclopropyl)-8-(methoxy-d3)-2-methyl-4-((1-(3-(pentafluoro-λ$^6$-sulfanyl)phenyl)ethyl)amino)pyrido[4,3-*d*]pyrimidine-7(6*H*)-one**

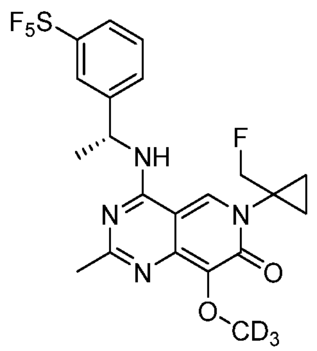

The title compound was prepared in the same manner as in Example **23** except for replacing methyl (*R*)-2-(6-((1-(3-(difluoromethyl)-2-fluorophenyl)ethyl)amino)-5-(1,3-dioxolan-2-yl)-2-methylpyrimidin-4-yl)-acetate in step **2** with methyl (*R*)-2-(5-(1,3-dioxolan-2-yl)-2-methyl-6-((1-(3-(pentafluoro-λ$^6$-sulfanyl)phenyl)ethyl)amino)pyrimidin-4-yl)acetate.

$^1$H NMR (400 MHz, DMSO-$d_6$) δ 8.89 (s, 1H), 8.81 (d, *J* = 7.3 Hz, 1H), 7.95 (s, 1H), 7.78 (d, *J* = 8.2 Hz, 1H), 7.70 (d, *J* = 7.7 Hz, 1H), 7.59 (t, *J* = 7.9 Hz, 1H), 5.65-5.55 (m, 1H), 4.81-4.46 (m, 2H), 2.25 (s, 3H), 1.60 (d, *J* = 7.0 Hz, 3H), 1.38-1.21 (m, 4H). LC/MS(m/z, MH$^+$): 512.1.

**Example 32 (*R*)-2-(1-(4-((1-(3-(difluoromethyl)-2-fluorophenyl)ethyl)amino)-8-methoxy-2-methyl-7-oxopyrido[4,3-*d*]pyrimidine-6(7*H*)-yl)cyclopropyl)acetonitrile**

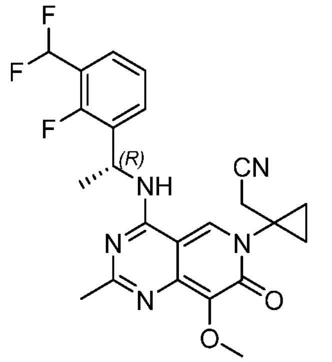

The title compound was prepared in the same manner as in Example **14** except for replacing 1-aminocyclopropanecarbonitrile hydrochloride in step **1** with 2-(1-aminocyclopropyl)acetonitrile hydrochloride.

$^1$HNMR (400 MHz, $CDCl_3$) δ 9.02 (s, 1H), 8.83 (d, *J* = 7.1 Hz, 1H), 7.63 (t, *J* = 7.3 Hz, 1H), 7.52 (t, *J* = 7.0 Hz, 1H), 7.31 (t, *J* = 7.7 Hz, 1H), 7.23 (t, *J* = 54.4 Hz, 1H), 5.82-5.73 (m, 1H), 3.78 (s, 3H), 3.27-3.10 (m, 2H), 2.22 (s, 3H), 1.59 (d, *J* = 7.1 Hz, 3H), 1.42-1.13 (m, 4H). LC/MS(m/z, $MH^+$): 458.2.

**Example 33 (*R*)-4-((1-(3-(difluoromethyl)-2-fluorophenyl)ethyl)amino)-6-(1-(fluoromethyl) cyclopropyl)-8-hydroxy-2-methylpyrido[4,3-*d*]pyrimidine-7(6*H*)-one**

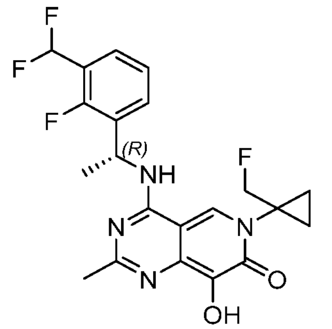

**Step 1: synthesis of 2-(6-((*R*)-1-(3-(difluoromethyl)-2-fluorophenyl)ethyl)amino)-5-(1,3-dioxolan-2-yl)-2-methylpyrimidin-4-yl)-*N*-(1-(fluoromethyl)cyclopropyl)-2-hydroxyacetamide**

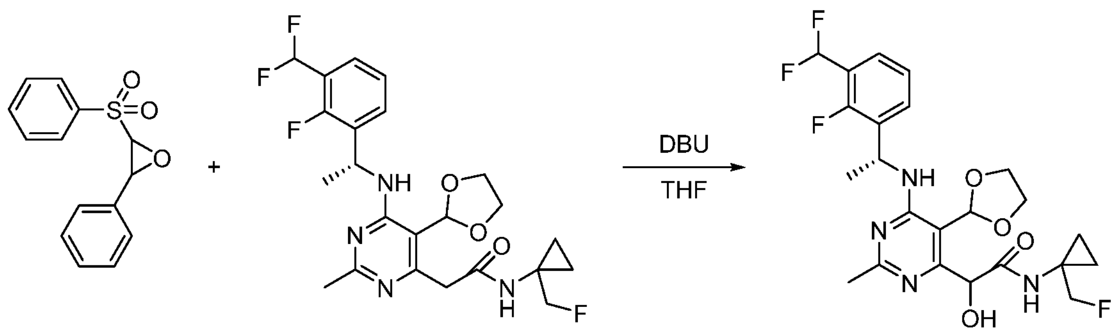


(*R*)-2-(6-((1-(3-(Difluoromethyl)-2-fluorophenyl)ethyl)amino)-5-(1,3-dioxolan-2-yl)-2-methylpyrimidin-4-yl)-*N*-(1-(fluoromethyl)cyclopropyl)acetamide (1.10 g) and 2-phenyl-3-(phenylsulfonyl)oxirane (714 mg) were dissolved in 10 mL of tetrahydrofuran. 1,8-Diazabicyclo[5.4.0]undec-7-ene (416 mg) was added. The reaction was completed after the mixture was stirred at room temperature overnight. The reaction mixture was concentrated and purified by column chromatography (petroleum ether/ethyl acetate gradient elution) to give the title compound.

**Step 2: synthesis of (*R*)-4-((1-(3-(difluoromethyl)-2-fluorophenyl)ethyl)amino)-6-(1-(fluoromethyl)cyclopropyl)-8-hydroxy-2-methylpyrido[4,3-*d*]pyrimidine-7(6*H*)-one**

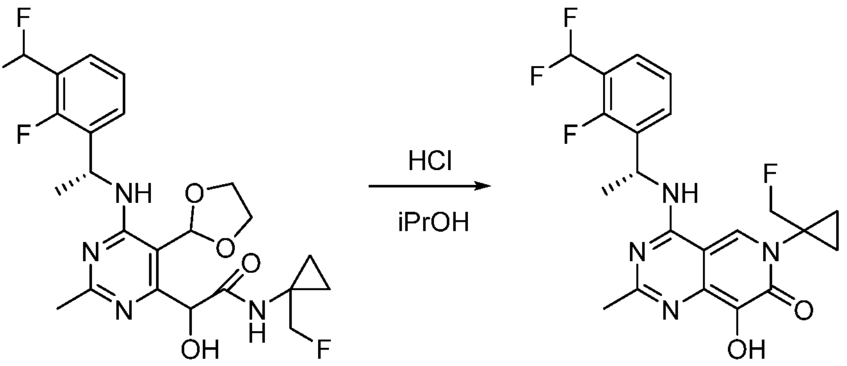


2-(6-((*R*)-1-(3-(Difluoromethyl)-2-fluorophenyl)ethyl)amino)-5-(1,3-dioxolan-2-yl)-2-methylpyrimidin-4-yl)-*N*-(1-(fluoromethyl)cyclopropyl)-2-hydroxyacetamide (460 mg) was dissolved in 5 mL of isopropanol. 1.85 mL of a 5 M hydrochloric acid solution was added, and the reaction was completed after the mixture was reacted at 50 °C for 1 h. 20 mL of water was added, and the mixture was extracted with dichloromethane. The organic phase was concentrated, slurried with dichloromethane, and subjected to suction filtration. The filter cake was dried to give the title compound.
$^1$H NMR (400 MHz, DMSO-$d_6$) δ 8.73 (d, *J* = 5.9 Hz, 1H), 8.66 (s, 1H), 7.66 (t, *J* = 7.3 Hz, 1H), 7.52 (t, *J* = 6.9 Hz, 1H), 7.33 (t, *J* = 7.7 Hz, 1H), 7.24 (t, *J* = 54.4 Hz, 1H), 5.81-5.71 (m, 1H), 4.82-4.50 (m, 2H), 2.22 (s, 3H), 1.59 (d, *J* = 7.1 Hz, 3H), 1.41-1.21 (m, 4H). LC/MS(m/z, MH$^+$): 437.2.

**Example 34: (*R*)-4-((1-(3-(difluoromethyl)-2-fluorophenyl)ethyl)amino)-6-(1-(fluoromethyl) cyclopropyl)-2-methyl-8-morpholinylpyrido[4,3-*d*]pyrimidine-7(6*H*)-one**

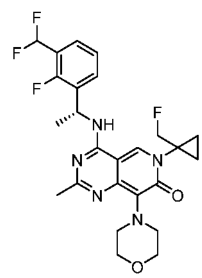

**Step 1: synthesis of 2-(6-(((*R*)-1-(3-(difluoromethyl)-2-fluorophenyl)ethyl)amino)-5-(1,3-dioxolan-2-yl)-2-methylpyrimidin-4-yl)-*N*-(1-(fluoromethyl)cyclopropyl)-2-morpholinylacetamide**

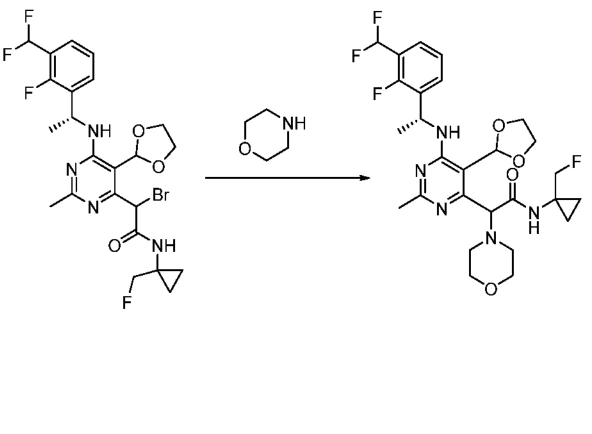

2-Bromo-2-(6-((*R*)-1-(3-(difluoromethyl)-2-fluorophenyl)ethyl)amino)-5-(1,3-dioxolan-2-yl)-2-methylpyrimidin-4-yl)-*N*-(1-(fluoromethyl)cyclopropyl)acetamide (100 mg) was added to a reaction flask. 1 mL of morpholine was added. The reaction was completed after the mixture was stirred at room temperature for 1 h. The reaction mixture was concentrated and purified by column chromatography (dichloromethane/methanol gradient elution) to give the title compound.

**Step 2: synthesis of (*R*)-4-((1-(3-(difluoromethyl)-2-fluorophenyl)ethyl)amino)-6-(1-(fluoromethyl)cyclopropyl)-2-methyl-8-morpholinylpyrido[4,3-*d*]pyrimidine-7(6*H*)-one**

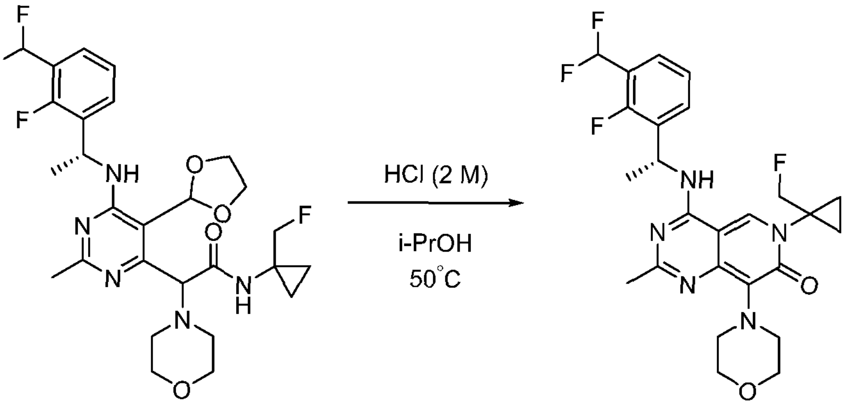


2-(6-(((*R*)-1-(3-(Difluoromethyl)-2-fluorophenyl)ethyl)amino)-5-(1,3-dioxolan-2-yl)-2-methylpyrimidin-4-yl)-*N*-(1-(fluoromethyl)cyclopropyl)-2-morpholinylacetamide (80.0 mg) was added to a reaction flask. Isopropanol (1.4 mL) was added, and then diluted hydrochloric acid (2 M, 0.2 mL) was added. The reaction was completed after the mixture was heated to 50 °C and stirred for 4 h. The reaction mixture was concentrated and purified by preparative HPLC (mobile phase: acetonitrile/water gradient elution) to give the title compound.

$^1H$ NMR (400 MHz, DMSO-$d_6$) δ 8.81 (s, 1H), 8.63 (d, *J* = 7.1 Hz, 1H), 7.62 (t, *J* = 7.3 Hz, 1H), 7.51 (t, *J* = 7.0 Hz, 1H), 7.31 (t, *J* = 7.7 Hz, 1H), 7.23 (t, *J* = 54.4 Hz, 1H), 5.74 (m, 1H), 4.80-4.45 (m, 2H), 3.63 (t, *J* = 4.6 Hz, 4H), 3.30-3.23 (m, 4H), 2.19 (s, 3H), 1.57 (d, *J* = 7.1 Hz, 3H), 1.36-1.20 (m, 4H). LC/MS(m/z, $MH^+$): 506.2.

**Example 35 (*R*)-8-(cyclopropylmethoxy)-4-((1-(3-(difluoromethyl)-2-fluorophenyl)ethyl)amino)-6-(1-(fluoromethyl)cyclopropyl)-2-methylpyrido[4,3-*d*]pyrimidine-7(6*H*)-one**

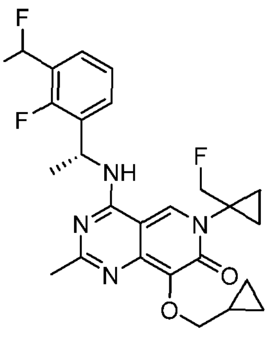

The title compound was prepared in the same manner as in Example **23** except for replacing methanol-$d_3$ in step **4** with hydroxymethylcyclopropane.

$^1H$ NMR (400 MHz, DMSO-$d_6$) δ 8.93 (s, 1H), 8.77 (d, *J* = 7.1 Hz, 1H), 7.64 (t, *J* = 7.4 Hz, 1H), 7.52 (t, *J* = 7.0 Hz, 1H), 7.32 (t, *J* = 7.7 Hz, 1H), 7.23 (t, *J* = 54.4 Hz, 1H), 5.75 (m, 1H), 4.84-4.45 (m, 2H), 3.86 (d, *J* = 6.9 Hz, 2H), 2.21 (s, 3H), 1.58 (d, *J* = 7.1 Hz, 3H), 1.37-1.20 (m, 4H), 1.18-1.08 (m, 1H), 0.46-0.38 (m, 2H), 0.27-0.19 (m, 2H). LC/MS(m/z, $MH^+$): 491.2.

**Example 36 (*R*)-4-((1-(3-(difluoromethyl)-2-fluorophenyl)ethyl)amino)-6-(1-(fluoromethyl) cyclopropyl)-8-isopropoxy-2-methylpyrido[4,3-*d*]pyrimidine-7(6*H*)-one**

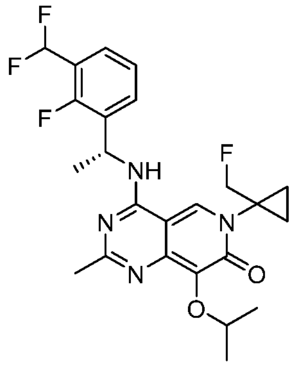

The title compound was prepared in the same manner as in Example **23** except for replacing methanol-$d_3$ in step **4** with isopropanol.

$^1$H NMR (400 MHz, DMSO-$d_6$) δ 8.91 (s, 1H), 8.76 (d, *J* = 7.1 Hz, 1H), 7.65 (t, *J* = 7.3 Hz, 1H), 7.52 (t, *J* = 6.9 Hz, 1H), 7.32 (t, *J* = 7.7 Hz, 1H), 7.23 (t, *J* = 54.4 Hz, 1H), 5.76 (m, 1H), 4.79-4.48 (m, 3H), 2.20 (s, 3H), 1.58 (d, *J* = 7.1 Hz, 3H), 1.38-1.22 (m, 4H), 1.17 (dd, *J* = 6.2, 3.6 Hz, 6H). LC/MS (m/z, MH$^+$): 479.2.

**Example 37 (*R*)-8-(3,3-difluorocyclobutoxy)-4-((1-(3-(difluoromethyl)-2-fluorophenyl)ethyl) amino)-6-(1-(fluoromethyl)cyclopropyl)-2-methylpyrido[4,3-*d*]pyrimidine-7(6*H*)-one**

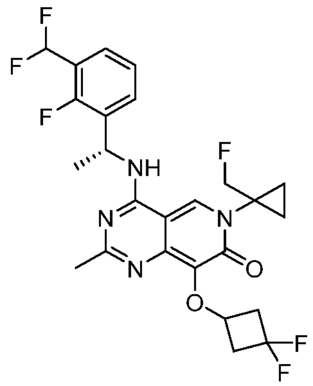

The title compound was prepared in the same manner as in Example **23** except for replacing methanol-$d_3$ in step **4** with 3,3-difluorocyclobutanol.

$^1$H NMR (400 MHz, DMSO-$d_6$) δ 9.00 (s, 1H), 8.86 (d, *J* = 7.0 Hz, 1H), 7.65 (t, *J* = 7.3 Hz, 1H), 7.52 (t, *J* = 7.0 Hz, 1H), 7.32 (t, *J* = 7.7 Hz, 1H), 7.23 (t, *J* = 54.4 Hz, 1H), 5.76 (m, 1H), 4.91-4.77 (m, 1H), 4.77-4.49 (m, 2H), 2.92-2.79 (m, 4H), 2.22 (s, 3H), 1.58 (d, *J* = 7.1 Hz, 3H), 1.47-1.21 (m, 4H). LC/MS(m/z, MH$^+$): 527.2.

**Example 38 (*R*)-4-((1-(3-(difluoromethyl)-2-fluorophenyl)ethyl)amino)-6-(1-(fluoromethyl) cyclopropyl)-2-methyl-8-(4-methylpiperazin-1-yl)pyrido[4,3-*d*]pyrimidine-7(6*H*)-one**

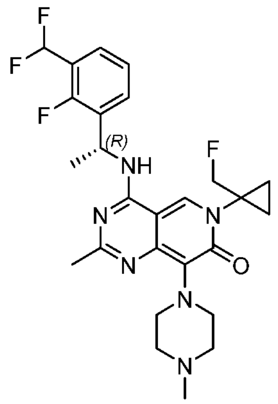

The title compound was prepared in the same manner as in Example **34** except for replacing morpholine in step **1** with *N*-methylpiperazine.

$^1$H NMR (400 MHz, DMSO-$d_6$) δ 8.78 (s, 1H), 8.62 (d, *J* = 7.1 Hz, 1H), 7.62 (t, *J* = 7.4 Hz, 1H), 7.51 (t, *J* = 6.8 Hz, 1H), 7.31 (t, *J* = 7.7 Hz, 1H), 7.23 (t, *J* = 54.4 Hz, 1H), 5.80-5.69 (m, 1H), 4.80-4.46 (m, 2H), 3.31-3.24 (m, 4H), 2.45-2.31 (m, 4H), 2.21 (s, 3H), 2.18 (s, 3H), 1.57 (d, *J* = 7.1 Hz, 3H), 1.35-1.19 (m, 4H). LC/MS(m/z, MH$^+$): 519.2.

**Example 39 4-(((*R*)-1-(3-(difluoromethyl)-2-fluorophenyl)ethyl)amino)-6-(1-(fluoromethyl) cyclopropyl)-2-methyl-8-((*R*)-3-methylmorpholine)pyrido[4,3-*d*]pyrimidine-7(6*H*)-one**

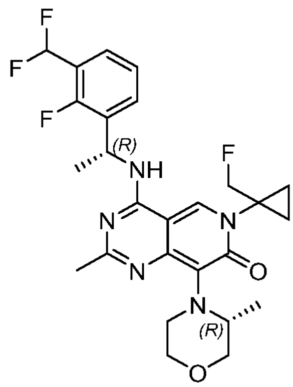

The title compound was prepared in the same manner as in Example **34** except for replacing morpholine in step **1** with 3-(*R*)-3-methylmorpholine.

$^1$H NMR (400 MHz, DMSO-$d_6$) δ 8.94 (s, 1H), 8.71 (d, *J* = 7.1 Hz, 1H), 7.65 (t, *J* = 7.2 Hz, 1H), 7.52 (t, *J* = 6.8 Hz, 1H), 7.32 (t, *J* = 7.7 Hz, 1H), 7.23 (t, *J* = 54.4 Hz, 1H), 5.76 (m, 1H), 4.63 (m, 2H), 4.01-3.85 (m, 1H), 3.76-3.53 (m, 3H), 3.22-2.96 (m, 3H), 2.21 (s, 3H), 1.57 (d, *J* = 7.1 Hz, 3H), 1.37-1.17 (m, 4H), 0.68 (d, *J* = 6.3 Hz, 3H). LC/MS(m/z, MH$^+$): 520.2.

**Example 40 4-(((*R*)-1-(3-(difluoromethyl)-2-fluorophenyl)ethyl)amino)-6-(1-(fluoromethyl) cyclopropyl)-2-methyl-8-((*S*)-3-methylmorpholine)pyrido[4,3-*d*]pyrimidine-7(6*H*)-one**

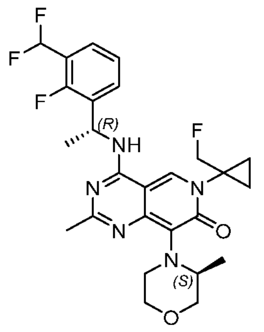

The title compound was prepared in the same manner as in Example **34** except for replacing morpholine in step **1** with 3-(*S*)-3-methylmorpholine.

$^1$H NMR (400 MHz, DMSO-$d_6$) δ 8.95 (s, 1H), 8.71 (d, *J* = 7.0 Hz, 1H), 7.64 (t, *J* = 7.2 Hz, 1H), 7.51 (t, *J* = 7.0 Hz, 1H), 7.31 (t, *J* = 7.6 Hz, 1H), 7.23 (t, *J* = 54.4 Hz, 1H), 5.80-5.69 (m, 1H), 4.87-4.38 (m, 2H), 4.01-3.87 (m, 1H), 3.73-3.55 (m, 3H), 3.24-2.95 (m, 3H), 2.20 (s, 3H), 1.57 (d, *J* = 7.1 Hz, 3H), 1.40-1.17 (m, 4H), 0.69 (d, *J* = 6.4 Hz, 3H). LC/MS(m/z, MH$^+$): 520.2.

**Example 41 4-(((*R*)-1-(3-(difluoromethyl)-2-fluorophenyl)ethyl)amino)-8-((2*S*,6*R*)-2,6-dimethylmorpholino)-6-(1-(fluoromethyl)cyclopropyl)-2-methylpyrido[4,3-*d*]pyrimidine-7(6*H*)-one**

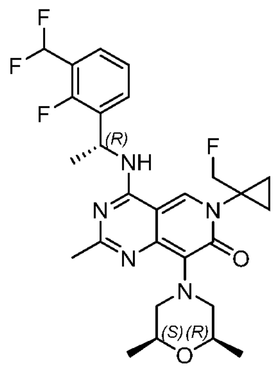

The title compound was prepared in the same manner as in Example **34** except for replacing morpholine in step **1** with (2*S*,6*R*)-2,6-dimethylmorpholine.

$^1$H NMR (400 MHz, DMSO-$d_6$) δ 8.79 (s, 1H), 8.62 (d, *J* = 7.1 Hz, 1H), 7.62 (t, *J* = 7.2 Hz, 1H), 7.51 (t, *J* = 6.9 Hz, 1H), 7.31 (t, *J* = 7.7 Hz, 1H), 7.23 (t, *J* = 54.4 Hz, 1H), 5.74 (m, 1H), 4.79-4.45 (m, 2H), 3.76-3.58 (m, 2H), 3.18 (t, *J* = 10.9 Hz, 2H), 2.94 (dd, *J* = 22.2, 11.8 Hz, 2H), 2.18 (s, 3H), 1.57 (d, *J* = 7.1 Hz, 3H), 1.34-1.20 (m, 4H), 1.03 (dd, *J* = 6.2, 2.0 Hz, 6H). LC/MS(m/z, MH$^+$): 534.2.

**Example 42 4-(((*R*)-1-(3-(difluoromethyl)-2-fluorophenyl)ethyl)amino)-6-(1-(fluoromethyl) cyclopropyl)-2-methyl-8-((*R*)-2-methylmorpholine)pyrido[4,3-*d*]pyrimidine-7(6*H*)-one**

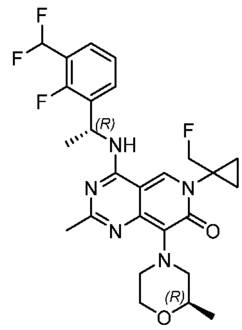

The title compound was prepared in the same manner as in Example **34** except for replacing morpholine in step **1** with (*R*)-2-methylmorpholine.

$^1H$ NMR (400 MHz, DMSO-$d_6$) δ 8.80 (s, 1H), 8.63 (d, *J* = 7.1 Hz, 1H), 7.62 (t, *J* = 7.3 Hz, 1H), 7.51 (t, *J* = 7.0 Hz, 1H), 7.31 (t, *J* = 7.7 Hz, 1H), 7.23 (t, *J* = 54.4 Hz, 1H), 5.78-5.69 (m, 1H), 4.81-4.38 (m, 2H), 3.75-3.70 (m, 1H), 3.66-3.55 (m, 2H), 3.40-3.33 (m, 1H), 3.22-3.07 (m, 2H), 3.07-2.95 (m, 1H), 2.19 (s, 3H), 1.57 (d, *J* = 7.1 Hz, 3H), 1.33-1.20 (m, 4H), 1.03 (d, *J* = 6.2 Hz, 3H). LC/MS(m/z, $MH^+$): 520.2.

**Example 43 4-(((*R*)-1-(3-(difluoromethyl)-2-fluorophenyl)ethyl)amino)-6-(1-(fluoromethyl) cyclopropyl)-2-methyl-8-((*S*)-2-methylmorpholine)pyrido[4,3-*d*]pyrimidine-7(6*H*)-one**

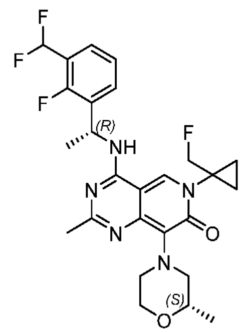

The title compound was prepared in the same manner as in Example **34** except for replacing morpholine in step **1** with (*S*)-2-methylmorpholine.

$^1H$ NMR (400 MHz, DMSO-$d_6$) δ 8.80 (s, 1H), 8.63 (d, *J* = 7.1 Hz, 1H), 7.62 (t, *J* = 7.3 Hz, 1H), 7.51 (t, *J* = 7.0 Hz, 1H), 7.31 (t, *J* = 7.7 Hz, 1H), 7.23 (t, *J* = 54.4 Hz, 1H), 5.74 (m, 1H), 4.78-4.47 (m, 2H), 3.75-3.69 (m, 1H), 3.67-3.55 (m, 2H), 3.38 (td, *J* = 12.0, 3.1 Hz, 1H), 3.14 (dd, *J* = 48.0, 12.0 Hz, 2H), 3.03-2.94 (m, 1H), 2.19 (s, 3H), 1.57 (d, *J* = 7.1 Hz, 3H), 1.36-1.18 (m, 4H), 1.03 (d, *J* = 6.2 Hz,3H). LC/MS(m/z, $MH^+$): 520.2.

**Example 44 8-(3-oxa-8-azabicyclo[3.2.1]oct-8-yl)-4-(((*R*)-1-(3-(difluoromethyl)-2-fluorophenyl) ethyl)amino)-6-(1-(fluoromethyl)cyclopropyl)-2-methylpyrido[4,3-*d*]pyrimidine-7(6*H*)-one**

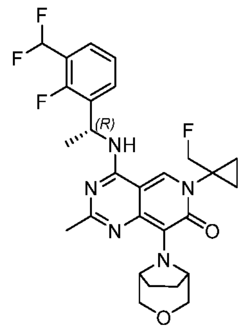

The title compound was prepared in the same manner as in Example **34** except for replacing morpholine in step **1** with 3-oxa-8-azabicyclo[3.2.1]octane.

$^1$H NMR (400 MHz, DMSO-*d*$_6$) δ 8.56 (s, 1H), 8.51 (d, *J* = 7.1 Hz, 1H), 7.62 (t, *J* = 7.1 Hz, 1H), 7.51 (t, *J* = 7.0 Hz, 1H), 7.31 (t, *J* = 7.7 Hz, 1H), 7.23 (t, *J* = 54.4 Hz, 1H), 5.79-5.70 (m, 1H), 4.81-4.45 (m, 4H), 3.80 (d, *J* = 10.3 Hz, 2H), 3.47 (d, *J* = 10.4 Hz, 2H), 2.16 (s, 3H), 1.94-1.82 (m, 4H), 1.57 (d, *J* = 7.1 Hz, 3H), 1.35-1.20 (m, 3H). LC/MS(m/z, MH$^+$): 532.2.

**Example 45 (*R*)-4-((1-(3-(difluoromethyl)-2-fluorophenyl)ethyl)amino)-6-(1-(fluoromethyl) cyclopropyl)-8-(4-hydroxy-4-methylpiperidin-1-yl)-2-methylpyrido[4,3-*d*]pyrimidine-7(6*H*)-one**

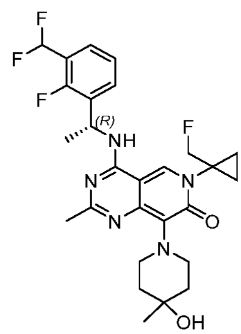

The title compound was prepared in the same manner as in Example **34** except for replacing morpholine in step **1** with 4-hydroxy-4-methylpiperidine.

$^1$H NMR (400 MHz, DMSO-*d*$_6$) δ 8.72 (s, 1H), 8.56 (d, *J* = 7.1 Hz, 1H), 7.62 (t, *J* = 7.1 Hz, 1H), 7.51 (t, *J* = 6.8 Hz, 1H), 7.31 (t, *J* = 7.7 Hz, 1H), 7.23 (t, *J* = 54.4 Hz, 1H), 5.74 (m, 1H), 4.85-4.44 (m, 2H), 4.13 (s, 1H), 3.43-3.34 (m, 2H), 3.18-3.08 (m, 2H), 2.18 (s, 3H), 1.57 (d, *J* = 7.1 Hz, 3H), 1.54-1.45 (m, 4H), 1.35-1.18 (m, 4H), 1.14 (s, 3H). LC/MS(m/z, MH$^+$): 534.2.

**Example 46 (*R*)-6-(1-(fluoromethyl)cyclopropyl)-2-methyl-8-morpholino-4-((1-(3-(pentafluoro-λ$^6$-sulfanyl)phenyl)ethyl)amino)pyrido[4,3-*d*]pyrimidine-7(6*H*)-one**

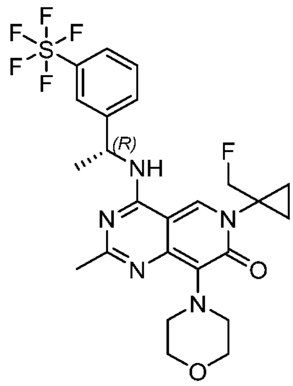

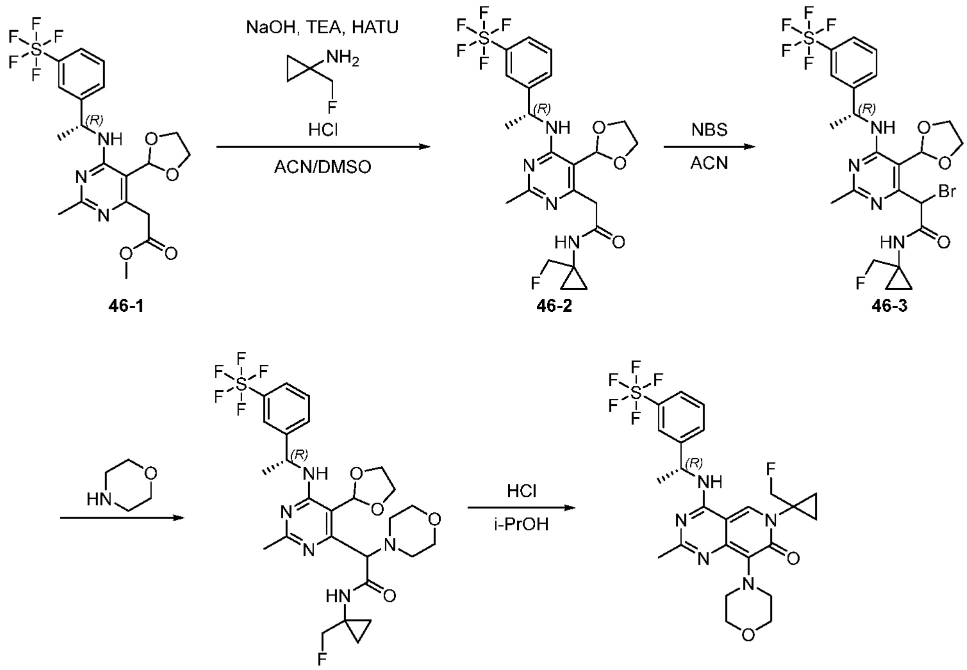


46-1 46-2 46-3

46-4 46-5

For the synthesis of **46-3,** reference was made to step **2** and step **3** of Example **23.** For the synthesis of **46-5,** reference was made to Example **34.** The title compound was prepared by the above-mentioned technical route.

$^1$H NMR (400 MHz, DMSO-$d_6$) δ 8.75 (s, 1H), 8.65 (d, *J* = 7.4 Hz, 1H), 7.94 (s, 1H), 7.77 (dd, *J* = 8.2, 1.6 Hz, 1H), 7.69 (d, *J* = 7.8 Hz, 1H), 7.58 (t, *J* = 8.0 Hz, 1H), 5.59 (m, 1H), 4.77-4.47 (m, 2H), 3.66-3.59 (m, 4H), 3.30-3.25 (m, 4H), 2.23 (s, 3H), 1.59 (d, *J* = 7.1 Hz, 3H), 1.34-1.21 (m, 4H). LC/MS(m/z, MH$^+$): 564.2.

**Example 47 (*R*)-3-(1-((6-(1-(fluoromethyl)cyclopropyl))-2-methyl-8-morpholino-7-oxo-6,7-dihydropyrido[4,3-*d*]pyrimidin-4-yl)amino)ethyl)-2-methylbenzonitrile**

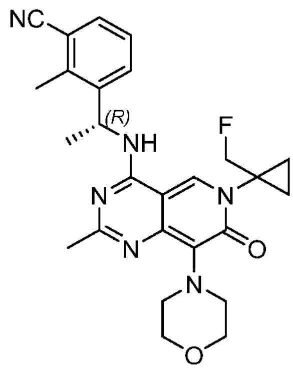

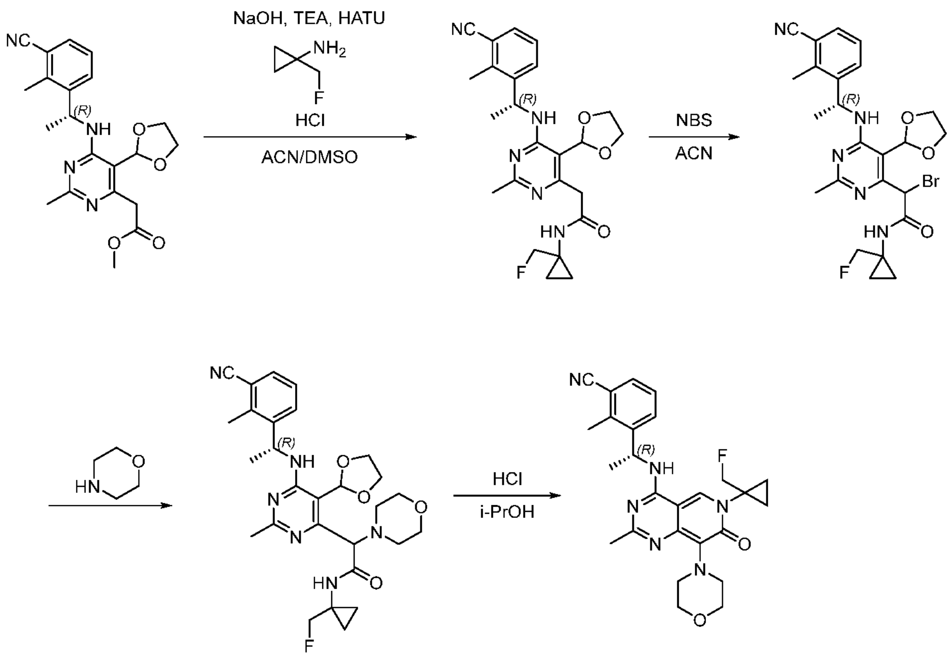


Reference was made to step **2** and step **3** of Example **23** and the synthesis method of Example **34.** The title compound was prepared by the above-mentioned technical route.

$^{1}H$ NMR (400 MHz, DMSO-$d_6$) δ 8.79 (s, 1H), 8.71 (br, 1H), 7.71 (d, *J* = 7.8 Hz, 1H), 7.65 (d, *J* = 6.8 Hz, 1H), 7.39 (t, *J* = 7.8 Hz, 1H), 5.60 (m, 1H), 4.62 (m, 2H), 3.63 (t, *J* = 4.6 Hz, 4H), 3.30-3.19 (m, 4H), 2.66 (s, 3H), 2.19 (s, 3H), 1.51 (d, *J* = 7.0 Hz, 3H), 1.34-1.19 (m, 4H). LC/MS(m/z, $MH^+$): 477.2.

**Example 48 (*R*)-4-((1-(3-(difluoromethyl)-2-fluorophenyl)ethyl)amino)-8-(methoxy-d$_3$)-2-methyl-6-morpholinepyrido[4,3-*d*]pyrimidine-7(6*H*)-one**

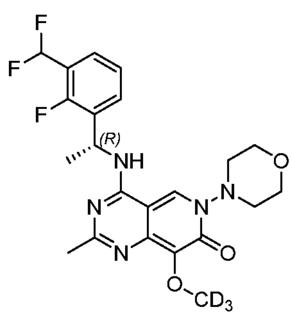

The title compound was prepared in the same manner as in Example **20** except for replacing the solution of

sodium methoxide in methanol in step **4** with methanol-$d_3$($CD_3OH$) and sodium *tert*-butoxide.

$^1H$ NMR (400 MHz, DMSO-$d_6$) δ 9.03 (s, 1H), 8.80 (d, *J* = 7.2 Hz, 1H), 7.67 (t, *J* = 7.2 Hz, 1H), 7.52 (t, *J* = 6.8 Hz, 1H), 7.32 (t, *J* = 7.7 Hz, 1H), 7.23 (t, *J* = 54.3 Hz, 1H), 5.80-5.70 (m, 1H), 4.15-3.36 (m, 8H), 2.21 (s, 3H), 1.55 (d, *J* = 7.0 Hz, 3H). LC/MS(m/z, $MH^+$): 467.2.

**Example 49 (*R*)-8-(4-acetylpiperazin-1-yl)-4-((1-(3-(difluoromethyl)-2-fluorophenyl)ethyl) amino-6-1-fluoromethlcclorol-2-methpyrido[4,3-*d*]pyrimidine-7(6*H*)-one**

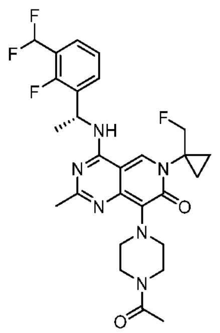

**Step 1 synthesis of *tert*-butyl 4-(1-(6-(((*R*)-1-(3-(difluoromethyl)-2-fluorophenyl)ethyl)amino)-5-(1,3-dioxolan-2-yl)-2-methylpyrimidin-4-yl)-2-((1-(fluoromethyl)cyclopropyl)amino)-2-oxoethyl)piperazine-1-carboxylate**

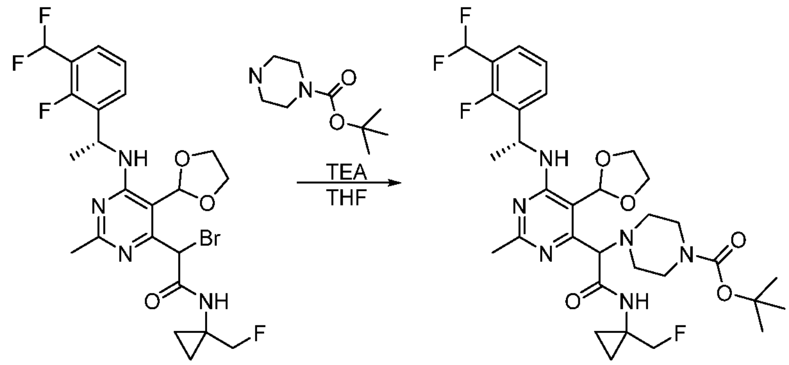


2-Bromo-2-(6-(((*R*)-1-(3-(difluoromethyl)-2-fluorophenyl)ethyl)amino)-5-(1,3-dioxolan-2-yl)-2-methylpyrimidin-4-yl)-N (1-(fluoromethyl)cyclopropyl)acetamide (0.15 g) was dissolved in tetrahydrofuran (5 mL). *tert*-Butyl piperazine-1-carboxylate (248 mg) and triethylamine (54.1 mg) were added, and the reaction was completed after the mixture was stirred at room temperature for 2 h. 20 mL of water was added to the system, and the mixture was extracted with ethyl acetate and separated. The organic phase was washed with a saturated aqueous sodium chloride solution, dried over anhydrous magnesium sulfate, filtered, concentrated, and purified by column chromatography (petroleum ether/ethyl acetate gradient elution) to give the title compound.

**Step 2 synthesis of (*R*)-4-((1-(3-(difluoromethyl)-2-fluorophenyl)ethyl)amino)-6-(1-(fluoromethyl)cyclopropyl)-2-methyl-8-(piperazin-1-yl)pyrido[4,3-*d*]pyrimidine-7(6*H*)-one**

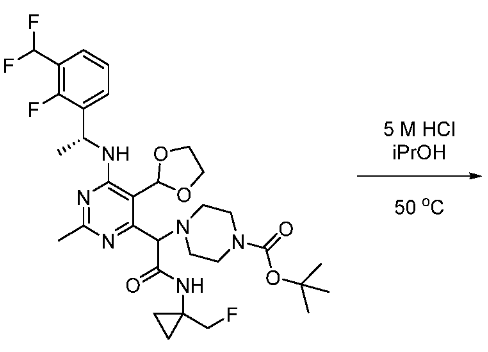


*tert*-Butyl 4-(1-(6-(((R)-1-(3-(difluoromethyl)-2-fluorophenyl)ethyl)amino)-5-(1,3-dioxolan-2-yl)-2-methylpyrimidin-4-yl)-2-((1-(fluoromethyl)cyclopropyl)amino)-2-oxoethyl)piperazine-1-carboxylate (0.150 g) was dissolved in isopropanol (6 mL). Diluted hydrochloric acid (5 mol/L, 224 μL) was added. The reaction was completed after the mixture was heated to 50 °C and stirred for 2 h. The reaction mixture was concentrated and purified by column chromatography (petroleum ether/ethyl acetate gradient elution) to give the title compound.

**Step 3 synthesis of (*R*)-8-(4-acetylpiperazin-1-yl)-4-((1-(3-(difluoromethyl)-2-fluorophenyl)ethyl)amino)-6-(1-(fluoromethyl)cyclopropyl)-2-methylpyrido[4,3-*d*]pyrimidine-7(6*H*)-one**

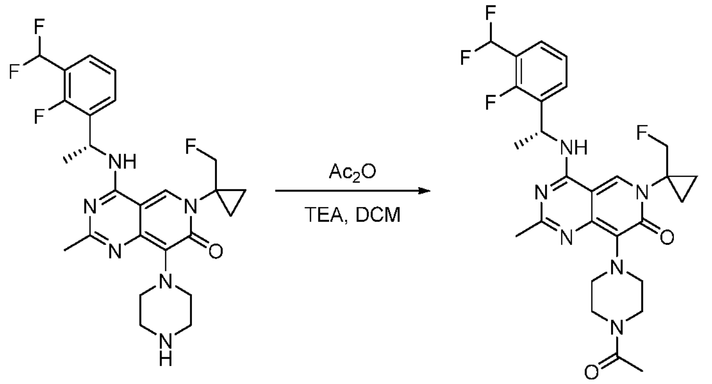


(*R*)-4-((1-(3-(Difluoromethyl)-2-fluorophenyl)ethyl)amino)-6-(1-(fluoromethyl)cyclopropyl)-2-methyl-8-(piperazin-1-yl)pyrido[4,3-*d*]pyrimidine-7(6*H*)-one (0.10 g) was dissolved in dichloromethane (5 mL). Triethylamine (51.3 mg) and acetic anhydride (30.4 mg) were added, and the reaction was completed after the mixture was stirred at room temperature for 3 h. The reaction mixture was concentrated and purified by column chromatography (dichloromethane/methanol gradient elution) to give the title compound.

$^1H$ NMR (400 MHz, DMSO-$d_6$) δ 8.83 (s, 1H), 8.66 (d, *J* = 7.1 Hz, 1H), 7.62 (t, *J* = 7.3 Hz, 1H), 7.51 (t, *J* = 7.0 Hz, 1H), 7.31 (t, *J* = 7.7 Hz, 1H), 7.23 (t, *J* = 54.4 Hz, 1H), 5.74 (m, 1H), 4.80-4.44 (m, 2H), 3.55-3.44 (m, 4H), 3.30-3.24 (m, 2H), 3.23-3.17 (m, 2H), 2.19 (s, 3H), 2.01 (s, 3H), 1.57 (d, *J* = 7.1 Hz, 3H), 1.35-1.18 (m, 4H). LC/MS(m/z, $MH^+$): 547.2.

**Example 50 (*R*)-4-((1-(3-(difluoromethyl)-2-fluorophenyl)ethyl)amino)-6-(1-(fluoromethyl) cyclopropyl)-2-methyl-8-(2-oxa-6-azaspiro[3.3]heptan-6-yl)pyrido[4,3-*d*]pyrimidine-7(6*H*)-one**

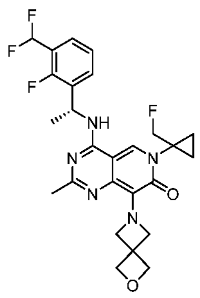

The title compound was prepared in the same manner as in Example **34** except for replacing morpholine in step 1 with 2-oxa-6-aza-spiro[3,3]heptane.

$^1$H NMR (400 MHz, DMSO-$d_6$) δ 8.45 (d, *J* = 7.1 Hz, 1H), 8.37 (s, 1H), 7.60 (t, *J* = 7.6 Hz, 1H), 7.53-7.46 (m, 1H), 7.30 (t, *J* = 7.8 Hz, 1H), 7.23 (t, *J* = 54.4 Hz, 1H), 5.77-5.66 (m, 1H), 4.64 (s, 4H), 4.68-4.58 (m, 2H), 4.46 (s, 4H), 2.14 (s, 3H), 1.55 (d, *J* = 7.0 Hz, 3H), 1.32-1.15 (m, 4H). LC/MS(m/z, MH$^+$): 518.2.

**Example 51 (*R*)-4-((1-(3-(difluoromethyl)-2-fluorophenyl)ethyl)amino)-8-(2,2-dimethylmorpholine) -6-(1-(fluoromethyl)cyclopropyl)-2-methylpyrido[4,3-*d*]pyrimidine-7(6*H*)-one**

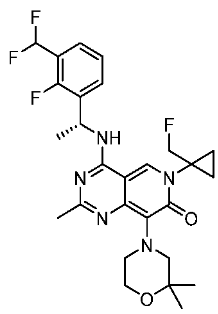

The title compound was prepared in the same manner as in Example **34** except for replacing morpholine in step **1** with 2,2-dimethylmorpholine.

$^1$H NMR (400 MHz, DMSO-$d_6$) δ 8.79 (s, 1H), 8.65 (d, *J* = 7.0 Hz, 1H), 7.62 (t, *J* = 7.3 Hz, 1H), 7.51 (t, *J* = 6.8 Hz, 1H), 7.31 (t, *J* = 7.7 Hz, 1H), 7.23 (t, *J* = 54.4 Hz, 1H), 5.78-5.70 (m, 1H), 4.82-4.43 (m, 2H), 3.71 (t, *J* = 4.8 Hz, 2H), 3.25-3.17 (m, 2H), 3.02 (s, 2H), 2.18 (s, 3H), 1.57 (d, *J* = 7.1 Hz, 3H), 1.34-1.21 (m, 4H), 1.19 (s, 6H). LC/MS(m/z, MH$^+$): 534.2.

**Example 52 (*R*)-4-((1-(3-(difluoromethyl)-2-fluorophenyl)ethyl)amino)-8-(3,3-dimethylmorpholine) -6-(1-(fluoromethyl)cyclopropyl)-2-methylpyrido[4,3-*d*]pyrimidine-7(6*H*)-one**

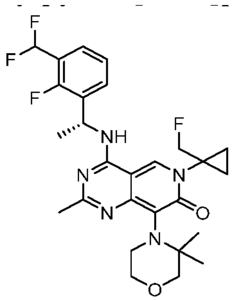

The title compound was prepared in the same manner as in Example **34** except for replacing morpholine in step **1** with 3,3-dimethylmorpholine.

$^1$H NMR (400 MHz, DMSO-$d_6$) δ 8.96 (s, 1H), 8.75-8.66 (m, 1H), 7.71-7.60 (m, 1H), 7.52 (t, *J* = 6.8 Hz, 1H), 7.35-7.28 (m, 1H), 7.38-7.09 (m, 1H), 5.80-5.70 (m, 1H), 4.81-4.39 (m, 2H), 3.78-3.58 (m, 2H), 3.13-2.96 (m, 1H), 2.20 (d, *J* = 1.3 Hz, 3H), 1.57 (d, *J* = 6.9 Hz, 3H), 1.36-1.19 (m, 4H), 1.09-0.97 (m, 6H). LC/MS(m/z, MH$^+$): 534.2.

**Example 53 8-((1*R*,4*R*)-2-oxa-5-azabicyclo[2.2.1]heptan-5-yl)-4-(((*R*)-1-(3-(difluoromethyl)-2-fluorophenyl)ethyl)amino)-6-(1-(fluoromethyl)cyclopropyl)-2-methylpyrido[4,3-*d*]pyrimidine-7(6*H*)-one**

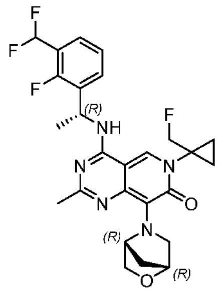

The title compound was prepared in the same manner as in Example **34** except for replacing morpholine in step **1** with (1*R*,4*R*)-2-oxa-5-azabicyclo[2.2.1]heptane.

$^1$H NMR (400 MHz, DMSO-$d_6$) δ 8.50-8.46 (m, 2H), 7.61 (t, *J* = 7.5 Hz, 1H), 7.51 (t, *J* = 7.1 Hz, 1H), 7.31 (t, *J* = 7.7 Hz, 1H), 7.23 (t, *J* = 54.4 Hz, 1H), 5.79-5.71 (m, 1H), 5.20 (s, 1H), 4.75-4.55 (m, 2H), 4.46 (s, 1H), 4.24 (d, *J* = 10.1 Hz, 1H), 3.93 (d, *J* = 7.4 Hz, 1H), 3.77 (d, *J* = 6.0 Hz, 1H), 3.15 (d, *J* = 11.2 Hz, 1H), 2.14 (s, 3H), 1.74 (dd, *J* = 25.1, 8.9 Hz, 2H), 1.57 (d, *J* = 7.1 Hz, 3H), 1.36-1.19 (m, 4H). LC/MS(m/z, MH$^+$): 518.2.

**Example 54 8-((1*R*,5*S*)-8-oxa-3-azabicyclo[3.2.1]octan-3-yl)-4-(((*R*)-1-(3-(difluoromethyl)-2-fluorophenyl)ethyl)amino)-6-(1-(fluoromethyl)cyclopropyl)-2-methylpyrido[4,3-*d*]pyrimidine-7(6*H*)-one**

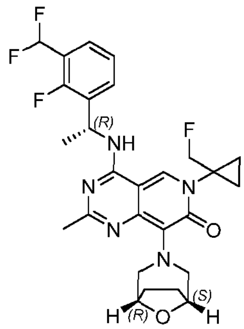

The title compound was prepared in the same manner as in Example **34** except for replacing morpholine in step **1** with (1*R*,5*S*)-8-oxa-3-azabicyclo[3.2.1]octane.

$^1$H NMR (400 MHz, DMSO-$d_6$) δ 8.81 (s, 1H), 8.64 (d, *J* = 7.1 Hz, 1H), 7.61 (t, *J* = 7.2 Hz, 1H), 7.51 (t, *J* = 7.0 Hz, 1H), 7.31 (t, *J* = 7.7 Hz, 1H), 7.23 (t, *J* = 54.4 Hz, 1H), 5.73 (m, 1H), 4.79-4.40 (m, 2H), 4.18 (s, 2H), 3.53 (t, *J* = 10.2 Hz, 2H), 2.96-2.86 (m, 2H), 2.25 (d, *J* = 10.4 Hz, 2H), 2.19 (s, 3H), 1.74-1.64 (m, 2H), 1.57 (d, *J* = 7.1 Hz, 3H), 1.37-1.14 (m, 4H). LC/MS(m/z, MH$^+$): 532.2.

**Example 55 (*R*)-4-((1-(3-(difluoromethyl)-2-fluorophenyl)ethyl-1-*d*)amino)-6-(1-(fluoromethyl) cyclopropyl)-2-methyl-8-morpholinylpyrido[4,3-d]pyrimidine-7(6*H*)-one**

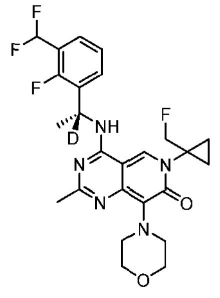

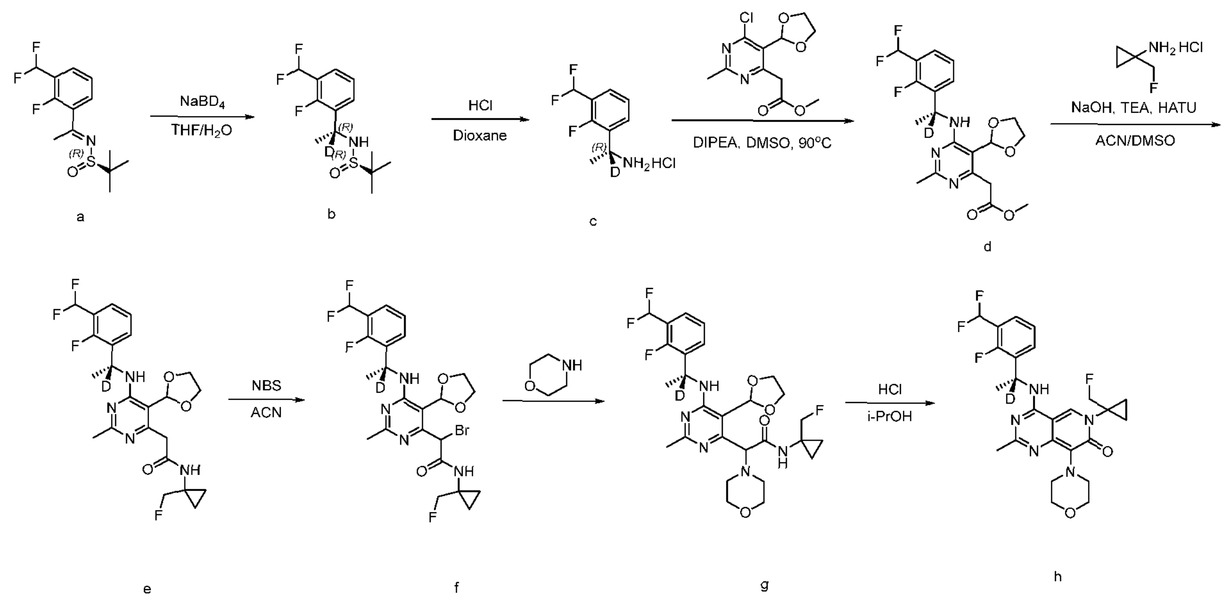


**Step 1: synthesis of (*R*)-*N*-((*R*)-1-(3-(difluoromethyl))-2-fluorophenyl)ethyl-1-*d*)-2-methylpropane-2-sulfinamide**

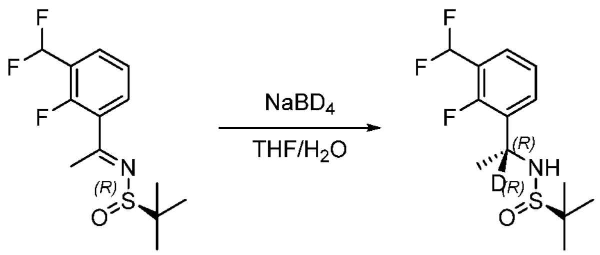


(*R,E*)-*N*-(1-(3-(Difluoromethyl)-2-fluorophenyl)ethylidene)-2-methylpropane-2-sulfinamide (10.0 g) was dissolved in a mixed solvent of 76 mL of tetrahydrofuran and 16 mL of water. The system was purged with nitrogen and cooled to -50 °C. Sodium borondeuteride ($NaBD_4$, 1.58 g) was added in batches. After the addition, the reaction was completed after the mixture was naturally warmed and reacted for 3 h. The reaction mixture was quenched with water and extracted with dichloromethane. The organic phase was dried over anhydrous sodium sulfate, filtered, concentrated, and purified by column chromatography (petroleum ether/ethyl acetate gradient elution) to give the title compound.

**Step 2: synthesis of (*R*)-1-(3-(difluoromethyl)-2-fluorophenyl)ethan-1-*d*-1-amine hydrochloride**

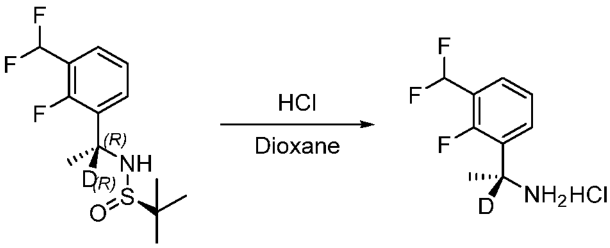


(*R*)-*N*-((*R*)-1-(3-(Difluoromethyl))-2-fluorophenyl)ethyl-1-*d*)-2-methylpropane-2-sulfinamide (3.30 g) was dissolved in 10 mL of a solution of hydrochloric acid in dioxane. After the mixture was stirred at room temperature for reaction for 1 h, 40 mL of isopropyl ether was added to the reaction mixture, and white solid was precipitated. The mixture was stirred for 1 h and subjected to suction filtration, and the filter cake was dried to give the title compound.

**Step 3: synthesis of methyl (*R*)-2-(6-((1-(3-(difluoromethyl)-2-fluorophenyl)ethyl-1-*d*)amino)-5-(1,3-dioxolan-2-yl)-2-methylpyrimidin-4-yl)acetate**

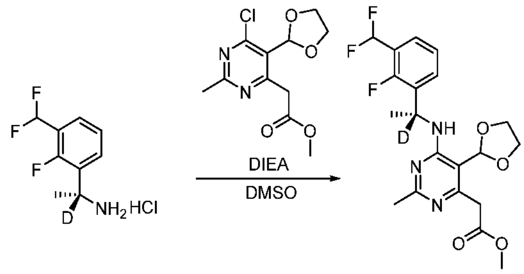


(*R*)-1-(3-(Difluoromethyl)-2-fluorophenyl)ethan-1-*d*-1-amine hydrochloride (5.00 g) and methyl 2-(6-chloro-5-(1,3-dioxolan-2-yl)-2-methylpyrimidin-4-yl)acetate (6.00 g) were placed in a reaction flask. *N,N*-Diisopropylethylamine (7.10 g) and 30 mL of dimethyl sulfoxide were added, and the reaction was completed after the mixture was stirred at 90 °C for 5 h under argon atmosphere. The reaction mixture was quenched by pouring it into water and extracted with ethyl acetate. The organic phase was dried over anhydrous sodium sulfate, filtered, concentrated, and purified by column chromatography (petroleum ether/ethyl acetate gradient elution) to give the title compound.

For the reactions of compound d-h, reference was made to step 2 and step 3 of Example 23 and the synthesis method of Example **34.** The title compound was prepared according to the above-mentioned technical route.

$^1$H NMR (400 MHz, DMSO-$d_6$) δ 8.82 (s, 1H), 8.66 (s, 1H), 7.63 (t, *J* = 7.3 Hz, 1H), 7.51 (t, *J* = 7.0 Hz, 1H), 7.31 (t, *J* = 7.7 Hz, 1H), 7.23 (t, *J* = 54.4 Hz, 1H), 4.85-4.40 (m, 2H), 3.63 (t, *J* = 4.6 Hz, 4H), 3.32-3.20 (m, 4H), 2.20 (s, 3H), 1.56 (s, 3H), 1.40-1.17 (m, 4H). LC/MS(m/z, MH$^+$): 507.1.

**Example 56 6-(1-(fluoromethyl)cyclopropyl)-2-methyl-4-(((*R*)-1-(2-methyl-3-(trifluoromethyl) phenyl)ethyl)amino)-8-((*S*)-3-methylmorpholine)pyrido[4,3-*d*]pyrimidine-7(6*H*)-one**

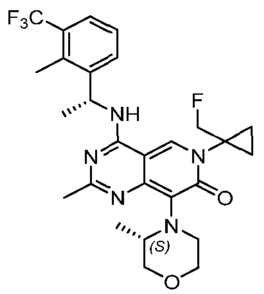

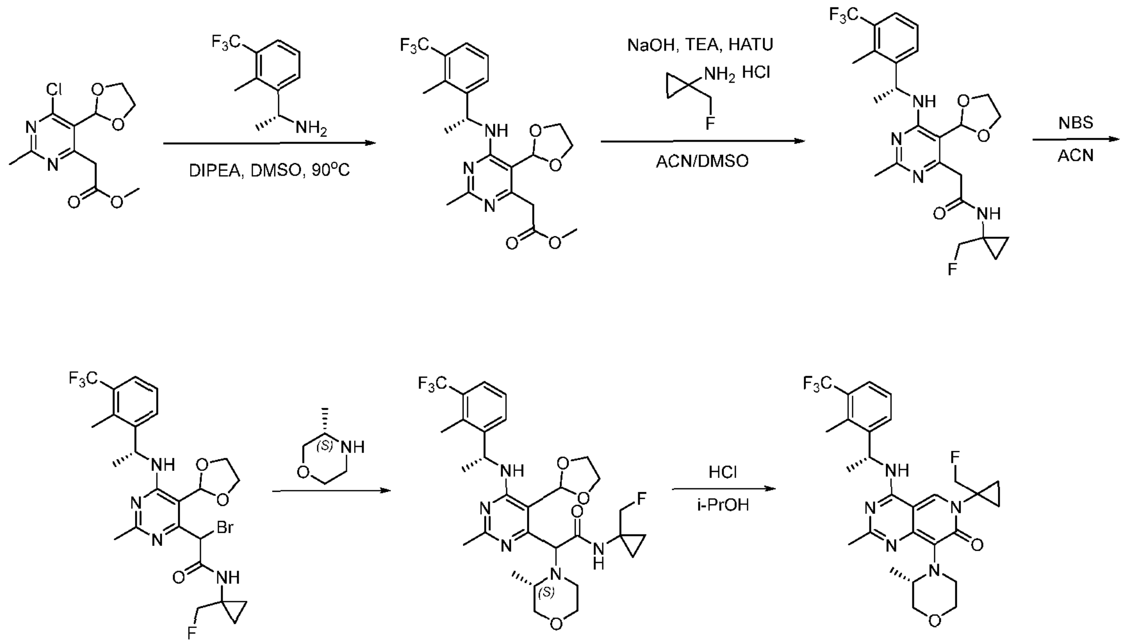


Reference was made to step **1-step 3** of Example **23** and the synthesis method of Example **34.** The title compound was prepared according to the above-mentioned technical route.

$^1$H NMR (400 MHz, DMSO-$d_6$) δ 8.94 (s, 1H), 8.79 (d, *J* = 6.7 Hz, 1H), 7.72 (d, *J* = 7.7 Hz, 1H), 7.55 (d, *J* = 7.6 Hz, 1H), 7.38 (t, *J* = 7.8 Hz, 1H), 5.72-5.61 (m, 1H), 4.87-4.36 (m, 2H), 3.99-3.87 (m, 1H), 3.70-3.53 (m, 3H), 3.20-2.94 (m, 3H), 2.58 (s, 3H), 2.19 (s, 3H), 1.53 (d, *J* = 7.0 Hz, 3H), 1.36-1.16 (m, 4H), 0.67 (d, *J* = 6.3 Hz, 3H). LC/MS(m/z, MH$^+$): 534.2.

**Example 57 4-(((*R*)-1-(3-(difluoromethyl)-2-fluorophenyl)ethyl)amino)-6-(1-(fluoromethyl) cyclopropyl)-8-((*S*)-3-isopropylmorpholino)-2-methylpyrido[4,3-*d*]pyrimidine-7(6*H*)-one**

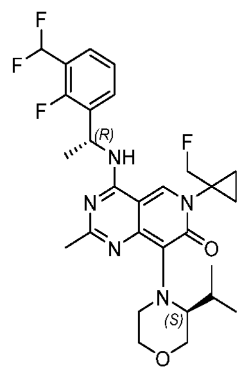

The title compound was prepared in the same manner as in Example **34** except for replacing morpholine in step **1** with (*S*)-3-isopropylmorpholine.

$^1$H NMR (400 MHz, DMSO-$d_6$) δ 8.84 (s, 1H), 8.64 (d, *J* = 7.2 Hz, 1H), 7.65 (t, *J* = 7.5 Hz, 1H), 7.51 (t, *J* = 6.9 Hz, 1H), 7.32 (t, *J* = 7.7 Hz, 1H), 7.23 (t, *J* = 54.4 Hz, 1H), 5.75 (m, 1H), 4.85-4.41 (m, 2H), 3.86-3.77 (m, 1H), 3.64-3.55 (m, 3H), 3.53-3.45 (m, 1H), 3.31-3.22 (m, 1H), 3.10-2.99 (m, 1H), 2.19 (s, 3H), 1.96-1.77 (m, 1H), 1.57 (d, *J* = 7.1 Hz, 3H), 1.43-1.14 (m, 4H), 0.73 (dd, *J* = 17.2, 6.8 Hz, 6H). LC/MS(m/z, MH$^+$): 548.2.

**Example 58 4-(((*R*)-1-(3-(difluoromethyl)-2-fluorophenyl)ethyl)amino)-8-((*S*)-3-ethylmorpholino)-6-(1-(fluoromethyl)cyclopropyl)-2-methylpyrido[4,3-*d*]pyrimidine-7(6*H*)-one**

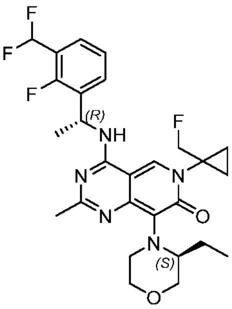

The title compound was prepared in the same manner as in Example **34** except for replacing morpholine in step **1** with (*S*)-2-ethylmorpholine.

$^1$H NMR (400 MHz, DMSO-$d_6$) δ 8.90 (s, 1H), 8.68 (d, *J* = 5.1 Hz, 1H), 7.65 (t, *J* = 7.3 Hz, 1H), 7.51 (t, *J* = 6.8 Hz, 1H), 7.32 (t, *J* = 7.7 Hz, 1H), 7.24 (t, *J* = 54.4 Hz, 1H), 5.80-5.70 (m, 1H), 4.84-4.37 (m, 2H), 3.82-3.68 (m, 2H), 3.66-3.54 (m, 2H), 3.41-3.34 (m, 1H), 3.31-3.21 (m, 1H), 3.05-2.96 (m, 1H), 2.20 (s, 3H), 1.57 (d, *J* = 7.0 Hz, 3H), 1.41-1.16 (m, 6H), 0.66 (t, *J* = 7.5 Hz, 3H). LC/MS(m/z, MH$^+$): 534.2.

**Example 59 (*R*)-8-amino-4-((1-(3-(difluoromethyl)-2-fluorophenyl)ethyl)amino)-6-(1-(fluoromethyl)cyclopropyl)-2-methylpyrido[4,3-*d*]pyrimidine-7(6*H*)-one**

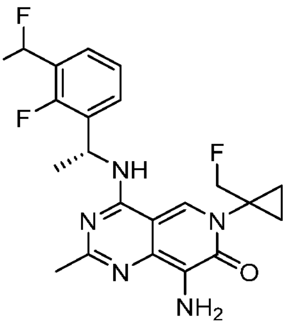

**Step 1: synthesis of 2-(dibenzylamino)-2-(6-((*R*)-1-(3-(difluoromethyl)-2-fluorophenyl) ethyl)amino)-5-(1,3-dioxolan-2-yl)-2-methylpyrimidin-4-yl)-*N*-(1-(fluoromethyl)cyclopropyl)acetamide**

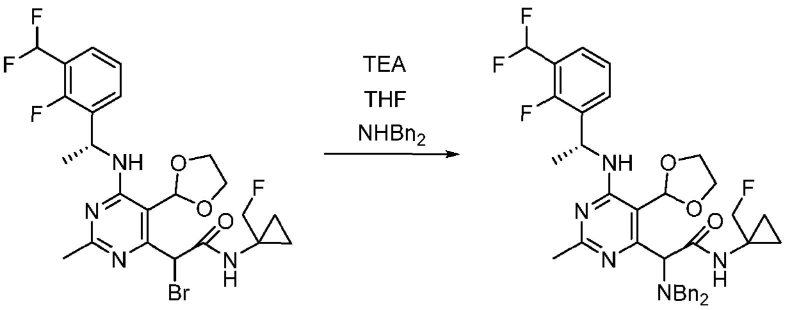


2-Bromo-2-(6-((*R*)-1-(3-(difluoromethyl)-2-fluorophenyl)ethyl)amino)-5-(1,3-dioxolan-2-yl)-2-methylpyrimidin-4-yl)-*N*-(1-(fluoromethyl)cyclopropyl)acetamide (290 mg) and dibenzylamine (112 mg) were dissolved in 3 mL of tetrahydrofuran. Triethylamine (52.2 mg) was added. The mixture was reacted at 50 °C for 16 h. After the reaction was completed, the reaction mixture was poured into water, extracted with dichloromethane, dried over anhydrous sodium sulfate, filtered, concentrated, and purified by column chromatography (petroleum ether/ethyl acetate gradient elution) to give the title compound.

**Step 2: synthesis of (*R*)-8-(dibenzylamino)-4-((1-(3-(difluoromethyl)-2-fluorophenyl) ethyl)amino)-6-(1-(fluoromethyl)cyclopropyl)-2-methylpyrido[4,3-*d*]pyrimidine-7(6*H*)-one**

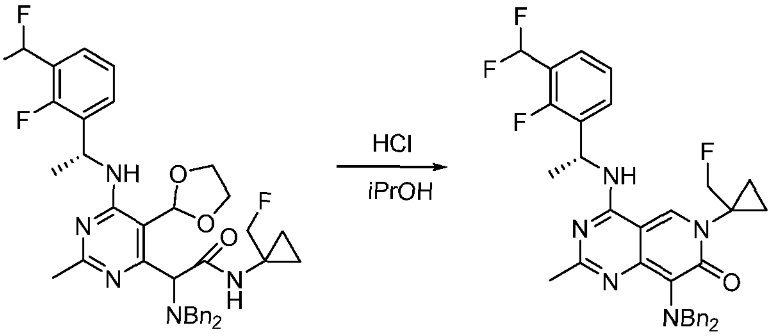


2-(Dibenzylamino)-2-(6-((*R*)-1-(3-(difluoromethyl)-2-fluorophenyl)ethyl)amino)-5-(1,3-dioxolan-2-yl)-2-methylpyrimidin-4-yl)-*N*-(1-(fluoromethyl)cyclopropyl)acetamide (370 mg) was dissolved in 3 mL of isopropanol. 1.3 mL of 2 M hydrochloric acid solution was added, and the mixture was reacted at 50 °C for 2 h. After the reaction was completed, the reaction mixture was neutralized with a saturated sodium bicarbonate solution and extracted with dichloromethane. The organic phase was dried, concentrated, and purified by column chromatography (petroleum ether/ethyl acetate gradient elution) to give the title compound.

**Step 3: synthesis of (*R*)-8-amino-4-((1-(3-(difluoromethyl)-2-fluorophenyl)ethyl)amino)-6-(1-(fluoromethyl)cyclopropyl)-2-methylpyrido[4,3-*d*]pyrimidine-7(6*H*)-one**

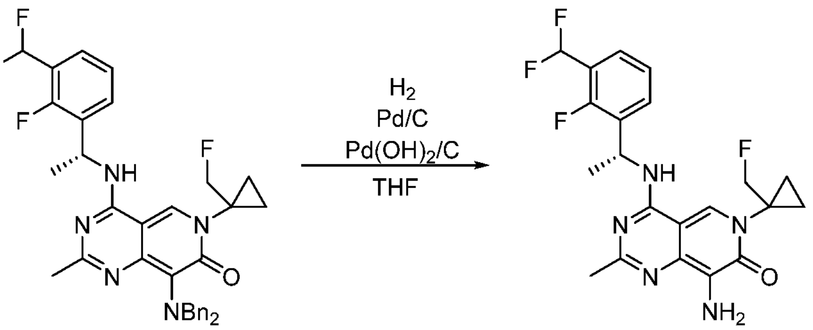


(*R*)-8-(Dibenzylamino)-4-((1-(3-(difluoromethyl)-2-fluorophenyl)ethyl)amino)-6-(1-(fluoromethyl)cyclopropyl)-2-methylpyrido[4,3-*d*]pyrimidine-7(6*H*)-one (186 mg) was dissolved in 4 mL of tetrahydrofuran. 20 mg of palladium on carbon and 20 mg of palladium hydroxide on carbon were added. The system was purged with hydrogen, and the mixture was reacted at room temperature for 24 h. After the reaction was completed, the mixture was filtered to remove the catalyst, and the filtrate was concentrated and purified by column chromatography (dichloromethane/methanol gradient elution) to give the title compound.

$^1$H NMR (400 MHz, DMSO-$d_6$) δ 8.55 (d, *J* = 7.2 Hz, 1H), 8.37 (s, 1H), 7.63 (t, *J* = 7.2 Hz, 1H), 7.51 (t, *J* = 6.9 Hz, 1H), 7.31 (t, *J* = 7.7 Hz, 1H), 7.24 (t, *J* = 54.4 Hz, 1H), 5.77 (m, 1H), 5.01 (s, 2H), 4.80-4.50 (m, 2H), 2.21 (s, 3H), 1.57 (d, *J* = 7.1 Hz, 3H), 1.40-1.18 (m, 4H). LC/MS(m/z, $MH^+$): 436.2.

**Example 60 (*R*)-4-((1-(3-(difluoromethyl)-2-fluorophenyl)ethyl)amino)-6-(1-(fluoromethyl) cyclopropyl)-2-methyl-8-((1-methyl-1*H*-pyrazol-4-yl)oxy)pyrido[4,3-*d*]pyrimidine-7(6*H*)-one**

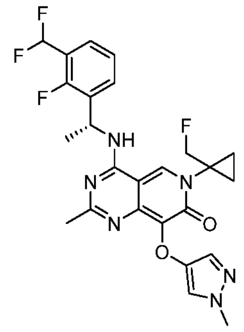

The title compound was prepared in the same manner as in Example **23** except for replacing methanol-$d_3$($CD_3OH$) in step **4** with 1-methyl-1*H*-pyrazol-4-ol.

$^1H$ NMR (400 MHz, DMSO-$d_6$) δ 9.10 (s, 1H), 8.94 (d, *J* = 6.9 Hz, 1H), 7.66 (t, *J* = 7.3 Hz, 1H), 7.53 (t, *J* = 6.9 Hz, 1H), 7.33 (t, *J* = 7.7 Hz, 1H), 7.26 (s, 1H), 7.24 (t, *J* = 54.3 Hz, 1H), 7.06 (s, 1H), 5.77 (m, 1H), 4.80-4.43 (m, 2H), 3.66 (s, 3H), 2.20 (s, 3H), 1.59 (d, *J* = 7.0 Hz, 3H), 1.41-1.21 (m, 4H). LC/MS(m/z, $MH^+$): 517.2.

**Example 61 (*R*)-4-((1-(3-(difluoromethyl)-2-fluorophenyl)ethyl)amino)-6-(1-(fluoromethyl) cyclopropyl)-2-methyl-8-(oxetan-3-ylamino)pyrido[4,3-*d*]pyrimidine-7(6*H*)-one**

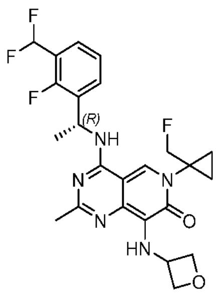

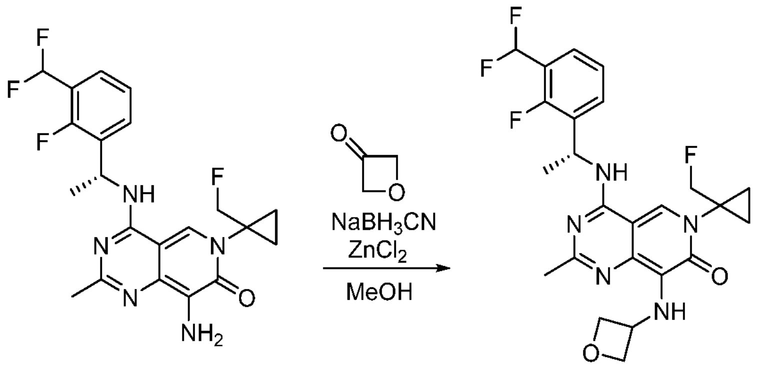


(*R*)-8-Amino-4-((1-(3-(difluoromethyl)-2-fluorophenyl)ethyl)amino)-6-(1-(fluoromethyl)cyclopropyl)-2-methyl-pyrido[4,3-*d*]pyrimidine-7(6*H*)-one (0.03 g), 3-oxetanone (0.012 g), sodium cyanoborohydride (0.01 g), and zinc chloride (0.03 g) were added to 1 mL of methanol. The reaction was completed after the mixture was stirred at 50 °C for 3 h. The mixture was quenched with water and extracted with ethyl acetate. The organic phase was dried over anhydrous sodium sulfate, filtered, and concentrated to give the title compound.

$^1H$ NMR (400 MHz, DMSO-$d_6$) δ 7.91-7.83 (m, 1H), 7.61 (s, 1H), 7.45 (t, *J* = 6.7 Hz, 1H), 7.34-7.06 (m, 2H), 5.89-5.79 (m, 1H), 5.46 (s, 1H), 5.11-5.04 (m, 1H), 4.70-4.43 (m, 2H), 4.03-3.95 (m, 1H), 3.65-3.57 (m, 1H), 3.53-3.35 (m, 3H), 2.30 (s, 3H), 1.39-1.13 (m, 7H). LC/MS(m/z, $MH^+$): 492.2.

**Example 62 4-(((*R*)-1-(3-(difluoromethyl)-2-fluorophenyl)ethyl)amino)-6-(1-(fluoromethyl) cyclopropyl)-8-(3-hydroxy-3-methylpyrrolidin-1-yl)-2-methylpyrido[4,3-*d*]pyrimidine-7(6*H*)-one**

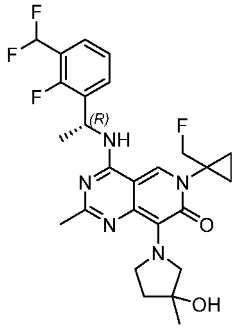

The title compound was prepared in the same manner as in Example **34** except for replacing morpholine in step **1** with 3-hydroxy-3-methylpyrrole.

$^1$H NMR (400 MHz, DMSO-$d_6$) δ 8.70 (d, *J* = 2.4 Hz, 1H), 8.62 (d, *J* = 7.1 Hz, 1H), 7.63 (t, *J* = 7.3 Hz, 1H), 7.51 (t, *J* = 7.0 Hz, 1H), 7.32 (t, *J* = 7.7 Hz, 1H), 7.24 (t, *J* = 54.4 Hz, 1H), 5.79-5.70 (m, 1H), 5.33 (d, *J* = 11.6 Hz, 1H), 4.83-4.44 (m, 2H), 3.79-3.65 (m, 1H), 3.62-3.50 (m, 2H), 3.39-3.30 (m, 1H), 2.18 (s, 3H), 1.80-1.65 (m, 2H), 1.57 (d, *J* = 7.1 Hz, 3H), 1.34-1.20 (m, 7H). LC/MS(m/z, MH$^+$): 520.2.

**Example 63 (*R*)-2-methyl-3-(1-((2-methyl-6-morpholine-7-oxo-6,7-dihydropyrido[4,3-*d*]pyrimidin-4-yl)amino)ethyl)benzonitrile**

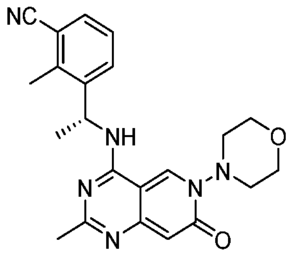

The title compound was prepared in the same manner as in Example **18** except for replacing (R)-1-(3-(pentafluoro-λ$^6$-sulfanyl)phenyl)ethane-1-amine in step **1** with (*R*)-3-(1-aminoethyl)-2-benzonitrile.

**Example 64 (*R*)-5-(4-((1-(3-cyano-2-methylphenyl)ethyl)amino)-2-methyl-6-morpholine-7-oxo-6,7-dihydropyrido[4,3-*d*]pyrimidin-8-yl)pyridinecarbonitrile**

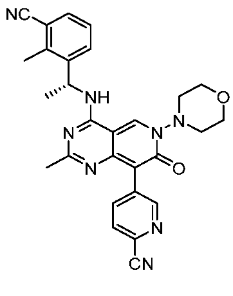

The title compound was prepared in the same manner as in Example **22** except for replacing (*R*)-2-methyl-6-morpholine-4-((1-(3-(pentafluoro-$\lambda^6$-sulfanyl)phenyl)ethyl)amino)pyrido[4,3-*d*]pyrimidine-7(6*H*)-one in step **1** with (*R*)-2-methyl-3-(1-((2-methyl-6-morpholine-7-oxo-6,7-dihydropyrido[4,3-*d*]pyrimidin-4-yl)amino)ethyl)benzonitrile.

$^1$H NMR (400 MHz, DMSO-$d_6$) $\delta$ 9.35 (s, 1H), 9.10 (d, *J* = 4.2 Hz, 1H), 8.83 (d, *J* = 1.4 Hz, 1H), 8.14 (dd, *J* = 8.1, 2.0 Hz, 1H), 8.01 (d, *J* = 8.1 Hz, 1H), 7.79 (d, *J* = 7.8 Hz, 1H), 7.68 (d, *J* = 7.8 Hz, 1H), 7.42 (t, *J* = 7.8 Hz, 1H), 5.70-5.59 (m, 1H), 4.23-3.40 (m, 8H), 2.67 (s, 3H), 2.17 (s, 3H), 1.53 (d, *J* = 7.0 Hz, 3H). LC/MS(m/z, MH$^+$): 507.2.

**Example 65 (*R*)-4-((1-(3-(difluoromethyl)-2-fluorophenyl)ethyl)amino)-6-(1-(fluoromethyl) cyclopropyl)-2-methyl-8-(methyl(1-methyl-1*H*-pyrazol-4-yl)amino)pyrido[4,3-*d*]pyrimidine-7(6*H*)-one**

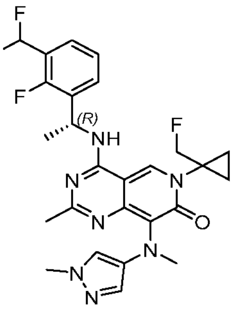

The title compound was prepared in the same manner as in Example **34** except for replacing morpholine in step **1** with *N*,1-dimethyl-1*H*-pyrazole-4-amine.

$^1$H NMR (400 MHz, DMSO-$d_6$) $\delta$ 9.08 (s, 1H), 8.85 (s, 1H), 7.67 (t, *J* = 7.3 Hz, 1H), 7.53 (t, *J* = 6.9 Hz, 1H), 7.33 (t, *J* = 7.7 Hz, 1H), 7.24 (t, *J* = 54.4 Hz, 1H), 6.81 (s, 1H), 6.60 (s, 1H), 5.78 (m, 1H), 4.79-4.46 (m, 2H), 3.65 (s, 3H), 3.03 (s, 3H), 2.18 (s, 3H), 1.59 (d, *J* = 7.1 Hz, 3H), 1.27 (m, 4H). LC/MS(m/z, MH$^+$): 530.2.

**Example 66 (*R*)-4-((1-(3-(difluoromethyl)-2-fluorophenyl)ethyl)amino)-6-(1-(fluoromethyl) cyclopropyl)-2-methyl-8-(methylsulfonyl)pyrido[4,3-*d*]pyrimidine-7(6*H*)-one**

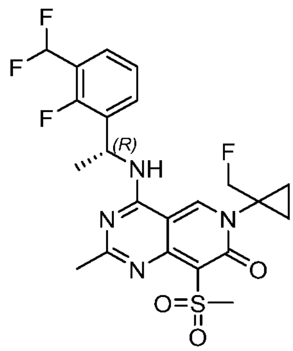

**Step 1: synthesis of 2-(6-(((*R*)-1-(3-(difluoromethyl)-2-fluorophenyl)ethyl)amino)-5-(1,3-dioxolan-2-yl)-2-methylpyrimidin-4-yl)-*N*-(1-(fluoromethyl)cyclopropyl)-2-(methylsulfonyl)acetamide**

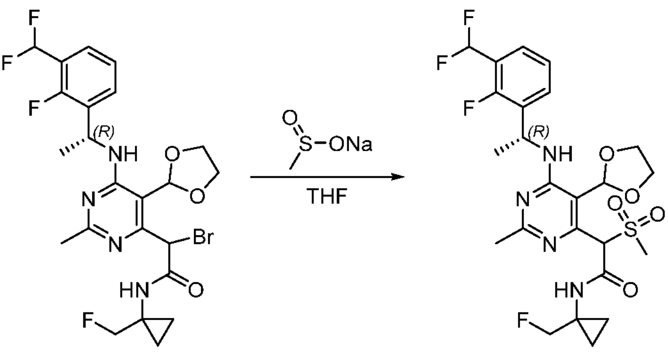


2-Bromo-2-(6-(((*R*)-1-(3-(difluoromethyl)-2-fluorophenyl)ethyl)amino)-5-(1,3-dioxolan-2-yl)-2-methylpyrimidin-4-yl)-*N*-(1-(fluoromethyl)cyclopropyl)acetamide (88.0 mg) was dissolved in tetrahydrofuran (1 mL). Sodium methanesulfinate (160 mg) was added. The reaction was completed after the mixture was stirred at room temperature for 3 h. The reaction mixture was concentrated and purified by column chromatography (petroleum ether/ethyl acetate gradient elution) to give the title compound.

**Step 2: synthesis of (*R*)-4-((1-(3-(difluoromethyl)-2-fluorophenyl)ethyl)amino)-6-(1-(fluoromethyl) cyclopropyl)-2-methyl-8-(methylsulfonyl)pyrido[4,3-*d*]pyrimidine-7(6*H*)-one**

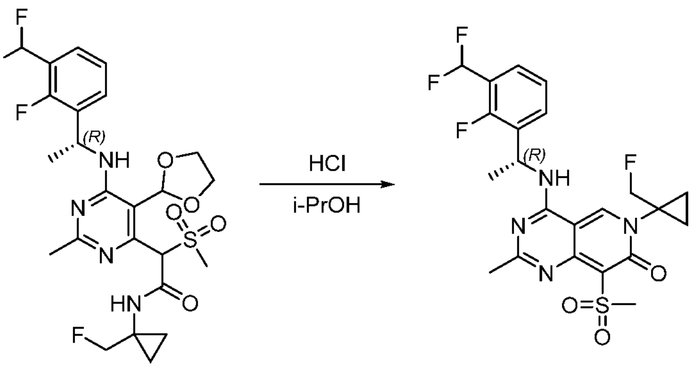


The title compound was prepared in the same manner as in **step 2** of Example **34.**

$^1$H NMR (400 MHz, DMSO-$d_6$) δ 9.48 (s, 1H), 9.19 (d, *J* = 6.9 Hz, 1H), 7.66 (t, *J* = 7.2 Hz, 1H), 7.54 (t, *J* = 6.9 Hz, 1H), 7.33 (t, *J* = 7.7 Hz, 1H), 7.24 (t, *J* = 54.3 Hz, 1H), 5.82-5.71 (m, 1H), 4.85-4.47 (m, 2H), 3.33 (s, 3H), 2.27 (s, 3H), 1.60 (d, *J* = 7.1 Hz, 3H), 1.45-1.18 (m, 4H). LC/MS(m/z, MH$^+$): 499.2.

**Example 67 (*R*)-5-(4-((1-(3-cyano-2-methylphenyl)ethyl)amino)-6-(1-(fluoromethyl)cyclopropyl)-2-methyl-7-oxo-6,7-dihydropyrido[4,3-*d*]pyrimidin-8-yl)pyridinecarbonitrile**

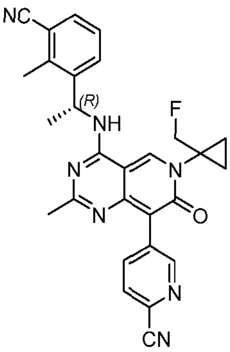

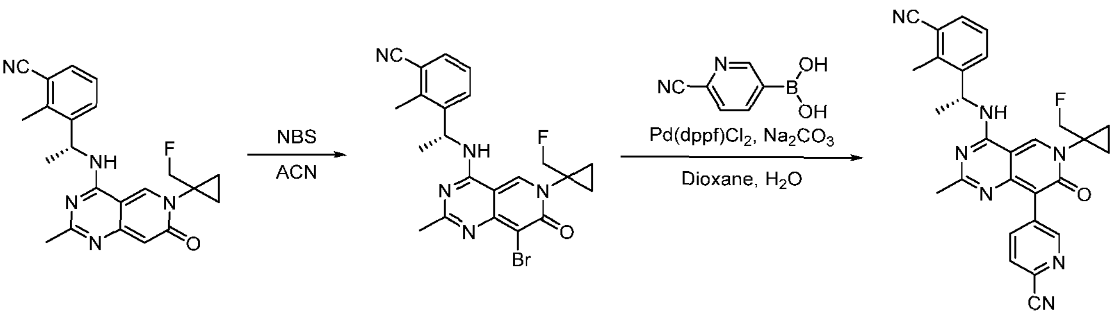


Reference was made to the synthesis method of Example **22,** and the title compound was prepared according to the above-mentioned technical route.

$^1H$ NMR (400 MHz, DMSO-$d_6$) δ 9.29 (s, 1H), 9.12 (d, *J* = 6.8 Hz, 1H), 8.85 (d, *J* = 1.4 Hz, 1H), 8.16 (dd, *J* = 8.1, 2.1 Hz, 1H), 7.99 (d, *J* = 8.1 Hz, 1H), 7.75 (d, *J* = 7.6 Hz, 1H), 7.67 (d, *J* = 6.9 Hz, 1H), 7.41 (t, *J* = 7.8 Hz, 1H), 5.71-5.56 (m, 1H), 4.86-4.47 (m, 2H), 2.66 (s, 3H), 2.16 (s, 3H), 1.55 (d, *J* = 7.0 Hz, 3H), 1.38 - 1.19 (m, 4H). LC/MS(m/z, $MH^+$): 494.2.

**Example 68 4-(((*R*)-1-(3-(difluoromethyl)-2-fluorophenyl)ethyl)amino)-2-methyl-8-((*S*)-3-methylmorpholine)-6-morpholinepyrido[4,3-*d*]pyrimidine-7(6*H*)-one**

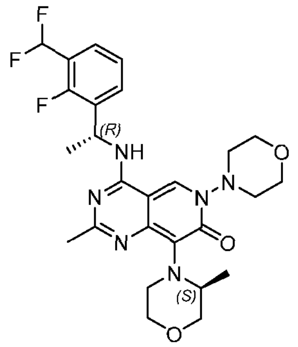

The title compound was prepared in the same manner as in Example **20** except for replacing the solution of sodium methoxide in methanol in step **4** with (*S*)-3-methylmorpholine.

$^1H$ NMR (400 MHz, DMSO-$d_6$) δ 9.03 (s, 1H), 8.68 (d, *J* = 7.1 Hz, 1H), 7.67 (t, *J* = 7.3 Hz, 1H), 7.52 (t, *J* = 6.9 Hz, 1H), 7.31 (t, *J* = 7.7 Hz, 1H), 7.23 (t, *J* = 54.4 Hz, 1H), 5.74 (m, 1H), 4.01-3.37 (m, 12H), 3.21-3.07 (m, 2H), 3.06-2.96 (m, 1H), 2.21 (s, 3H), 1.55 (d, *J* = 7.1 Hz, 3H), 0.71 (d, *J* = 6.4 Hz, 3H). LC/MS(m/z, $MH^+$): 533.2.

**Example 69 (*R*)-4-((1-(3-(difluoromethyl)-2-fluorophenyl)ethyl)amino)-2-methyl-8-(4-methylpiperazin-1-yl)-6-morpholinepyrido[4,3-*d*]pyrimidine-7(6*H*)-one**

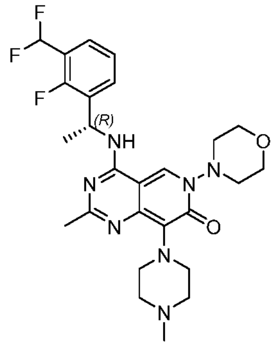

The title compound was prepared in the same manner as in Example **20** except for replacing the solution of sodium methoxide in methanol in step **4** with *N*-methylpiperazine.

$^1H$ NMR (400 MHz, DMSO-$d_6$) δ 8.84 (s, 1H), 8.58 (d, *J* = 7.2 Hz, 1H), 7.65 (t, *J* = 7.3 Hz, 1H), 7.51 (t, *J* = 7.0 Hz, 1H), 7.30 (t, *J* = 7.7 Hz, 1H), 7.23 (t, *J* = 54.4 Hz, 1H), 5.74 (m, 1H), 4.12-3.37 (m, 8H), 3.30-3.22 (m, 4H), 2.40-2.31 (m, 4H), 2.19 (s, 3H), 2.18 (s, 3H), 1.54 (d, *J* = 7.1 Hz, 3H).

LC/MS(m/z, $MH^+$): 532.2.

**Example 70 (*R*)-2-methyl-4-((1-(2-methyl-3-(trifluoromethyl)phenyl)ethyl)amino)-8-(4-methylpiperazin-1-yl)-6-morpholinepyrido[4,3-*d*]pyrimidine-7(6*H*)-one**

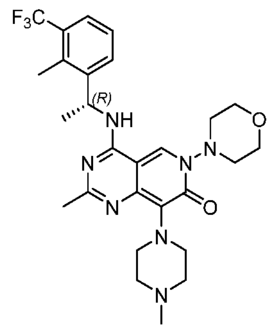

Reference was made to the synthesis method of Example **20,** except for replacing (*R*)-1-(3-(difluoromethyl)-2-fluorophenyl)ethane-1-amine in step **1** with (*R*)-1-(2-methyl-3-(trifluoromethyl)phenyl)ethane-1-amine, and simultaneously replacing the solution of sodium methoxide in methanol in step **4** with *N*-methylpiperazine. The title compound was prepared in the same manner.

$^1H$ NMR (400 MHz, DMSO-$d_6$) δ 8.82 (s, 1H), 8.66 (d, *J* = 7.0 Hz, 1H), 7.74 (d, *J* = 7.9 Hz, 1H), 7.55 (d, *J* = 7.8 Hz, 1H), 7.38 (t, *J* = 7.7 Hz, 1H), 5.71-5.62 (m, 1H), 4.04-3.39 (m, 8H), 3.30-3.22 (m, 4H), 2.58 (s, 3H), 2.40-2.30 (m, 4H), 2.19 (s, 3H), 2.18 (s, 3H), 1.50 (d, *J* = 7.0 Hz, 3H). LC/MS(m/z, $MH^+$): 546.2.

**Example 71 (*R*)-3-(1-((8-(4-cyanophenyl)-6-(1-(fluoromethyl)cyclopropyl)-2-methyl-7-oxo-6,7-dihydropyrido[4,3-*d*]pyrimidin-4-yl)amino)ethyl)-2-methylbenzonitrile**

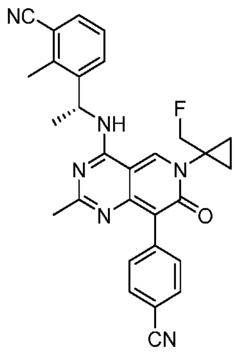

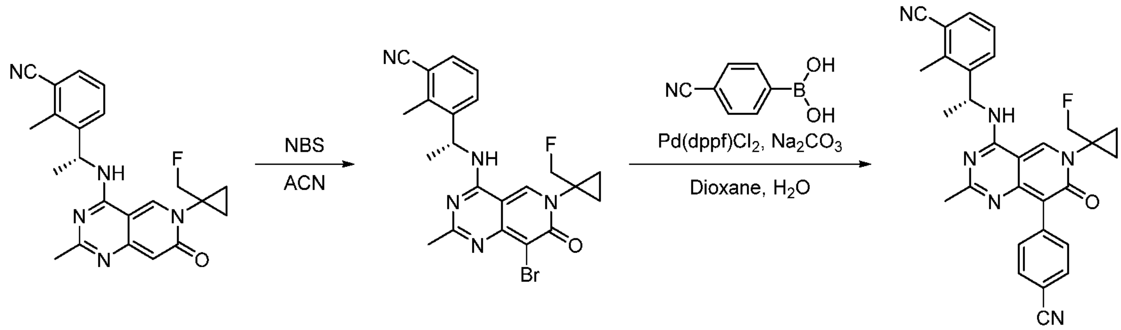


Reference was made to the synthesis method of Example **22,** and the title compound was prepared according to the above-mentioned technical route.

$^1$H NMR (400 MHz, DMSO-$d_6$) δ 9.24 (s, 1H), 9.03 (d, *J* = 6.8 Hz, 1H), 7.79-7.72 (m, 3H), 7.69-7.60 (m, 3H), 7.41 (t, *J* = 7.8 Hz, 1H), 5.69-5.57 (m, 1H), 4.84-4.45 (m, 2H), 2.66 (s, 3H), 2.13 (s, 3H), 1.54 (d, *J* = 7.0 Hz, 3H), 1.44-1.19 (m, 4H). LC/MS(m/z, MH$^+$): 493.2.

**Example 72 (*R*)-4-((4-((1-(3-(difluoromethyl)-2-fluorophenyl)ethyl)amino)-6-(1-(fluoromethyl) cyclopropyl)-2-methyl-7-oxo-6,7-dihydropyrido[4,3-*d*]pyrimidin-8-yl)oxy)pyridinecarbonitrile**

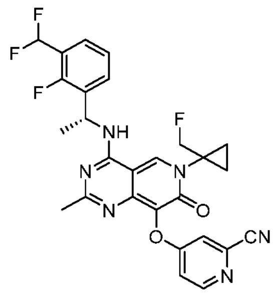

The title compound was prepared in the same manner as in Example **23** except for replacing methanol-$d_3$ in step **4** with 2-cyano-4-hydroxypyridine.

$^1$H NMR (400 MHz, DMSO-$d_6$) δ 9.25 (s, 1H), 9.11 (d, *J* = 7.1 Hz, 1H), 8.50 (d, *J* = 5.8 Hz, 1H), 7.69 (t, *J* = 7.3 Hz, 1H), 7.65 (d, *J* = 2.5 Hz, 1H), 7.54 (t, *J* = 7.0 Hz, 1H), 7.39-7.10 (m, 3H), 5.84-5.74 (m, 1H), 4.79-4.47 (m, 2H), 2.16 (s, 3H), 1.61 (d, *J* = 7.1 Hz, 3H), 1.39-1.20 (m, 4H). LC/MS(m/z, MH$^+$): 539.2.

**Example 73 (*R*)-8-((1-acetylpiperidin-4-yl)oxy)-4-((1-(3-(difluoromethyl)-2-fluorophenyl) ethyl)amino)-6-(1-(fluoromethyl)cyclopropyl)-2-methylpyrido[4,3-*d*]pyrimidine-7(6*H*)-one**

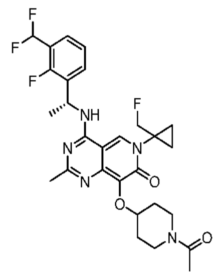

The title compound was prepared in the same manner as in Example **23** except for replacing methanol-$d_3$ in step **4** with 1-(4-hydroxypiperidin-1-yl)ethanone.

$^1$H NMR (400 MHz, DMSO-$d_6$) δ 8.97 (s, 1H), 8.82 (s, 1H), 7.66 (t, *J* = 7.2 Hz, 1H), 7.53 (t, *J* = 6.9 Hz, 1H), 7.33 (t, *J* = 7.7 Hz, 1H), 7.24 (t, *J* = 54.3 Hz, 1H), 5.82-5.72 (m, 1H), 4.82-4.46 (m, 3H), 3.80-3.72 (m, 1H), 3.70-3.61 (m, 1H), 3.55-3.38 (m, 1H), 3.32-3.24 (m, 1H), 2.22 (s, 3H), 1.98 (d, *J* = 2.9 Hz, 3H), 1.79-1.54 (m, 7H), 1.38-1.19 (m, 4H). LC/MS(m/z, MH$^+$): 562.2.

**Example 74 (*R*)-3-(1-((6-(1-(fluoromethyl)cyclopropyl)-2-methyl-8-(4-methylpiperazin-1-yl)-7-oxo-6,7-dihydropyrido[4,3-*d*]pyrimidin-4-yl)amino)ethyl)-2-methylbenzonitrile**

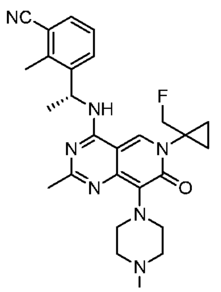

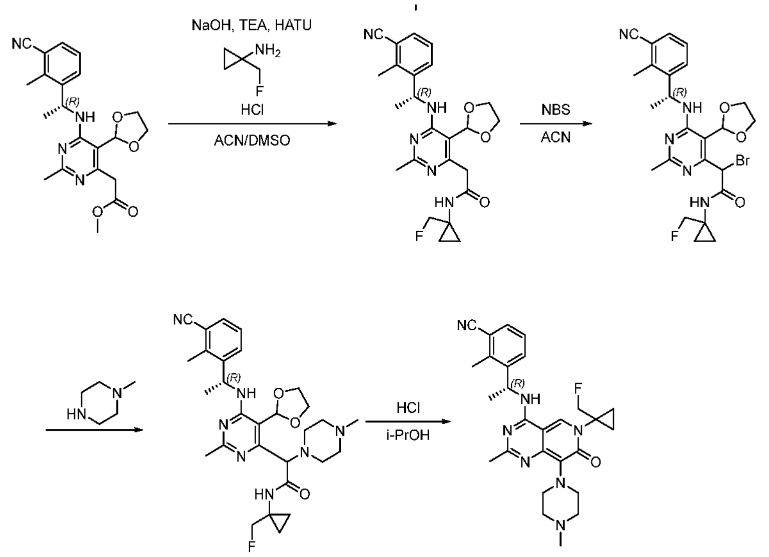


Reference was made to step **2** and step **3** of Example **23** and the synthesis method of Example **34.** The title compound was prepared by the above-mentioned technical route.

$^1$H NMR (400 MHz, DMSO-$d_6$) δ 8.74 (s, 1H), 8.67 (d, *J* = 6.9 Hz, 1H), 7.71 (d, *J* = 7.8 Hz, 1H), 7.67-7.62 (m, 1H), 7.38 (t, *J* = 7.8 Hz, 1H), 5.64-5.53 (m, 1H), 4.81-4.47 (m, 2H), 3.31-3.19 (m, 4H), 2.66 (s, 3H), 2.34 (t, *J* = 4.6 Hz, 4H), 2.19 (s, 3H), 2.17 (s, 3H), 1.51 (d, *J* = 7.0 Hz, 3H), 1.33-1.19 (m, 4H).

LC/MS(m/z, MH$^+$): 490.2.

**Example 75 (*R*)-1-(4-((1-(3-(difluoromethyl)-2-fluorophenyl)ethyl)amino)-6-(1-(fluoromethyl) cyclopropyl)-2-methyl-7-oxo-6,7-dihydropyrido[4,3-*d*]pyrimidin-8-yl)-3-methylazetidine-3-carbonitrile**

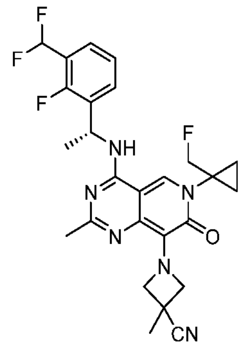

**Step 1: synthesis of 2-(3-cyano-3-methylazetidin-1-yl)-2-(6-(((*R*)-1-(3-(difluoromethyl)-2-fluorophenyl)ethyl)amino)-5-(1,3-dioxolan-2-yl)-2-methylpyrimidin-4-yl)-*N*-(1-(fluoromethyl)cyclopropyl)acetamide**

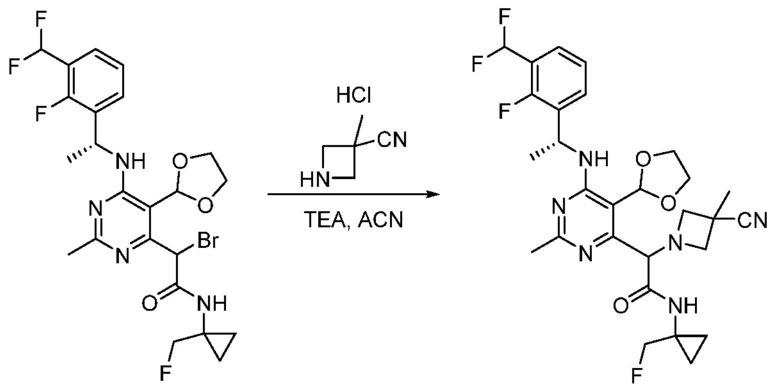


2-Bromo-2-(6-((*R*)-1-(3-(difluoromethyl)-2-fluorophenyl)ethyl)amino)-5-(1,3-dioxolan-2-yl)-2-methylpyrimidin-4-yl)-*N*-(1-(fluoromethyl)cyclopropyl)acetamide (100 mg) was added to a reaction flask. 3-Methyl-3-cyanoazetidine hydrochloride (100 mg), triethylamine (300 mg), and acetonitrile (10 mL) were added. The reaction was completed after the mixture was stirred at room temperature for 1 h. The reaction mixture was concentrated and purified by column chromatography (dichloromethane/methanol gradient elution) to give the title compound.

**Step 2: synthesis of (*R*)-1-(4-((1-(3-(difluoromethyl)-2-fluorophenyl)ethyl)amino)-6-(1-(fluoromethyl)cyclopropyl)-2-methyl-7-oxo-6,7-dihydropyrido[4,3-*d*]pyrimidin-8-yl)-3-methylazetidine-3-carbonitrile**

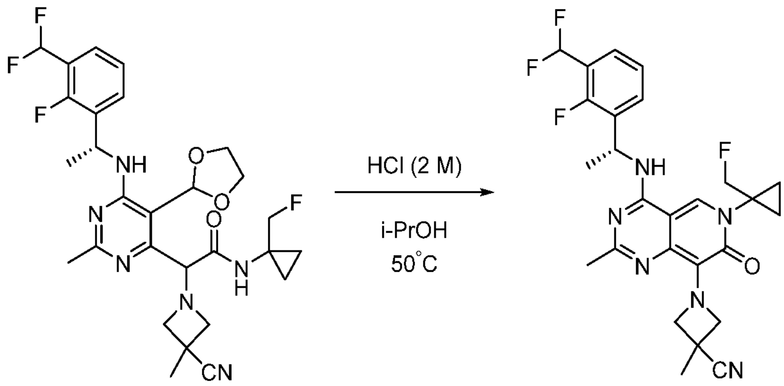


2-(3-Cyano-3-methylazetidin-1-yl)-2-(6-(((*R*)-1-(3-(difluoromethyl)-2-fluorophenyl)ethyl)amino)-5-(1,3-dioxolan-2-yl)-2-methylpyrimidin-4-yl)-*N*-(1-(fluoromethyl)cyclopropyl)acetamide (80.0 mg) was added to a reaction flask. Isopropanol (1.4 mL) was added, and then diluted hydrochloric acid (2 M, 0.2 mL) was added. The reaction was completed after the mixture was heated to 50 °C and stirred for 4 h. The reaction mixture was concentrated and purified by preparative HPLC (mobile phase: acetonitrile/water gradient elution) to give the title compound.

$^1$H NMR (400 MHz, DMSO-$d_6$) δ 8.54 (d, *J* = 7.2 Hz, 1H), 8.50 (s, 1H), 7.61 (t, *J* = 7.1 Hz, 1H), 7.51 (t, *J* = 6.9 Hz, 1H), 7.31 (t, *J* = 7.7 Hz, 1H), 7.23 (t, *J* = 54.4 Hz, 1H), 5.81-5.68 (m, 1H), 4.76-4.43 (m, 4H), 4.23 (d, *J* = 9.2 Hz, 2H), 2.14 (s, 3H), 1.56 (d, *J* = 7.1 Hz, 3H), 1.55 (s, 3H), 1.33-1.17 (m, 4H). LC/MS(m/z, $MH^+$): 515.2.

**Example 76 (*R*)-2-methyl-3-(1-((2-methyl-8-(4-methylpiperazin-1-yl)-6-morpholine-7-oxo-6,7-dihydropyrido[4,3-*d*]pyrimidin-4-yl)amino)ethyl)benzonitrile**

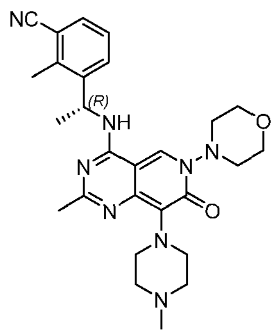

**Step 1: synthesis of methyl (*R*)-2-(6-((1-(3-cyano-2-methylphenyl)ethyl)amino)-5-(1,3-dioxolan-2-yl)-2-methylpyrimidin-4-yl)acetate**

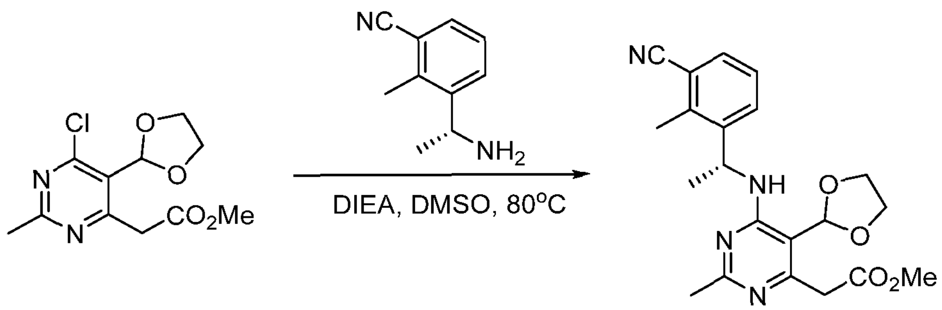


(*R*)-3-(1-aminoethyl)-2-methylbenzonitrile (5.00 g) and methyl 2-(6-chloro-5-(1,3-dioxolan-2-yl)-2-methylpyrimidin-4-yl)acetate (8.51 g) were added to dimethyl sulfoxide (35 mL), and *N,N*-diisopropylethylamine (10.1 g) was added.

The reaction was completed after the mixture was heated to 80 °C and stirred for 6 h under argon atmosphere. The reaction mixture was poured into saturated brine and extracted with ethyl acetate. The organic phase was dried over anhydrous sodium sulfate, filtered, concentrated, and purified by column chromatography (petroleum ether/ethyl acetate gradient elution) to give the title compound.

**Step 2: synthesis of (*R*)-2-(6-((1-(3-cyano-2-methylphenyl)ethyl)amino)-5-(1,3-dioxolan-2-yl)-2-methylpyrimidin-4-yl)-*N*-morpholinylacetamide**

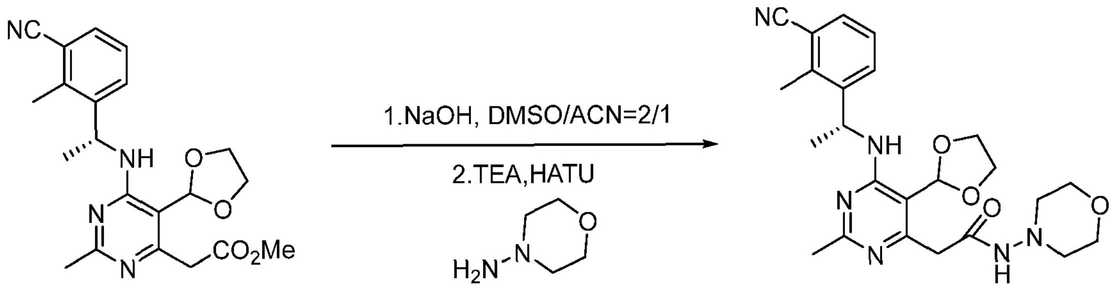


Methyl (R)-2-(6-((1-(3-cyano-2-methylphenyl)ethyl)amino)-5-(1,3-dioxolan-2-yl)-2-methylpyrimidin-4-yl)acetate (7.00 g) was dissolved in a mixed solution (70 mL) of dimethyl sulfoxide/acetonitrile = 2/1. Sodium hydroxide (2.83 g) was added, and the mixture was stirred at room temperature for half an hour. Triethylamine (3.57 g), *N*-aminomorpholine (2.16 g), and 2-(7-azabenzotriazol-1-yl)-*N,N,N',N'*-tetramethyluronium hexafluorophosphate (9.99 g) were slowly added sequentially, and the reaction was completed after the mixture was stirred at room temperature for 2 h. Saturated brine was added, and the mixture was extracted with ethyl acetate. The organic layers were combined. The organic phase was dried over anhydrous sodium sulfate, filtered, concentrated, and purified by column chromatography (petroleum ether/ethyl acetate gradient elution) to give the title compound.

**Step 3: synthesis of 2-chloro-2-(6-(((*R*)-1-(3-cyano-2-methylphenyl)ethyl)amino)-5-(1,3-dioxolan-2-yl)-2-methylpyrimidin-4-yl)-*N*-morpholineacetamide**

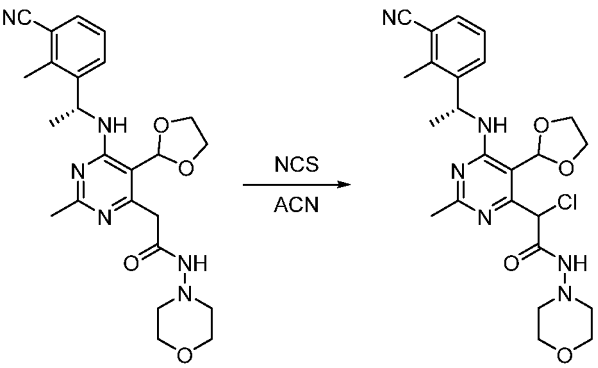


(*R*)-2-(6-((1-(3-Cyano-2-methylphenyl)ethyl)amino)-5-(1,3-dioxolan-2-yl)-2-methylpyrimidin-4-yl)-*N*-morpholinylacetamide (2.70 g) was added to 20 mL of acetonitrile. A solution (10 mL) of *N*-chlorosuccinimide (772 mg) in acetonitrile was slowly added. The reaction was completed after the mixture was stirred at room temperature for 1 h. The reaction mixture was concentrated and purified by column chromatography (petroleum ether/ethyl acetate gradient elution) to give the title compound.

**Step 4: synthesis of 2-(6-(((*R*)-1-(3-cyano-2-methylphenyl)ethyl)amino)-5-(1,3-dioxolan-2-yl)-2-methylpyrimidin-4-yl)-2-(4-methylpiperazin-1-yl)-*N*-morpholineacetamide**

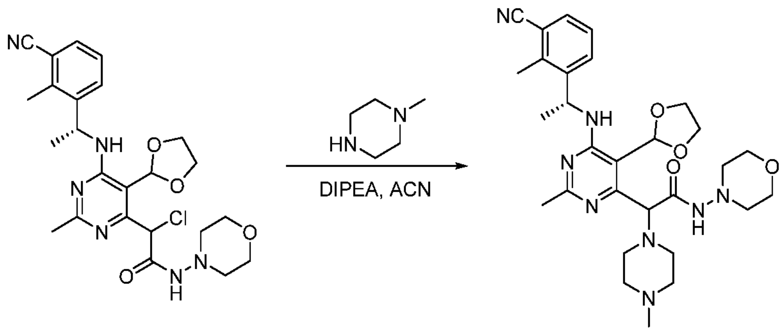


2-Chloro-2-(6-(((*R*)-1-(3-cyano-2-methylphenyl)ethyl)amino)-5-(1,3-dioxolan-2-yl)-2-methylpyrimidin-4-yl)-*N*-morpholineacetamide (200 mg) and *N*-methylpiperazine (200 mg) were placed in a reaction flask. 10 mL of acetonitrile was added. *N*,*N*-Diisopropylethylamine (194 mg) was added. The reaction was completed after the mixture was stirred at room temperature for 10 h. The mixture was quenched with water and extracted with ethyl acetate. The organic phase was dried over anhydrous sodium sulfate, filtered, concentrated, and purified by column chromatography (dichloromethane/methanol gradient elution) to give the title compound.

**Step 5: synthesis of (*R*)-2-methyl-3-(1-((2-methyl-8-(4-methylpiperazin-1-yl)-6-morpholine-7-oxo-6,7-dihdrorido[4,3-*d*]pyrimidin-4-yl)amino)ethyl)benzonitrile**

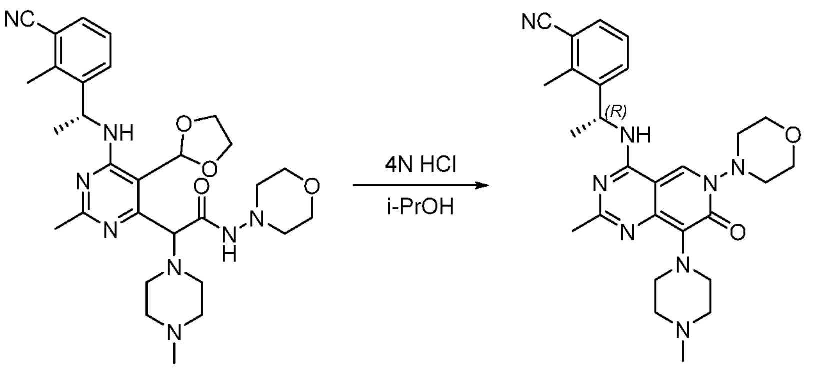


2-(6-(((*R*)-1-(3-Cyano-2-methylphenyl)ethyl)amino)-5-(1,3-dioxolan-2-yl)-2-methylpyrimidin-4-yl)-2-(4-methylpiperazin-1-yl)-*N*-morpholineacetamide (100 mg) was dissolved in 2 mL of isopropanol. 4 M hydrochloric acid solution (139 μL) was added. The reaction was completed after the mixture was heated to 50 °C and stirred for reaction for 1.5 h. The reaction mixture was concentrated and purified by preparative HPLC (mobile phase: acetonitrile/water gradient elution) to give the title compound.

$^1$H NMR (400 MHz, DMSO-$d_6$) δ 8.81 (s, 1H), 8.65 (d, *J* = 7.0 Hz, 1H), 7.75 (d, *J* = 8.0 Hz, 1H), 7.65 (d, *J* = 7.5 Hz, 1H), 7.38 (t, *J* = 7.8 Hz, 1H), 5.59 (m, 1H), 4.23-3.37 (m, 8H), 3.30-3.20 (m, 4H), 2.66 (s, 3H), 2.40-2.30 (m, 4H), 2.21 (s, 3H), 2.18 (s, 3H), 1.49 (d, *J* = 7.0 Hz, 3H). LC/MS(m/z, MH$^+$): 503.2.

**Example 77 (*R*)-4-((1-(3-(difluoromethyl)-2-fluorophenyl)ethyl)amino)-6-(1-(fluoromethyl) cyclorol-2-methyl-8-(methylamino)pyrido[4,3-*d*]pyrimidine-7(6*H*)-one**

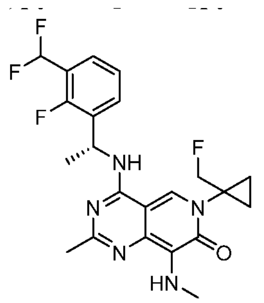

**Step 1: synthesis of 2-(benzyl(methyl)amino)-2-(6-(((*R*)-1-(3-(difluoromethyl)-2-fluorophenyl) ethyl)amino)-5-(1,3-dioxolan-2-yl)-2-methylpyrimidin-4-yl)-*N*-(1-(fluoromethyl)cyclopropyl)acetamide**

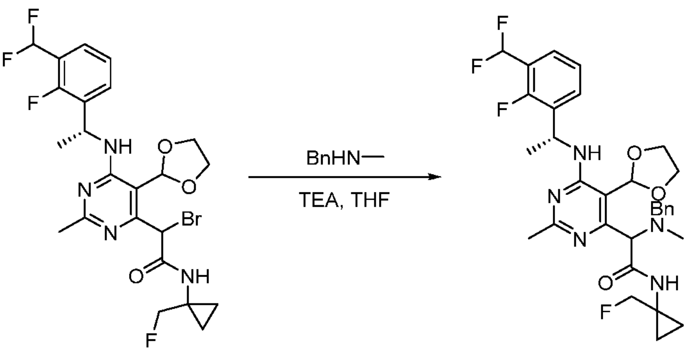


2-Bromo-2-(6-(((*R*)-1-(3-(difluoromethyl)-2-fluorophenyl)ethyl)amino)-5-(1,3-dioxolan-2-yl)-2-methylpyrimidin-4-yl)-*N*-(1-(fluoromethyl)cyclopropyl)acetamide (150 mg) and *N*-methylbenzylamine (38.3 mg) were dissolved in 2 mL of tetrahydrofuran. Triethylamine (27.0 mg) was added. The mixture was reacted at 50 °C overnight. After the reaction was completed, the reaction mixture was poured into water and extracted with dichloromethane. The organic phase was dried over anhydrous sodium sulfate, filtered, concentrated, and purified by column chromatography (petroleum ether/ethyl acetate gradient elution) to give the title compound.

**Step 2: synthesis of (*R*)-8-(benzyl(methyl)amino)-4-((1-(3-(difluoromethyl)-2-fluorophenyl) ethyl)amino)-6-(1-(fluoromethyl)cyclopropyl)-2-methylpyrido[4,3-*d*]pyrimidine-7(6*H*)-one**

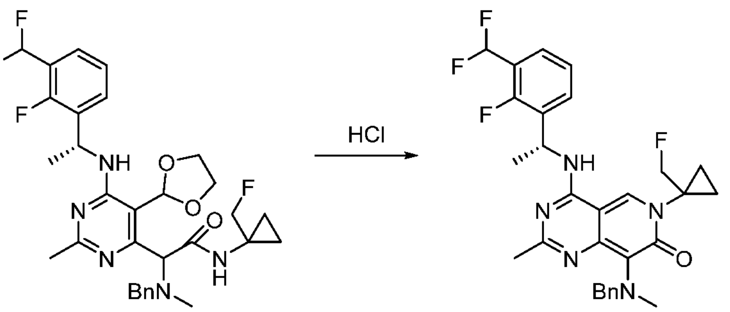


2-(Benzyl(methyl)amino)-2-(6-(((*R*)-1-(3-(difluoromethyl)-2-fluorophenyl)ethyl)amino)-5-(1,3-dioxolan-2-yl)-2-methylpyrimidin-4-yl)-*N*-(1-(fluoromethyl)cyclopropyl)acetamide (140 mg) was dissolved in 2 mL of isopropanol. 0.58 mL of 2 M hydrochloric acid solution was added. The reaction was completed after the mixture was reacted at 50 °C for 2 h. The pH was adjusted to 7-8 with a saturated sodium bicarbonate solution. The mixture was extracted with dichloromethane. The organic phase was dried over anhydrous sodium sulfate, filtered, concentrated, and purified by column chromatography (petroleum ether/ethyl acetate gradient elution) to give the title compound.

**Step 3:** synthesis **of (*R*)-4-((1-(3-(difluoromethyl)-2-fluorophenyl)ethyl)amino)-6-(1-(fluoromethyl)cyclopropyl)-2-methyl-8-(methylamino)pyrido[4,3-*d*]pyrimidine-7(6*H*)-one**

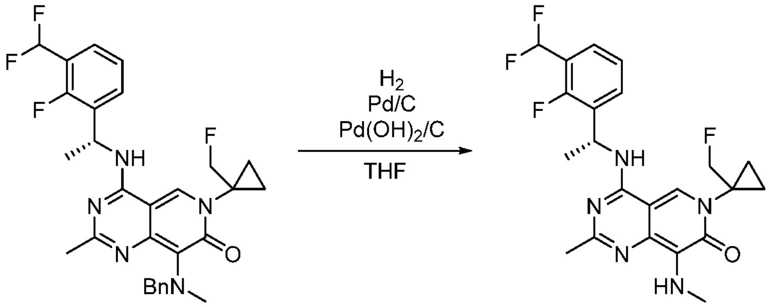


(*R*)-8-(Benzyl(methyl)amino)-4-((1-(3-(difluoromethyl)-2-fluorophenyl)ethyl)amino)-6-(1-(fluoromethyl)cyclopropyl)-2-methylpyrido[4,3-*d*]pyrimidine-7(6*H*)-one (120 mg) was dissolved in 4 mL of tetrahydrofuran. 12 mg of palladium/carbon and 12 mg of palladium hydroxide/carbon were added. The system was purged with hydrogen, and the reaction was completed after the mixture was reacted at room temperature for 4 h. The mixture was filtered, and the filtrate was concentrated and purified by column chromatography (dichloromethane/methanol gradient elution) to give the title compound.

$^1$H NMR (400 MHz, DMSO-$d_6$) $\delta$ 8.49 (d, *J* = 7.2 Hz, 1H), 8.37 (s, 1H), 7.62 (t, *J* = 7.3 Hz, 1H), 7.51 (t, *J* = 6.8 Hz, 1H), 7.31 (t, *J* = 7.7 Hz, 1H), 7.24 (t, *J* = 54.4 Hz, 1H), 5.82-5.71 (m, 1H), 5.30-5.18 (m, 1H), 4.80-4.48 (m, 2H), 3.18 (d, *J* = 4.1 Hz, 3H), 2.18 (s, 3H), 1.57 (d, *J* = 7.1 Hz, 3H), 1.35-1.20 (m, 4H). LC/MS(m/z, $MH^+$): 450.2.

**Example 78 (*R*)-4-((1-(3-(difluoromethyl)-2-fluorophenyl)ethyl-1-*d*)amino)-2-methyl-6,8-dimorpholinepyrido[4,3-*d*]pyrimidine-7(6*H*)-one**

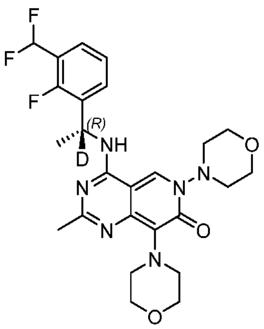

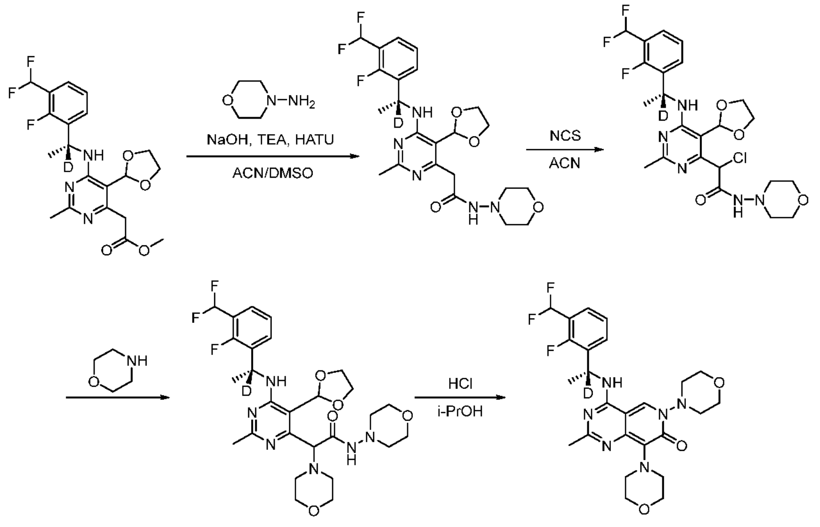


Reference was made to the preparation method of Example **76,** and the title compound was prepared according to the above-mentioned technical route.

$^1H$ NMR (400 MHz, DMSO-$d_6$) δ 8.89 (s, 1H), 8.62 (s, 1H), 7.66 (t, *J* = 7.3 Hz, 1H), 7.52 (t, *J* = 6.9 Hz, 1H), 7.31 (t, *J* = 7.7 Hz, 1H), 7.23 (t, *J* = 54.4 Hz, 1H), 4.11-3.37 (m, 12H), 3.30-3.22 (m, 4H), 2.21 (s, 3H), 1.54 (s, 3H). LC/MS(m/z, $MH^+$): 520.2.

**Example 79 4-(((*R*)-1-(3-(difluoromethyl)-2-fluorophenyl))ethyl)amino)-2-methyl-8-((*S*)-2-methylmorpholine)-6-morpholinepyrido[4,3-*d*]pyrimidine-7(6*H*)-one**

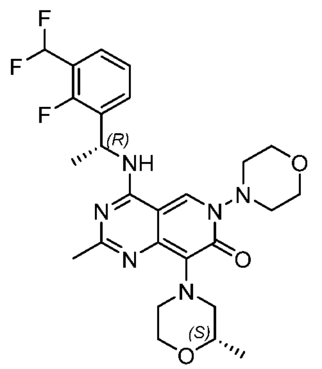

The title compound was prepared in the same manner as in Example **20** except for replacing the solution of sodium methoxide in methanol in step **4** with (*S*)-2-methylmorpholine.

$^1H$ NMR (400 MHz, DMSO-$d_6$) δ 8.89 (s, 1H), 8.62 (s, 1H), 7.65 (t, *J* = 7.5 Hz, 1H), 7.52 (t, *J* = 7.1 Hz, 1H), 7.31 (t, *J* = 7.7 Hz, 1H), 7.23 (t, *J* = 54.4 Hz, 1H), 5.79-5.70 (m, 1H), 4.15-3.35 (m, 12H), 3.23-3.13 (m, 1H), 3.12-2.95 (m, 2H), 2.21 (s, 3H), 1.55 (d, *J* = 7.1 Hz, 3H), 1.04 (d, *J* = 6.2 Hz, 3H). LC/MS(m/z, $MH^+$): 533.2.

**Example 80 (*R*)-3-(1-((6-(1-(fluoromethyl)cyclopropyl)-2-methyl-7-oxo-8-(3-(trifluoromethyl)-5,6-dihydro-[1,2,4]triazoto[4,3-*a*]pyrazin-7(8*H*)-yl)-6,7-dihydropyrido[4,3-*d*]pyrimidin-4-yl)amino)ethyl)-2-methylbenzonitrile**

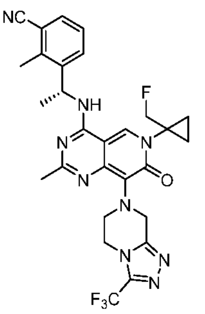

**Step 1: synthesis of 2-(6-(((*R*)-1-(3-cyano-2-methylphenyl)ethyl)amino)-5-(1,3-dioxolan-2-yl)-2-methylpyrimidin-4-yl)-*N*-(1-(fluoromethyl)cyclopropyl)-2-(3-(trifluoromethyl)-5,6-dihydro-[1,2,4]triazolo[4,3-*a*]pyrazin-7(8*H*)-yl)acetamide**

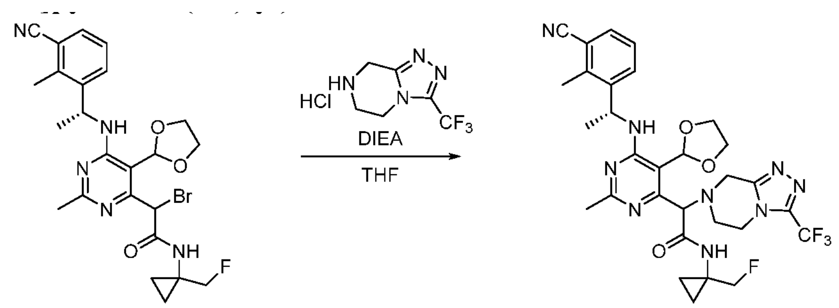


2-Bromo-2-(6-(((*R*)-1-(3-cyano-2-methylphenyl)ethyl)amino)-5-(1,3-dioxolan-2-yl)-2-methylpyrimidin-4-yl)-*N*-(1-(fluoromethyl)cyclopropyl)acetamide (200 mg) and 3-(trifluoromethyl)-5,6,7,8-tetrahydro-[1,2,4]triazolo[4,3-*a*]pyrazine hydrochloride (257 mg) were placed in a reaction flask. 2 mL of tetrahydrofuran was added. *N,N*-Diisopropylethylamine (194 mg) was added. The reaction was completed after the mixture was stirred at room temperature for 10 h. The mixture was quenched with water and extracted with ethyl acetate. The organic phase was dried over anhydrous sodium sulfate, filtered, concentrated, and purified by column chromatography (petroleum ether/ethyl acetate gradient elution) to give the title compound.

**Step 2: synthesis of (*R*)-3-(1-((6-(1-(fluoromethyl)cyclopropyl)-2-methyl-7-oxo-8-(3-(trifluoromethyl)-5,6-dihydro-[1,2,4]triazolo[4,3-*a*]pyrazin-7(8*H*)-yl)-6,7-dihydropyrido[4,3-*d*]pyrimidin-4-yl)amino)ethyl)-2-methylbenzonitrile**

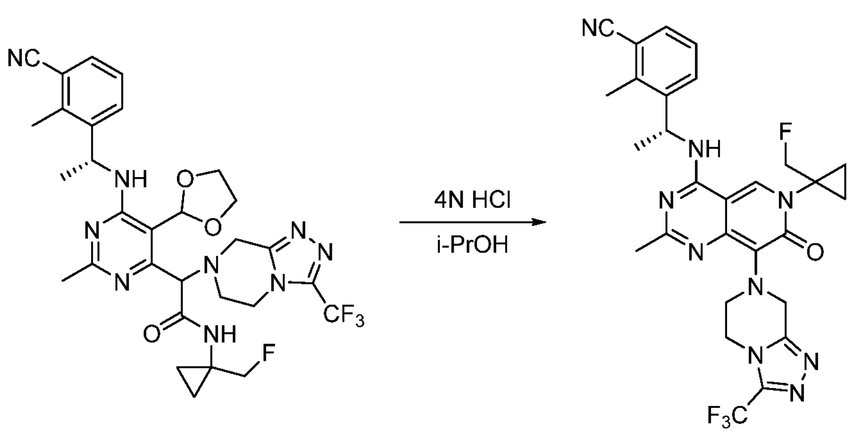


2-(6-(((*R*)-1-(3-Cyano-2-methylphenyl)ethyl)amino)-5-(1,3-dioxolan-2-yl)-2-methylpyrimidin-4-yl)-*N*-(1-(fluoromethyl)cyclopropyl)-2-(3-(trifluoromethyl)-5,6-dihydro-[1,2,4]triazolo[4,3-*a*]pyrazin-7(8*H*)-yl)acetamide (90 mg) was dissolved in 2 mL of isopropanol. 4 M hydrochloric acid solution (139 μL) was added. The reaction was completed after the mixture was heated to 50 °C and stirred for reaction for 1.5 h. The reaction mixture was concentrated and purified by preparative HPLC (mobile phase: acetonitrile/water gradient elution) to give the title compound.

$^1$H NMR (400 MHz, DMSO-$d_6$) δ 8.92 (s, 1H), 8.85 (d, *J* = 6.8 Hz, 1H), 7.71 (d, *J* = 7.6 Hz, 1H), 7.65 (d, *J* = 7.7 Hz, 1H), 7.39 (t, *J* = 7.8 Hz, 1H), 5.63-5.54 (m, 1H), 4.85-4.36 (m, 4H), 4.00 (t, *J* = 4.9 Hz, 2H), 3.59 (t, *J* = 5.2 Hz, 2H), 2.64 (s, 3H), 1.94 (s, 3H), 1.51 (d, *J* = 7.0 Hz, 3H), 1.38-1.16 (m, 4H). LC/MS(m/z, MH$^+$): 582.2.

**Example 81 (*R*)-2-methyl-6,8-dimorpholine-4-((1-(3-(pentafluoro-λ$^6$-sulfanyl)phenyl)ethyl) amino)pyrido[4,3-*d*]pyrimidine-7(6*H*)-one**

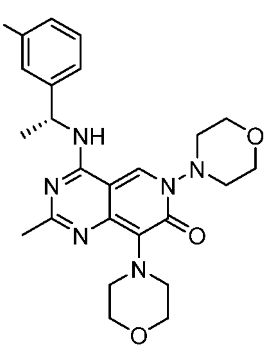

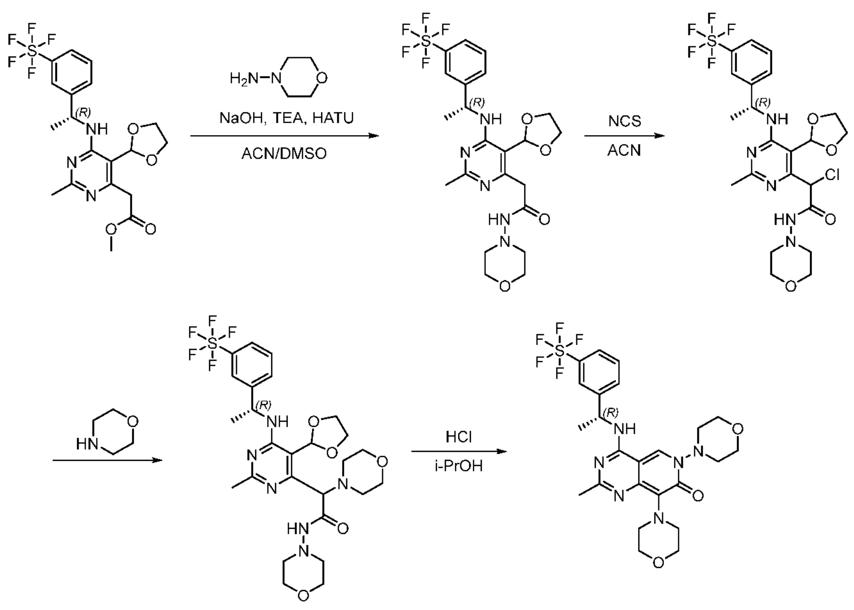


Reference was made to the synthesis method of Example **76,** and the title compound was prepared according to the above-mentioned technical route.

$^1$H NMR (400 MHz, DMSO-$d_6$) δ 8.81 (s, 1H), 8.61 (d, *J* = 7.6 Hz, 1H), 7.96 (s, 1H), 7.77 (d, *J* = 8.3 Hz, 1H), 7.71 (d, *J* = 7.7 Hz, 1H), 7.58 (t, *J* = 8.0 Hz, 1H), 5.60-5.51 (m, 1H), 4.13-3.37 (m, 12H), 3.31-3.24 (m, 4H), 2.23 (s, 3H), 1.57 (d, *J* = 7.0 Hz, 3H). LC/MS(m/z, MH$^+$): 577.2.

**Example 82 (*R*)-2-methyl-8-(4-methylpiperazin-1-yl)-6-morpholine-4-((1-(3-(pentafluoro-λ$^6$-sulfanyl)phenyl)ethyl)amino)pyrido[4,3-*d*]pyrimidine-7(6*H*)-one**

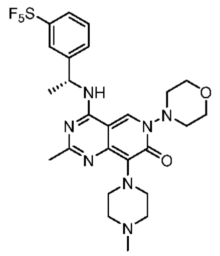

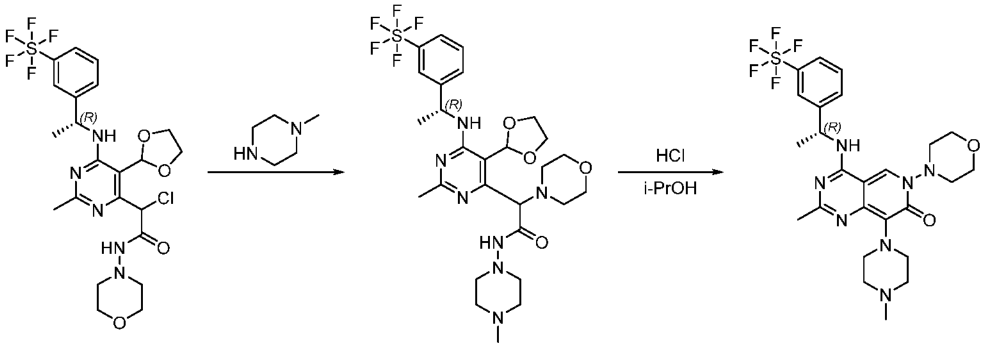


Reference was made to the synthesis method of Example **76,** and the title compound was prepared according to the above-mentioned technical route.

$^1$H NMR (400 MHz, DMSO-$d_6$) δ 8.77 (s, 1H), 8.58 (d, *J* = 7.3 Hz, 1H), 7.95 (s, 1H), 7.79-7.75 (m, 1H), 7.70 (d, *J* = 7.6 Hz, 1H), 7.58 (t, *J* = 8.0 Hz, 1H), 5.59-5.50 (m, 1H), 4.27-3.39 (m, 8H), 3.32-3.26 (m, 4H), 2.47-2.37 (m, 4H), 2.24 (s, 3H), 2.23 (s, 3H), 1.56 (d, *J* = 7.1 Hz, 3H). LC/MS(m/z, MH$^+$): 590.2.

**Example 83 (*R*)-*N*-(4-((1-(3-(difluoromethyl)-2-fluorophenyl)ethyl)amino)-6-(1-(fluoromethyl) cyclopropyl)-2-methyl-7-oxo-6,7-dihydropyrido[4,3-*d*]pyrimidine-8-yl)acetamide**

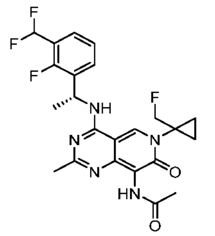

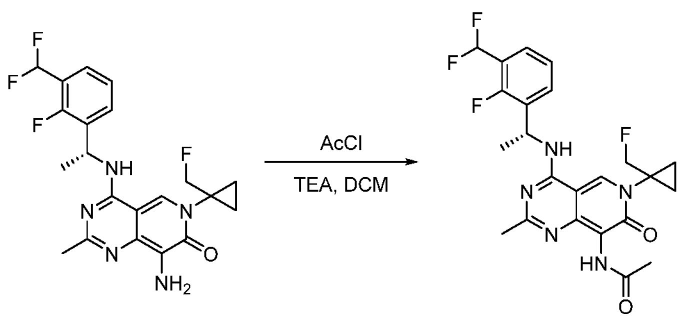


(*R*)-8-Amino-4-((1-(3-(difluoromethyl)-2-fluorophenyl)ethyl)amino)-6-(1-(fluoromethyl)cyclopropyl)-2-methylpyrido[4,3-*d*]pyrimidine-7(6*H*)-one (100 mg) was added to 10 mL of anhydrous dichloromethane. Triethylamine (100 mg) was added. Acetyl chloride (20.0 mg) was added dropwise to the reaction mixture at 0 °C. The reaction was completed after the mixture was stirred at room temperature for 1 h. The reaction mixture was concentrated and purified by preparative HPLC (mobile phase: acetonitrile/water gradient elution) to give the title compound.

$^1H$ NMR (400 MHz, DMSO-$d_6$) δ 9.13 (s, 1H), 9.05-8.74 (m, 2H), 7.65 (t, $J$ = 7.3 Hz, 1H), 7.52 (t, $J$ = 7.0 Hz, 1H), 7.32 (t, $J$ = 7.7 Hz, 1H), 7.24 (t, $J$ = 54.4 Hz, 1H), 5.76 (m, 1H), 4.82-4.46 (m, 2H), 2.20 (s, 3H), 1.91 (s, 3H), 1.59 (d, $J$ = 7.1 Hz, 3H), 1.40-1.21 (m, 4H). LC/MS(m/z, $MH^+$): 478.2.

**Example 84 (*R*)-2-methyl-3-(1-((2-methyl-8-(4-methyl-3-oxopiperazin-1-yl)-6-morpholine-7-oxo-6,7-dihydropyrido[4,3-*d*]pyrimidin-4-yl)amino)ethyl)benzonitrile**

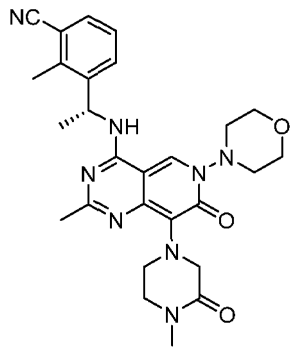

The title compound was prepared in the same manner as in Example **76** except for replacing N-methylpiperazine in step **4** with 1-methylpiperazine-2-one.

$^1H$ NMR (400 MHz, DMSO-$d_6$) δ 8.92 (s, 1H), 8.76 (d, $J$ = 7.0 Hz, 1H), 7.75 (d, $J$ = 7.8 Hz, 1H), 7.69-7.59 (m, 1H), 7.39 (t, $J$ = 7.8 Hz, 1H), 5.64-5.55 (m, 1H), 4.02-3.49 (m, 8H), 3.42 (t, $J$ = 5.2 Hz, 2H), 3.21 (t, $J$ = 5.2 Hz, 2H), 2.84 (s, 3H), 2.66 (s, 3H), 2.19 (s, 3H), 1.49 (d, $J$ = 7.0 Hz, 3H). LC/MS(m/z, $MH^+$): 517.2.

**Example 85 6-(1-(fluoromethyl)cyclopropyl)-2-methyl-4-(((*R*)-1-(2-methyl-3-(trifluoromethyl) phenyl)ethyl)amino)-8-((*R*)-2-methylmorpholine)pyrido[4,3-*d*]pyrimidine-7(6*H*)-one**

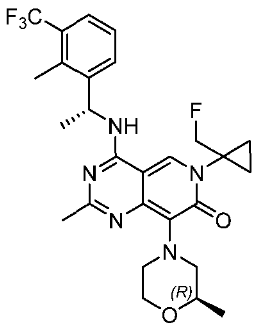

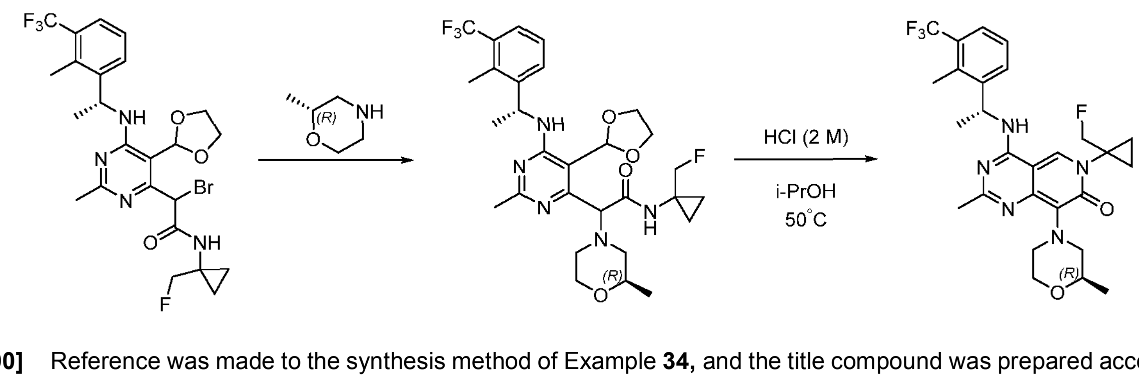


Reference was made to the synthesis method of Example **34,** and the title compound was prepared according to the above-mentioned technical route.

$^1H$ NMR (400 MHz, DMSO-$d_6$) δ 8.87-8.68 (m, 2H), 7.70 (d, *J* = 7.8 Hz, 1H), 7.55 (d, *J* = 7.7 Hz, 1H), 7.38 (t, *J* = 7.8 Hz, 1H), 5.72-5.61 (m, 1H), 4.83-4.41 (m, 2H), 3.75-3.69 (m, 1H), 3.66-3.55 (m, 2H), 3.32-3.29 (m, 1H), 3.19-3.05 (m, 2H), 3.05-2.97 (m, 1H), 2.57 (s, 3H), 2.19 (s, 3H), 1.52 (d, *J* = 7.0 Hz, 3H), 1.34-1.19 (m, 4H), 1.02 (d, *J* = 6.2 Hz, 3H). LC/MS(m/z, MH⁺): 534.2.

**Example 86 (*R*)-4-((1-(3-(difluoromethyl)-2-fluorophenyl)ethyl)amino)-6-(1-(fluoromethyl) cyclopropyl)-2-methyl-7-oxo-6,7-dihydropyrido[4,3-*d*]pyrimidin-8-yl-4-methylpiperazine-1-carboxylate**

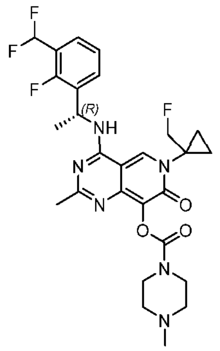

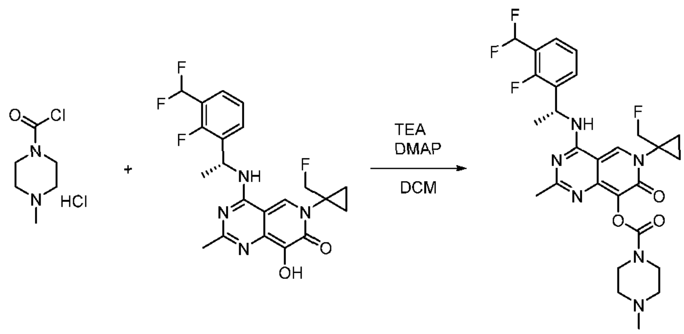


(*R*)-4-((1-(3-(Difluoromethyl)-2-fluorophenyl)ethyl)amino)-6-(1-(fluoromethyl)cyclopropyl)-8-hydroxy-2-methylpyrido[4,3-*d*]pyrimidine-7(6*H*)-one (42.0 mg) and 4-methylpiperazine-1-carbonyl chloride hydrochloride (22.9 mg) were dissolved in 2 mL of dichloromethane. Triethylamine (19.4 mg) and 4-dimethylaminopyridine (11.7 mg) were added. The reaction was completed after the mixture was stirred at 40 °C for 6 h. 10 mL of water was added, and the mixture was extracted with dichloromethane. The organic phase was dried over anhydrous sodium sulfate, filtered, concentrated, and purified by preparative HPLC (mobile phase: acetonitrile/water gradient elution) to give the title compound.

$^1H$ NMR (400 MHz, DMSO-$d_6$) δ 9.11 (s, 1H), 8.97 (d, *J* = 7.0 Hz, 1H), 7.66 (t, *J* = 7.3 Hz, 1H), 7.53 (t, *J* = 7.0 Hz, 1H), 7.32 (t, *J* = 7.7 Hz, 1H), 7.24 (t, *J* = 54.4 Hz, 1H), 5.81-5.71 (m, 1H), 4.81-4.45 (m, 2H), 3.64-3.49 (m, 2H), 3.44-3.36 (m, 2H), 2.43-2.30 (m, 4H), 2.22 (s, 3H), 2.20 (s, 3H), 1.59 (d, *J* = 7.1 Hz, 3H), 1.36-1.21 (m, 4H). LC/MS(m/z, MH⁺): 563.2.

**Example 87 (*R*)-2-methyl-3-(1-((2-methyl-6-morpholine-7-oxo-8-(piperazin-1-yl)-6,7-dihydropyrido [4,3-*d*]pyrimidin-4-yl)amino)ethyl)benzonitrile**

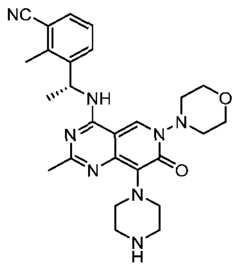

**Step 1: synthesis of *tert*-butyl 4-(1-(6-(((*R*)-1-(3-cyano-2-methylphenyl)ethyl)amino)-5-(1,3-dioxolan-2-yl)-2-methylpyrimidin-4-yl)-2-(morpholineamino)-2-oxoethyl)piperazine-1-carboxylate**

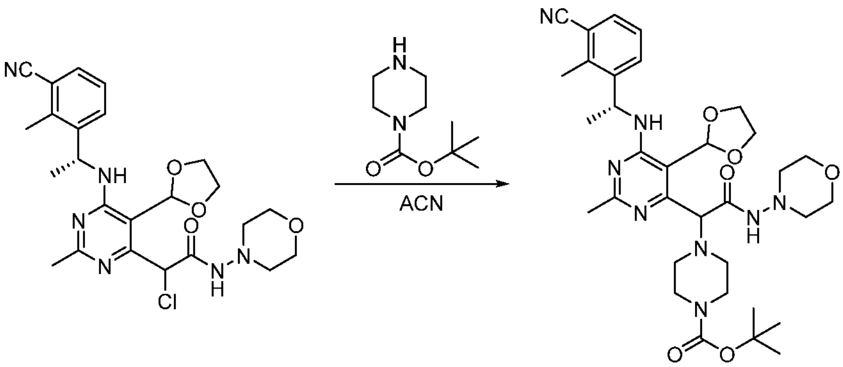


2-Chloro-2-(6-(((*R*)-1-(3-cyano-2-methylphenyl)ethyl)amino)-5-(1,3-dioxolan-2-yl)-2-methylpyrimidin-4-yl)-*N*-morpholinylacetamide (170 mg) was added to 4 mL of acetonitrile. *tert*-Butyl piperazine-1-carboxylate (190 mg) was added. The reaction was completed after the mixture was stirred at 50 °C for 10 h. The reaction mixture was concentrated and purified by column chromatography (petroleum ether/ethyl acetate gradient elution) to give the title compound.

**Step 2: synthesis of (*R*)-2-methyl-3-(1-((2-methyl-6-morpholine-7-oxo-8-(piperazin-1-yl)-6,7-dihydropyrido[4,3-*d*]pyrimidin-4-yl)amino)ethyl)benzonitrile**

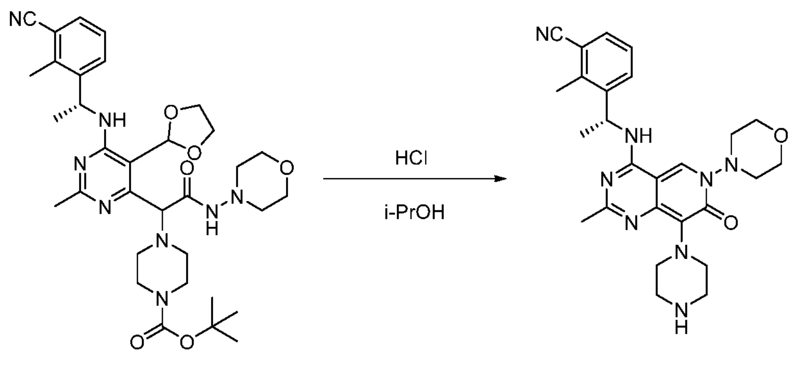


*tert*-Butyl 4-(1-(6-(((*R*)-1-(3-cyano-2-methylphenyl)ethyl)amino)-5-(1,3-dioxolan-2-yl)-2-methylpyrimidin-4-yl)-2-(morpholineamino)-2-oxoethyl)piperazine-1-carboxylate (80.0 mg) was dissolved in 1.5 mL of isopropanol. 4 N diluted hydrochloric acid 120 μL was added. The reaction was completed after the mixture was stirred at 50 °C for 8 h. The reaction mixture was concentrated and purified by preparative HPLC (mobile phase: acetonitrile/water gradient elution) to give the title compound.

$^1$H NMR (400 MHz, DMSO-$d_6$) δ 8.81 (s, 1H), 8.66 (d, *J* = 6.8 Hz, 1H), 7.75 (d, *J* = 7.6 Hz, 1H), 7.65 (d, *J* =

7.1 Hz, 1H), 7.38 (t, *J* = 7.8 Hz, 1H), 5.63-5.54 (m, 1H), 3.88 -3.25 (m, 8H), 3.26-3.15 (m, 4H), 2.84-2.71 (m, 4H), 2.66 (s, 3H), 2.21 (s, 3H), 1.49 (d, *J* = 7.0 Hz, 3H). LC/MS(m/z, $MH^+$): 489.2.

**Example 88 (*R*)-3-(1-((8-(1,1-dioxidothiomorpholine))-2-methyl-6-morpholinyl-7-oxo-6,7-dihydropyrido[4,3-*d*]pyrimidin-4-yl)amino)ethyl)-2-methylbenzonitrile**

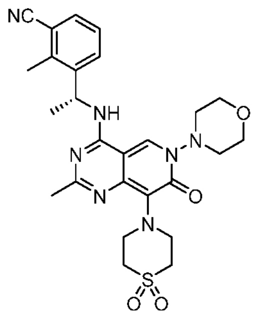

The title compound was prepared in the same manner as in Example **76** except for replacing N-methylpiperazine in step **4** with thiomorpholine-1,1-dioxide.

$^1$H NMR (400 MHz, DMSO-$d_6$) δ 8.92 (s, 1H), 8.79 (d, *J* = 6.0 Hz, 1H), 7.75 (d, *J* = 7.8 Hz, 1H), 7.66 (d, *J* = 6.9 Hz, 1H), 7.39 (t, *J* = 7.8 Hz, 1H), 5.66-5.54 (m, 1H), 4.16-3.40 (m, 12H), 3.23-3.10 (m, 4H), 2.66 (s, 3H), 2.25 (s, 3H), 1.50 (d, *J* = 7.0 Hz, 3H). LC/MS(m/z, $MH^+$): 538.2.

**Example 89 (*R*)-2-methyl-3-(1-((2-methyl-6-morpholinyl-7-oxo-8-(6-(pyrrolidin-1-yl)pyridin-3-yl)-6,7-dihydropyrido[4,3-*d*]pyrimidin-4-yl)amino)ethyl)benzonitrile**

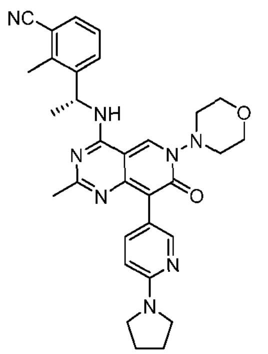

**Step 1: synthesis of 8-bromo-4-hydroxy-2-methyl-6-morpholinepyrido[4,3-*d*]pyrimidine-7(6*H*)-one**

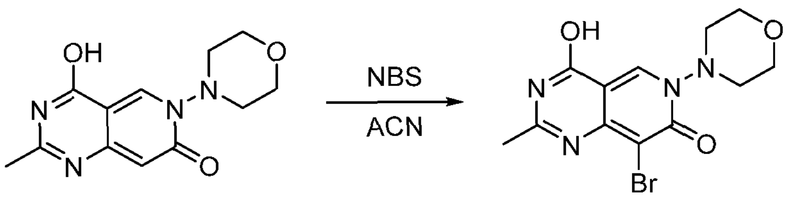


4-Hydroxy-2-methyl-6-morpholinylpyrido[4,3-*d*]pyrimidine-7(6*H*)-one (400 mg) was added to 4 mL of acetonitrile, and a solution of *N*-bromosuccinimide (0.27 g) in acetonitrile was slowly added dropwise. The reaction was completed after the mixture was reacted at room temperature for 2 h. The mixture was quenched with water and filtered.

The filter cake was washed with water, collected, and dried to give the title compound.

**Step 2: synthesis of 4-hydroxy-2-methyl-6-morpholinyl-8-(6-(pyrrolidin-1-yl)pyridin-3-yl)pyrido[4,3-*d*]rimidine-7(6*H*)-one**

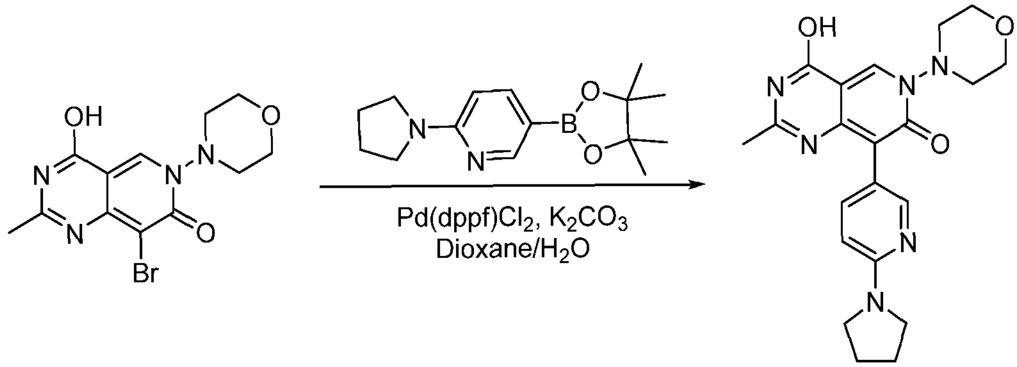


8-Bromo-4-hydroxy-2-methyl-6-morpholinepyrido[4,3-*d*]pyrimidine-7(6*H*)-one (200 mg), 2-pyrrolyl-5-pyridineboronate (200 mg), 1,1'-bis(diphenylphosphino)ferrocene dichloropalladium (42.9 mg), and potassium carbonate (0.16 g) were added to 2 mL of a mixed solution of dioxane/water (4:1). The reaction was completed after the mixture was heated to 90 °C and stirred for 2 h under argon atmosphere. The reaction mixture was concentrated and purified by column chromatography (petroleum ether/ethyl acetate gradient elution) to give the title compound.

**Step 3: synthesis of (*R*)-2-methyl-3-(1-((2-methyl-6-morpholinyl-7-oxo-8-(6-(pyrrolidin-1-yl)pyridin-3-yl)-6,7-dihydropyrido[4,3-*d*]pyrimidin-4-yl)amino)ethyl)benzonitrile**

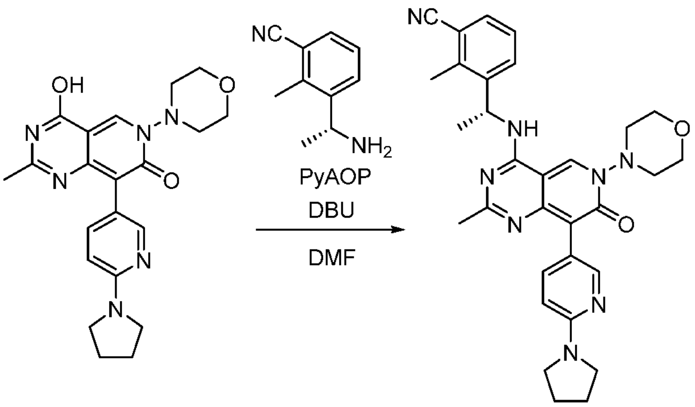


4-Hydroxy-2-methyl-6-morpholinyl-8-(6-(pyrrolidin-1-yl)pyridin-3-yl)pyrido[4,3-*d*]pyrimidine-7(6*H*)-one (100 mg), (*R*)-3-(1-aminoethyl)-2-methylbenzonitrile (47.1 mg), and 1,8-diazabicyclo[5.4.0]undec-7-ene (112 mg) were added to *N,N*-dimethylformamide (2 mL). After the mixture was stirred at room temperature for 20 min, (7-azabenzotriazole-1-oxy)tripyrrolidinephosphonium hexafluorophosphate (190 mg) was added. The reaction was completed after the mixture was stirred at room temperature for another 1 h. The mixture was diluted with ethyl acetate and washed with a saturated sodium chloride solution. The organic phases were combined, dried over anhydrous sodium sulfate, filtered, concentrated, and purified by column chromatography (dichloromethane/methanol gradient elution) to give the title compound.

$^1$H NMR (400 MHz, DMSO-$d_6$) δ 9.14 (s, 1H), 8.88 (d, *J* = 6.6 Hz, 1H), 8.13 (d, *J* = 2.1 Hz, 1H), 7.78 (d, *J* = 7.7 Hz, 1H), 7.67 (d, *J* = 7.6 Hz, 1H), 7.58 (d, *J* = 8.6 Hz, 1H), 7.40 (t, *J* = 7.8 Hz, 1H), 6.42 (d, *J* = 8.8 Hz, 1H), 5.75-5.54 (m, 1H), 4.31-3.45 (m, 8H), 3.44-3.37 (m, 4H), 2.67 (s, 3H), 2.15 (s, 3H), 1.99-1.90 (m, 4H), 1.52 (d, *J* = 7.0 Hz, 3H). LC/MS(m/z, $MH^+$): 551.2.

**Example 90 (*R*)-4-((1-(3-(difluoromethyl)-2-fluorophenyl))ethyl-2,2,2-*d*$_3$)amino)-6-(1-(fluoromethyl)cyclopropyl)-2-methyl-8-morpholinylpyrido[4,3-*d*]pyrimidine-7(6*H*)-one**

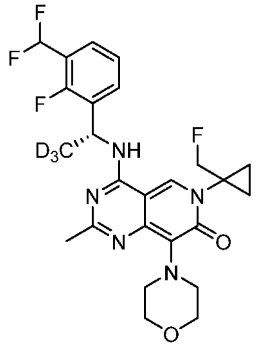

**Step 1: synthesis of (*S,E*)-*N*-(3-(difluoromethyl)-2-fluorobenzylidene)-2-methylpropane-2-sulfinamide**

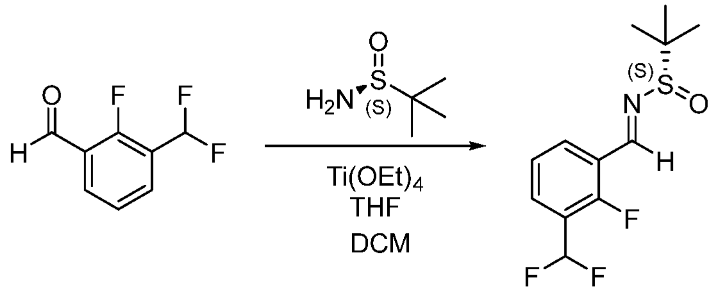


3-(Difluoromethyl)-2-fluorobenzaldehyde (38.6 g) and (*S*)-*tert*-butylsulfinamide (29.7 g) were dispersed in tetrahydrofuran under nitrogen atmosphere. Tetraethyl titanate (151 g) was added, and the mixture was stirred at room temperature for reaction. After 14 h, the reaction mixture was quenched by adding it dropwise to 275 mL of purified water, and the mixture was thoroughly stirred and dispersed. After the mixture was stirred for 3 h, dichloromethane (620 mL) was added, and the mixture was stirred for another 1 h. Then the mixture was filtered through celite, and the filter cake was washed with 2 × 60 mL of dichloromethane. Then the filtrates were combined and the aqueous phase was removed. The organic phase was washed with purified water (560 mL), directly dried over anhydrous sodium sulfate, filtered, and concentrated to remove the solvent to give the title compound.

**Step 2: synthesis of (*S*)-*N*-((*R*)-1-(3-(difluoromethyl)-2-fluorophenyl)ethyl-2,2,2-d$_3$)-2-methylpropane-2-sulfoximide**

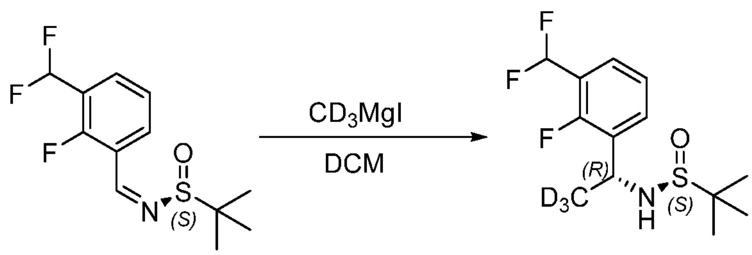


(*S,Z*)-*N*-(3-(Difluoromethyl)-2-fluorobenzyl)-2-methylpropane-2-sulfoximide (11.0 g) was added to 110 mL of dichloromethane under argon atmosphere, and the mixture was cooled to -40 °C. Then a solution of methyl-d$_3$-magnesium

iodide (16.8 g) in diethyl ether was slowly added dropwise for 30 min. The mixture was reacted for 3 h while maintaining the temperature. The reaction was completed after the mixture was then naturally warmed to room temperature and reacted for 12 h. The reaction was quenched with a 20% aqueous ammonium chloride solution and separated. The aqueous phase was extracted with dichloromethane. The organic layers were combined, dried over anhydrous sodium sulfate, filtered, concentrated, and purified by column chromatography (ethyl acetate/petroleum ether gradient elution) to give the title compound.

**Step 3: synthesis of (*R*)-1-(3-(difluoromethyl)-2-fluorophenyl)ethan-2,2,2-d$_3$-1-amine hydrochloride**

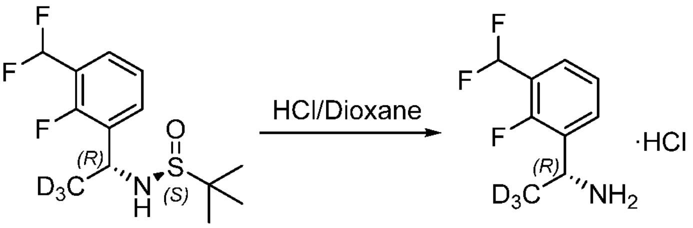


(*S*)-*N*-((*R*)-1-(3-(Difluoromethyl)-2-fluorophenyl)ethyl-2,2,2-d$_3$)-2-methylpropane-2-sulfoximide (1.70 g) was added to 15 mL of a solution of hydrochloric acid in dioxane (4 mol/L). The reaction was completed after the mixture was stirred at room temperature for 2 h. The reaction mixture was concentrated, slurried with isopropyl ether, and filtered. The filter cake was collected and dried to give the title compound.

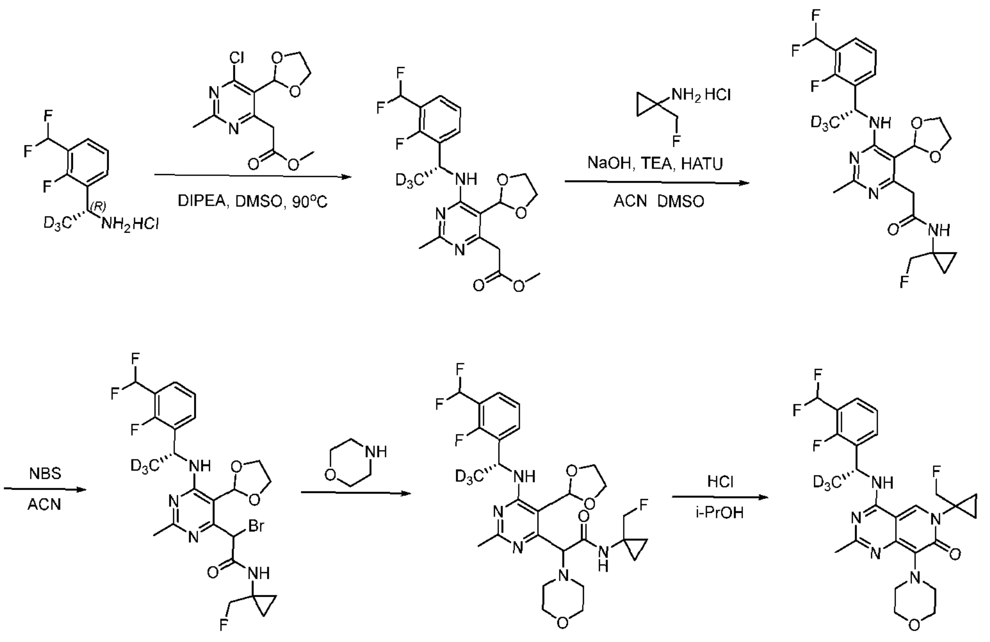


Reference was made to the preparation method of Example **55,** and the title compound was prepared according to the above-mentioned technical route.

$^1$H NMR (400 MHz, DMSO-$d_6$) δ 8.82 (s, 1H), 8.66 (d, *J* = 6.9 Hz, 1H), 7.62 (t, *J* = 7.2 Hz, 1H), 7.51 (t, *J* = 6.7 Hz, 1H), 7.31 (t, *J* = 7.7 Hz, 1H), 7.23 (t, *J* = 54.4 Hz, 1H), 5.72 (d, *J* = 7.0 Hz, 1H), 4.81-4.42 (m, 2H), 3.63 (t, *J* = 4.5 Hz, 4H), 3.30-3.21 (m, 4H), 2.19 (s, 3H), 1.37-1.18 (m, 4H). LC/MS(m/z, MH$^+$): 509.2.

**Example 91 (*R*)-2-methyl-3-(1-((2-methyl-8-(1-methylcyclopropoxy)-6-morpholinyl-7-oxo-6,7-dihydropyrido[4,3-*d*]pyrimidin-4-yl)amino)ethyl)benzonitrile**

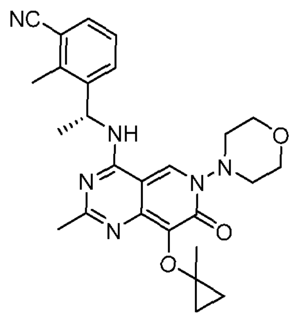

**Step 1: synthesis of 2-(6-(((*R*)-1-(3-cyano-2-methylphenyl)ethyl)amino)-5-(1,3-dioxolan-2-yl)-2-methylpyrimidin-4-yl)-2-(1-methylcyclopropoxy)-*N*-morpholineacetamide**

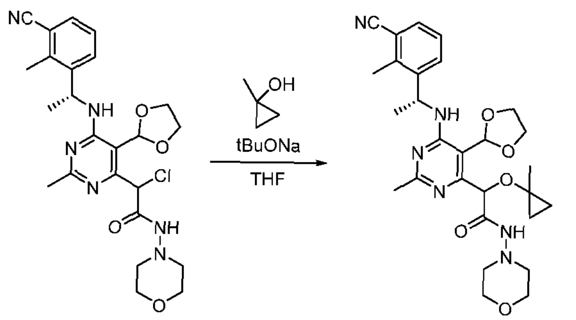


2-Chloro-2-(6-(((*R*)-1-(3-cyano-2-methylphenyl)ethyl)amino)-5-(1,3-dioxolan-2-yl)-2-methylpyrimidin-4-yl)-*N*-morpholinoacetamide (150 mg) was dissolved in 1 mL of tetrahydrofuran. Then 1-methylcyclopropan-1-ol (43.1 mg) and sodium *tert*-butoxide (86.3 mg) were added thereto, and the reaction was completed after the mixture was reacted at room temperature for 4 h. The reaction mixture was concentrated and purified by column chromatography (petroleum ether/ethyl acetate gradient elution) to give the title compound.

**Step 2 synthesis of (*R*)-2-methyl-3-(1-((2-methyl-8-(1-methylcyclopropoxy)-6-morpholinyl-7-oxo-6,7-dihydropyrido[4,3-*d*]pyrimidin-4-yl)amino)ethyl)benzonitrile**

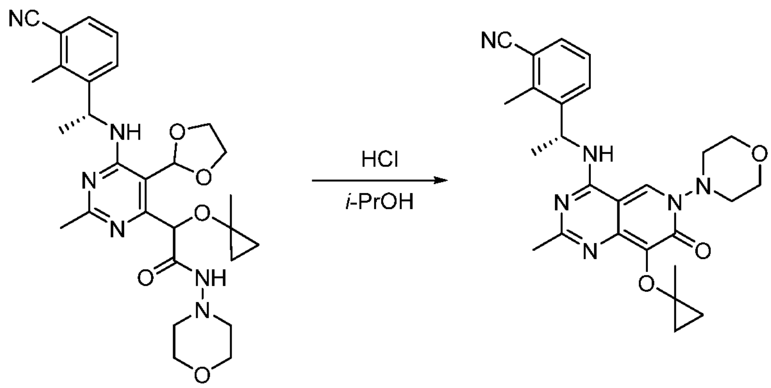


2-(6-(((*R*)-1-(3-Cyano-2-methylphenyl)ethyl)amino)-5-(1,3-dioxolan-2-yl)-2-methylpyrimidin-4-yl)-2-(1-methylcyclopropoxy)-*N*-morpholineacetamide (80.0 mg) was dissolved in 1.5 mL of isopropanol. 4 N diluted hydrochloric acid 120 μL was added. The reaction was completed after the mixture was stirred at 50 °C for 8 h. The reaction mixture was concentrated and purified by preparative HPLC (mobile phase: acetonitrile/water gradient elution) to give the title compound.

$^1H$ NMR (400 MHz, DMSO-$d_6$) δ 9.00 (s, 1H), 8.84 (d, *J* = 6.6 Hz, 1H), 7.77 (d, *J* = 7.5 Hz, 1H), 7.66 (d, *J* = 7.6 Hz, 1H), 7.39 (t, *J* = 7.8 Hz, 1H), 5.65-5.55 (m, 1H), 4.34-3.42 (m, 8H), 2.67 (s, 3H), 2.22 (s, 3H), 1.53-1.46 (m, 6H), 1.08-1.02 (m, 2H), 0.36-0.31 (m, 2H). LC/MS(m/z, MH$^+$): 475.2.

**Example 92 (*R*)-3-(1-((8-(4,4-difluoropiperidin-1-yl)-2-methyl-6-morpholine-7-oxo-6,7-dihydropyrido[4,3-*d*]pyrimidin-4-yl)amino)ethyl)-2-methylbenzonitrile**

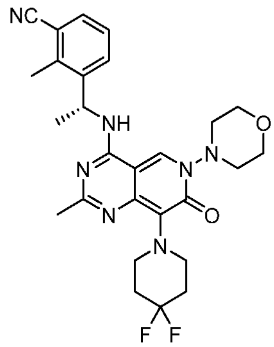

The title compound was prepared in the same manner as in Example **76** except for replacing N-methylpiperazine in step **4** with 4,4-difluoropiperidine.

$^1H$ NMR (400 MHz, DMSO-$d_6$) δ 8.86 (s, 1H), 8.71 (d, *J* = 5.6 Hz, 1H), 7.75 (d, *J* = 7.6 Hz, 1H), 7.65 (d, *J* = 7.6 Hz, 1H), 7.39 (t, *J* = 7.8 Hz, 1H), 5.65-5.54 (m, 1H), 4.28-3.34 (m, 8H), 3.33-3.26 (m, 4H), 2.66 (s, 3H), 2.22 (s, 3H), 2.09-1.93 (m, 4H), 1.49 (d, *J* = 7.0 Hz, 3H).

LC/MS(m/z,MH$^+$): 524.2.

**Example 93 (*R*)-4-((1-(3-(difluoromethyl)-2-fluorophenyl)ethyl)amino)-6-(1-(fluoromethyl) cyclopropyl)-2-methyl-8-((1-methyl-1*H*-pyrazol-4-yl)amino)pyrido[4,3-*d*]pyrimidine-7(6*H*)-one**

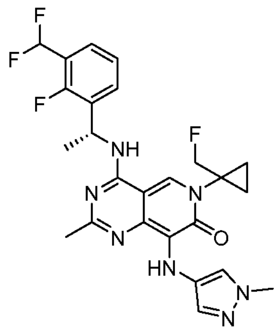

The title compound was prepared in the same manner as in Example **59** except for replacing dibenzylamine in step **1** with *N*-benzyl-1-methyl-1*H*-pyrazole-4-amine.

$^1H$ NMR (400 MHz, DMSO-$d_6$) δ 8.80-8.63 (m, 2H), 7.65 (t, *J* = 7.4 Hz, 1H), 7.52 (t, *J* = 7.1 Hz, 1H), 7.33 (t, *J* = 7.7 Hz, 1H), 7.24 (t, *J* = 54.3 Hz, 1H), 7.18 (s, 1H), 7.07 (s, 1H), 6.78 (s, 1H), 5.83-5.73 (m, 1H), 4.83-4.48 (m, 2H), 3.69 (s, 3H), 2.20 (s, 3H), 1.59 (d, *J* = 7.1 Hz, 3H), 1.38-1.21 (m, 4H). LC/MS(m/z, MH$^+$): 516.2.

**Example 94 (*R*)-4-((1-(3-(difluoromethyl)-2-fluorophenyl)ethyl)amino)-6-(1-(fluoromethyl) cyclopropyl)-8-(4-isopropylpiperazin-1-yl)-2-methylpyrido[4,3-*d*]pyrimidine-7(6*H*)-one**

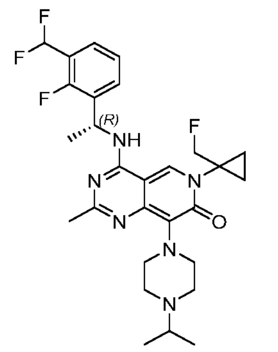

The title compound was prepared in the same manner as in Example **34** except for replacing morpholine in step **1** with 1-isopropylpiperazine.

$^1$H NMR (400 MHz, DMSO-$d_6$) $\delta$ 8.77 (s, 1H), 8.61 (d, *J* = 7.1 Hz, 1H), 7.62 (t, *J*= 7.2 Hz, 1H), 7.47 (dd, *J* = 34.6, 27.9 Hz, 1H), 7.31 (t, *J* = 7.7 Hz, 1H), 7.17 (d, *J* = 54.3 Hz, 1H), 5.74 (m, 1H), 4.79-4.46 (m, 2H), 3.32-3.23 (m, 4H), 2.72-2.61 (m, 1H), 2.18 (s, 3H), 1.56 (d, *J* = 7.1 Hz, 3H), 1.34-1.17 (m, 4H), 0.99 (d, *J* = 6.4 Hz, 6H). LC/MS(m/z, MH$^+$): 547.2.

**Example 95 (*R*)-4-((1-(3-(difluoromethyl)-2-fluorophenyl)ethyl)amino)-6-(1-(fluoromethyl) cydopropyt)-2-methyt-8-(4-(oxetan-3-yt)piperazin-l-yt)pyrido[4,3-*d*]pyrimidine-7(6*H*)-one**

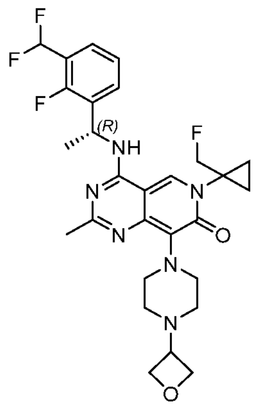

The title compound was prepared in the same manner as in Example **34** except for replacing morpholine in step **1** with 1-(oxetan-3-yl)piperazine.

$^1$H NMR (400 MHz, DMSO-$d_6$) $\delta$ 8.79 (s, 1H), 8.63 (br, 1H), 7.62 (t, *J* = 7.2 Hz, 1H), 7.51 (t, *J* = 6.8 Hz, 1H), 7.31 (t, *J* = 7.7 Hz, 1H), 7.23 (t, *J* = 54.4 Hz, 1H), 5.74 (m, 1H), 4.79-4.55 (m, 2H), 4.53 (t, *J* = 6.4 Hz, 2H), 4.45 (t, *J* = 6.0 Hz, 2H), 3.45-3.37 (m, 1H), 3.33-3.25 (m, 4H), 2.36-2.24 (m, 4H), 2.19 (s, 3H), 1.57 (d, *J* = 7.1 Hz, 3H), 1.34-1.21 (m, 4H). LC/MS(m/z, MH$^+$): 561.2.

**Example 96 (*R*)-8-(diethylamino)-4-((1-(3-(difluoromethyl)-2-fluorophenyl)ethyl)amino)-6-(1-(fluoromethyl)cyclopropyl)-2-methylpyrido[4,3-*d*]pyrimidine-7(6*H*)-one**

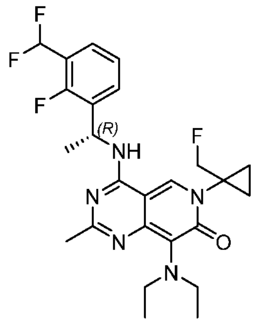

The title compound was prepared in the same manner as in Example **34** except for replacing morpholine in step **1** with diethylamine.

$^1$H NMR (400 MHz, DMSO-$d_6$) δ 8.88 (s, 1H), 8.68 (s, 1H), 7.65 (t, *J* = 7.0 Hz, 1H), 7.52 (t, *J* = 6.9 Hz, 1H), 7.32 (t, *J* = 7.7 Hz, 1H), 7.23 (t, *J* = 54.4 Hz, 1H), 5.81-5.71 (m, 1H), 4.81-4.44 (m, 2H), 3.30-3.17 (m, 4H), 2.20 (s, 3H), 1.57 (d, *J* = 6.9 Hz, 3H), 1.36-1.16 (m, 4H), 0.86 (t, *J* = 7.1 Hz, 6H). LC/MS(m/z, MH$^+$): 492.2.

**Example 97 4-(((*R*)-1-(3-(difluoromethyl)-2-fluorophenyl)ethyl)amino)-6-(1-(fluoromethyl) cyclopropyl)-8-((*R*)-hexahydropyrrolo[1,2-*a*]pyrazin-2(1*H*)-yl)-2-methylpyrido[4,3-*d*]pyrimidine-7(6*H*)-one**

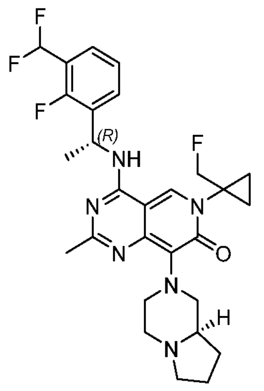

The title compound was prepared in the same manner as in Example **34** except for replacing morpholine in step **1** with (*R*)-octahydropyrrolo[1,2-*a*]pyrazine.

$^1$H NMR (400 MHz, DMSO-$d_6$) δ 8.78 (s, 1H), 8.62 (d, *J* = 6.7 Hz, 1H), 7.62 (t, *J* = 7.2 Hz, 1H), 7.51 (t, *J* = 6.9 Hz, 1H), 7.31 (t, *J* = 7.7 Hz, 1H), 7.23 (t, *J* = 54.4 Hz, 1H), 5.74 (m, 1H), 4.83-4.42 (m, 2H), 3.40-3.35 (m, 1H), 3.33-3.20 (m, 2H), 3.13-2.83 (m, 3H), 2.18 (s, 3H), 2.13-1.99 (m, 1H),1.78-1.60 (m, 3H), 1.57 (d, *J* = 7.1 Hz, 3H), 1.36-1.18 (m, 4H). LC/MS(m/z, MH$^+$): 545.2.

**Example 98 (*R*)-4-((1-(3-(difluoromethyl)-2-fluorophenyl)ethyl)amino)-6-(1-(fluoromethyl) cyclopropyl)-2-methyl-8-(2-methyl-2,4,5,7-tetrahydro-6*H*-pyrazolo[3,4-*c*]pyridin-6-yl)pyrido[4,3-*d*]pyrimidine-7(6*H*)-one**

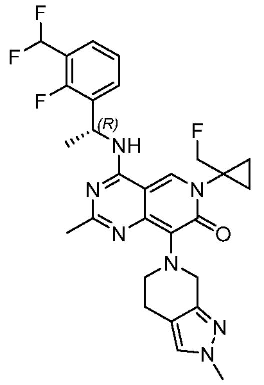

The title compound was prepared in the same manner as in Example **34** except for replacing morpholine in step **1** with 2-methyl-4,5,6,7-tetrahydro-2*H*-pyrazolo[3,4-c]pyridine.

$^1$H NMR (400 MHz, DMSO-$d_6$) δ 8.83 (s, 1H), 8.67 (s, 1H), 7.64 (t, *J* = 7.2 Hz, 1H), 7.52 (t, *J* = 6.9 Hz, 1H), 7.35-7.29 (m, 2H), 7.24 (t, *J* = 54.4 Hz, 1H), 5.81-5.70 (m, 1H), 4.82-4.45 (m, 2H), 4.28 (s, 2H), 3.73 (s, 3H), 3.48-3.36 (m, 2H), 2.78-2.68 (m, 2H), 2.20 (s, 3H), 1.58 (d, *J* = 7.0 Hz, 3H), 1.40-1.18 (m, 4H). LC/MS(m/z, MH$^+$): 556.2.

**Example 99 2-methyl-3-((*R*)-1-((2-methyl-6-morpholine-7-oxo-8-((*R*)-3-oxotetrahydro-3*H*-oxazolo[3,4-*a*]pyrazin-7(1*H*)-yl)-6,7-dihydropyrido[4,3-*d*]pyrimidin-4-yl)amino)ethyl)benzonitrile**

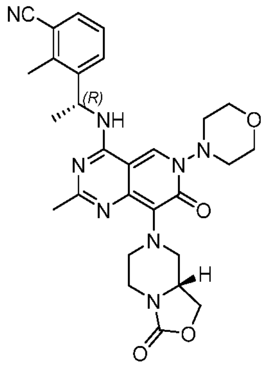

The title compound was prepared in the same manner as in Example **76** except for replacing N-methylpiperazine in step **4** with (*R*)-hexahydro-3*H*-oxazolo[3,4-*a*]pyrazin-3-one.

$^1$H NMR (400 MHz, DMSO-$d_6$) δ 8.89 (s, 1H), 8.74 (d, *J* = 5.9 Hz, 1H), 7.74 (d, *J* = 7.8 Hz, 1H), 7.65 (d, *J* = 6.8 Hz, 1H), 7.38 (t, *J* = 7.8 Hz, 1H), 5.64-5.55 (m, 1H), 4.38-4.30 (m, 1H), 3.97-3.87 (m, 2H), 3.83-3.62 (m, 8H), 3.58-3.49 (m, 1H), 3.38-3.34 (m, 1H), 3.23-3.06 (m, 4H), 2.66 (s, 3H), 2.23 (s, 3H), 1.49 (d, *J* = 7.0 Hz, 3H). LC/MS(m/z, MH$^+$): 545.2.

**Example 100 (*R*)-4-((1-(3-(difluoromethyl)-2-fluorophenyl)ethyl)amino)-2-methyl-8-(1-methyl-1*H*-pyrazolo[3,4-*b*]pyridin-5-yl)-6-morpholinepyrido[4,3-*d*]pyrimidine-7(6*H*))-one**

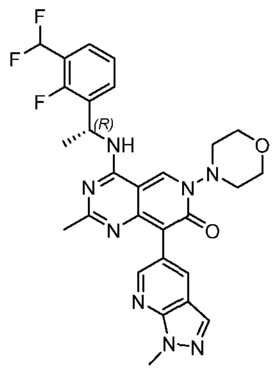

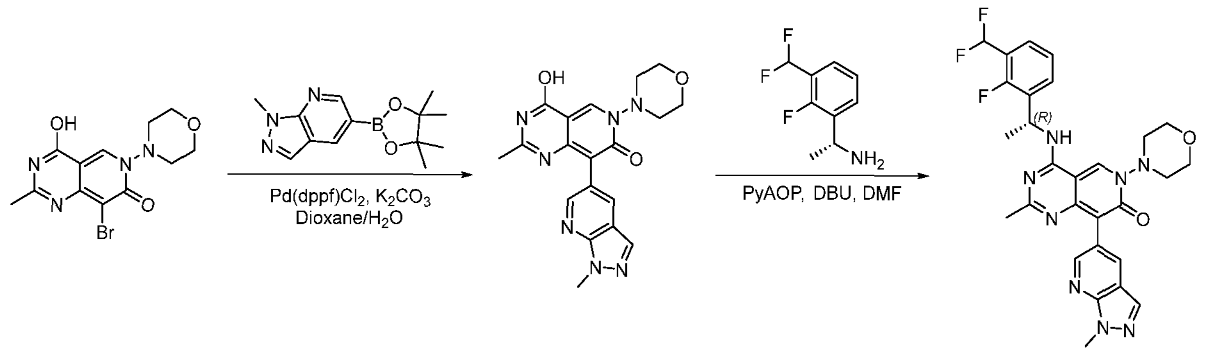


Reference was made to the synthesis method of Example **89,** and the title compound was prepared according to the above-mentioned technical route.

$^1$H NMR (400 MHz, DMSO-$d_6$) δ 9.32 (s, 1H), 8.92 (d, *J* = 7.1 Hz, 1H), 8.55 (d, *J* = 1.9 Hz, 1H), 8.23 (d, *J* = 1.9 Hz, 1H), 8.13 (s, 1H), 7.71 (t, *J* = 7.3 Hz, 1H), 7.54 (t, *J* = 6.6 Hz, 1H), 7.34 (t, *J* = 7.7 Hz, 1H), 7.24 (t, *J* = 54.3 Hz, 1H), 5.83-5.74 (m, 1H), 4.07 (s, 3H), 3.99-3.42 (m, 8H), 2.12 (s, 3H), 1.59 (d, *J* = 7.0 Hz, 3H). LC/MS(m/z, MH$^+$): 565.2.

**Example 101 (*R*)-1-(4-((1-(3-(difluoromethyl)-2-fluorophenyl)ethyl)amino)-6-(1-(fluoromethyl) cyclopropyl)-2-methyl-7-oxo-6,7-dihydropyrido[4,3-*d*]pyrimidin-8-yl)-1*H*-pyrazole-4-carbonitrile**

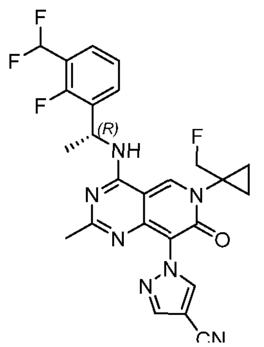

The title compound was prepared in the same manner as in Example **23** except for replacing methanol-d$_3$(CD$_3$OH) in step **4** with 1*H*-pyrazole-4-carbonitrile.

$^1$H NMR (400 MHz, DMSO-$d_6$) δ 9.43 (s, 1H), 9.18 (d, *J* = 7.0 Hz, 1H), 8.53 (s, 1H), 8.19 (s, 1H), 7.67 (t, *J* = 7.2 Hz, 1H), 7.54 (t, *J* = 7.0 Hz, 1H), 7.34 (t, *J* = 7.7 Hz, 1H), 7.24 (t, *J* = 54.3 Hz, 1H), 5.78 (m, 1H), 4.80-4.50 (m, 2H), 2.12 (s, 3H), 1.61 (d, *J* = 7.1 Hz, 3H), 1.45-1.29 (m, 4H). LC/MS(m/z, MH$^+$): 512.2.

**Example 102 (*R*)-8-(2-aminopyrimidin-5-yl)-4-((1-(3-(difluoromethyl)-2-fluorophenyl)ethyl) amino)-2-methyl-6-morpholinepyrido[4,3-*d*]pyrimidine-7(6*H*)-one**

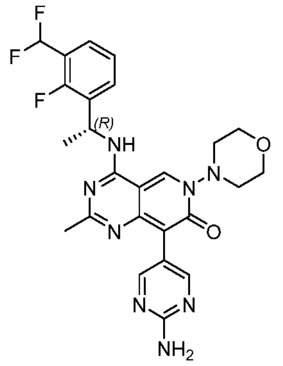

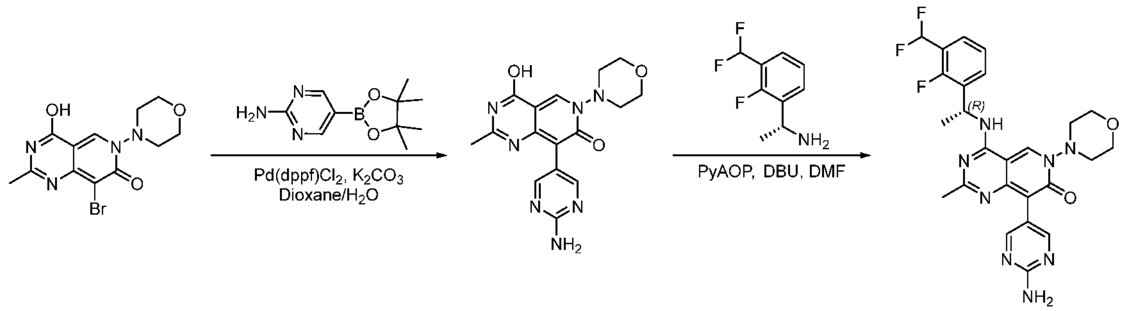


Reference was made to the synthesis method of Example **89,** and the title compound was prepared according to the above-mentioned technical route.

$^1$H NMR (400 MHz, DMSO-$d_6$) δ 9.22 (s, 1H), 8.89 (d, *J* = 6.3 Hz, 1H), 8.35 (s, 2H), 7.70 (t, *J* = 7.5 Hz, 1H), 7.53 (t, *J* = 7.0 Hz, 1H), 7.33 (t, *J* = 7.6 Hz, 1H), 7.24 (t, *J* = 54.3 Hz, 1H), 6.57 (s, 2H), 5.82-5.72 (m, 1H), 4.07-3.41 (m, 8H), 2.16 (s, 3H), 1.57 (d, *J* = 7.0 Hz, 3H).

LC/MS(m/z, $MH^+$): 527.2.

**Example 103 (*R*)-4-((1-(3-(difluoromethyl)-2-fluorophenyl)ethyl)amino)-8-((dimethyl(oxo)-λ$^6$-sulfanylidene)amino)-6-(1-(fluoromethyl)cyclopropyl)-2-methylpyrido[4,3-*d*]pyrimidine-7(6*H*)-one**

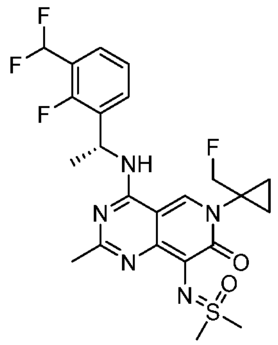

The title compound was prepared in the same manner as in Example **34** except for replacing morpholine in step **1** with dimethylsulfoximine.

$^1$H NMR (400 MHz, DMSO-$d_6$) δ 8.71 (s, 1H), 7.64 (t, *J* = 7.0 Hz, 1H), 7.51 (t, *J* = 6.8 Hz, 1H), 7.32 (t, *J* = 7.7 Hz, 1H), 7.24 (t, *J* = 54.4 Hz, 1H), 5.81-5.72 (m, 1H), 4.80-4.45 (m, 2H), 3.32-3.25 (m, 6H), 2.21 (s, 3H), 1.58 (d, *J* = 7.0 Hz, 3H), 1.37-1.20 (m, 4H). LC/MS(m/z, $MH^+$): 512.2.

**Example 104 (*R*)-4-((1-(3-(difluoromethyl)-2-fluorophenyl)ethyl)amino)-6-(1-(fluoromethyl)cyclopropyl)-2-methyl-8-(1*H*-pyrazolo[4,3-*b*]pyridin-1-yl)pyrido[4,3-*d*]pyrimidine-7(6*H*)-one**

**(*R*)-4-((1-(3-(difluoromethyl)-2-fluorophenyl)ethyl)amino)-6-(1-(fluoromethyl)cyclopropyl)-2-methyl-8-(2*H*-pyrazolo[4,3-*b*]pyridin-2-yl)pyrido[4,3-*d*]pyrimidine-7(6*H*)-one**

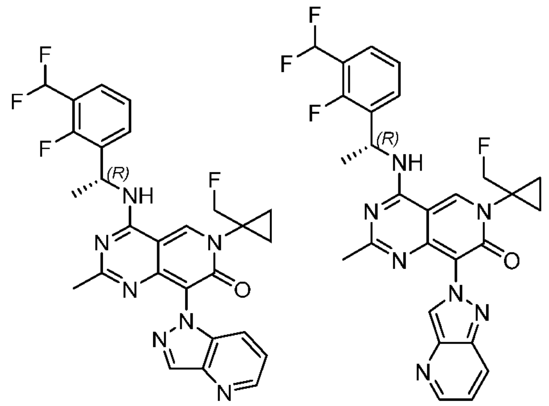

The title compound was prepared in the same manner as in Example **23** except for replacing methanol-$d_3$($CD_3OH$) in step **4** with 1*H*-pyrazolo[4,3-*b*]pyridine.

$^1$H NMR (400 MHz, DMSO-$d_6$) δ 9.49-9.44 (m, 1H), 9.24-9.14 (m, 1H), 8.60-8.40 (m, 2H), 7.74-7.65 (m, 1H), 7.63-7.50 (m, 2H), 7.40-7.08 (m, 3H), 5.85-5.74 (m, 1H), 4.87-4.44 (m, 2H), 2.09-1.99 (m, 3H), 1.66-1.59 (m, 3H), 1.50-1.28 (m, 4H). LC/MS(m/z, MH$^+$): 538.2.

**Example 105 4-(((*R*)-1-(3-(difluoromethyl)-2-fluorophenyl)ethyl)amino)-6-(1-(fluoromethyl) cyclopropyl)-2-methyl-8-((((*S*)-oxetan-2-yl)methyl)amino)pyrido[4,3-*d*]pyrimidine-7(6*H*)-one**

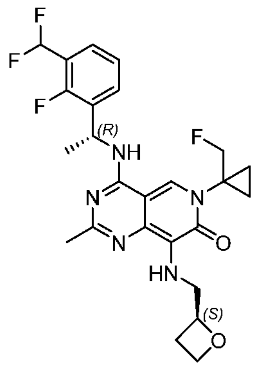

The title compound was prepared in the same manner as in Example **77** except for replacing N-methylbenzylamine in step **1** with (S)-N-benzyl-1-(oxetan-2-yl)methylamine.

$^1$H NMR (400 MHz, DMSO-$d_6$) δ 8.57 (d, *J* = 7.2 Hz, 1H), 8.46 (s, 1H), 7.63 (t, *J* = 7.4 Hz, 1H), 7.51 (t, *J* = 6.8 Hz, 1H), 7.32 (t, *J* = 7.7 Hz, 1H), 7.24 (t, *J* = 54.4 Hz, 1H), 5.81-5.70 (m, 1H), 5.52-5.42 (m, 1H), 4.86-4.78 (m, 1H), 4.76-4.51 (m, 2H), 4.51-4.43 (m, 1H), 4.39-4.33 (m, 1H), 3.96-3.87 (m, 1H), 2.47-2.31 (m, 2H), 2.18 (s, 3H), 1.57 (d, *J* = 7.1 Hz, 3H), 1.36-1.20 (m, 4H).

LC/MS(m/z, MH$^+$): 506.2.

**Example 106 (*R*)-8-(2-cyclopropyl-2,6-dihydropyrrolo[3,4-*c*]pyrazol-5(4*H*)-yl)-4-((1-(3-(difluoromethyl)-2-fluorophenyl))ethyl)amino)-6-(1-(fluoromethyl)cyclopropyl)-2-methylpyrido[4,3-*d*]pyrimidine-7(6*H*)-one**

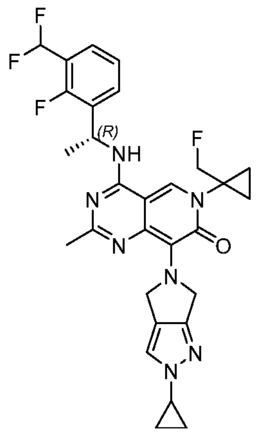

The title compound was prepared in the same manner as in Example **34** except for replacing morpholine in step **1** with 2-cyclopropyl-2,4,5,6-tetrahydropyrrolo[3,4-c]pyrazole.

$^1$H NMR (400 MHz, DMSO-$d_6$) δ 8.92 (s, 1H), 8.76 (br, 1H), 7.64 (t, *J* = 7.3 Hz, 1H), 7.52 (t, *J* = 6.8 Hz, 1H), 7.49 (s, 1H), 7.32 (t, *J* = 7.7 Hz, 1H), 7.24 (t, *J* = 54.4 Hz, 1H), 5.82-5.71 (m, 1H), 4.86-4.32 (m, 6H), 3.70-3.62 (m, 1H), 2.22 (s, 3H), 1.59 (d, *J* = 7.0 Hz, 3H), 1.37-1.20 (m, 4H), 1.05-0.98 (m, 2H), 0.97-0.89 (m, 2H). LC/MS(m/z, MH$^+$): 568.2.

**Example 107 4-(((*R*)-1-(3-(difluoromethyl)-2-fluorophenyl)ethyl)amino)-8-((3*S*,4*R*)-3,4-difluoropyrrolidin-1-yl)-6-(1-(fluoromethyl)cyclopropyl)-2-methylpyrido[4,3-*d*]pyrimidine-7(6*H*)-one**

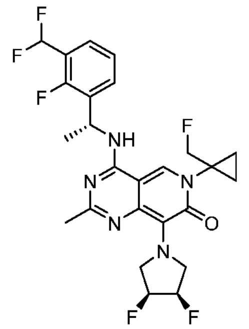

The title compound was prepared in the same manner as in Example **34** except for replacing morpholine in step **1** with (3*S*,4*R*)-3,4-difluoropyrrolidine.

$^1$H NMR (400 MHz, DMSO-$d_6$) δ 8.77 (s, 1H), 8.69 (br, 1H), 7.63 (t, *J* = 7.2 Hz, 1H), 7.52 (t, *J* = 6.9 Hz, 1H), 7.32 (t, *J* = 7.7 Hz, 1H), 7.24 (t, *J* = 54.4 Hz, 1H), 5.75 (m, 1H), 5.38-5.27 (m, 1H), 5.26-5.14 (m, 1H), 4.83-4.44 (m, 2H), 4.16-3.92 (m, 2H), 3.91-3.71 (m, 2H), 2.20 (s, 3H), 1.57 (d, *J* = 7.1 Hz, 3H), 1.35-1.20 (m, 4H). LC/MS(m/z, MH$^+$): 526.2.

**Example 108 (*R*)-2-methyl-3-(1-((2-methyl-6,8-dimorpholine-7-oxo-6,7-dihydropyrido[4,3-*d*]pyrimidin-4-yl)amino)ethyl)benzonitrile**

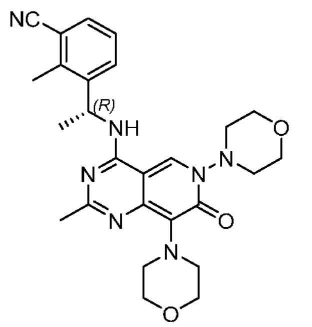

The title compound was prepared in the same manner as in Example **76** except for replacing *N*-methylpiperazine in step **4** with morpholine.

$^1$H NMR (400 MHz, DMSO-$d_6$) δ 8.86 (s, 1H), 8.70 (br, 1H), 7.75 (d, *J* = 7.7 Hz, 1H), 7.66 (d, *J* = 7.3 Hz, 1H), 7.39 (t, *J* = 7.8 Hz, 1H), 5.70-5.48 (m, 1H), 4.23-3.40 (m, 12H), 3.30-3.16 (m, 4H), 2.66 (s, 3H), 2.23 (s, 3H), 1.49 (d, *J* = 7.0 Hz, 3H). LC/MS(m/z, MH$^+$): 490.2.

**Example 109 (*R*)-4-((1-(3-(difluoromethyl)-2-fluorophenyl)ethyl)amino)-6-(1-(fluoromethyl)cyclopropyl)-2-methyl-8-(4-((3-methyloxetan-3-yl)methyl)piperazin-1-yl)pyrido[4,3-*d*]pyrimidine-7(6*H*)-one**

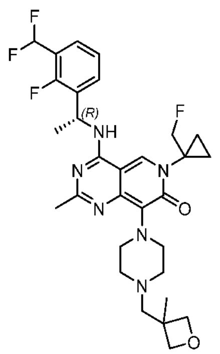

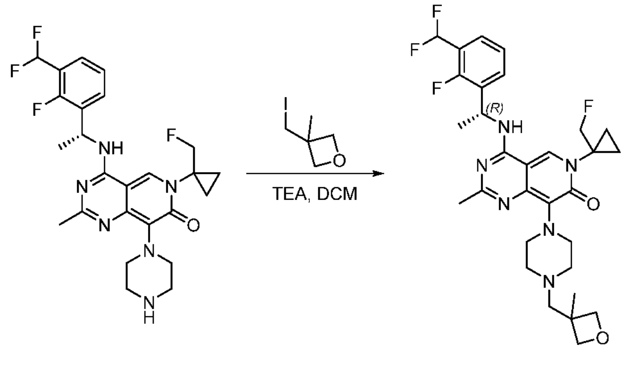


(*R*)-4-((1-(3-(Difluoromethyl)-2-fluorophenyl)ethyl)amino)-6-(1-(fluoromethyl)cyclopropyl)-2-methyl-8-(piperazin-1-yl)pyrido[4,3-*d*]pyrimidine-7(6*H*)-one (0.10 g) was dissolved in dichloromethane (5 mL). Triethylamine (51.3 mg) and 3-(iodomethyl)-3-methyloxetane (30.0 mg) were added, and the reaction was completed after the mixture was stirred at room temperature for 3 h. The reaction mixture was concentrated and purified by column chromatography (dichloromethane/methanol gradient elution) to give the title compound.

$^1$H NMR (400 MHz, DMSO-$d_6$) δ 8.78 (s, 1H), 8.61 (d, *J* = 7.3 Hz, 1H), 7.62 (t, *J* = 7.2 Hz, 1H), 7.51 (t, *J* = 6.7 Hz, 1H), 7.31 (t, *J* = 7.7 Hz, 1H), 7.23 (t, *J* = 54.3 Hz, 1H), 5.78-5.69 (m, 1H), 4.83-4.46 (m, 2H), 4.37 (d, *J* = 5.6 Hz, 2H), 4.19 (d, *J* = 5.6 Hz, 2H), 3.31 (s, 2H), 3.30-3.21 (m, 4H), 2.35-2.26 (m, 4H), 2.18 (s, 3H), 1.56 (d, *J* = 7.1 Hz, 3H), 1.33 (s, 3H), 1.31-1.21 (m, 4H).

LC/MS(m/z, MH+): 589.2.

**Example 110 2-methyl-3-((*R*)-1-((2-methyl-8-((*S*)-3-methylmorpholine)-6-morpholine-7-oxo-6,7-dihydropyrido[4,3-*d*]pyrimidin-4-yl)amino)ethyl)benzonitrile**

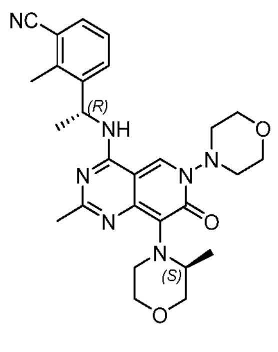

The title compound was prepared in the same manner as in Example **76** except for replacing *N*-methylpiperazine in step **4** with (*S*)-3-methylmorpholine.

$^1$H NMR (400 MHz, DMSO-$d_6$) δ 8.99 (s, 1H), 8.76 (br, 1H), 7.77 (d, *J* = 7.7 Hz, 1H), 7.65 (d, *J* = 7.6 Hz, 1H), 7.39 (t, *J* = 7.8 Hz, 1H), 5.65-5.51 (m, 1H), 4.00-3.50 (m, 12H), 3.21-3.06 (m, 2H), 3.05-2.95 (m, 1H), 2.67 (s, 3H), 2.23 (s, 3H), 1.49 (d, *J* = 7.0 Hz, 3H), 0.69 (d, *J* = 6.3 Hz, 3H).

LC/MS(m/z, MH+): 504.2.

**Example 111 (*R*)-4-((1-(3-(difluoromethyl)-2-fluorophenyl)ethyl)amino)-6-(1-(fluoromethyl) cyclorol-2-methyl-8-3-methylsulfonylazetidin-1-yl)pyrido[4,3-*d*]pyrimidine-7(6*H*)-one**

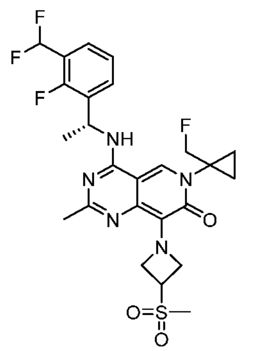

The title compound was prepared in the same manner as in Example **34** except for replacing morpholine in step **1** with 3-(methylsulfonyl)azetidine.

$^1$H NMR (400 MHz, DMSO-$d_6$) δδ 8.52 (d, *J* = 5.7 Hz, 1H), 8.46 (s, 1H), 7.61 (t, *J* = 7.3 Hz, 1H), 7.51 (t, *J* = 6.8 Hz, 1H), 7.31 (t, *J* = 7.7 Hz, 1H), 7.23 (t, *J* = 54.4 Hz, 1H), 5.74 (m, 1H), 4.83-4.39 (m, 6H), 4.27-4.16 (m, 1H), 2.97 (s, 3H), 2.15 (s, 3H), 1.56 (d, *J* = 7.1 Hz, 3H), 1.40 - 1.17 (m, 4H). LC/MS(m/z, MH+): 554.2.

**Example 112 4-(((*R*)-1-(3-(difluoromethyl)-2-fluorophenyl)ethyl-1-*d*)amino)-6-(1-(fluoromethyl) cyclopropyl)-2-methyl-8-((*S*)-3-methylmorpholine)pyrido[4,3-*d*]pyrimidine-7(6*H*)-one**

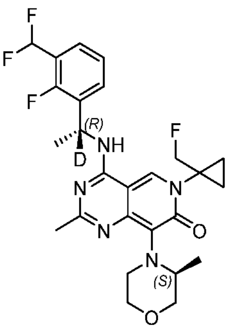

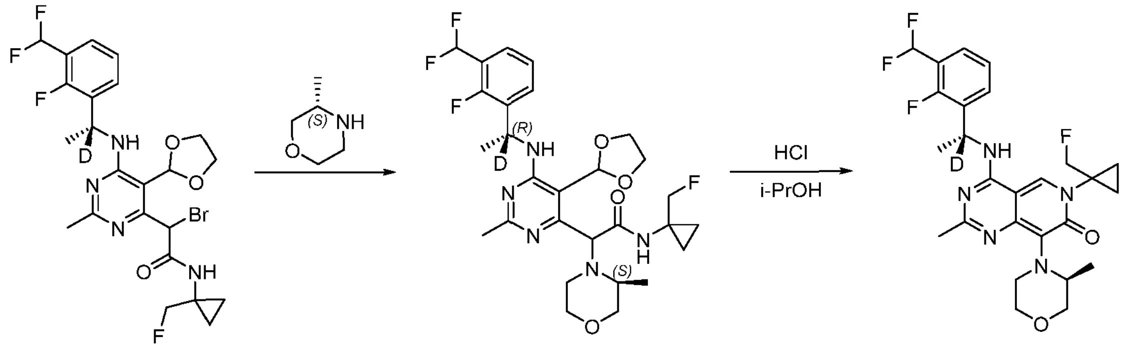


Reference was made to the synthesis method of Example **55,** and the title compound was prepared according to the above-mentioned technical route.

$^1$H NMR (400 MHz, DMSO-$d_6$) δ 8.95 (s, 1H), 8.74 (s, 1H), 7.64 (t, *J* = 7.2 Hz, 1H), 7.51 (t, *J* = 7.0 Hz, 1H), 7.31 (t, *J* = 7.6 Hz, 1H), 7.23 (t, *J* = 54.4 Hz, 1H), 4.87-4.38 (m, 2H), 4.01-3.87 (m, 1H), 3.73-3.55 (m, 3H), 3.24-2.95 (m, 3H), 2.20 (s, 3H), 1.57 (s, 3H), 1.40-1.17 (m, 4H), 0.69 (d, *J* = 6.4 Hz, 3H). LC/MS(m/z, MH$^+$): 521.2.

**Example 113 4-(((*R*)-1-(3-(difluoromethyl)-2-fluorophenyl)ethyl-2,2,2-d$_3$)amino)-6-(1-(fluoromethyl)cyclopropyl)-2-methyl-8-((*S*)-3-methylmorpholine)pyrido[4,3-*d*]pyrimidine-7(6*H*)-one**

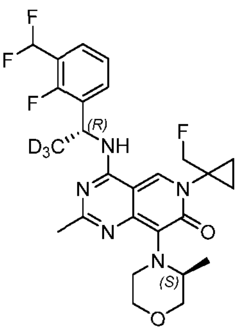

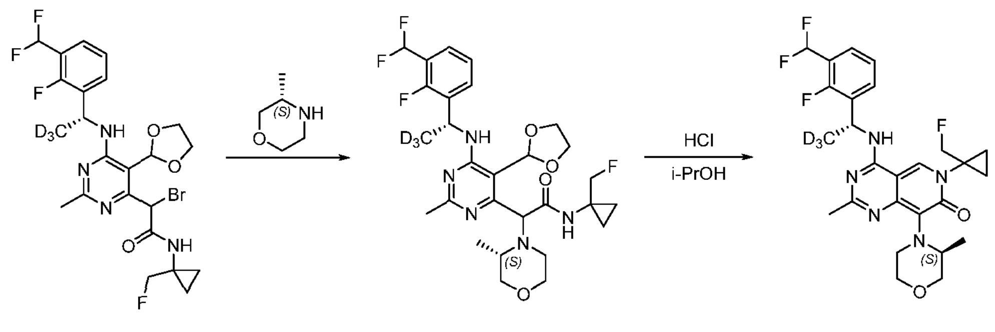

Reference was made to the synthesis method of Example **90,** and the title compound was prepared according to the above-mentioned technical route.

$^1$H NMR (400 MHz, DMSO-$d_6$) δ 8.95 (s, 1H), 8.71 (d, *J* = 7.0 Hz, 1H), 7.64 (t, *J* = 7.2 Hz, 1H), 7.51 (t, *J* = 7.0 Hz, 1H), 7.31 (t, *J* = 7.6 Hz, 1H), 7.23 (t, *J* = 54.4 Hz, 1H), 5.75 (d, *J* = 7.0 Hz, 1H), 4.87-4.38 (m, 2H), 4.01-3.87 (m, 1H), 3.73-3.55 (m, 3H), 3.24-2.95 (m, 3H), 2.20 (s, 3H), 1.40-1.17 (m, 4H), 0.69 (d, *J* = 6.4 Hz, 3H). LC/MS(m/z, MH$^+$): 523.2.

**Example 114 4-(((*R*)-1-(3-(1,1-difluoroethyl)-2-fluorophenyl)ethyl)amino)-6-(1-(fluoromethyl) cyclopropyl)-2-methyl-8-((*S*)-3-methylmorpholine)pyrido[4,3-*d*]pyrimidine-7(6*H*)-one**

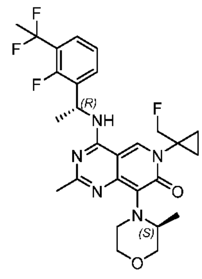

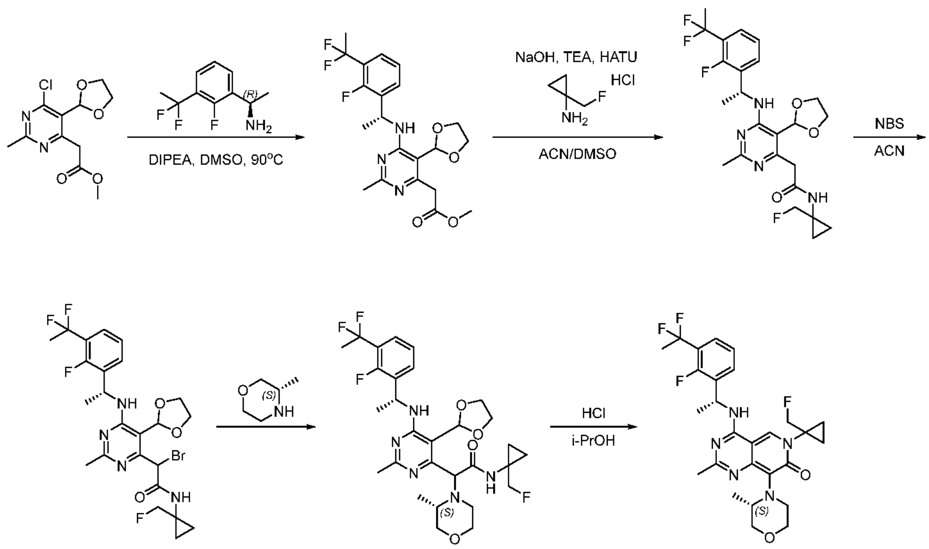


Reference was made to step **1**-step **3** of Example **23** and the synthesis method of Example **34.** The title compound was prepared according to the above-mentioned technical route.

$^1$H NMR (400 MHz, DMSO) δ 8.97 (s, 1H), 8.72 (br, 1H), 7.60 (t, *J* = 7.1 Hz, 1H), 7.45 (t, *J* = 7.1 Hz, 1H), 7.28 (t, *J* = 7.8 Hz, 1H), 5.81-5.68 (m, 1H), 4.84-4.44 (m, 2H), 3.99-3.86 (m, 1H), 3.70-3.55 (m, 3H), 3.22-2.96 (m, 3H), 2.21 (s, 3H), 2.02 (t, *J* = 19.1 Hz, 3H), 1.57 (d, *J* = 6.8 Hz, 3H), 1.35-1.19 (m, 4H), 0.68 (d, *J* = 6.3 Hz, 3H). LC/MS(m/z, MH$^+$): 534.2.

**Example 115 (*R*)-3-((4-((1-(3-(difluoromethyl)-2-fluorophenyl)ethyl)amino)-2-methyl-6-morpholino-7-oxo-6,7-dihydropyrido[4,3-*d*]pyrimidin-8-yl)oxy)-5-fluorobenzonitrile**

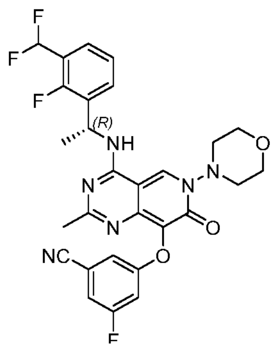

The title compound was prepared in the same manner as in Example **20** except for replacing the solution of sodium methoxide in methanol in step **4** with 3-fluoro-5-hydroxybenzonitrile and sodium *tert*-butoxide.

$^1H$ NMR (400 MHz, DMSO-$d_6$) $\delta$ 9.27 (s, 1H), 9.03 (d, *J* = 6.9 Hz, 1H), 7.72 (t, *J* = 7.3 Hz, 1H), 7.54 (t, *J* = 7.0 Hz, 1H), 7.46-7.40 (m, 1H), 7.39-7.10 (m, 4H), 5.86-5.75 (m, 1H), 4.05-3.36 (m, 8H), 2.18 (s, 3H), 1.59 (d, *J* = 7.0 Hz, 3H). LC/MS(m/z, $MH^+$): 569.2.

**Example 116 (*R*)-4-((1-(3-(difluoromethyl)-2-fluorophenyl)ethyl)amino)-6-(1-(fluoromethyl) cyclopropyl)-2-methyl-8-(morpholine-4-carbonyl)pyrido[4,3-*d*]pyrimidine-7(6*H*)-one**

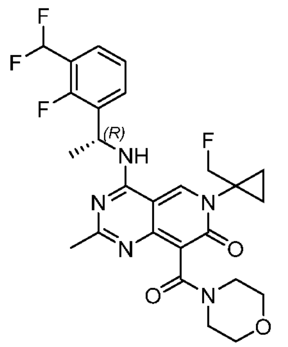

**Step 1: synthesis of dimethyl (*R*)-2-(6-(1-(3-(difluoromethyl)-2-fluorophenyl)ethyl)amino)-5-(1,3-dioxolan-2-yl)-2-methylpyrimidin-4-yl)malonate**

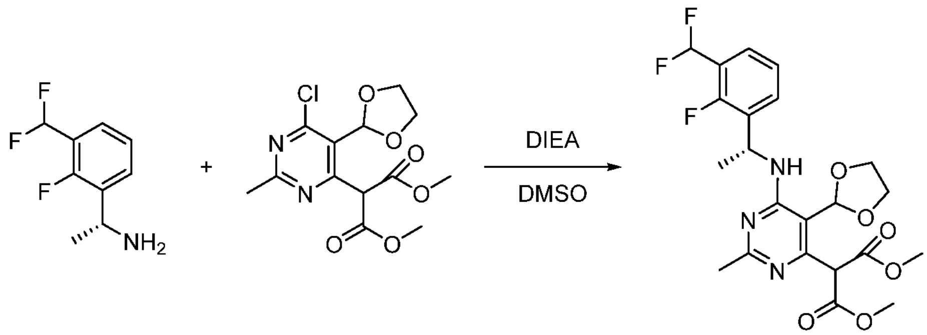


Dimethyl 2-(6-chloro-5-(1,3-dioxolan-2-yl)-2-methylpyrimidin-4-yl)malonate (1.00 g) and (*R*)-1-(3-(difluoromethyl)-2-fluorophenyl)ethane-1-amine (572 mg) were dissolved in 10 mL of dimethyl sulfoxide. Diisopropylethylamine (781 mg) was added, and the reaction was completed after the mixture was heated to 85 °C and stirred for 6 h. 100 mL of water was added to the reaction mixture, and the mixture was extracted with ethyl acetate. The organic phases were combined, dried over anhydrous sodium sulfate, filtered, concentrated, and purified by column chromatography (petroleum ether/ethyl acetate gradient elution) to give the title compound.

**Step 2: synthesis of dimethyl (*R*)-2-(6-(1-(3-(difluoromethyl)-2-fluorophenyl)ethyl)amino)-5-formyl-2-methylpyrimidin-4-yl)malonate**

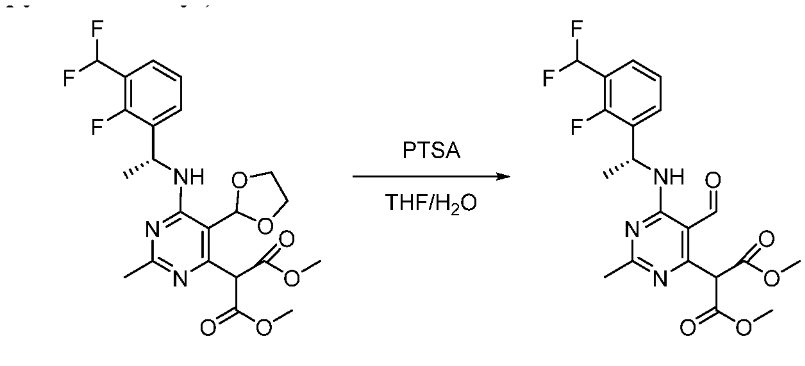


Dimethyl (*R*)-2-(6-(1-(3-(difluoromethyl)-2-fluorophenyl)ethyl)amino)-5-(1,3-dioxolan-2-yl)-2-methylpyrimidin-4-yl)malonate (830 mg) was dissolved in 8 mL of tetrahydrofuran and 8 mL of water. *p*-Toluenesulfonic acid (295 mg) was added, and the reaction was completed after the mixture was stirred at room temperature for 10 h. 50 mL of ethyl acetate was added to the reaction mixture for extraction, and the organic phases were combined, washed with a saturated sodium chloride solution, dried over anhydrous sodium sulfate, filtered, concentrated, and purified by column chromatography (petroleum ether/ethyl acetate gradient elution) to give the title compound.

**Step 3: synthesis of methyl (*R*)-4-((1-(3-(difluoromethyl)-2-fluorophenyl)ethyl)amino)-6-(1-(fluoromethyl)cyclopropyl)-2-methyl-7-oxo-6,7-dihydropyrido[4,3-*d*]pyrimidine-8-carboxylate**

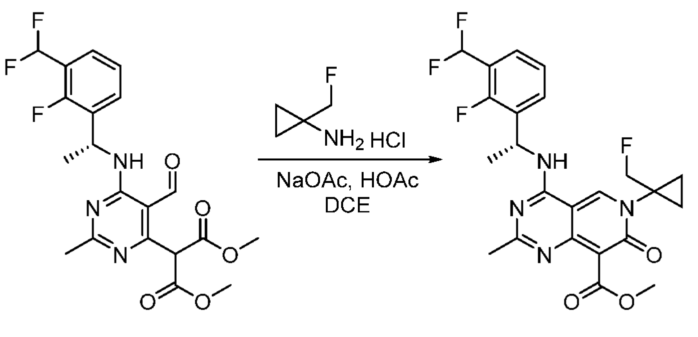


1-(Fluoromethyl)cyclopropane-1-amine hydrochloride (165 mg), dimethyl (*R*)-2-(6-(1-(3-(difluoromethyl)-2-fluorophenyl)ethyl)amino)-5-formyl-2-methylpyrimidin-4-yl)malonate (580 mg) was added to 1,2-dichloroethane (5 mL). Sodium acetate (108 mg) and acetic acid (79.2 mg) were added, and the reaction was completed after the mixture was stirred at 90 °C for 6 h. 20 mL of a saturated sodium bicarbonate solution was added to the reaction mixture, and the mixture was extracted with dichloromethane. The organic phase was dried over anhydrous sodium sulfate, filtered, concentrated, and purified by column chromatography (petroleum ether/ethyl acetate gradient elution) to give the title compound.

**Step 4: synthesis of (*R*)-4-((1-(3-(difluoromethyl)-2-fluorophenyl)ethyl)amino)-6-(1-(fluoromethyl)cyclopropyl)-2-methyl-8-(morpholinyl-4-carbonyl)pyrido[4,3-*d*]pyrimidine-7(6*H*)-one**

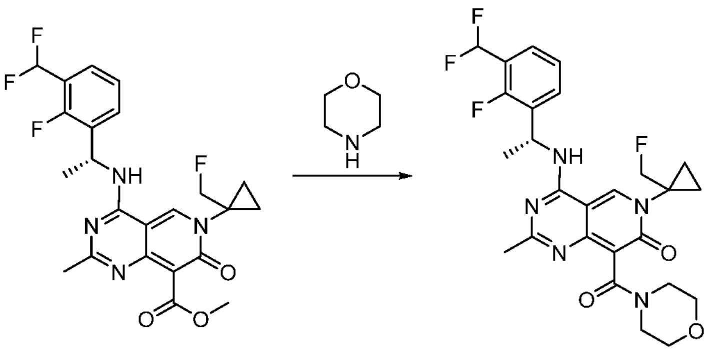

Methyl (*R*)-4-((1-(3-(difluoromethyl)-2-fluorophenyl)ethyl)amino)-6-(1-(fluoromethyl)cyclopropyl)-2-methyl-7-oxo-6,7-dihydropyrido[4,3-*d*]pyrimidine-8-carboxylate (100 mg) was dissolved in 0.5 mL of morpholine, and the reaction was completed after the mixture was stirred at 90 °C for 6 h. The reaction mixture was concentrated and purified by column chromatography (dichloromethane/methanol gradient elution) to give the title compound.

$^1$H NMR (400 MHz, DMSO-$d_6$) δ 9.22 (s, 1H), 9.05-8.91 (m, 1H), 7.70-7.62 (m, 1H), 7.56-7.50 (m, 1H), 7.38-7.10 (m, 2H), 5.81-5.71 (m, 1H), 4.81-4.42 (m, 2H), 3.71-3.63 (m, 1H), 3.62-3.56 (m, 2H), 3.54-3.48 (m, 2H), 3.48-3.42 (m, 1H), 3.18-3.08 (m, 2H), 2.21-2.15 (m, 3H), 1.61-1.55 (m, 3H), 1.37-1.20 (m, 4H). LC/MS(m/z, MH$^+$): 534.2.

**Example 117 (*R*)-6-(1-(fluoromethyl)cyclopropyl)-2-methyl-4-((1-(2-methyl-3-nitrophenyl) ethyl)amino)-8-morpholinylpyrido[4,3-*d*]pyrimidine-7(6*H*)-one**

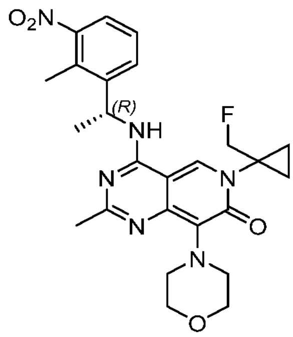

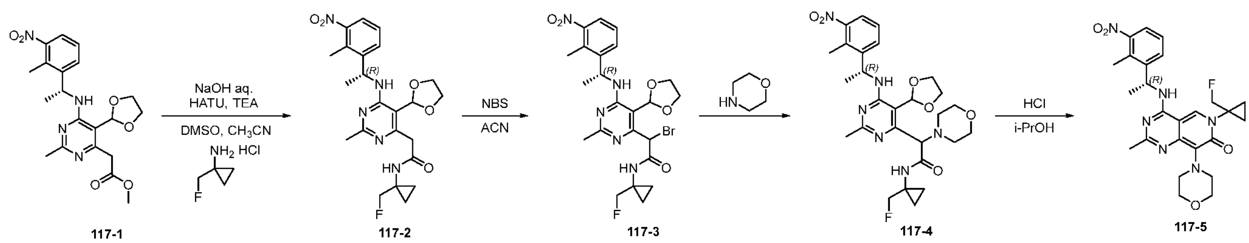


117-1 117-2 117-3 117-4 117-5

For the synthesis of 117-2 and 117-3, reference was made to step 2 and step 3 of Example 16. For the synthesis of 117-4 and 117-5, reference was made to Example 34. The title compound was prepared according to the above-mentioned technical route.

$^1$H NMR (400 MHz, DMSO) δ 8.80 (s, 1H), 8.76 (br, 1H), 7.74-7.63 (m, 2H), 7.42 (t, *J*= 7.9 Hz, 1H), 5.69-5.59 (m, 1H), 4.91-4.33 (m, 2H), 3.63 (t, *J*= 4.6 Hz, 4H), 3.31-3.18 (m, 4H), 2.49 (s, 3H), 2.21 (s, 3H), 1.53 (d, *J*= 7.0 Hz, 3H), 1.37-1.19 (m, 4H). LC/MS(m/z, MH$^+$): 497.2.

**Example 118 (*R*)-4-((4-((1-(3-(difluoromethyl)-2-fluorophenyl)ethyl)amino)-2-methyl-6-morpholino-7-oxo-6,7-dihydropyrido[4,3-*d*]pyrimidin-8-yl)oxy)pyridinecarbonitrile**

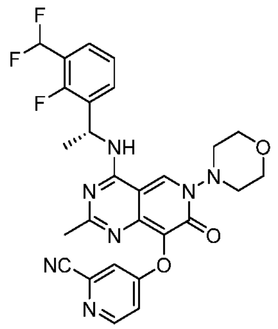

**Step 1 synthesis of 2-((2-cyanopyridin-4-yl)oxy)-2-(6-(((*R*)-1-(3-(difluoromethyl)-2-fluorophenyl) ethyl)amino)-5-(1,3-dioxolan-2-yl)-2-methylpyrimidin-4-yl)-*N*-morpholinoacetamide**

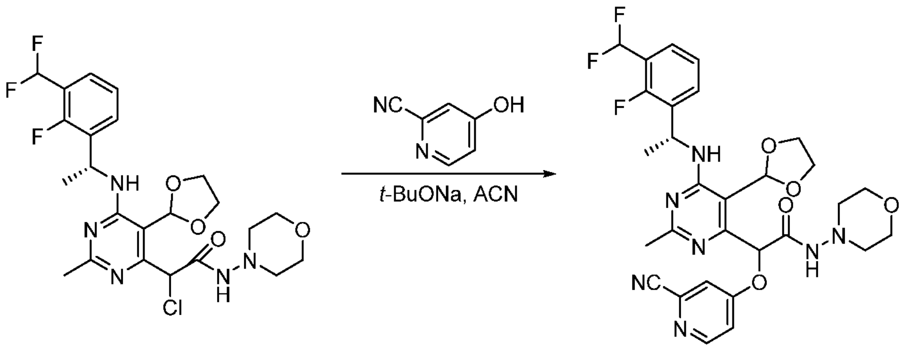


2-Chloro-2-(6-((*R*)-1-(3-(difluoromethyl)-2-fluorophenyl)ethyl)amino)-5-(1,3-dioxolan-2-yl)-2-methylpyrimidin-4-yl)-*N*-morpholinoacetamide (100 mg) was dissolved in 5 mL of acetonitrile. 4-Hydroxypyridinecarbonitrile (200 mg) and sodium *tert*-butoxide (200 mg) were added. The reaction was completed after the mixture was stirred at room temperature for 2 h. The reaction mixture was poured into a saturated ammonium chloride solution and extracted with dichloromethane. The organic phase was dried over anhydrous sodium sulfate, filtered, concentrated, and purified by column chromatography (dichloromethane/methanol gradient elution) to give the title compound.

**Step 2 synthesis of (*R*)-4-((4-((1-(3-(difluoromethyl)-2-fluorophenyl)ethyl)amino)-2-methyl-6-morpholino-7-oxo-6,7-dihydropyrido[4,3-*d*]pyrimidin-8-yl)oxy)pyridinecarbonitrile**

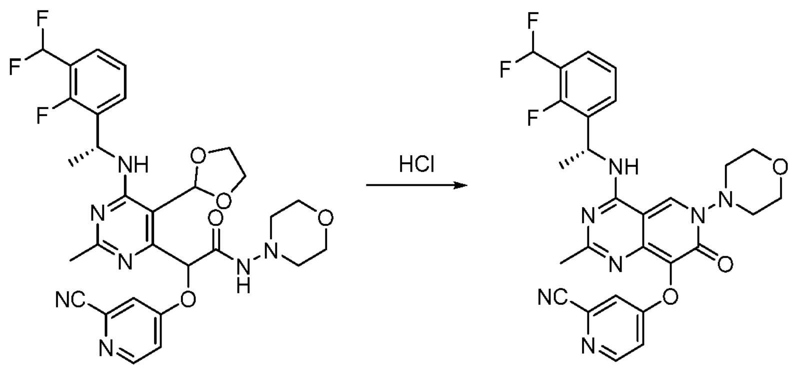


The title compound was prepared with reference to the synthesis method in step **5** of Example **20.**

$^1$H NMR (400 MHz, DMSO) δ 9.31 (s, 1H), 9.08 (d, *J*= 7.2 Hz, 1H), 8.52 (d, *J*= 5.8 Hz, 1H), 7.72 (t, *J*= 7.3 Hz, 1H), 7.67 (d, *J*= 2.5 Hz, 1H), 7.54 (t, *J*= 7.0 Hz, 1H), 7.35 (t, *J*= 8.1 Hz, 1H), 7.26 (dd, *J* = 5.8, 2.6 Hz, 1H), 7.24 (t, *J*=

54.0 Hz, 1H), 5.86-5.76 (m, 1H), 3.91-3.38 (m, 8H), 2.17 (s, 3H), 1.59 (d, $J$= 7.0 Hz, 3H). LC/MS(m/z, $MH^+$): 552.2.

**Example 119 (*R*)-8-(difluoromethoxy)-4-((1-(3-(difluoromethyl)-2-fluorophenyl)ethyl)amino)-6-(1-(fluoromethyl)cyclopropyl)-2-methylpyrido[4,3-*d*]pyrimidine-7(6*H*)-one**

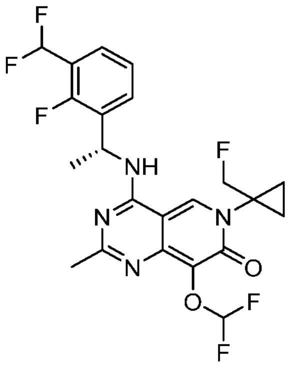

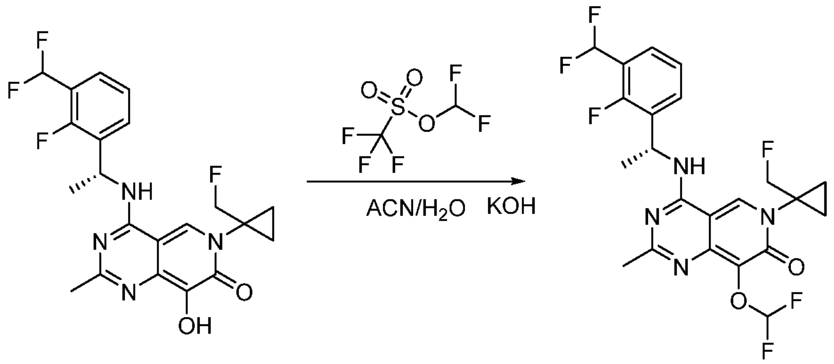


(*R*)-4-((1-(3-(Difluoromethyl)-2-fluorophenyl)ethyl)amino)-6-(1-(fluoromethyl)cyclopropyl)-8-hydroxy-2-methylpyrido[4,3-*d*]pyrimidine-7(6*H*)-one (50.0 mg) was dissolved in 0.4 mL of acetonitrile and 0.4 mL of a 6 M potassium hydroxide solution. Difluoromethyl trifluoromethanesulfonate (22.9 mg) was added, and the reaction was completed after the mixture was reacted at room temperature for 5 min. A 1 N hydrochloric acid solution was added to adjust the pH to 7-8, and the mixture was extracted with dichloromethane. The organic phase was dried over anhydrous sodium sulfate, filtered, concentrated, and purified by column chromatography (dichloromethane/methanol gradient elution) to give the title compound.

$^1H$ NMR (400 MHz, DMSO) $\delta$ 9.16 (s, 1H), 9.03 (d, $J$= 6.8 Hz, 1H), 7.65 (t, $J$= 7.4 Hz, 1H), 7.53 (t, $J$ = 6.9 Hz, 1H), 7.38-6.88 (m, 3H), 5.82-5.71 (m, 1H), 4.82-4.47 (m, 2H), 2.24 (s, 3H), 1.59 (d, $J$ = 7.1 Hz, 3H), 1.40-1.21 (m, 4H). LC/MS(m/z, $MH^+$): 487.2.

**Example 120 (*R*)-4-((1-(3-(difluoromethyl)-2-fluorophenyl)ethyl)amino)-2-methyl-6-morpholino -8-(pyrazolo[1,5-*a*]pyrimidin-6-yl)pyrido[4,3-*d*]pyrimidine-7(6*H*)-one**

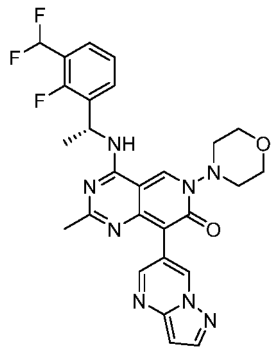

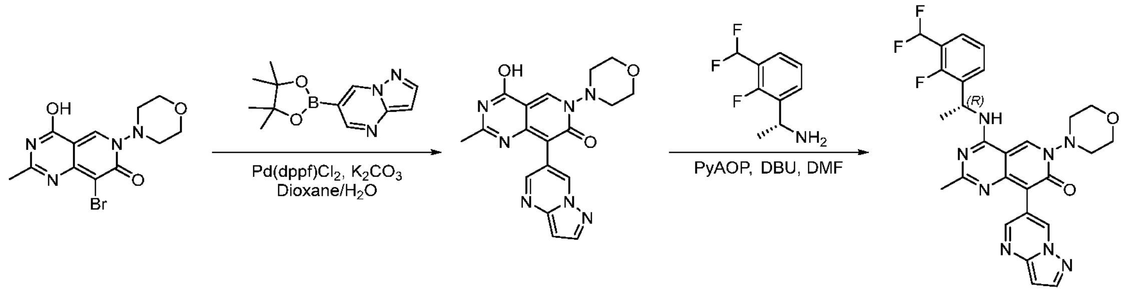


Reference was made to the synthesis method of Example **89,** and the title compound was prepared according to the above-mentioned technical route.

$^1$H NMR (400 MHz, DMSO) δ 9.36 (s, 1H), 9.11-9.06 (m, 1H), 9.01 (d, *J* = 7.1 Hz, 1H), 8.66 (d, *J*= 2.0 Hz, 1H), 8.21 (d, *J* = 2.3 Hz, 1H), 7.71 (t, *J* = 7.3 Hz, 1H), 7.54 (t, *J* = 6.8 Hz, 1H), 7.34 (t, *J* = 7.7 Hz, 1H), 7.24 (t, *J*= 54.3 Hz, 1H), 6.73-6.67 (m, 1H), 5.85-5.73 (m, 1H), 4.18-3.38 (m, 8H), 2.18 (s, 3H), 1.59 (d, *J*= 7.0 Hz, 3H). LC/MS(m/z, $MH^+$): 551.2.

**Example 121 (*R*)-4-((1-(3-(difluoromethyl)-2-fluorophenyl)ethyl)amino)-2-methyl-8-((3-methloxetan-3-yl)methoxy)-6-morpholinylpyrido[4,3-*d*]pyrimidine-7(6*H*)-one**

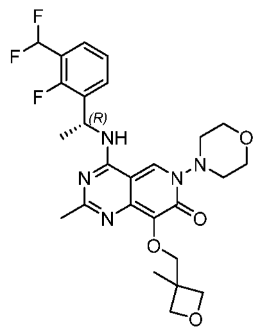

The title compound was prepared in the same manner as in Example **118** except for replacing 4-hydroxypyridinecarbonitrile in step 1 with (3-methyloxetan-3-yl)methanol.

$^1$H NMR (400 MHz, DMSO-$d_6$) δ 9.05 (s, 1H), 8.82 (d, *J*= 7.0 Hz, 1H), 7.68 (t, *J*= 7.3 Hz, 1H), 7.52 (t, *J* = 6.9 Hz, 1H), 7.32 (t, *J* = 7.7 Hz, 1H), 7.24 (t, *J* = 54.3 Hz, 1H), 5.81-5.70 (m, 1H), 4.63 (d, *J* = 5.5 Hz, 2H), 4.24 (d, *J* = 5.6 Hz, 2H), 4.07-3.98 (m, 2H), 3.94-3.39 (m, 8H), 2.22 (s, 3H), 1.55 (d, *J* = 7.0 Hz, 3H), 1.37 (s, 3H). LC/MS(m/z, $MH^+$): 534.2.

**Example 122 (*R*)-4-((1-(3-(difluoromethyl)-2-fluorophenyl)ethyl)amino)-6-(1-(fluoromethyl) cyclopropyl)-2-methyl-8-(pyridin-2-ylmethoxy)pyrido[4,3-*d*]pyrimidine-7(6*H*)-one**

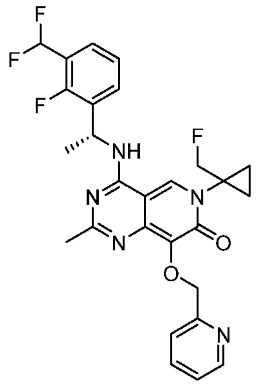

The title compound was prepared in the same manner as in Example **23** except for replacing methanol-d3 in step **4** with 2-pyridinemethanol.

$^1$H NMR (400 MHz, DMSO-$d_6$) δ 9.00 (s, 1H), 8.87 (d, *J* = 7.2 Hz, 1H), 8.46 (d, *J* = 4.8 Hz, 1H), 7.99 (d, *J* = 7.8 Hz, 1H), 7.86-7.76 (m, 1H), 7.65 (t, *J* = 7.2 Hz, 1H), 7.52 (t, *J* = 7.0 Hz, 1H), 7.39-7.10 (m, 3H), 5.82-5.72 (m, 1H), 5.20 (s, 2H), 4.85-4.45 (m, 2H), 2.24 (s, 3H), 1.58 (d, *J*= 7.1 Hz, 3H), 1.40-119 (m, 4H). LC/MS(m/z, $MH^+$): 528.2.

**Example 123 (*R*)-4-((1-(3-(difluoromethyl)-2-fluorophenyl)ethyl)amino)-8-(3,5-dimethylisoxazol- 4-yl)-6-(1-(fluoromethyl)cyclopropyl)-2-methylpyrido[4,3-*d*]pyrimidine-7(6*H*)-one**

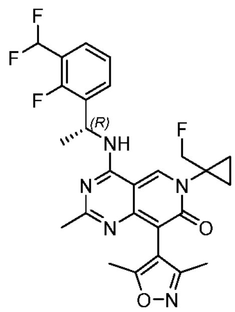

The title compound was prepared in the same manner as in Example **12** except for replacing (1-methyl-1*H*-pyrazol-4-yl)boronic acid with 3,5-dimethylisoxazole-4-boronic acid pinacol ester.

$^1$H NMR (400 MHz, DMSO-$d_6$) δ 9.27 (s, 1H), 8.98 (d, *J* = 7.0 Hz, 1H), 7.69 (t, *J* = 7.2 Hz, 1H), 7.53 (t, *J* = 7.2 Hz, 1H), 7.34 (t, *J* = 7.7 Hz, 1H), 7.24 (t, *J* = 54.4 Hz, 1H), 5.83-5.74 (m, 1H), 4.91-4.50 (m, 2H), 2.17 (s, 3H), 2.14 (d, *J*= 14.3 Hz, 3H), 2.00 (d, *J* = 14.2 Hz, 3H), 1.60 (d, *J*= 7.1 Hz, 3H), 1.29 (d, *J*= 49.9 Hz, 4H). LC/MS(m/z, $MH^+$): 516.2.

**Example 124 (*R*)-4-((1-(3-(difluoromethyl)-2-fluorophenyl)ethyl-1-*d*)amino)-8-(1,1-dioxidothiomorpholinyl)-6-(1-(fluoromethyl)cyclopropyl)-2-methylpyrido[4,3-*d*]pyrimidine-7(6*H*)-one**

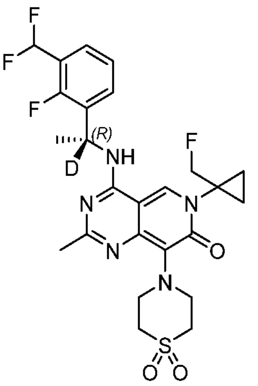

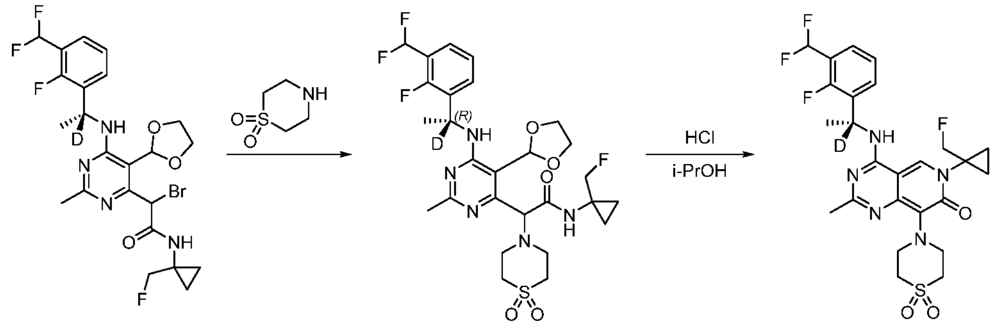


Reference was made to the synthesis method of Example **55,** and the title compound was prepared according to the above-mentioned technical route.

$^1$H NMR (400 MHz, DMSO-$d_6$) $\delta$ 8.88 (s, 1H), 8.76 (s, 1H), 7.62 (t, *J* = 7.2 Hz, 1H), 7.51 (t, *J* = 6.8 Hz, 1H), 7.31 (t, *J* = 7.7 Hz, 1H), 7.23 (t, *J* = 54.4 Hz, 1H), 4.93-4.35 (m, 2H), 3.66-3.53 (m, 4H), 3.25-3.09 (m, 4H), 2.21 (s, 3H), 1.57 (s, 3H), 1.38-1.21 (m, 4H). LC/MS(m/z, MH$^+$): 555.2.

**Example 125 (*R*)-4-((1-(3-(difluoromethyl)-2-fluorophenyl)ethyl-1-*d*)amino)-6-(1-(fluoromethyl) cyclopropyl)-2-methyl-8-(4-(methylsulfonyl)piperidin-1-yl)pyrido[4,3-*d*]pyrimidine-7(6*H*)-one**

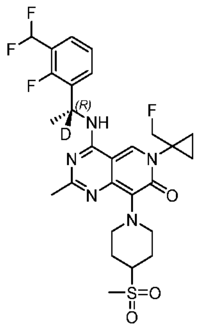

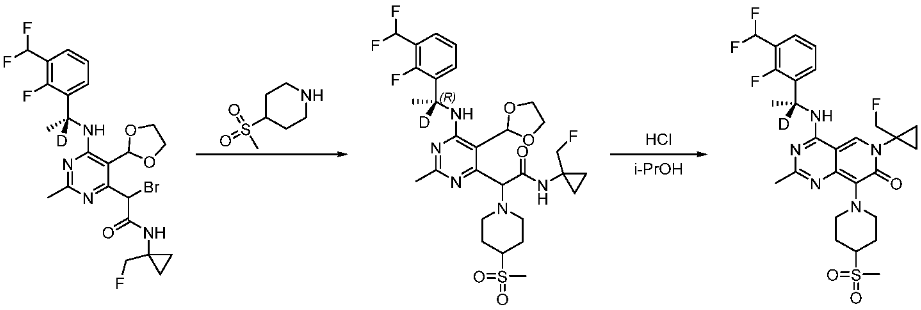


Reference was made to the synthesis method of Example **55,** and the title compound was prepared according to the above-mentioned technical route.

$^1$H NMR (400 MHz, DMSO-$d_6$) δ 8.81 (s, 1H), 8.65 (s, 1H), 7.63 (t, *J* = 7.0 Hz, 1H), 7.52 (t, *J* = 6.8 Hz, 1H), 7.32 (t, *J* = 7.7 Hz, 1H), 7.23 (t, *J* = 54.4 Hz, 1H), 4.81-4.46 (m, 2H), 3.47-3.37 (m, 2H), 3.29-3.12 (m, 3H), 2.93 (s, 3H), 2.20 (s, 3H), 2.01-1.92 (m, 2H), 1.80-1.64 (m, 2H), 1.56 (s, 3H), 1.36-1.19 (m, 4H). LC/MS(m/z, MH$^+$): 583.2.

**Example 126 (*R*)-8-((1-acetylpiperidin-4-yl)amino)-4-((1-(3-(difluoromethyl)-2-fluorophenyl) ethyl)amino)-6-(1-(fluoromethyl)cyclopropyl)-2-methylpyrido[4,3-*d*]pyrimidine-7(6*H*)-one**

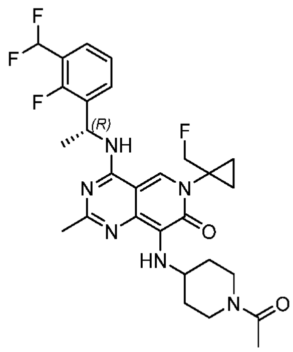

The title compound was prepared in the same manner as in Example 77 except for replacing N-methylbenzylamine in step **1** with 1-(4-(benzylamino)piperidin-1-yl)ethane-1-one.

$^1$H NMR (400 MHz, DMSO-$d_6$) δ 8.59 (d, *J*= 5.3 Hz, 1H), 8.47 (s, 1H), 7.63 (t, *J*= 7.2 Hz, 1H), 7.51 (t, *J*= 6.9 Hz, 1H), 7.32 (t, *J*= 7.7 Hz, 1H), 7.24 (t, *J*= 54.4 Hz, 1H), 5.81-5.72 (m, 1H), 5.07-4.94 (m, 1H), 4.79-4.48 (m, 3H), 4.23-4.14 (m, 1H), 3.79-3.65 (m, 1H), 3.14-3.01 (m, 1H), 2.73-2.62 (m, 1H), 2.20 (s, 3H), 1.97 (s, 3H), 1.93-1.78 (m, 2H), 1.57 (d, *J*= 7.1 Hz, 3H), 1.37-1.21 (m, 4H), 1.17-1.03 (m, 1H). LC/MS(m/z, MH$^+$): 561.3.

**Example 127 4-(((*R*)-1-(3-(difluoromethyl)-2-fluorophenyl)ethyl)amino)-8-((3*S*,5*S*)-3,5-dimethylmorpholinyl)-6-(1-(fluoromethyl)cyclopropyl)-2-methylpyrido[4,3-*d*]pyrimidine-7(6*H*)-one**

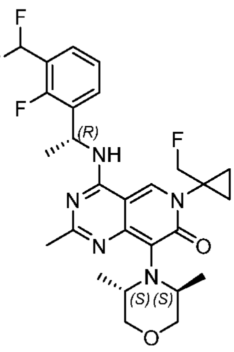

The title compound was prepared in the same manner as in Example **34** except for replacing morpholine in step 1 with (3S,5S)-3,5-dimethylmorpholine.

$^1$H NMR (400 MHz, DMSO-$d_6$) δ 8.95 (s, 1H), 8.70 (d, *J*= 6.6 Hz, 1H), 7.66 (t, *J*= 7.3 Hz, 1H), 7.52 (t, *J* = 6.8 Hz, 1H), 7.32 (t, *J* = 7.7 Hz, 1H), 7.24 (t, *J* = 54.4 Hz, 1H), 5.82-5.70 (m, 1H), 4.88-4.37 (m, 2H), 3.94-3.60 (m, 4H), 3.30-3.23 (m, 2H), 2.20 (s, 3H), 1.57 (d, *J* = 7.1 Hz,3H), 1.37-1.15 (m, 4H), 0.83-0.66 (m, 6H). LC/MS(m/z, $MH^+$): 534.2.

**Example 128 (*R*)-5-(4-((1-(3-(difluoromethyl)-2-fluorophenyl)ethyl)amino)-2-methyl-6-morpholinyl-7-oxo-6,7-dihydropyrido[4,3-*d*]pyrimidin-8-yl)-1*H*-pyrazole-3-carbonitrile**

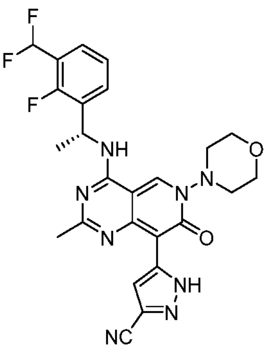

**Step 1: synthesis of 5-bromo-1-(tetrahydro-2*H*-pyran-2-yl)-1*H*-pyrazole-3-carbonitrile**

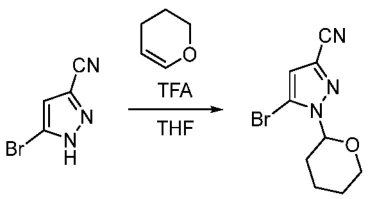


5-Bromo-1*H*-pyrazole-3-carbonitrile (500 mg) was dissolved in 5 mL of tetrahydrofuran, and 3,4-dihydro-2H-pyran (978 mg) and trifluoroacetic acid (66 mg) were added. The reaction was completed after the mixture was stirred at 60 °C for 4 h. The reaction mixture was concentrated and purified by column chromatography (petroleum ether/ethyl acetate gradient elution) to give the title compound.

Step 2: **synthesis of 1-(tetrahydro-2*H*-pyran-2-yl)-5-(4,4,5,5-tetramethyl-1,3,2-dioxaborolan-2-yl)-1*H*-pyrazole-3-carbonitrile**

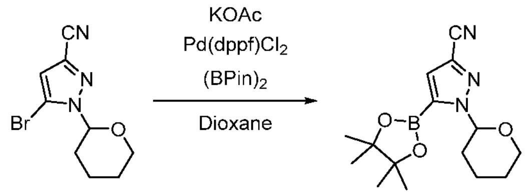


5-Bromo-1-(tetrahydro-2*H*-pyran-2-yl)-1*H*-pyrazole-3-carbonitrile (100 mg) was dissolved in 1 mL of 1,4-dioxane, and bis(pinacolato)diboron (99.1 mg), [1,1'-bis(diphenylphosphino)ferrocene]palladium dichloride (28.3 mg), and potassium acetate (76.6 mg) were added. The reaction was completed after the mixture was heated to 100 °C and stirred for 6 h under argon atmosphere. The reaction mixture was concentrated and directly used in the next step.

**Step 3: synthesis of 5-(4-hydroxy-2-methyl-6-morpholino-7-oxo-6,7-dihydropyrido[4,3-*d*]pyrimidin-8-yl)-1-(tetrahydro-2*H*-pyrin-2-yl))-1*H*-pyrazole-3-carbonitrile**

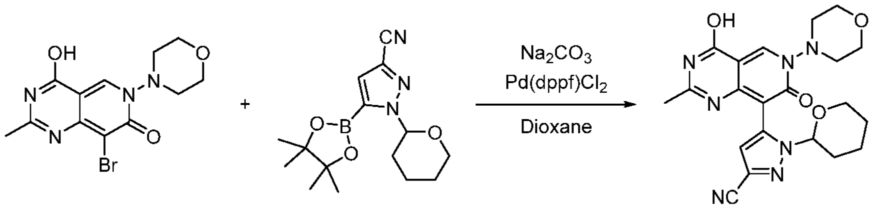


The crude product obtained in the previous step and 8-bromo-4-hydroxy-2-methyl-6-morpholinopyrido[4,3-*d*]pyrimidine-7(6*H*)-one (100 mg) were added to 5 mL of 1,4-dioxane, and [1,1'-bis(diphenylphosphino)ferrocene]palladium dichloride (21.2 mg) and sodium carbonate (81.2 mg) were added. The reaction was completed after the mixture was heated to 100 °C and stirred for 4 h under argon atmosphere. The reaction mixture was concentrated and purified by column chromatography (dichloromethane/methanol gradient elution) to give the title compound.

**Step 4: synthesis of 5-(4-(((*R*)-1-(3-(difluoromethyl)-2-fluorophenyl)ethyl)amino)-2-methyl-6-morpholinyl-7-oxo-6,7-dihydropyrido[4,3-*d*]pyrimidin-8-yl)-1-(tetrahydro-2*H*-pyran-2-yl)-1*H* pyrazole-3-carbonitrile**

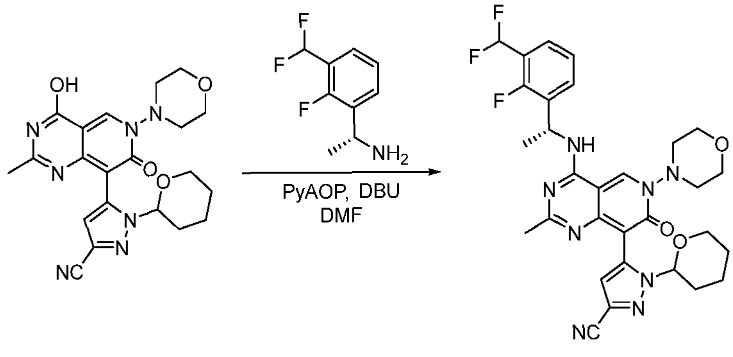


5-(4-Hydroxy-2-methyl-6-morpholino-7-oxo-6,7-dihydropyrido[4,3-d]pyrimidin-8-yl)-1-(tetrahydro-2*H*-pyran-2-yl))-1*H*-pyrazole-3-carbonitrile (100 mg) was dissolved in 1 mL of *N,N*-dimethylformamide. Then (R)-1-(3-(difluoromethyl)-2-fluorophenyl)ethan-1-amine (51.9 mg) and 1,8-diazabicyclo[5.4.0]undec-7-ene (104 mg) were added. Finally, (7-azabenzotriazole-1-oxy)tripyrrolephosphonium hexafluorophosphate (178 mg) was added. The reaction was completed after the mixture was stirred at room temperature for 8 h. 20 mL of saturated brine and 40 mL of ethyl acetate were added, and the mixture was separated. The organic phase was concentrated and purified by column chromatography (dichloromethane/methanol gradient elution) to give the title compound.

**Step 5: synthesis of (*R*)-5-(4-((1-(3-(difluoromethyl)-2-fluorophenyl)ethyl)amino)-2-methyl-6-morpholinyl-7-oxo-6,7-dihydropyrido[4,3-*d*]pyrimidin-8-yl)-1*H*-pyrazole-3-carbonitrile**

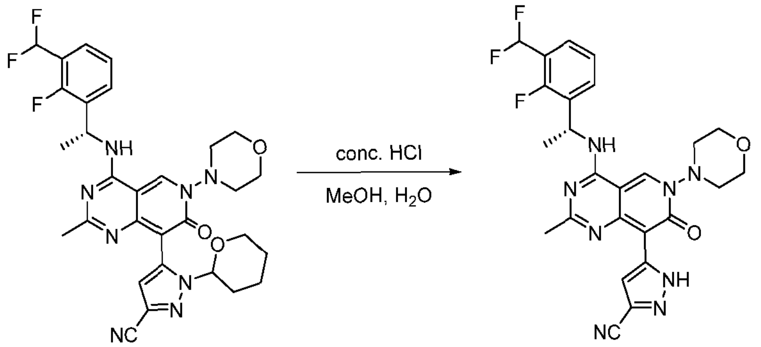


5-(4-(((*R*)-1-(3-(Difluoromethyl)-2-fluorophenyl)ethyl)amino)-2-methyl-6-morpholinyl-7-oxo-6,7-dihydropyrido[4,3-*d*]pyrimidin-8-yl)-1-(tetrahydro-2*H*-pyran-2-yl)-1*H*-pyrazole-3-carbonitrile (50.0 mg) was dissolved in 1 mL of methanol. 0.5 mL of concentrated hydrochloric acid was added, and the reaction was completed after the mixture was stirred at room temperature for 3 h. The reaction mixture was adjusted to pH 8-9 with ammonia in methanol and purified by column chromatography (dichloromethane/methanol gradient elution) to give the title compound.

$^1$H NMR (400 MHz, DMSO-$d_6$) δ 14.30 (s, 1H), 9.42 (s, 1H), 9.18 (d, *J* = 7.2 Hz, 1H), 7.80-7.76 (m, 1H), 7.71 (t, *J* = 7.2 Hz, 1H), 7.54 (t, *J* = 6.8 Hz, 1H), 7.34 (t, *J* = 7.7 Hz, 1H), 7.25 (t, *J* = 54.3 Hz, 1H), 5.89-5.74 (m, 1H), 4.39-3.38 (m, 8H), 2.42 (s, 3H), 1.60 (d, *J*= 7.0 Hz, 3H).

LC/MS(m/z, $MH^+$): 525.2.

**Example 129 (*R*)-4-((1-(3-(difluoromethyl)-2-fluorophenyl)ethyl)amino)-6-(1-(fluoromethyl) cyclopropyl)-2-methyl-8-(1-methyl-1*H*-pyrazolo[4,3-*b*]pyridin-6-yl)pyrido[4,3-*d*]pyrimidine-7(6*H*)-one**

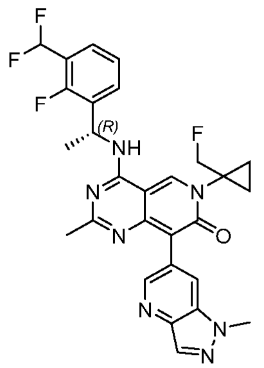

The title compound was prepared in the same manner as in Example **12** except for replacing (1-methyl-1*H*-pyrazol-4-yl)boronic acid with 1-methyl-6-(4,4,5,5-tetramethyl-1,3,2-dioxaborolan-2-yl)-1*H*-pyrazolo[4,3-*b*]pyridine.

$^1$H NMR (400 MHz, DMSO-$d_6$) δ 9.29 (s, 1H), 8.99 (d, *J* = 6.8 Hz, 1H), 8.50 (d, *J*= 1.7 Hz, 1H), 8.20 (d, *J* = 0.8 Hz, 1H), 8.14-8.09 (m, 1H), 7.69 (t, *J* = 7.2 Hz, 1H), 7.54 (t, *J* = 7.0 Hz, 1H), 7.35 (t, *J* = 7.7 Hz, 1H), 7.24 (t, *J* = 54.4 Hz, 1H), 5.86-5.73 (m, 1H), 4.94-4.44 (m, 2H), 4.05 (s, 3H), 2.11 (s, 3H), 1.62 (d, *J*= 7.1 Hz, 3H), 1.44-1.22 (m, 4H). LC/MS(m/z, $MH^+$): 552.2.

**Example 130 (*R*)-3-(1-((2-methyl-6-morpholinyl-7-oxo-6,7-dihydropyrido[4,3-*d*]pyrimidin-4-yl)amino)ethyl)-2-(trifluoromethyl)benzonitrile**

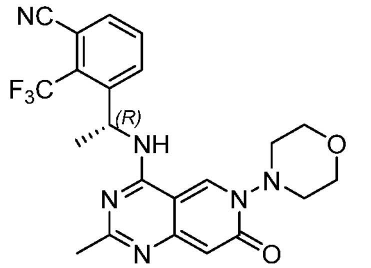

The title compound was prepared in the same manner as in Example **18** except for replacing (*R*)-1-(3-(pentafluoro-λ$^6$-sulfanyl)phenyl)ethane-1-amine in step **1** with (*R*)-3-(1-aminoethyl)-2-(trifluoromethyl)benzonitrile.

$^1$H NMR (400 MHz, DMSO-$d_6$) δ 9.26 (s, 1H), 9.02 (d, *J* = 6.1 Hz, 1H), 8.09 (d, *J* = 8.1 Hz, 1H), 8.01 (d, *J*= 7.4 Hz, 1H), 7.85 (t, *J*= 7.9 Hz, 1H), 6.10 (s, 1H), 5.76-5.66 (m, 1H), 4.14-3.37 (m, 8H), 2.10 (s, 3H), 1.55 (d, *J*= 7.0 Hz, 3H). LC/MS(m/z, MH$^+$): 459.2.

**Example 131 (*R*)-4-((1-(3-(difluoromethyl)-2-fluorophenyl)ethyl)amino)-6-(1-(fluoromethyl) cyclopropyl)-2-methyl-8-(4-(2,2,2-trifluoroethyl)piperazin-1-yl)pyrido[4,3-*d*]pyrimidine-7(6*H*)-one**

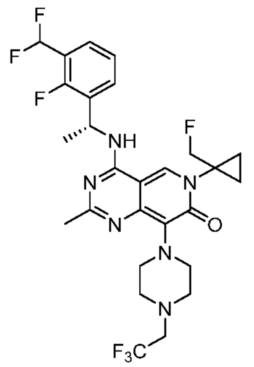

The title compound was prepared in the same manner as in Example **49** except for replacing acetic anhydride in step **3** with 2,2,2-trifluoroethyl trifluoromethanesulfonate.

$^1$H NMR (400 MHz, DMSO-$d_6$) δ 8.80 (s, 1H), 8.63 (br, 1H), 7.62 (t, *J*= 7.2 Hz, 1H), 7.51 (t, *J*= 6.9 Hz, 1H), 7.31 (t, *J* = 7.6 Hz, 1H), 7.23 (t, *J* = 54.4 Hz, 1H), 5.79-5.69 (m, 1H), 4.80-4.45 (m, 2H), 3.31-3.23 (m, 4H), 3.22-3.12 (m, 2H), 2.71-2.62 (m, 4H), 2.19 (s, 3H), 1.57 (d, *J*= 7.0 Hz, 3H), 1.35-1.17 (m, 4H). LC/MS(m/z, MH$^+$): 587.2.

**Example 132 (*R*)-8-(4-(cyclopropylmethyl)piperazin-1-yl)-4-((1-(3-(difluoromethyl)-2-fluorophenyl)ethyl)amino)-6-(1-(fluoromethyl)cyclopropyl)-2-methylpyrido[4,3-*d*]pyrimidine-7(6*H*)-one**

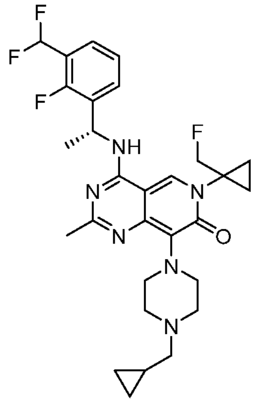

The title compound was prepared in the same manner as in Example **49** except for replacing acetic anhydride in step 3 with (iodomethyl)cyclopropane.

$^1$H NMR (400 MHz, DMSO-$d_6$) $\delta$ 8.77 (s, 1H), 8.61 (d, *J*= 7.1 Hz, 1H), 7.62 (t, *J*= 7.3 Hz, 1H), 7.51 (t, *J* = 6.6 Hz, 1H), 7.31 (t, *J* = 7.7 Hz, 1H), 7.24 (t, *J* = 54.4 Hz, 1H), 5.79-5.68 (m, 1H), 4.86-4.41 (m, 2H), 3.32-3.23 (m, 4H), 2.18 (s, 3H), 1.56 (d, *J*= 7.1 Hz, 3H), 1.34-1.11 (m, 5H), 0.55-0.36 (m, 2H), 0.11-0.04 (m, 2H). LC/MS(m/z, MH$^+$): 559.2.

**Example 133 (*R*)-4-((1-(3-(difluoromethyl)-2-fluorophenyl)ethyl)amino)-6-(1-(fluoromethyl) cyclopropyl)-2-methyl-8-(4-(oxetan-3-ylmethyl)piperazin-1-yl)pyrido[4,3-*d*]pyrimidine-7(6*H*)-one**

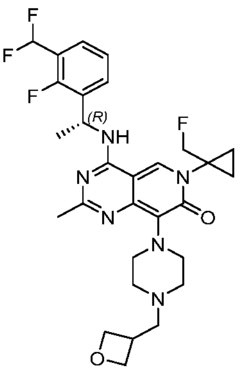

The title compound was prepared in the same manner as in Example **49** except for replacing acetic anhydride in step 3 with 3-iodomethyloxetane.

$^1$H NMR (400 MHz, DMSO-$d_6$) $\delta$ 8.78 (s, 1H), 8.62 (d, *J*= 7.1 Hz, 1H), 7.62 (t, *J*= 7.4 Hz, 1H), 7.50 (d, *J* = 6.6 Hz, 1H), 7.31 (t, *J* = 7.5 Hz, 1H), 7.24 (t, *J* = 54.5 Hz, 1H), 5.79-5.68 (m, 1H), 4.89-4.42 (m, 4H), 4.27 (t, *J*= 5.9 Hz, 2H), 3.30-3.16 (m, 4H), 2.45-2.31 (m, 4H), 2.18 (s, 3H), 1.57 (d, *J*= 6.9 Hz, 3H), 1.39-1.17 (m, 4H). LC/MS(m/z, MH$^+$): 575.2.

**Example 134 (*R*)-2-(4-(4-((1-(3-(difluoromethyl)-2-fluorophenyl)ethyl)amino)-6-(1-(fluoromethyl)cyclopropyl)-2-methyl-7-oxo-6,7-dihydropyrido[4,3-*d*]pyrimidin-8-yl)piperazin-1-yl)acetonitrile**

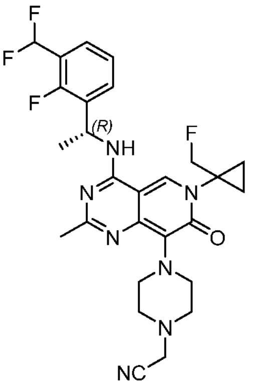

The title compound was prepared in the same manner as in Example **49** except for replacing acetic anhydride in step 3 with bromoacetonitrile.

$^1$H NMR (400 MHz, DMSO-$d_6$) $\delta$ 8.81 (s, 1H), 8.65 (d, *J*= 6.4 Hz, 1H), 7.62 (t, *J*= 7.4 Hz, 1H), 7.51 (t, *J* = 6.8 Hz, 1H), 7.31 (t, *J* = 7.7 Hz, 1H), 7.24 (t, *J* = 54.4 Hz, 1H), 5.80-5.69 (m, 1H), 4.82-4.45 (m, 2H), 3.74 (s, 2H), 3.33-3.27 (m, 4H), 2.57-2.52 (m, 4H), 2.19 (s, 3H), 1.57 (d, *J* = 7.1 Hz, 3H), 1.37-1.21 (m, 4H). LC/MS(m/z, MH$^+$): 544.2.

**Example 135 4-(((*R*)-1-(3-(difluoromethyl)-2-fluorophenyl)ethyl)amino)-6-(1-(fluoromethyl) cyclopropyl)-8-((*S*)-3-(hydroxymethyl)morpholinyl)-2-methylpyrido[4,3-*d*]pyrimidine-7(6*H*)-one**

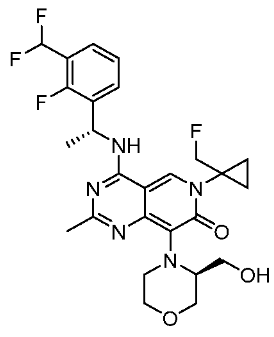

The title compound was prepared in the same manner as in Example **34** except for replacing morpholine in step 1 with (*S*)-morpholin-3-ylmethanol.

$^1$H NMR (400 MHz, DMSO-$d_6$) δ 9.03 (s, 1H), 8.89 (br, 1H), 7.66 (t, *J*= 7.2 Hz, 1H), 7.53 (t, *J*= 6.9 Hz, 1H), 7.33 (t, *J*= 7.7 Hz, 1H), 7.24 (t, *J*= 54.4 Hz, 1H), 5.82-5.69 (m, 1H), 5.06 (s, 1H), 4.87-4.43 (m, 2H), 3.78-3.70 (m, 2H), 3.66-3.53 (m, 2H), 3.50-3.39 (m, 2H), 3.31-3.19 (m, 2H), 2.99-2.90 (m, 1H), 2.21 (s, 3H), 1.58 (d, *J*=7.1 Hz, 3H), 1.39-1.17 (m, 4H). LC/MS(m/z, MH$^+$): 536.2.

**Example 136 4-(((*R*)-1-(3-(difluoromethyl)-2-fluorophenyl)ethyl)amino)-6-(1-(fluoromethyl) cyclopropyl)-8-((*R*)-3-(fluoromethyl)morpholinyl)-2-methylpyrido[4,3-*d*]pyrimidine-7(6*H*)-one**

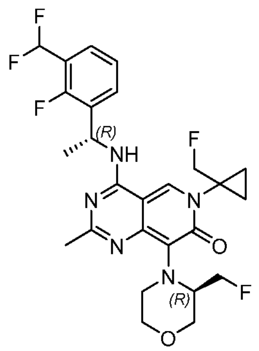

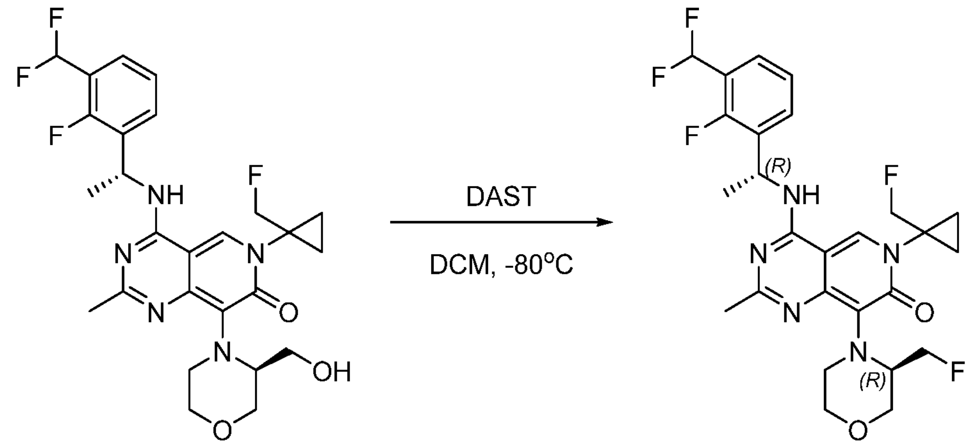

4-(((*R*)-1-(3-(Difluoromethyl)-2-fluorophenyl)ethyl)amino)-6-(1-(fluoromethyl)cyclopropyl)-8-((*S*)-3-(hydroxymethyl)morpholinyl)-2-methylpyrido[4,3-d]pyrimidine-7(6H)-one (20.0 mg) was added to 2 mL of anhydrous dichloromethane. The mixture was cooled to -80 °C under argon atmosphere. Diethylaminosulfur trifluoride (20.0 mg) was added dropwise, after which the reaction was completed after the mixture was stirred for another 3 h. The reaction mixture was added dropwise to ice water, and the pH was adjusted to 7-8 with an aqueous sodium bicarbonate solution. The mixture was extracted with ethyl acetate. The organic layer was concentrated and purified by column chromatography (dichloromethane/methanol gradient elution) to give the title compound.

$^1H$ NMR (400 MHz, DMSO-$d_6$) δ 8.93 (s, 1H), 8.73 (d, *J* = 7.0 Hz, 1H), 7.65 (t, *J* = 7.3 Hz, 1H), 7.52 (t, *J*= 6.9 Hz, 1H), 7.32 (t, *J*= 7.7 Hz, 1H), 7.24 (t, *J*= 54.4 Hz, 1H), 5.81-5.68 (m, 1H), 4.83-4.30 (m, 4H), 3.94-3.83 (m, 1H), 3.83-3.75 (m, 1H), 3.72-3.60 (m,3H), 3.59-3.51 (m, 1H), 2.99-2.86 (m, 1H), 2.21 (s, 3H), 1.57 (d, *J*= 7.1 Hz, 3H), 1.39-1.19 (m, 4H). LC/MS(m/z, $MH^+$): 538.2.

**Example 137 (R)-4-((1-(3-(difluoromethyl)-2-fluorophenyl)ethyl)amino)-6-(1-(fluoromethyl) cyclopropyl)-2-methyl-*N*-(methyl-d3)-7-oxo-6,7-dihydropyrido[4,3-*d*]pyrimidine-8-carboxamide**

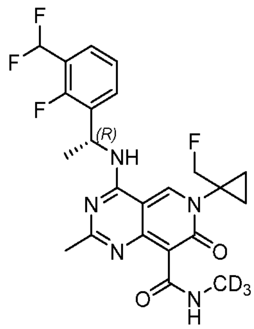

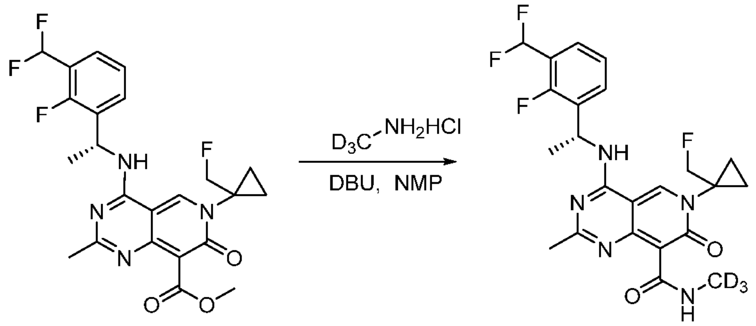


Methyl (*R*)-4-((1-(3-(difluoromethyl)-2-fluorophenyl)ethyl)amino)-6-(1-(fluoromethyl)cyclopropyl)-2-methyl-7-oxo-6,7-dihydropyrido[4,3-d]pyrimidine-8-carboxylate (80.0 mg) and deuterated methylamine hydrochloride (235 mg) were dissolved in 0.8 mL of *N*-methyl-2-pyrrolidone. 1,8-Diazacyclo[5,4,0]undecene-7 (509 mg) was added, and the reaction was completed after the mixture was stirred at 50 °C for 6 h. 20 mL of ethyl acetate was added to the reaction mixture for dilution, and the mixture was extracted with saturated sodium chloride. The organic phase was dried with anhydrous sulfuric acid, filtered, concentrated, and purified by column chromatography (dichloromethane/methanol gradient elution) to give the title compound.

$^1H$ NMR (400 MHz, DMSO-$d_6$) δ 9.20 (s, 1H), 8.92 (d, *J* = 6.6 Hz, 1H), 7.73 (s, 1H), 7.63 (t, *J* = 7.3 Hz, 1H), 7.55-7.49 (m, 1H), 7.32 (t, *J* = 7.7 Hz, 1H), 7.24 (t, *J* = 54.4 Hz, 1H), 5.79-5.70 (m, 1H), 4.78-4.51 (m, 2H), 2.16 (s, 3H), 1.59 (d, *J*= 7.0 Hz, 3H), 1.37-1.22 (m, 4H).

LC/MS(m/z, $MH^+$): 481.2.

**Example 138 (*R*)-4-((1-(3-(difluoromethyl)-2-fluorophenyl)ethyl)amino)-8-(6,7-dihydro-4*H*-pyrazolo[5,1-*c*][1,4]oxazin-3-yl)-2-methyl-6-morpholinylpyrido[4,3-*d*]pyrimidine-7(6*H*)-one**

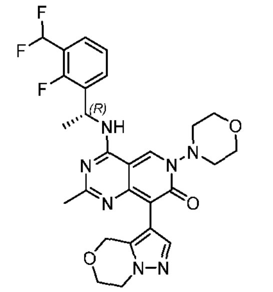

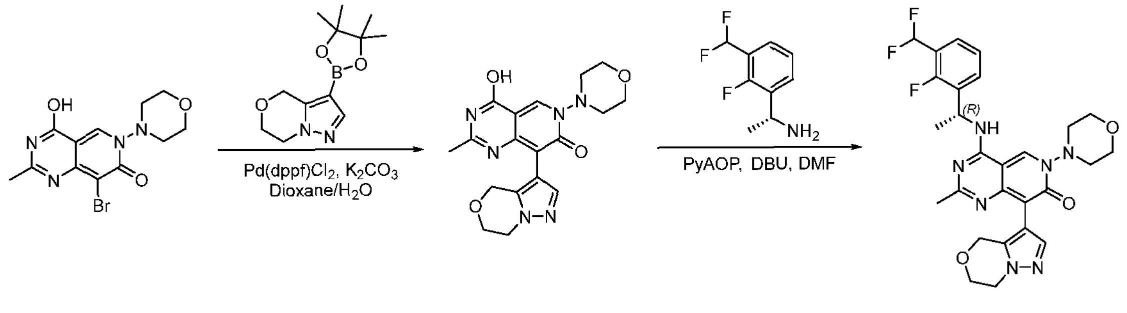


Reference was made to the synthesis method of Example **89,** and the title compound was prepared according to the above-mentioned technical route.

$^1$H NMR (400 MHz, DMSO-d$_6$) δ 9.19 (s, 1H), 8.84 (d, *J*= 7.2 Hz, 1H), 7.73-7.66 (m, 2H), 7.53 (t, *J* = 7.1 Hz, 1H), 7.33 (t, *J* = 7.7 Hz, 1H), 7.24 (t, *J* = 54.3 Hz, 1H), 5.84-5.69 (m, 1H), 4.72-4.58 (m, 2H), 4.25-3.44 (m, 12H), 2.21 (s, 3H), 1.57 (d, *J*= 7.0 Hz, 3H). LC/MS(m/z, MH$^+$): 556.2.

**Example 139 4-(((*R*)-1-(3-(difluoromethyl)-2-fluorophenyl)ethyl)amino)-6-(1-(fluoromethyl) cyclopropyl)-8-(3-hydroxytetrahydrofuran-3-yl)-2-methylpyrido[4,3-*d*]pyrimidine-7(6*H*)-one**

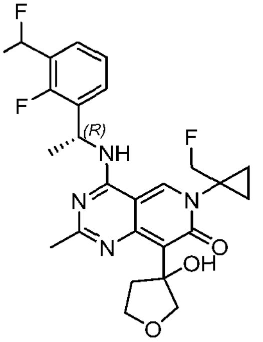

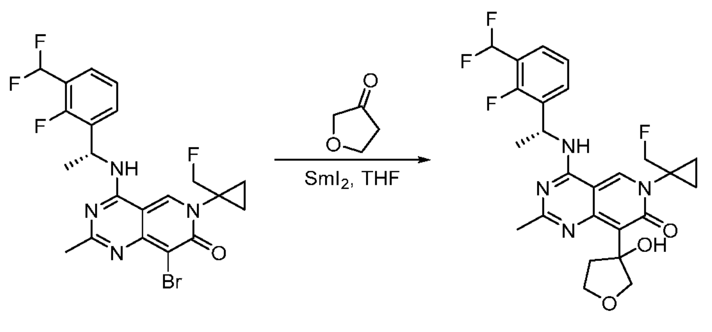


(*R*)-8-Bromo-4-((1-(3-(difluoromethyl)-2-fluorophenyl)ethyl)amino)-6-(1-(fluoromethyl)cyclopropyl)-2-methylpyrido[4,3-*d*]pyrimidine-7(6*H*)-one (100 mg) was dissolved in 1 mL of anhydrous tetrahydrofuran, and dihydro-3(2*H*)-furanone (34.5 mg) was added. The mixture was cooled to -78 °C under argon atmosphere. A solution of samarium diiodide in tetrahydrofuran (0.1 M, 10 mL) was added dropwise, after which the reaction was completed after the mixture was stirred for another 3 h. The reaction was quenched with 5 mL of a saturated ammonium chloride solution. The mixture was diluted with 20 mL of ethyl acetate and filtered through celite. The filtrate was concentrated and purified by column chromatography (dichloromethane/methanol gradient elution) to give the title compound.

$^1$H NMR (400 MHz, DMSO-$d_6$) δ 9.23 (s, 1H), 9.06 (d, *J*= 7.0 Hz, 1H), 8.80 (s, 1H), 7.64 (t, *J*= 7.2 Hz, 1H), 7.53 (t, *J*= 6.9 Hz, 1H), 7.33 (t, *J*= 7.7 Hz, 1H), 7.24 (t, *J*= 54.3 Hz, 1H), 5.84-5.71 (m, 1H), 4.84-4.40 (m, 2H), 4.09-3.98 (m, 1H), 3.95-3.82 (m, 2H), 3.75-3.65 (m, 1H), 2.89-2.73 (m, 1H), 2.22 (s, 3H), 2.02-1.87 (m, 1H), 1.60 (d, *J*= 7.0 Hz, 3H), 1.41-1.17 (m, 4H).

LC/MS(m/z, MH$^+$): 507.2.

**Example 140 4-(((*R*)-1-(3-(difluoromethyl)-2-fluorophenyl)ethyl)amino)-6-(1-(fluoromethyl) cyclopropyl)-8-(3-methoxytetrahydrofuran-3-yl)-2-methylpyrido[4,3-*d*]pyrimidine-7(6*H*)-one**

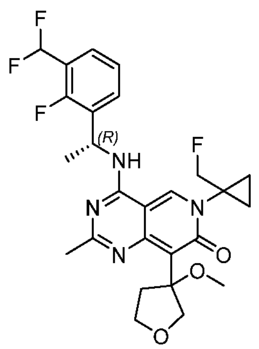

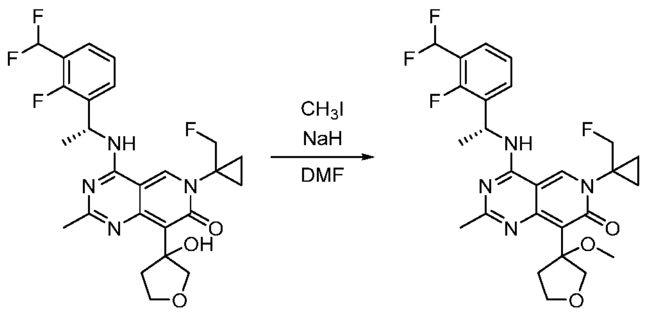


4-(((*R*)-1-(3-(Difluoromethyl)-2-fluorophenyl)ethyl)amino)-6-(1-(fluoromethyl)cyclopropyl)-8-(3-hydroxytetrahydrofuran-3-yl)-2-methylpyrido[4,3-*d*]pyrimidine-7(6*H*)-one (30.0 mg) was dissolved in 1 mL of anhydrous *N,N*-dimethylformamide. Sodium hydride (60% content, 4.74 mg) was added thereto. After the mixture was stirred at room temperature for 20 min, iodomethane (8.41 mg) was added, and the reaction was completed after the mixture was stirred for another 6 h. The reaction was quenched with 2 mL of water. The mixture was diluted with 20 mL of ethyl acetate. The organic phase was washed with saturated brine, dried over anhydrous sodium sulfate, filtered, concentrated, and purified by column chromatography (dichloromethane/methanol gradient elution) to give the title compound.

$^1$H NMR (400 MHz, DMSO-$d_6$) δ 9.22 (s, 1H), 8.86 (d, *J*= 7.0 Hz, 1H), 7.66 (t, *J*= 7.4 Hz, 1H), 7.52 (t, *J* = 7.1 Hz, 1H), 7.33 (t, *J* = 7.6 Hz, 1H), 7.24 (t, *J* = 54.3 Hz, 1H), 5.83-5.71 (m, 1H), 4.85-4.37 (m, 3H), 3.76-3.60 (m, 2H), 3.26-3.16 (m, 1H), 2.98-2.92 (m, 3H), 2.20 (s, 3H), 2.08-1.95 (m, 1H), 1.58 (d, *J*= 7.1 Hz, 3H), 1.37-1.18 (m, 4H). LC/MS(m/z, MH$^+$): 521.2.

**Example 141 (*R*)-4-((1-(3-(difluoromethyl)-2-fluorophenyl)ethyl)amino)-8-(2,3-dihydroimidazo [1,2-*a*]pyridin-7-yl)-6-(1-(fluoromethyl)cyclopropyl)-2-methylpyrido[4,3-*d*]pyrimidine-7(6*H*)-one**

Step 1: synthesis of 6-bromo-2,3-dihydroimidazol[1,2-*a*]pyridine

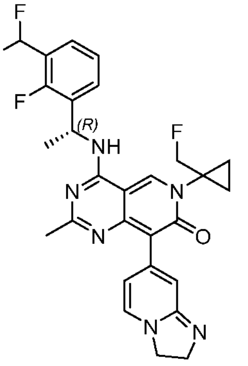

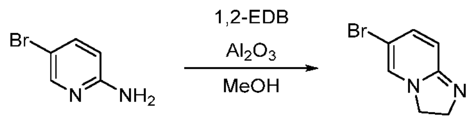


5-Bromopyridine-2-amine (1.00 g), basic alumina (58.9 mg), and 1,2-dibromoethane (2.17 g) were added to a microwave reaction tube. Then 1 mL of methanol was added. The reaction was completed after the mixture was microwaved to 100 °C and stirred for 12 h. The reaction mixture was concentrated and purified by column chromatography (dichloromethane/methanol gradient elution) to give the title compound.

**Step 2: synthesis of 6-(4,4,5,5-tetramethyl-1,3,2-dioxaborolan-2-yl)-2,3-dihydroimidazo[1,2-*a*]pyridine**

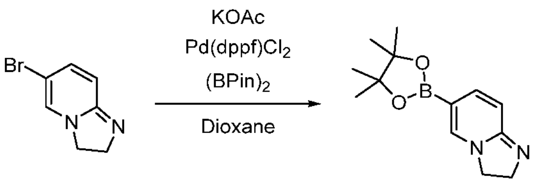


6-Bromo-2,3-dihydroimidazo[1,2-a]pyridine (50.0 mg) was added to 1 mL of 1,4-dioxane, and bis(pinacolato)diboron (57.4 mg), [1,1'-bis(diphenylphosphino)ferrocene]palladium dichloride (18.2 mg), and potassium acetate (49.3 mg) were added. The reaction was completed after the mixture was heated to 100 °C and stirred for 6 h under argon atmosphere. The reaction mixture was cooled and concentrated to give a crude product.

**Step 3: synthesis of (*R*)-4-((1-(3-(difluoromethyl)-2-fluorophenyl)ethyl)amino)-8-(2,3-dihydroimidazo[1,2-*a*]pyridin-7-yl)-6-(1-(fluoromethyl)cyclopropyl)-2-methylpyrido[4,3-*d*]pyrimidine-7(6*H*)-one**

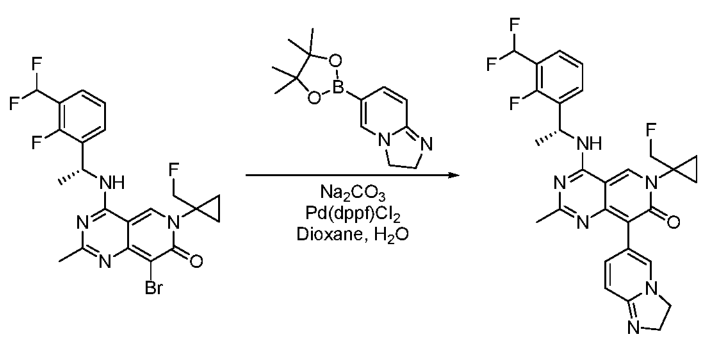


The crude product obtained in step 2 and (*R*)-8-bromo-4-((1-(3-(difluoromethyl)-2-fluorophenyl)ethyl)amino)-6-(1-(fluoromethyl)cyclopropyl)-2-methylpyrido[4,3-d]pyrimidine-7(6*H*)-one (50.0 mg) were added to 5 mL of a mixed

solvent of dioxane/water (v:v, 4:1), and [1,1'-bis(diphenylphosphino)ferrocene]palladium dichloride (7.27 mg) and sodium carbonate (21.2 mg) were added. The reaction was completed after the mixture was heated to 100 °C and stirred for 6 h under argon atmosphere. The reaction mixture was concentrated and purified by column chromatography (dichloromethane/methanol gradient elution) to give the title compound.

$^1H$ NMR (400 MHz, DMSO-$d_6$) δ 9.22 (br, 1H), 9.02 (br, 1H), 8.25-8.12 (m, 1H), 7.92-7.78 (m, 1H), 7.72-7.64 (m, 1H), 7.56-7.50 (m, 1H), 7.34 (t, *J*= 7.5 Hz, 1H), 7.24 (t, *J*= 54.3 Hz, 1H), 6.87-6.74 (m, 1H), 5.83-5.72 (m, 1H), 4.80-4.42 (m, 4H), 3.93-3.80 (m, 2H), 2.18 (s, 3H), 1.60 (d, *J*= 6.9 Hz, 3H), 1.38-1.18 (m, 4H). LC/MS(m/z, $MH^+$): 539.2.

**Example 142 (*R*)-8-(4-cyclopropylpiperazin-1-yl)-4-((1-(3-(difluoromethyl)-2-fluorophenyl) ethyl)amino)-6-(1-(fluoromethyl)cyclopropyl)-2-methylpyrido[4,3-*d*]pyrimidine-7(6*H*)-one**

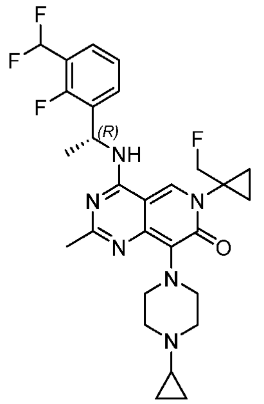

The title compound was prepared in the same manner as in Example **34** except for replacing morpholine in step 1 with 1-cyclopropylpiperazine.

$^1H$ NMR (400 MHz, DMSO-$d_6$) δ 8.78 (s, 1H), 8.62 (d, *J*= 4.6 Hz, 1H), 7.62 (t, *J*= 7.3 Hz, 1H), 7.51 (t, *J* = 6.9 Hz, 1H), 7.31 (t, *J* = 7.7 Hz, 1H), 7.23 (t, *J* = 54.4 Hz, 1H), 5.85-5.66 (m, 1H), 4.82-4.46 (m, 2H), 3.29-3.17 (m, 4H), 2.70-2.55 (m, 5H), 2.18 (s, 3H), 1.57 (d, *J* = 7.0 Hz, 3H), 1.39-1.19 (m, 4H), 0.51-0.26 (m, 4H). LC/MS(m/z, $MH^+$): 545.2.

**Example 143 (*R*)-4-((1-(3-(difluoromethyl)-2-fluorophenyl)ethyl)amino)-6-(1-(fluoromethyl) cyclopropyl)-2-methyl-8-(4-(3-methyloxetane)-3-yl)piperazin-1-yl)pyrido[4,3-*d*]pyrimidine-7(6*H*)-one**

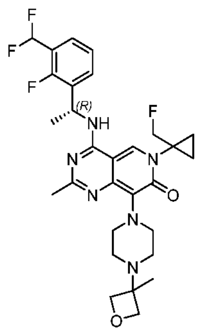

The title compound was prepared in the same manner as in Example **34** except for replacing morpholine in step 1 with 1-(3-methyloxetan-3-yl)piperazine.

$^1H$ NMR (400 MHz, DMSO-$d_6$) δ 8.79 (s, 1H), 8.63 (s, 1H), 7.62 (t, *J* = 7.2 Hz, 1H), 7.51 (t, *J* = 7.1 Hz, 1H), 7.31 (t, *J* = 7.7 Hz, 1H), 7.23 (t, *J* = 54.4 Hz, 1H), 5.80-5.67 (m, 1H), 4.80-4.49 (m, 2H), 4.43 (d, *J*= 5.5 Hz, 2H), 4.12 (d, *J*= 5.5 Hz, 2H), 3.32-3.24 (m, 4H), 2.41-2.25 (m, 4H), 2.20 (s, 3H), 1.57 (d, *J*= 7.1 Hz, 3H), 1.34-1.18 (m, 7H). LC/MS(m/z, $MH^+$): 575.2.

**Example 144 (*R*)-4-((1-(3-(difluoromethyl)-2-fluorophenyl)ethyl)amino)-6-(1-(fluoromethyl)cyclopropyl)-8-(2-hydroxyprop-2-yl)-2-methylpyrido[4,3-*d*]pyrimidine-7(6*H*)-one**

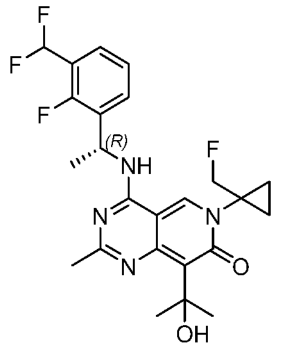

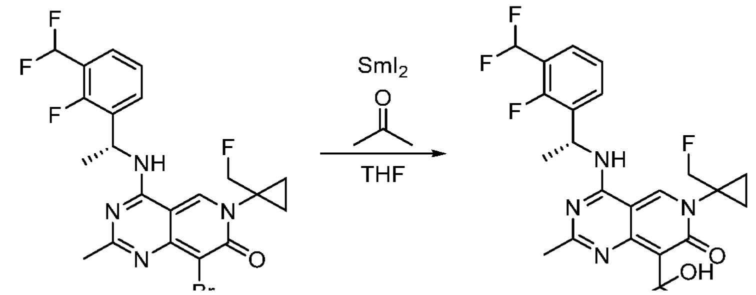


(*R*)-8-Bromo-4-((1-(3-(difluoromethyl)-2-fluorophenyl)ethyl)amino)-6-(1-(fluoromethyl)cyclopropyl)-2-methylpyrido[4,3-d]pyrimidine-7(6*H*)-one (50.0 mg) and acetone (8.72 mg) were dissolved in 2.5 mL of tetrahydrofuran. The mixture was cooled to -78 °C. 5 mL of a 0.1 M samarium diiodide solution was added dropwise, and the reaction was completed after the mixture was stirred for 4 h. The reaction mixture was poured into a saturated ammonium chloride solution and extracted with ethyl acetate. The organic phase was dried over anhydrous sodium sulfate, filtered, concentrated, and purified by column chromatography (dichloromethane/methanol gradient elution) to give the title compound.

$^1H$ NMR (400 MHz, DMSO-$d_6$) $\delta$ 9.25 (s, 1H), 9.17 (s, 1H), 9.01 (d, *J* = 7.0 Hz, 1H), 7.64 (t, *J* = 7.2 Hz, 1H), 7.53 (t, *J* = 6.9 Hz, 1H), 7.32 (t, *J* = 7.7 Hz, 1H), 7.24 (t, *J* = 54.4 Hz, 1H), 5.82 - 5.73 (m, 1H), 4.84 - 4.37 (m, 1H), 2.21 (s, 1H), 1.59 (d, *J* = 7.1 Hz, 1H), 1.53 (s, 1H), 1.51 (s, 1H), 1.28 (d, *J* = 38.8 Hz, 2H). LC/MS(m/z, $MH^+$): 493.2.

**Example 145 (*R*)-4-((1-(3-(difluoromethyl)-2-fluorophenyl)ethyl)amino)-6-(1-(fluoromethyl) cyclopropyl)-8-(4-hydroxy-1-(oxetan-3-yl)piperidin-4-yl)-2-methylpyrido[4,3-*d*]pyrimidine-7(6*H*)-one**

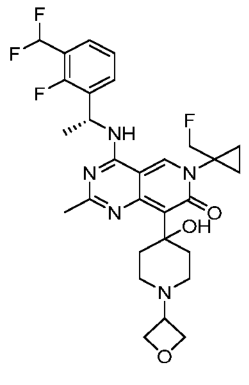

**Step 1: synthesis of *tert*-butyl (*R*)-4-(4-((1-(3-(difluoromethyl)-2-fluorophenyl)ethyl)amino)-6-(1-(fluoromethyl)cyclopropyl)-2-methyl-7-oxo-6,7-dihydropyrido[4,3-*d*]pyrimidin-8-yl)-4-hydroxypiperidine-1-carboxylate**

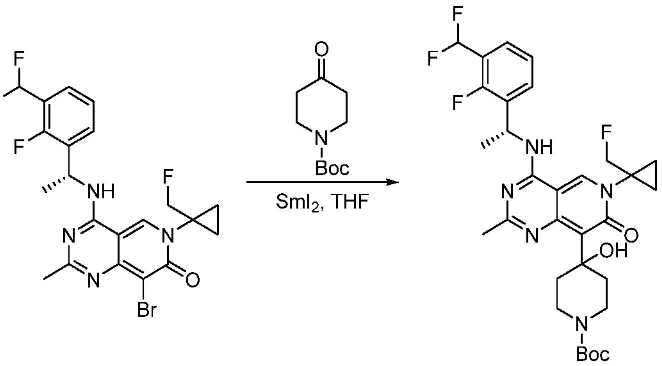


(R)-8-Bromo-4-((1-(3-(difluoromethyl)-2-fluorophenyl)ethyl)amino)-6-(1-(fluoromethyl)cyclopropyl)-2-methylpyrido[4,3-d]pyrimidine-7(6H)-one (200 mg) was dissolved in 2 mL of anhydrous tetrahydrofuran. *N-tert*-Butoxycarbonyl-4-piperidone (159 mg) was added. The mixture was cooled to -78 °C under argon atmosphere. A solution of samarium diiodide in tetrahydrofuran (0.1 M, 20 mL) was added dropwise, after which the reaction was completed after the mixture was stirred for another 3 h. The reaction was quenched with 2 mL of a saturated ammonium chloride solution. The mixture was diluted with 20 mL of ethyl acetate. The organic phase was dried over anhydrous sodium sulfate, filtered, concentrated, and purified by column chromatography (dichloromethane/methanol gradient elution) to give the title compound.

**Step 2: synthesis of (*R*)-4-((1-(3-(difluoromethyl)-2-fluorophenyl)ethyl)amino)-6-(1-(fluoromethyl)cyclopropyl)-8-(4-hydroxypiperidin-4-yl)-2-methylpyrido[4,3-*d*]pyrimidine-7(6*H*)-one**

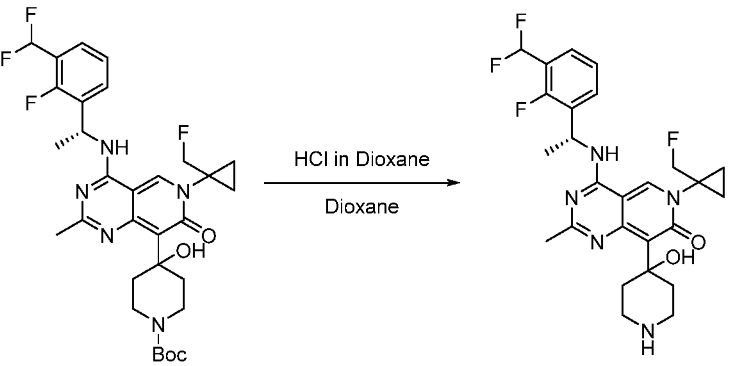


*tert*-Butyl (*R*)-4-(4-((1-(3-(difluoromethyl)-2-fluorophenyl)ethyl)amino)-6-(1-(fluoromethyl) cyclopropyl)-2-methyl-7-oxo-6,7-dihydropyrido[4,3-d]pyrimidin-8-yl)-4-hydroxypiperidine-1-carboxylate (60.0 mg) was dissolved in 1 mL of 1,4-dioxane. Then 0.5 mL of a 4 M solution of hydrochloric acid in dioxane was added thereto. After the mixture was reacted at room temperature for 30 min, the pH was adjusted to 8-9 with ammonia in methanol. The mixture was concentrated and purified by column chromatography (dichloromethane/methanol gradient elution) to give the title compound.

**Step 3: synthesis of (*R*)-4-((1-(3-(difluoromethyl)-2-fluorophenyl)ethyl)amino)-6-(1-(fluoromethyl)cyclopropyl)-8-(4-hydroxy-1-(oxetan-3-yl)piperidin-4-yl)-2-methylpyrido[4,3-*d*]pyrimidine-7(6*H*)-one**

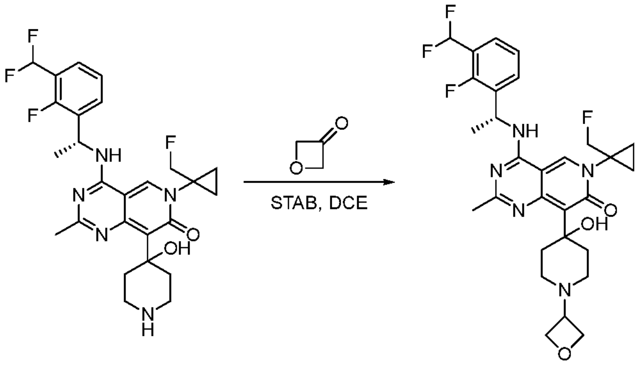


(*R*)-4-((1-(3-(Difluoromethyl)-2-fluorophenyl)ethyl)amino)-6-(1-(fluoromethyl)cyclopropyl)-8-(4-hydroxypiperidin-4-yl)-2-methylpyrido[4,3-d]pyrimidine-7(6H)-one (40.0 mg) was dissolved in 1 mL of 1,2-dichloroethane, and 3-oxetanone (11.1 mg) and sodium triacetoxyborohydride (19.8 mg) were added. The reaction was completed after the mixture was stirred at room temperature for 8 h. The reaction mixture was concentrated and purified by column chromatography (dichloromethane/methanol gradient elution) to give the title compound.

$^1$H NMR (400 MHz, DMSO-$d_6$) δ 9.55 (s, 1H), 9.20 (s, 1H), 9.04 (d, *J* = 7.0 Hz, 1H), 7.65 (t, *J* = 7.1 Hz, 1H), 7.53 (t, *J*= 7.0 Hz, 1H), 7.33 (t, *J*= 7.7 Hz, 1H), 7.24 (t, *J*= 54.3 Hz, 1H), 5.85-5.67 (m, 1H), 4.84-4.49 (m, 4H), 4.48-4.31 (m, 2H), 3.43-3.36 (m, 1H), 2.97-2.81 (m, 2H), 2.44-2.35 (m, 1H), 2.29-2.17 (m, 4H), 1.59 (d, *J*= 7.1 Hz, 3H), 1.41-1.19 (m, 6H). LC/MS(m/z, MH$^+$): 576.2.

**Example 146 (R)-4-((1-(3-(difluoromethyl)-2-fluorophenyl)ethyl)amino)-6-(1-(fluoromethyl) cyclopropyl)-2-methyl-8-(7-(oxetan-3-yl)-4,7-diazaspiro [2.5] octan-4-yl)pyrido [4,3-*d*]pyrimidine-7(6*H*)-one**

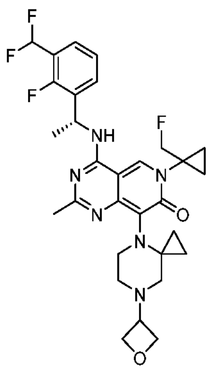

The title compound was prepared in the same manner as in Example **34** except for replacing morpholine in step 1 with 7-(oxetan-3-yl)-4,7-diazaspiro[2.5]octane.

$^1$H NMR (400 MHz, DMSO-$d_6$) δ 8.74 (s, 1H), 8.59 (d, *J*= 6.3 Hz, 1H), 7.63 (t, *J*= 7.2 Hz, 1H), 7.51 (t, *J* = 6.8 Hz, 1H), 7.31 (t, *J* = 7.7 Hz, 1H), 7.23 (t, *J* = 54.4 Hz, 1H), 5.79-5.68 (m, 1H), 4.87-4.35 (m, 6H), 3.55-3.38 (m, 2H), 2.47-2.36 (m, 2H), 2.31-2.20 (m, 2H), 2.17 (s, 3H), 1.56 (d, *J*= 7.1 Hz, 3H), 1.34-1.18 (m, 4H), 0.50-0.41 (m, 2H), 0.39-0.29 (m, 2H). LC/MS(m/z, MH$^+$): 587.2.

**Example 147 (*R*)-4-((1-(3-(difluoromethyl)-2-fluorophenyl)ethyl)amino)-6-(1-(fluoromethyl) cyclopropyl)-2-methyl-8-(7-oxa-2-azaspiro[3.5]nonan-2-yl)pyrido[4,3-*d*]pyrimidine-7(6*H*)-one**

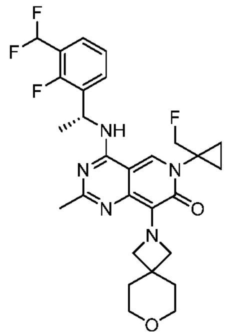

The title compound was prepared in the same manner as in Example **34** except for replacing morpholine in step 1 with 7-oxa-2-azaspiro[3.5]nonane.

$^1$H NMR (400 MHz, DMSO-$d_6$) δ 8.39 (d, *J*= 7.1 Hz, 1H), 8.33 (s, 1H), 7.60 (t, *J*= 7.5 Hz, 1H), 7.50 (t, *J* = 6.8 Hz, 1H), 7.30 (t, *J* = 7.7 Hz, 1H), 7.23 (t, *J* = 54.4 Hz, 1H), 5.77-5.68 (m, 1H), 4.77-4.42 (m, 2H), 4.12 (s, 4H), 3.55-3.44 (m, 4H), 2.12 (s, 3H), 1.70-1.63 (m, 4H), 1.55 (d, *J* = 7.1 Hz, 3H), 1.31-1.20 (m, 4H). LC/MS(m/z, $MH^+$): 546.2.

**Example 148 (*R*)-4-((1-(3-(difluoromethyl)-2-fluorophenyl)ethyl)amino)-6-(1-(fluoromethyl) cyclopropyl)-2-methyl-8-(((3-methyloxetan-3-yl)methyl)amino)pyrido[4,3-*d*]pyrimidine-7(6*H*)-one**

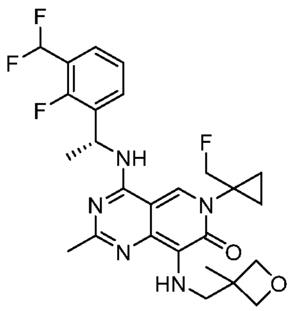

The title compound was prepared in the same manner as in Example 77 except for replacing N-methylbenzylamine in step 1 with (3-methyloxetan-3-yl)methylamine.

$^1$H NMR (400 MHz, DMSO-$d_6$) δ 8.57 (d, *J* = 7.2 Hz, 1H), 8.44 (s, 1H), 7.63 (t, *J* = 7.3 Hz, 1H), 7.51 (t, *J* = 6.8 Hz, 1H), 7.32 (t, *J* = 7.7 Hz, 1H), 7.24 (t, *J* = 54.4 Hz, 1H), 5.80-5.71 (m, 1H), 5.49-5.42 (m, 1H), 4.81-4.52 (m, 2H), 4.47 (d, *J*= 5.6 Hz, 2H), 4.16-4.09 (m, 2H), 3.84 (d, *J*= 7.0 Hz, 2H), 2.18 (s, 3H), 1.57 (d, *J* = 7.1 Hz, 3H), 1.35-1.21 (m, 7H). LC/MS(m/z, $MH^+$): 520.2.

**Example 149 4-(((*R*)-1-(3-(difluoromethyl)-2-fluorophenyl)ethyl)amino)-6-(1-(fluoromethyl) cyclopropyl)-2-methyl-8-(4-methyl-3-(trifluoromethyl)piperazin-1-yl)pyrido[4,3-*d*]pyrimidine-7(6*H*)-one**

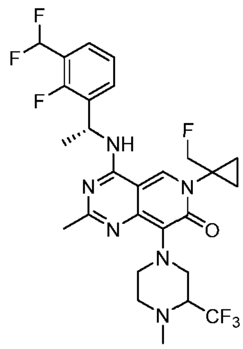

The title compound was prepared in the same manner as in Example **34** except for replacing morpholine in step 1 with 1-methyl-2-(trifluoromethyl)piperazine.

$^1$H NMR (400 MHz, DMSO-$d_6$) δ 8.84 (s, 1H), 8.69 (d, *J*= 4.5 Hz, 1H), 7.62 (t, *J*= 7.3 Hz, 1H), 7.51 (t, *J*= 7.0 Hz, 1H), 7.31 (t, *J*= 7.7 Hz, 1H), 7.23 (t, *J*= 54.4 Hz, 1H), 5.81-5.69 (m, 1H), 4.85-4.40 (m, 2H), 3.58-3.46 (m, 1H), 3.40-3.35 (m, 1H), 3.30-3.19 (m, 2H), 3.13-3.02 (m, 1H), 2.85-2.77 (m, 1H), 2.44-2.31 (m, 4H), 2.19 (s, 3H), 1.57 (d, *J*= 7.1 Hz, 3H), 1.35-1.21 (m, 4H).

LC/MS(m/z, MH$^+$): 587.2.

**Example 150 (*R*)-4-((1-(3-(difluoromethyl)-2-fluorophenyl)ethyl)amino)-6-(1-(fluoromethyl) cyclopropyl)-8-(3-methoxyoxetan-3-yl)-2-methylpyrido[4,3-*d*]pyrimidine-7(6*H*)-one**

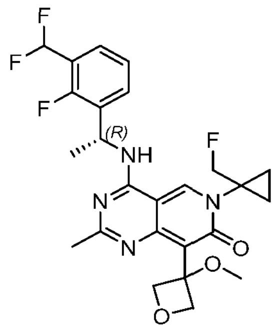

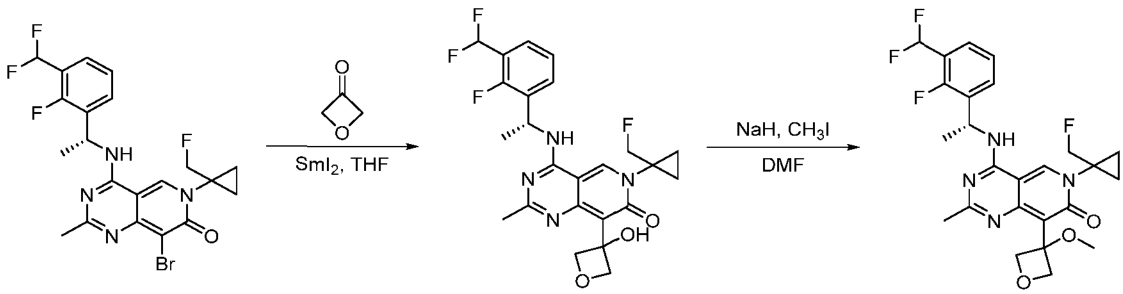


Reference was made to the synthesis methods of Example **139** and Example **140,** and the title compound was prepared according to the above-mentioned technical route.

$^1$H NMR (400 MHz, DMSO-$d_6$) δ 9.25 (s, 1H), 8.95 (d, *J* = 6.9 Hz, 1H), 7.66 (t, *J* = 7.1 Hz, 1H), 7.53 (t, *J*= 6.8 Hz, 1H), 7.33 (t, *J*= 7.6 Hz, 1H), 7.24 (t, *J*= 54.3 Hz, 1H), 5.81-5.71 (m, 1H), 4.91-4.39 (m, 6H), 3.05 (s, 3H), 2.17 (s, 3H), 1.59 (d, *J*= 6.9 Hz, 3H), 1.40-1.20 (m, 4H).

LC/MS(m/z, MH$^+$): 507.2.

**Example 151 (*R*)-4-((1-(3-(difluoromethyl)-2-fluorophenyl)ethyl)amino)-6-(1-(fluoromethyl) cyclopropyl)-2-methyl-8-(2-oxa-7-azaspiro[3.5]nonan-7-yl)pyrido[4,3-*d*]pyrimidine-7(6*H*)-one**

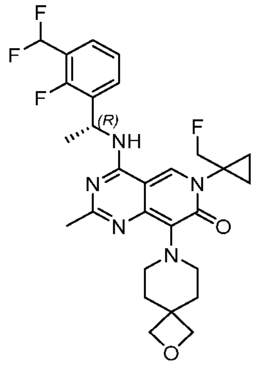

The title compound was prepared in the same manner as in Example **34** except for replacing morpholine in step 1 with 2-oxa-7-azaspiro[3.5]nonane.

$^1$H NMR (400 MHz, DMSO-$d_6$) δ 8.79 (s, 1H), 8.63 (br, 1H), 7.62 (t, *J*= 7.5 Hz, 1H), 7.51 (t, *J*= 6.9 Hz, 1H), 7.31 (t, *J* = 7.7 Hz, 1H), 7.23 (t, *J* = 54.3 Hz, 1H), 5.82-5.66 (m, 1H), 4.81-4.43 (m, 2H), 4.33 (s, 4H), 3.21-3.03 (m, 4H), 2.19 (s, 3H), 1.89-1.77 (m, 4H), 1.57 (d, *J* = 7.1 Hz, 3H), 1.33-1.19 (m, 4H). LC/MS(m/z, MH$^+$): 546.2.

**Example 152 4-(((*R*)-1-(3-(difluoromethyl)-2-fluorophenyl)ethyl)amino)-6-(1-(fluoromethyl) cyclopropyl)-2-methyl-8-(6-(oxetan-3-yl)-3,6-diazabicyclo[3.1.1]heptan-3-yl)pyrido[4,3-*d*]pyrimidine-7(6*H*)-one**

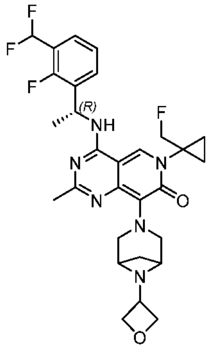

**Synthesis of 6-(oxetan-3-yl)-3,6-diazabicyclo[3.1.1]heptane:**

**Step 1: synthesis of tert-butyl 6-(oxetan-3-yl)-3,6-diazabicyclo[3.1.1]heptane-3-carboxylate**

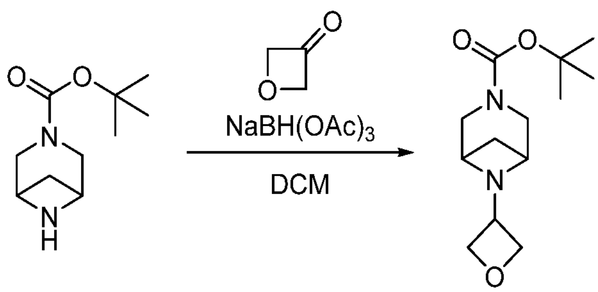


*tert*-Butyl 3,6-diazabicyclo[3.1.1]heptane-3-carboxylate (140 mg) and 3-oxetanone (101 mg) were dissolved in 5 mL of dichloromethane. Sodium triacetoxyborohydride (299 mg) was added. The reaction was completed after the mixture was stirred at room temperature for 6 h. The reaction mixture was poured into water and extracted with dichloromethane. The organic phase was dried over anhydrous sodium sulfate, filtered, concentrated, and purified by column

chromatography (petroleum ether/ethyl acetate gradient elution) to give the title compound.

**Step 2: synthesis of 6-(oxetan-3-yl)-3,6-diazabicyclo[3.1.1]heptane**

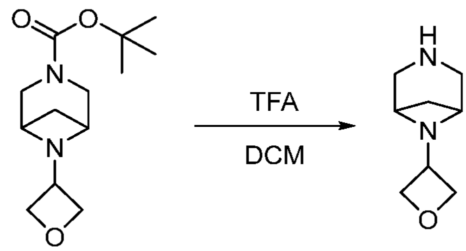


*tert*-Butyl 6-(oxetan-3-yl)-3,6-diazabicyclo[3.1.1]heptane-3-carboxylate (74.0 mg) was dissolved in 1 mL of dichloromethane. 220 μL of trifluoroacetic acid was added. The reaction was completed after the mixture was stirred at room temperature for 2 h. The reaction mixture was concentrated. 10 mL of methanol was added. The pH was adjusted to 8-9 with a saturated sodium bicarbonate solution. The mixture was subjected to suction filtration, and the filtrate was concentrated to give the title compound. The title compound was prepared in the same manner as in Example **34** except for replacing morpholine in step 1 with 6-(oxetan-3-yl)-3,6-diazabicyclo[3.1.1]heptane.

$^1H$ NMR (400 MHz, DMSO) δ 8.97 (s, 1H), 8.76 (d, *J*= 6.9 Hz, 1H), 7.64 (t, *J*= 7.2 Hz, 1H), 7.52 (t, *J*= 7.1 Hz, 1H), 7.32 (t, *J*= 7.7 Hz, 1H), 7.24 (t, *J*= 54.4 Hz, 1H), 5.82-5.71 (m, 1H), 4.81-4.53 (m, 5H), 4.40-4.18 (m, 2H), 3.65-3.47 (m, 4H), 3.28-3.13 (m, 2H), 2.41-2.34 (m, 1H), 2.26 (s, 3H), 1.95-1.85 (m, 1H), 1.59 (d, *J*= 7.1 Hz, 3H), 1.37-1.20 (m, 4H). LC/MS(m/z, $MH^+$): 573.2.

**Example 153 (*R*)-4-((1-(3-(difluoromethyl)-2-fluorophenyl)ethyl)amino)-6-(1-(fluoromethyl) cyclopropyl)-2-methyl-8-((1-(oxetan-3-yl)piperidin-4-yl)amino)pyrido[4,3-*d*]pyrimidine-7(6*H*)-one**

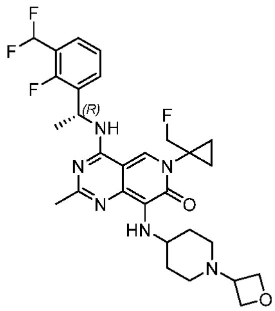

The title compound was prepared in the same manner as in Example **77** except for replacing N-methylbenzylamine in step 1 with **153-2.**

Synthesis of **153-2:**

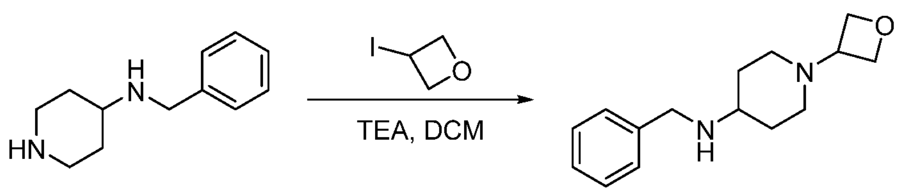


**153-1** **153-2**

**153-1** (380 mg), 3-iodooxetane (400 mg), and triethylamine (500 mg) were added to 10 mL of dichloromethane, and the reaction was completed after the mixture was stirred at room temperature for 1 h. The reaction mixture was concentrated and purified by column chromatography (dichloromethane/methanol gradient elution) to give **153-2.**

$^1H$ NMR (400 MHz, DMSO-$d_6$) δ 8.56 (d, *J*= 7.2 Hz, 1H), 8.43 (s, 1H), 7.63 (t, *J*= 7.2 Hz, 1H), 7.51 (t, *J* = 6.9 Hz, 1H), 7.31 (t, *J* = 7.7 Hz, 1H), 7.24 (t, *J* = 54.4 Hz, 1H), 5.82-5.70 (m, 1H), 5.02 (d, *J* = 9.1 Hz, 1H), 4.79-4.28 (m, 6H), 2.61-2.53 (m, 1H), 2.19 (s, 3H), 1.96-1.73 (m, 4H), 1.57 (d, *J*= 7.1 Hz, 3H), 1.38-1.17 (m, 7H). LC/MS(m/z, $MH^+$): 575.2.

The following compounds were synthesized with reference to the synthetic route and the steps of Example **34** and by replacing morpholine with the starting material A in the following table in the first step.

| Example | Starting material A | Structure | LC/MS (m/z, $MH^+$) | $^1H$ NMR |
|---|---|---|---|---|
| **154** | 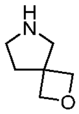 | 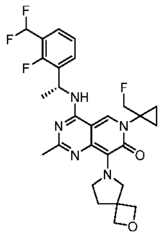 | 532.2 | $^1H$ NMR (400 MHz, DMSO-$d_6$) δ 8.67 (s, 1H), 8.55 (d, *J* = 7.1 Hz, 1H), 7.62 (t, *J* = 7.3 Hz, 1H), 7.51 (t, *J* = 6.9 Hz, 1H), 7.31 (t, *J* = 7.7 Hz, 1H), 7.23 (t, *J* = 54.4 Hz, 1H), 5.80-5.68 (m, 1H), 4.83-4.36 (m, 6H), 3.97-3.77 (m, 2H), 3.72-3.55 (m, 2H), 2.17 (s, 3H), 2.10-2.04 (m, 2H), 1.56 (d, *J* = 7.1 Hz, 3H), 1.35-1.18 (m, 4H). |
| **155** | 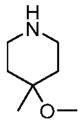 | 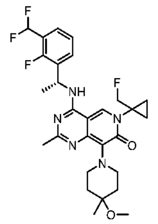 | 548.2 | $^1H$ NMR (400 MHz, DMSO-$d_6$) δ 8.74 (s, 1H), 8.58 (d, *J* = 7.0 Hz, 1H), 7.62 (t, *J* = 7.2 Hz, 1H), 7.51 (t, *J* = 6.7 Hz, 1H), 7.31 (t, *J* = 7.7 Hz, 1H), 7.24 (t, *J* = 54.4 Hz, 1H), 5.82-5.66 (m, 1H), 4.82-4.42 (m, 2H), 3.12 (s, 3H), 2.18 (s, 3H), 1.76-1.63 (m, 2H), 1.60-1.47 (m, 5H), 1.34-1.18 (m, 4H), 1.12 (s, 3H). |

**Example 156 (*R*)-*N*-(4-((1-(3-(difluoromethyl)-2-fluorophenyl)ethyl)amino)-6-(1-(fluoromethyl) cyclopropyl)-2-methyl-7-oxo-6,7-dihydropyrido[4,3-d]pyrimidine-8-yl)oxetane-3-carboxamide**

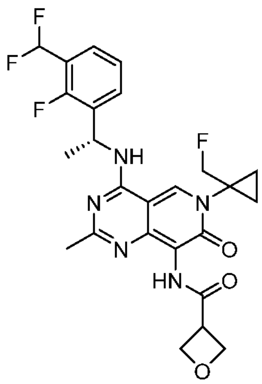

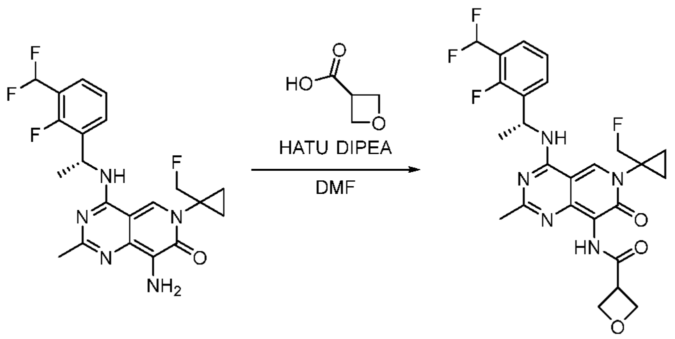


Oxetane-3-carboxylic acid (15.0 mg) was dissolved in *N,N*-dimethylformamide (0.5 mL). 2-(7-Azabenzotriazole)-*N,N,N',N'*-tetramethyluronium hexafluorophosphate (50.0 mg) was added, and then (*R*)-8-amino-4-((1-(3-(difluoromethyl)-2-fluorophenyl)ethyl)amino)-6-(1-(fluoromethyl)cyclopropyl)-2-methylpyrido[4,3-*d*]pyrimidine-7(6*H*)-one (50.0 mg) and *N,N*-diisopropylethylamine (133 mg) were added. The reaction was completed after the mixture was stirred at room temperature for 12 h. The reaction mixture was diluted with 20 mL of ethyl acetate and washed with a saturated aqueous sodium chloride solution. The organic layer was concentrated and purified by column chromatography (dichloromethane/methanol gradient elution) to give the title compound.

$^1$H NMR (400 MHz, DMSO-$d_6$) δ 9.16 (s, 1H), 8.93 (d, *J*= 6.9 Hz, 1H), 7.66 (t, *J*= 7.2 Hz, 1H), 7.53 (t, *J* = 6.9 Hz, 1H), 7.33 (t, *J* = 7.7 Hz, 1H), 7.24 (t, *J* = 54.4 Hz, 1H), 5.84-5.71 (m, 1H), 4.84-4.39 (m, 6H), 4.00-3.80 (m, 1H), 2.18 (s, 3H), 1.59 (d, *J*= 7.1 Hz, 3H), 1.40-1.23 (m, 4H).

LC/MS(m/z, MH$^+$): 520.2.

The following compound was synthesized with reference to the synthetic route and the steps of Example 156 and by replacing oxetane-3-carboxylic acid with the starting material A in the following table in the first step.

| Example | Starting material A | Structure | LC/MS (m/z, MH$^+$) | $^1$H NMR |
|---|---|---|---|---|
| **157** | 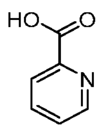 | 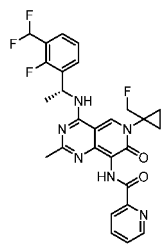 | 541.2 | $^1$H NMR (400 MHz, DMSO-$d_6$) δ 9.59 (s, 1H), 9.20 (s, 1H), 8.99 (s, 1H), 8.72 (d, *J*= 4.6 Hz, 1H), 8.12-8.01 (m, 2H), 7.71-7.63 (m, 2H), 7.53 (t, *J* = 7.0 Hz, 1H), 7.33 (t, *J* = 7.7 Hz, 1H), 7.25 (t, *J* = 54.4 Hz, 1H), 5.86-5.70 (m, 1H), 4.85-4.50 (m, 2H), 2.17 (s, 3H), 1.61 (d, *J*= 7.0 Hz, 3H), 1.41-1.22 (m, 4H). |

The following compounds may be synthesized with reference to the synthetic route and the steps of Example **34** and by replacing morpholine with the starting material A in the following table in the first step.

| Example | Starting material A | Structure | LC/MS (m/z, MH$^+$) | $^1$H NMR |
|---|---|---|---|---|
| **158** | 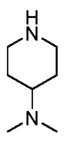 | 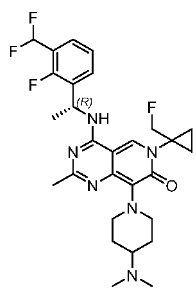 | 547.2 | $^1$H NMR (400 MHz, DMSO-$d_6$) δ 8.77 (s, 1H), 8.61 (d, *J* = 7.1 Hz, 1H), 7.62 (t, *J* = 7.3 Hz, 1H), 7.51 (t, *J* = 6.8 Hz, 1H), 7.31 (t, *J* = 7.7 Hz, 1H), 7.24 (t, *J* = 54.4 Hz, 1H), 5.81-5.68 (m, 1H), 4.83-4.46 (m, 2H), 3.24-3.12 (m, 2H), 2.24 (s, 6H), 2.18 (s, 3H), 1.76-1.66 (m, |

(continued)

| Example | Starting material A | Structure | LC/MS (m/z, MH+) | $^1$H NMR |
|---|---|---|---|---|
| | | | | 2H), 1.57 (d, *J* = 7.1 Hz, 3H), 1.54-1.43 (m, 2H), 1.33-1.21 (m, 4H). |
| **159** | 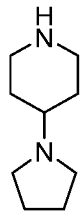 | 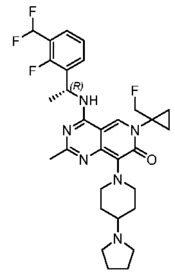 | 573.2 | $^1$H NMR (400 MHz, DMSO-$d_6$) δ 8.77 (s, 1H), 8.62 (d, *J* = 6.9 Hz, 1H), 7.62 (t, *J* = 7.1 Hz, 1H), 7.51 (t, *J* = 6.9 Hz, 1H), 7.31 (t, *J* = 7.7 Hz, 1H), 7.24 (t, *J* = 54.4 Hz, 1H), 5.79-5.70 (m, 1H), 4.83-4.40 (m, 2H), 3.26-3.12 (m, 2H), 2.18 (s, 3H), 1.90-1.80 (m, 2H), 1.78-1.64 (m, 4H), 1.61-1.44 (m, 5H), 1.34-1.22 (m, 4H). |

The following compound was synthesized with reference to the synthetic route and the steps of Example **23** and by replacing methanol-$d_3$ with the starting material A in the following table in step 4.

| Example | Starting material A | Structure | LC/MS (m/z, MH+) | $^1$H NMR |
|---|---|---|---|---|
| **160** | 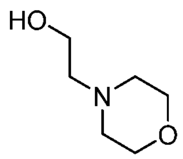 | 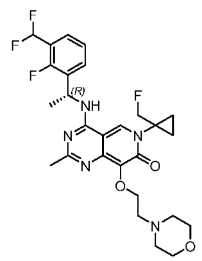 | 550.2 | $^1$H NMR (400 MHz, DMSO-$d_6$) δ 8.95 (s, 1H), 8.81 (d, *J* = 7.1 Hz, 1H), 7.64 (t, *J* = 7.4 Hz, 1H), 7.52 (t, *J* = 6.9 Hz, 1H), 7.32 (t, *J* = 7.7 Hz, 1H), 7.24 (t, *J* = 54.4 Hz, 1H), 5.82-5.68 (m, 1H), 4.83-4.46 (m, 2H), 4.21-4.04 (m, 2H), 3.58-3.44 (m, 4H), 2.62 (t, *J* = 5.6 Hz, 2H), 2.49-2.44 (m, 4H), 2.20 (s, 3H), 1.58 (d, *J* = 7.1 Hz, 3H), 1.37-1.22 (m, 4H). |

The following compounds were synthesized with reference to the synthetic route and the steps of Example **89** and by replacing 2-pyrrolyl-5-pyridineboronate with the starting material A in the following table in the second step.

| Example | Starting material A | Structure | LC/MS (m/z, MH+) | $^1$H NMR |
|---|---|---|---|---|
| **161** | 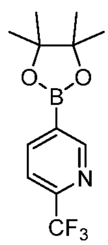 | 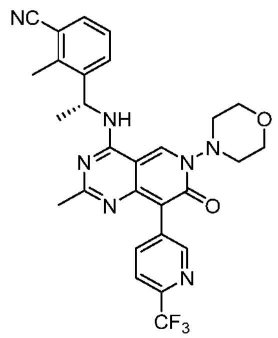 | 550.2. | $^1$H NMR (400 MHz, DMSO-$d_6$) δ 9.34 (s, 1H), 9.06 (d, *J* = 6.9 Hz, 1H), 8.81 (s, 1H), 8.15 (d, *J*= 8.5 Hz, 1H), 7.89 (d, *J* = 8.2 Hz, 1H), 7.79 (d, *J* = 7.6 Hz, 1H), 7.68 (d, *J* = 7.6 Hz, 1H), 7.42 (t, *J* = 7.8 Hz, 1H), 5.71-5.58 (m, 1H), 4.26-3.38 (m, 8H), 2.67 (s, 3H), 2.17 (s, 3H), 1.54 (d, *J* = 6.9 Hz, 3H). |

| | | | | |
|---|---|---|---|---|
| **162** | 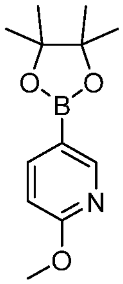 | 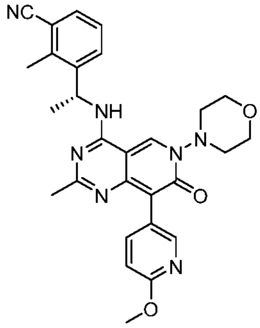 | 512.2. | $^1$H NMR (400 MHz, DMSO-$d_6$) δ 9.16 (s, 1H), 8.87 (d, *J* = 6.9 Hz, 1H), 8.13 (s, 1H), 7.75-7.64 (m, 2H), 7.60 (d, *J* = 7.7 Hz, 1H), 7.34 (t, *J* = 7.8 Hz, 1H), 6.73 (d, *J* = 8.6 Hz, 1H), 5.62-5.48 (m, 1H), 4.05-3.53 (m, 11H), 2.60 (s, 3H), 2.08 (s, 3H), 1.46 (d, *J* = 6.9 Hz, 3H). |
| **163** | 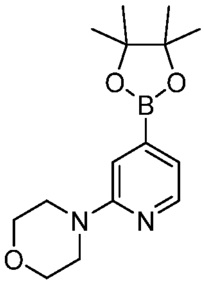 | 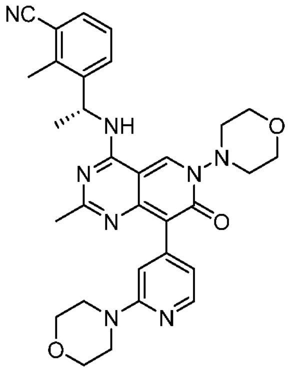 | 567.2. | $^1$H NMR (400 MHz, DMSO-$d_6$) δ 9.26 (s, 1H), 8.95 (d, *J* = 6.9 Hz, 1H), 8.08 (d, *J* = 5.1 Hz, 1H), 7.78 (d, *J* = 7.3 Hz, 1H), 7.67 (d, *J* = 6.8 Hz, 1H), 7.41 (t, *J* = 7.8 Hz, 1H), 6.80 (s, 1H), 6.71 (d, *J* = 5.2 Hz, 1H), 5.75-5.53 (m, 1H), 4.13-3.48 (m, 12H), 3.42-3.35 (m, 4H), 2.66 (s, 3H), 2.13 (s, 3H), 1.52 (d, *J* = 7.0 Hz, 3H). |
| **165** | 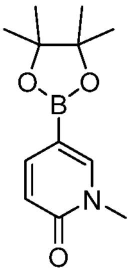 | 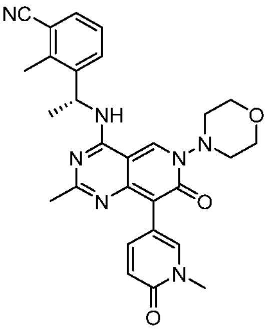 | 512.2. | $^1$H NMR (400 MHz, DMSO-$d_6$) δ 9.19 (s, 1H), 8.93 (d, *J* = 7.0 Hz, 1H), 7.82-7.75 (m, 2H), 7.70-7.63 (m, 1H), 7.58-7.52 (m, 1H), 7.40 (t, *J* = 7.8 Hz, 1H), 6.34 (d, *J* = 9.4 Hz, 1H), 5.69-5.55 (m, 1H), 4.22-3.37 (m, 11H), 2.67 (s, 3H), 2.19 (s, 3H), 1.52 (d, *J* = 7.0 Hz, 3H). |

The following compound was synthesized with reference to the synthetic route and the steps of Example **28** and by replacing potassium vinyltrifluoroborate with the starting material A in the following table.

| Example | Starting material A | Structure | LC/MS (m/z, MH+) | $^1$H NMR |
|---|---|---|---|---|
| 164 | 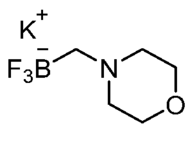 | 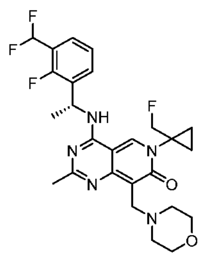 | 550.2 | $^1$H NMR (400 MHz, DMSO-$d_6$) δ 9.13 (s, 1H), 8.82 (d, *J* = 7.1 Hz, 1H), 7.66 (t, *J* = 7.1 Hz, 1H), 7.52 (t, *J* = 6.6 Hz, 1H), 7.32 (t, *J* = 7.7 Hz, 1H), 7.23 (t, *J* = 54.4 Hz, 1H), 5.81-5.71 (m, 1H), 4.82-4.44 (m, 2H), 3.81-3.66 (m, 2H), 3.53-3.40 (m, 4H), 2.43-2.32 (m, 4H), 2.20 (s, 3H), 1.58 (d, *J* = 7.1 Hz, 3H), 1.38-1.18 (m, 4H). |

The following compounds were synthesized with reference to the synthetic route and the steps of Example **144** and by replacing acetone with the starting material A.

| Example | Starting material A | Structure | LC/MS (m/z, MH+) | 1H NMR |
|---|---|---|---|---|
| **166** | 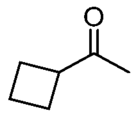 | 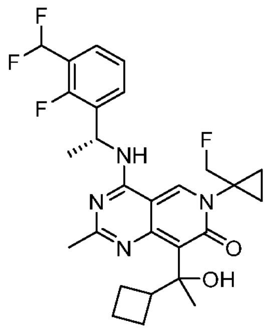 | 519.2. | $^1$H NMR (400 MHz, DMSO-$d_6$) δ 9.51 (s, 1H), 9.13 (s, 1H), 8.96 (d, *J* = 7.0 Hz, 1H), 7.65 (t, *J* = 7.3 Hz, 1H), 7.53 (t, *J* = 6.9 Hz, 1H), 7.32 (t, *J* = 7.7 Hz, 1H), 7.24 (t, *J* = 54.4 Hz, 1H), 5.84-5.72 (m, 1H), 4.82-4.41 (m, 2H), 2.21 (s, 3H), 2.18-2.09 (m, 1H), 1.94-1.82 (m, 1H), 1.72-1.61 (m, 2H), 1.61-1.46 (m, 4H), 1.38 (s, 3H), 1.35-1.22 (m, 5H). |
| **167** | 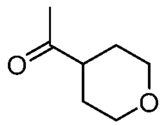 | 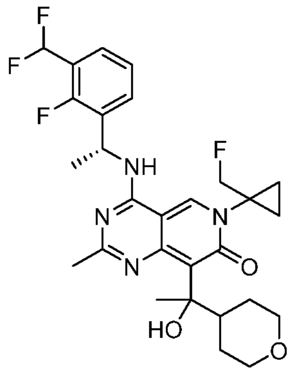 | 549.2. | $^1$H NMR (400 MHz, DMSO-$d_6$) δ 9.46 (s, 1H), 9.16 (s, 1H), 8.99 (d, *J* = 7.0 Hz, 1H), 7.66 (t, *J* = 7.2 Hz, 1H), 7.53 (t, *J* = 6.9 Hz, 1H), 7.33 (t, *J* = 7.7 Hz, 1H), 7.24 (t, *J* = 54.3 Hz, 1H), 5.83-5.73 (m, 1H), 4.77-4.40 (m, 2H), 3.93-3.82 (m, 1H), 3.79-3.71 (m, 1H), 3.26-3.16 (m, 1H), 3.15-3.06 (m, 1H), 2.20 (s, 3H), 1.59 (d, *J* = 7.1 Hz, 3H), 1.54-1.39 (m, 5H), 1.35-1.22 (m, 4H). |

The following compound was synthesized with reference to the synthetic route and the steps of Example **77** and by replacing *N*-methylbenzylamine with the starting material A in the first step.

| Example | Starting material A | Structure | LC/MS (m/z, MH+) | 1H NMR |
|---|---|---|---|---|
| **168** | 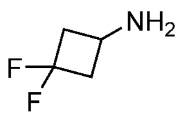 | 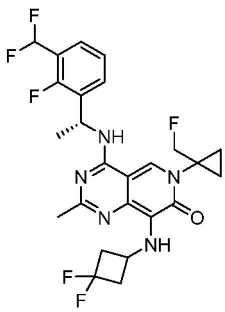 | 526.2 | $^1$H NMR (400 MHz, DMSO-$d_6$) δ 8.56 (d, *J* = 7.1 Hz, 1H), 8.46 (s, 1H), 7.63 (t, *J* = 7.2 Hz, 1H), 7.51 (t, *J* = 6.9 Hz, 1H), 7.31 (t, *J* = 7.7 Hz, 1H), 7.23 (t, *J*= 54.4 Hz, 1H), 5.81-5.70 (m, 1H), 5.45 (d, *J* = 8.0 Hz, 1H), 4.92-4.79 (m, 1H), 4.76-4.49 (m, 2H), 2.93-2.78 (m, 2H), 2.62-2.52 (m, 2H), 2.20 (s, 3H), 1.58 (d, *J* = 7.1 Hz, 3H), 1.37-1.21 (m, 4H). |

The following compound was synthesized with reference to the synthetic route and the steps of Example **34** and by replacing morpholine with the starting material A in the following table in the first step.

| Example | Starting material A | Structure | LC/MS (m/z, MH+) | $^1$H NMR |
|---|---|---|---|---|
| **169** | 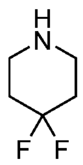 | 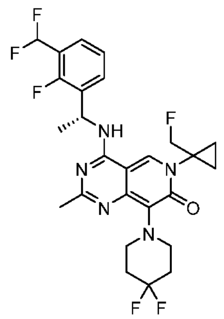 | 540.2 | $^1$H NMR (400 MHz, DMSO-$d_6$) δ 8.82 (s, 1H), 8.66 (d, *J* = 7.0 Hz, 1H), 7.62 (t, *J* = 7.4 Hz, 1H), 7.51 (t, *J* = 6.8 Hz, 1H), 7.31 (t, *J* = 7.7 Hz, 1H), 7.23 (t, *J* = 54.3 Hz, 1H), 5.83-5.68 (m, 1H), 4.80-4.59 (m, 2H), 3.36-3.32 (m, 4H), 2.20 (s, 3H), 2.08-1.93 (m, 4H), 1.57 (d, *J* = 7.1 Hz, 3H), 1.35-1.14 (m, 4H). |

The following compounds were prepared with reference to the preparation method of Example 12 and by replacing (1-methyl-1*H*-pyrazol-4-yl)boronic acid with the starting material A.

| Example | Starting material A | Structure | LC/MS (m/z, MH+) | $^1$H NMR |
|---|---|---|---|---|
| **170** | 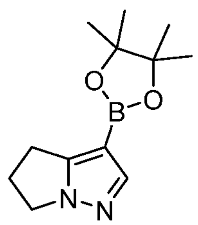 | 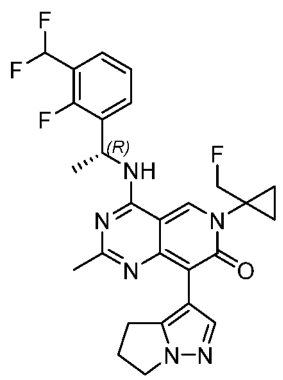 | 527.2 | $^1$H NMR (400 MHz, DMSO-$d_6$) δ 9.07 (s, 1H), 8.80 (d, *J* = 7.0 Hz, 1H), 7.88 (s, 1H), 7.66 (t, *J* = 7.4 Hz, 1H), 7.52 (t, *J* = 6.9 Hz, 1H), 7.33 (t, *J* = 7.7 Hz, 1H), 7.24 (t, *J* = 54.4 Hz, 1H), 5.84-5.72 (m, 1H), 4.89-4.47 (m, 2H), 4.06 (t, *J* = 7.2 Hz, 2H), 3.00-2.80 (m, 2H), 2.49-2.43 (m, 2H), 2.21 (s, 3H), 1.59 (d, *J* = 7.1 Hz, 3H), 1.43-1.14 (m, 4H). |
| **176** | 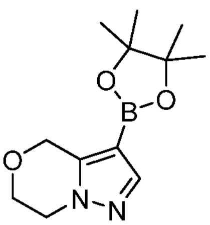 | 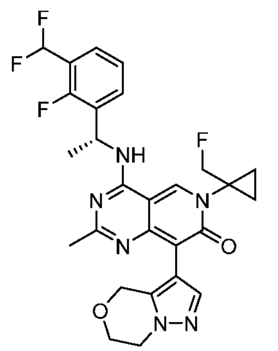 | 543.2 | $^1$H NMR (400 MHz, DMSO-$d_6$) δ 9.13 (s, 1H), 8.86 (d, *J* = 7.0 Hz, 1H), 7.74 (s, 1H), 7.67 (t, *J* = 7.3 Hz, 1H), 7.53 (t, *J* = 6.8 Hz, 1H), 7.33 (t, *J* = 7.7 Hz, 1H), 7.24 (t, *J* = 54.3 Hz, 1H), 5.83-5.71 (m, 1H), 4.9-4.43 (m, 4H), 4.22-3.97 (m, 4H), 2.21 (s, 3H), 1.59 (d, *J* = 7.1 Hz, 3H), 1.45-1.22 (m, 4H). |

**Example 171 (*R*)-3-(1-((8-(3,3-dimethylmorpholinyl)-6-(1-(fluoromethyl)cyclopropyl)-2-methyl-7-oxo-6,7-dihydropyrido[4,3-d]pyrimidin-4-yl)amino)ethyl)-2-methylbenzonitrile**

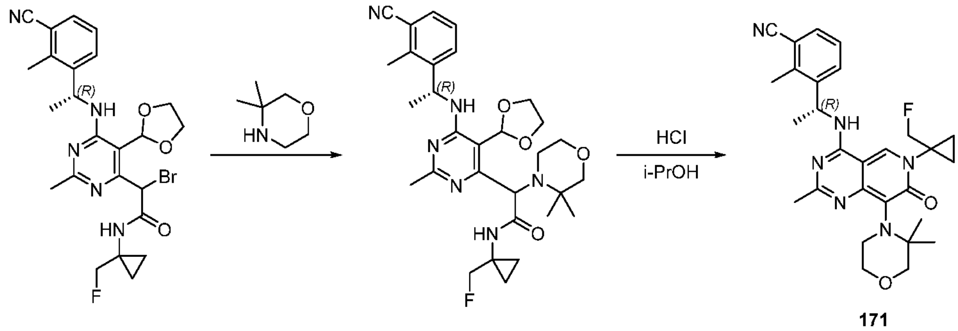


171

Reference was made to the synthesis method of Example 47, and the title compound was prepared according to the above-mentioned technical route.

$^1$H NMR (400 MHz, DMSO-$d_6$) δ 8.93 (d, *J* = 2.7 Hz, 1H), 8.74 (t, *J* = 7.5 Hz, 1H), 7.74 (t, *J* = 8.4 Hz, 1H), 7.65 (d, *J*= 7.5 Hz, 1H), 7.46-7.34 (m, 1H), 5.67-5.46 (m, 1H), 4.84-4.40 (m, 2H), 3.79-3.70 (m, 1H), 3.69-3.58 (m, 1H), 3.39-3.24 (m, 3H), 3.14-2.98 (m, 1H), 2.70-2.65 (m, 3H), 2.22 (s, 3H), 1.52 (d, *J* = 5.9 Hz, 3H), 1.38-1.14 (m, 4H), 1.10-0.97 (m, 6H). LC/MS(m/z, MH$^+$): 505.2.

**Example 172 (*R*)-4-(4-((1-(3-(difluoromethyl)-2-fluorophenyl)ethyl)amino)-6-(1-(fluoromethyl) cyclopropyl)-2-methyl-7-oxo-6,7-dihydropyrido[4,3-*d*]pyrimidin-8-yl)morpholine-3-one**

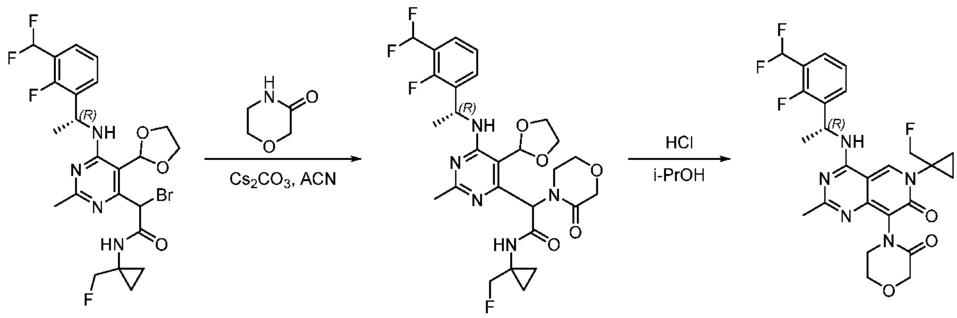


172-1 172-2 172

**Step 1: synthesis of 172-2**

**172-1** (100 mg) was added to 2 mL of acetonitrile. Cesium carbonate (116 mg) and 3-morpholinone (24.0 mg) were added, and the reaction was completed after the mixture was stirred at room temperature for 6 h. The reaction mixture was concentrated and purified by column chromatography (dichloromethane/methanol gradient elution) to give **172-2.**

**Step 2: synthesis of 172**

**172-2** (80.0 mg) was added to a reaction flask. Isopropanol (1.4 mL) was added, and then diluted hydrochloric acid (2 M, 0.2 mL) was added. The reaction was completed after the mixture was heated to 50 °C and stirred for 4 h. The reaction mixture was concentrated and purified by preparative HPLC (mobile phase: acetonitrile/water gradient elution) to give **172.**

$^1$H NMR (400 MHz, DMSO-$d_6$) δ 9.25 (s, 1H), 8.99 (d, *J* = 7.0 Hz, 1H), 7.65 (t, *J* = 7.1 Hz, 1H), 7.52 (t, *J*= 7.0 Hz, 1H), 7.31 (t, *J* = 7.8 Hz, 1H), 7.24 (t, *J* = 54.4 Hz, 1H), 5.81-5.68 (m, 1H), 4.78-4.52 (m, 2H), 4.22-4.06 (m, 2H), 4.01-3.84 (m, 2H), 3.59-3.39 (m, 2H), 2.19 (s, 3H), 1.60 (d, *J* = 7.0 Hz, 3H), 1.39-1.20 (m, 4H). LC/MS(m/z, MH$^+$): 520.2.

The following compounds were synthesized with reference to the synthetic route and the steps of Example **34** and by replacing morpholine with the starting material A in the following table in the first step.

| Example | Starting material A | Structure | LC/MS (m/z, MH+) | $^1$H NMR |
|---|---|---|---|---|
| 173 | 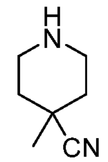 | 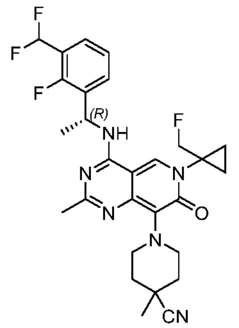 | 543.2. | $^1$H NMR (400 MHz, DMSO-$d_6$) δ8.81 (s, 1H), 8.65 (d, *J* = 7.1 Hz, 1H), 7.62 (t, *J* = 7.1 Hz, 1H), 7.51 (t, *J* = 6.8 Hz, 1H), 7.31 (t, *J* = 7.7 Hz, 1H), 7.24 (t, *J* = 54.4 Hz, 1H), 5.80-5.69 (m, 1H), 4.79-4.46 (m, 2H), 3.45-3.18 (m, 4H), 2.20 (s, 3H), 1.89-1.78 (m, 2H), 1.67-1.60 (m, 2H), 1.57 (d, *J* = 7.1 Hz, 3H), 1.42-1.19 (m, 7H). |
| 174 | 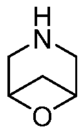 | 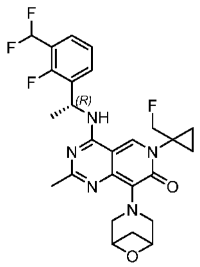 | 518.2 | $^1$H NMR (400 MHz, DMSO-$d_6$) δ 8.89 (s, 1H), 8.68 (d, *J* = 5.4 Hz, 1H), 7.64 (t, *J* = 7.3 Hz, 1H), 7.52 (t, *J* = 6.9 Hz, 1H), 7.32 (t, *J* = 7.7 Hz, 1H), 7.24 (t, *J* = 54.4 Hz, 1H), 5.84-5.69 (m, 1H), 4.81-4.51 (m, 2H), 4.51-4.42 (m, 2H), 4.04-3.82 (m, 2H), 3.55-3.40 (m, 2H), 3.04-2.89 (m, 1H), 2.44-2.38 (m, 1H), 2.21 (s, 3H), 1.58 (d, *J* = 7.1 Hz, 3H), 1.41-1.18 (m, 4H). |
| 175 | 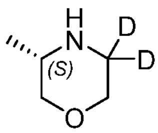 | 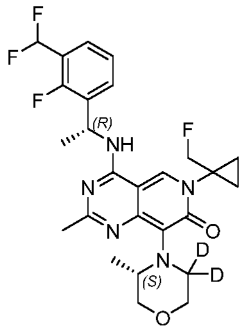 | 522.2 | $^1$H NMR (400 MHz, DMSO-$d_6$) δ 8.95 (s, 1H), 8.71 (d, *J* = 7.1 Hz, 1H), 7.65 (t, *J* = 7.3 Hz, 1H), 7.52 (t, *J* = 7.0 Hz, 1H), 7.32 (t, *J* = 7.7 Hz, 1H), 7.24 (t, *J* = 54.4 Hz, 1H), 5.80-5.70 (m, 1H), 4.86-4.36 (m, 2H), 3.99-3.90 (m, 1H), 3.71-3.54 (m, 3H), 3.23-3.11 (m, 1H), 2.20 (s, 3H), 1.57 (d, *J* = 7.1 Hz, 3H), 1.36-1.19 (m, 4H), 0.69 (d, *J* = 6.4 Hz, 3H). |
| 177 | 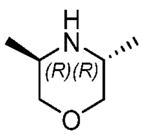 | 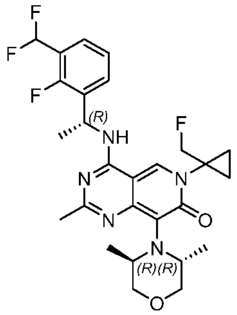 | 534.2 | $^1$H NMR (400 MHz, DMSO-$d_6$) δ 8.94 (s, 1H), 8.71 (d, *J* = 7.1 Hz, 1H), 7.66 (t, *J* = 7.3 Hz, 1H), 7.52 (t, *J* = 7.0 Hz, 1H), 7.32 (t, *J* = 7.6 Hz, 1H), 7.24 (t, *J* = 54.3 Hz, 1H), 5.82-5.71 (m, 1H), 4.83-4.47 (m, 2H), 3.91-3.79 (m, 2H), 3.76-3.65 (m, 2H), 3.30-3.22 (m, 2H), 2.21 (s, 3H), 1.57 (d, *J* = 7.1 Hz, 3H), 1.37-1.20 (m, 4H), 0.80 (d, *J* = 6.3 Hz, 3H), 0.72 (d, *J* = 6.4 Hz, 3H). |
| 178 | 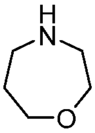 | 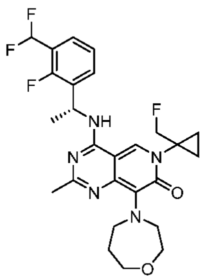 | 520.2 | $^1$H NMR (400 MHz, DMSO-$d_6$) δ 8.80 (s, 1H), 8.64 (d, *J* = 7.1 Hz, 1H), 7.63 (t, *J* = 7.3 Hz, 1H), 7.51 (t, *J* = 7.0 Hz, 1H), 7.31 (t, *J* = 7.7 Hz, 1H), 7.24 (t, *J* = 54.4 Hz, 1H), 5.84-5.67 (m, 1H), 4.83-4.47 (m, 2H), 3.86-3.76 (m, 2H), 3.72-3.62 (m, 2H), 3.44-3.36 (m, 4H), 2.19 (s, 3H), 1.97-1.83 (m, 2H), 1.57 (d, *J* = 7.1 Hz, 3H), 1.37-1.18 (m, 4H). |

(continued)

| Example | Starting material A | Structure | LC/MS (m/z, $MH^+$) | $^1H$ NMR |
|---|---|---|---|---|
| **179** | 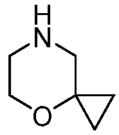 | 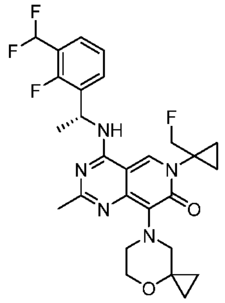 | 532.2 | $^1H$ NMR (400 MHz, DMSO-$d_6$) $\delta$ 8.79 (s, 1H), 8.64 (d, $J$ = 6.5 Hz, 1H), 7.62 (t, $J$ = 7.3 Hz, 1H), 7.51 (t, $J$ = 6.8 Hz, 1H), 7.31 (t, $J$ = 7.7 Hz, 1H), 7.23 (t, $J$ = 54.4 Hz, 1H), 5.79-5.68 (m, 1H), 4.82-4.44 (m, 2H), 3.76-3.64 (m, 2H), 3.48-3.36 (m, 2H), 3.30-3.19 (m, 2H), 2.19 (s, 3H), 1.57 (d, $J$ = 7.1 Hz, 3H), 1.36-1.19 (m, 4H), 0.72-0.47 (m, 4H) |

**Biological Activity and Related Properties Test**

**Experimental Example 1: Assay of Inhibitory Activity of Examples of the Present Invention on H358 Cell Proliferation**

The effect of the compounds of the present invention on H358 cell proliferation was assessed by CellTiter-Glo luminescent cell viability assay kit method.

Procedures:

H358 cells (ATCC, CRL-5807) were cultured in RPMI1640 (Hyclone, SH30256.01) complete medium containing 10% FBS (Gibco, 10100147) and 100 U/mL penicillin-streptomycin mixed solution (Gibco, 15140163). When the cells grew to 80-90%, the cells were digested, pipetted, and seeded in a 96-well plate (Corning, 4515) at 3000 cells/well (180 $\mu$L RPMI1640 complete medium), and then the 96-well plate was incubated overnight in an incubator at 37 °C with 5% $CO_2$.

After incubation overnight, 20 $\mu$L of a diluted test compound was added to each well using a multi-channel pipette, and the 96-well plate was placed in an incubator at 37 °C with 5% $CO_2$ for further culture. After 7 days, 100 $\mu$L of the supernatant was pipetted and discarded. 50 $\mu$L of CellTiter-Glo® 3D Cell Viability Assay liquid was added to each well. The plate was shaken at room temperature at 450 rpm for 30 min. After the cells were completely lysed, "Luminescence" on the microplate reader was selected for plate reading. In this experiment, the cell-free group (replaced with 1640 medium) was used as the 100% inhibition group, and the compound-free group in which the cell was added was used as the 0% inhibition group.

The inhibition percentage of H358 cell proliferation by the compounds of the present invention could be calculated by the following formula:

Inhibition percentage = 100% * (0% inhibition group signal value - signal value at a specific concentration of test compound) / (0% inhibition group signal value - 100% inhibition group signal value).

$IC_{50}$ values of the compounds were calculated from 8 concentration points using XLfit (ID Business Solutions Ltd., UK) software by the following formula:

$$Y = Bottom + (Top - Bottom) / (1 + 10^\wedge((logIC_{50} - X) \times slope\ factor))$$

Y is the inhibition percentage, X is the logarithmic value of the concentration of the test compound, Bottom is the minimum inhibition percentage, Top is the maximum inhibition percentage, and slope factor is the slope coefficient of the curve.

Results: under the condition of the experiment, the compounds of the examples of the present invention have good proliferation inhibitory activity on H358 cells. The corresponding activity test results for the test compounds were specified in Table 1.

Table 1. Results of H358 cell proliferation inhibitory activity test by the compounds of the examples of the present invention

| Example | $IC_{50}$ (nM) | Example | $IC_{50}$ (nM) | Example | $IC_{50}$ (nM) | Example | $IC_{50}$ (nM) |
|---|---|---|---|---|---|---|---|
| 1 | 19.6 | 52 | 26.5 | 96 | 50.5 | 146 | 69.9 |
| 3 | 61.4 | 55 | 24.3 | 97 | 11.8 | 150 | 68.7 |
| 6 | 80.0 | 56 | 17.4 | 98 | 44.0 | 151 | 73.1 |
| 7 | 74.7 | 57 | 73.1 | 100 | 80.8 | 152 | 13.6 |
| 10 | 41.6 | 58 | 57.9 | 103 | 129.8 | 154 | 116.5 |
| 11 | 50.8 | 59 | 161.5 | 104 | 144.8 | 155 | 123.5 |
| 13 | 54.1 | 60 | 161.9 | 108 | 76.4 | 157 | 84.8 |
| 16 | 187 | 61 | 22.8 | 109 | 23.7 | 158 | 12.5 |
| 19 | 69.6 | 62 | 71.6 | 110 | 50.4 | 159 | 51.5 |
| 20 | 98.2 | 64 | 40.7 | 112 | 21.2 | 162 | 96.5 |
| 22 | 128.7 | 65 | 66.4 | 113 | 15.6 | 164 | 135.3 |
| 23 | 118.5 | 66 | 79.0 | 114 | 24.6 | 171 | 52.7 |
| 25 | 35.7 | 67 | 117.9 | 117 | 13.3 | 174 | 36.2 |
| 29 | 78.7 | 68 | 25.2 | 118 | 136.1 | 175 | 36.2 |
| 30 | 33.6 | 69 | 22.9 | 119 | 72.1 | 176 | 131.7 |
| 31 | 133.2 | 70 | 45.2 | 120 | 46.0 | | |
| 32 | 134.5 | 71 | 114.2 | 122 | 102.0 | | |
| 34 | 12.3 | 72 | 73.8 | 124 | 41.9 | | |
| 35 | 153.7 | 73 | 71.8 | 125 | 35.6 | | |
| 36 | 64.8 | 74 | 84.7 | 127 | 90.0 | | |
| 38 | 11.7 | 76 | 108.4 | 131 | 88.4 | | |
| 39 | 32.7 | 78 | 71.1 | 132 | 37.3 | | |
| 40 | 14.2 | 82 | 30.4 | 133 | 34.9 | | |
| 41 | 52.0 | 83 | 90.9 | 135 | 65.2 | | |
| 42 | 50.5 | 85 | 97.2 | 136 | 42.3 | | |
| 43 | 50.8 | 86 | 120.6 | 138 | 115.2 | | |
| 45 | 31.4 | 89 | 134.3 | 139 | 86.6 | | |
| 46 | 74.8 | 90 | 35.4 | 142 | 24.1 | | |
| 47 | 23.7 | 91 | 147.7 | 143 | 15.8 | | |
| 48 | 47.2 | 94 | 16.8 | 144 | 105.0 | | |
| 49 | 10.1 | 95 | 10.2 | 145 | 64.6 | | |

**Experimental Example 2 Effect of the Compounds of the Present Invention on the p-ERK Pathway in H358 Cells**

The effect of the compounds of the present invention on H358 cell p-ERK pathway was assessed by Advanced phospho-ERK (Thr202/Tyr204) cellular kit method.

The experimental procedures were summarized as follows:

H358 cells (ATCC, CRL-5807) were cultured in RPMI1640 (ThermoFisher, A1049101) complete medium containing 10% FBS (Gibco, 10100147) and 100 U/mL penicillin-streptomycin mixed solution (Gibco, 15140163). When the coverage

of the cells in the culture vessel reached 80-90%, the cells were pipetted and seeded in a 96-well plate (Corning, 3599) at 50000 cells/well (90 μL RPMI1640 complete medium), and the plate was left to stand for 5 min, cultured in an incubator at 37 °C with 5% $CO_2$ for 6 h, replaced with a serum-free RPMI1640, and starved overnight.

After incubation overnight, 10 μL of a diluted test compound was added to each well using a multi-channel pipette, and the 96-well plate was cultured in an incubator at 37 °C with 5% $CO_2$ for 1 h. The supernatant was poured. The cells were washed once with PBS. 50 μL of a lysis buffer (Advanced phospho-ERK (Thr202/Tyr204) cellular kit, 64AERPEH) was added to each well. The plate was shaken at room temperature at 450 rpm for 1 h. After the cells were completely lysed, 16 μL of the supernatant was added to a 384-well plate (PE, 6007299), mixed energy donor Eu and energy acceptor D2 (D2/Eu = 1:1) were added, and the plate was incubated at room temperature for 4 h. HTRF on the microplate reader was selected for plate reading, Ratio = Signal 665 nm/Signal 620 nm $\times 10^4$. In this experiment, the cell-free group (replaced with 1640 medium) was used as the 100% inhibition group, and the compound-free group in which the cell was added was used as the 0% inhibition group.

The inhibition percentage of H358 cell p-ERK pathway by the compounds of the present invention could be calculated by the following formula: Inhibition percentage = 100% * (0% inhibition group signal value - signal value at a specific concentration of test compound) / (0% inhibition group signal value - 100% inhibition group signal value).

$IC_{50}$ values of the compounds were calculated from 8 concentration points using XLfit (ID Business Solutions Ltd., UK) software by the following formula:

$$Y = Bottom + (Top - Bottom) / (1 + 10^{\wedge}((logIC_{50} - X) \times slope\ factor))$$

Y is the inhibition percentage, X is the logarithmic value of the concentration of the test compound, Bottom is the minimum inhibition percentage, Top is the maximum inhibition percentage, and slope factor is the slope coefficient of the curve. The default fitted curve was used to fit the slope of the S-shaped curve to determine the $IC_{50}$ value.

Table 2. Results of p-ERK inhibition test by the compounds of the present invention

| Example | $IC_{50}$ (nM) | Example | $IC_{50}$ (nM) | Example | $IC_{50}$ (nM) | Example | $IC_{50}$ (nM) |
|---|---|---|---|---|---|---|---|
| 1 | 20.7 | 52 | 41.4 | 98 | 55.6 | 139 | 81.2 |
| 2 | 126.6 | 53 | 176.0 | 99 | 111.5 | 140 | 131.3 |
| 6 | 111.5 | 54 | 124.4 | 100 | 49.8 | 142 | 43.7 |
| 8 | 92.9 | 55 | 30.3 | 101 | 50.1 | 143 | 34.8 |
| 11 | 88.2 | 56 | 42.4 | 103 | 82.4 | 144 | 40.0 |
| 13 | 131.2 | 57 | 67.5 | 104 | 57.5 | 145 | 138.0 |
| 16 | 28.0 | 58 | 111.6 | 105 | 98.4 | 150 | 42.8 |
| 17 | 33.7 | 61 | 16.4 | 106 | 48.4 | 151 | 41.7 |
| 18 | 79.8 | 62 | 133.2 | 107 | 46.9 | 152 | 76.3 |
| 19 | 52.5 | 65 | 27.0 | 108 | 61.3 | 155 | 59.8 |
| 20 | 41.9 | 66 | 76.8 | 109 | 35.5 | 158 | 144.8 |
| 22 | 180.5 | 68 | 15.3 | 110 | 73.6 | 159 | 137.8 |
| 23 | 44.9 | 69 | 38.7 | 111 | 109.9 | 162 | 77.5 |
| 24 | 70.4 | 70 | 34.5 | 112 | 108.7 | 163 | 43.9 |
| 25 | 107 | 71 | 155.9 | 113 | 70.0 | 164 | 69.3 |
| 29 | 71 | 72 | 72.4 | 114 | 54.8 | 169 | 120.9 |
| 30 | 29.5 | 73 | 103.5 | 117 | 22.4 | 170 | 138.0 |
| 31 | 73 | 75 | 171.3 | 119 | 54.6 | 171 | 75.6 |
| 32 | 48.9 | 76 | 112.8 | 120 | 87.4 | 172 | 166.7 |
| 34 | 9.18 | 77 | 77.2 | 121 | 106.8 | 173 | 98.3 |
| 36 | 69.1 | 78 | 39.6 | 122 | 111.3 | 174 | 20.4 |

(continued)

| Example | $IC_{50}$ (nM) | Example | $IC_{50}$ (nM) | Example | $IC_{50}$ (nM) | Example | $IC_{50}$ (nM) |
|---|---|---|---|---|---|---|---|
| 38 | 100.4 | 79 | 79.3 | 123 | 111.2 | 175 | 31.0 |
| 39 | 38.7 | 81 | 77.0 | 124 | 62.3 | 176 | 126.1 |
| 40 | 19.9 | 82 | 74.6 | 125 | 59.8 | | |
| 41 | 116.7 | 83 | 92.0 | 127 | 72.5 | | |
| 42 | 41.8 | 85 | 41.0 | 129 | 126.7 | | |
| 43 | 49.2 | 90 | 16.7 | 131 | 56.5 | | |
| 44 | 131.3 | 91 | 132.9 | 132 | 84.3 | | |
| 45 | 18.9 | 93 | 90.3 | 133 | 70.0 | | |
| 46 | 112.2 | 94 | 60.9 | 134 | 49.1 | | |
| 47 | 37.5 | 95 | 30.8 | 135 | 59.0 | | |
| 48 | 114.4 | 96 | 95.5 | 136 | 80.2 | | |
| 49 | 62.6 | 97 | 66.3 | 138 | 63.1 | | |

**Experimental Example 3. Determination of Pharmacokinetics of the Compounds of the Present Invention**

Test animals

Healthy adult female BALB/c nude mice were evenly divided into groups of 3 mice per group. 3 mice were administered intragastrically, and 3 mice were administered intravenously. The mice were purchased from Beijing Vital River Laboratory Animal Technology Co., Ltd., animal production license number: SCXK (Zhejiang) 2019-0001.

Drug preparation

A certain amount of the compound of the present invention was weighed and dissolved in 5% DMSO + 20% PG + 5% anhydrous ethanol + 10% solutol + 60% water to prepare 5 mg/mL for intragastric administration. A certain amount of the compound of the present invention was weighed and dissolved in 1% DMSO + 4% PG + 1% anhydrous ethanol + 2% solutol + 92% water to prepare 0.5 mg/mL for intravenous injection.

Administration method

Intragastric administration group: BALB/c nude mice were fasted overnight and then intragastrically administered the compound at a dose of 50 mg/kg and a volume of 10 mL/kg.
Intravenous administration group: BALB/c nude mice were fasted overnight and then intravenously administered the compound at a dose of 3 mg/kg and a volume of 6 mL/kg.

Procedures

After intragastric administration or intravenous administration, 40 μL of blood was collected from orbits of the mice at 5 min, 15 min, 30 min, 1 h, 2 h, 4 h, 6 h, 8 h, and 24 h after administration, anticoagulated with 5 μL of EDTA-K2, centrifuged at 12000 rpm at 4 °C for 5 min to separate the plasma, and stored at -20 °C.
Determination of plasma concentrations in mice after intragastric administration or intravenous administration of different concentrations of the test compounds: the sample was melted at room temperature and vortexed for 1 min; 10 μL of the sample was quantitatively transferred to a 2-mL 96-well plate; 50 μL of an internal standard solution (a solution of tolbutamide in acetonitrile) and 50 μL of a precipitant (acetonitrile:methanol 7:3) were added, and the plate was shaken (1000 rpm * 3 min); the mixture was centrifuged (4000 rpm * 15 min); 80 μL of the supernatant was transferred to a 2-mL 96-well plate; 80 μL of a diluent (water) was added; the mixture was shaken evenly (1000 rpm * 3 min), and 5 μL of the sample was analyzed. LC/MS/MS conditions: The mobile phase A was 0.1% aqueous formic acid solution. The mobile phase B was 0.1% formic acid in acetonitrile. The column was Waters T3 1.8 μm (2.1 mm * 30 mm). The column temperature was 40 °C. The gradient was 0-0.10 min, 20% B; 0.10-0.70 min, 20%-98% B; 0.70-1.00 min, 98%

B; 1.00-1.01 min, 98%-20% B; 1.01-1.50 min, 20% B. The flow rate was 0.6 mL/min.

Results

Under the experimental conditions, the test compounds of the present invention show relatively good pharmacokinetic properties, and the results are specified in Table 3 and Table 4.

Table 3. Pharmacokinetic parameters after single intragastric administration of the compounds in mice

| Example | Cmax (ng/mL) | $T_{max}$ (hr) | AUClast (hr*ng/mL) | $T_{1/2}$ (hr) |
|---|---|---|---|---|
| 1 | 9430 | 0.4 | 17813 | 1.0 |

Table 4. Pharmacokinetic parameters after single intravenous administration of the compounds in mice

| Example | C0 (ng/mL) | $T_{1/2}$ (hr) | AUClast (hr*ng/mL) | Vss (L/Kg) |
|---|---|---|---|---|
| 1 | 2960 | 0.5 | 619 | 3.8 |

**Experimental Example 4: Determination of Inhibitory Activity of Examples of the Present Invention on Binding of SOS1 to KRAS G12D**

The inhibitory effect of the compounds of the present invention on KRAS G12D:: SOS 1 binding was assessed using KRAS-G12D/SOS1 BINDING ASSAY KITS (Cisbio, Cat. No. 63ADK000CB21PEH).

Procedures:

The test compound (10 mM stock solution) was diluted 5-fold to 0.1 mM with 100% DMSO and diluted at a ratio of 1:3 in a 384-well dilution plate for 11 concentrations. 0.1 μL of the gradiently-diluted compound solution was transferred to a 384-well plate using Echo. 2 replicate wells were set for each compound. The solution was centrifuged at 1000 rpm/min for 1 min. 5 μL of 4X KRAS G12D (final concentration 1X) and a GTP solution (final concentration 10 μM, sigma, Cat. No. V900868) were transferred to the 384-well reaction plate. The mixture was centrifuged at 1000 rpm/min for 1 min and incubated at 25 °C for 15 min. 5 μL of a 4X SOS1 solution (final concentration 1X) was transferred to the 384-well reaction plate. The solution was centrifuged at 1000 rpm/min for 1 min and incubated at 25 °C for 45 min. The final concentrations of BAY-293 compound were 10, 3.33, 1.11, 0.37, 0.12, 0.04, 0.014, 0.0046, 0.0015, 0.0005, 0.00017, and 0 μM. The final concentrations of the test compound were 10, 3.33, 1.11, 0.37, 0.12, 0.04, 0.014, 0.0046, 0.0015, 0.0005, 0.00017, and 0 μM. The final concentrations of DMSO were all 0.5%. 10 μL of a 2X assay reagent solution was transferred to the 384-well reaction plate. The solution was centrifuged at 1000 rpm/min for 1 min and incubated at 4 °C for 180 min. An Envision multifunctional microplate reader was used to read at an excitation wavelength of 665 nm and an emission wavelength of 615 nm. The signal intensity of 665/615 ratio was used to characterize the degree of enzyme activity.
Data processing: compound $IC_{50}$ was fitted by Graphpad Prism 8 nonlinear regression equation:
Negative control: DMSO
$IC_{50}$ (half maximal inhibitory concentration) of the compound was obtained using the following nonlinear fitting formula:

$$Y = Bottom + (Top - Bottom) / (1 + 10^\wedge((LogIC_{50} - X) * HillSlope))$$

X: log value of the concentration of the test compound
Y: inhibition percentage

Bottom is the minimum inhibition percentage, Top is the maximum inhibition percentage, and HillSlope is the Hill slope.
Results: under the condition of the experiment, the test compounds have good inhibitory activity on KRAS G12D::SOS1 binding. The corresponding activity test results for the test compounds were specified in Table 5.

Table 5. Results of KRAS G12D::SOS1 binding inhibitory activity test by the compounds of the present invention

| Example | $IC_{50}$ (nM) |
|---|---|
| 20 | 7.46 |
| 23 | 4.17 |
| 34 | 6.55 |
| 40 | 3.84 |
| 55 | 6.30 |

**Experimental Example 5: Determination of Plasma Protein Binding Rate of the Compound of the Present Invention**

I. Experimental materials and instruments

1. CD-1 mouse plasma (BioIVT)
2. 96-well equilibrium dialysis plate (HTDialysis LLC, Gales Ferry, CT, HTD96B) and equilibrium dialysis membrane (MWCO 12-14K, #1101)
3. Positive control compound warfarin
4. ABI QTrap 5500 liquid chromatography-mass spectrometry system

II. Procedures

1. Preparation of a buffer with a concentration of 100 mM sodium phosphate and 150 mM NaCl: an alkaline solution with a concentration of 14.2 g/L $Na_2HPO_4$ and 8.77 g/L NaCl was prepared with ultrapure water, and an acidic solution with a concentration of 12.0 g/L $NaH_2PO_4$ and 8.77 g/L NaCl was prepared with ultrapure water. The alkaline solution was titrated with the acidic solution until the pH was 7.4 to prepare a buffer with a concentration of 100 mM sodium phosphate and 150 mM NaCl.
2. Preparation of a dialysis membrane: the dialysis membrane was soaked in ultrapure water for 60 min to separate the membrane into two pieces, then soaked with 20% ethanol for 20 min, and finally soaked with dialysis buffer for 20 min.
3. Preparation of plasma: the frozen plasma was quickly thawed at room temperature and then centrifuged at 3,220 g at 4 °C for 10 min to remove clots, and the supernatant was collected to a new centrifuge tube. The pH of the plasma was measured and recorded. The plasma with pH 7-8 was used.
4. Preparation of a plasma sample containing the compound: A 10 mM stock solution of the compound or the positive control compound was diluted with DMSO to give a 200 μM working solution. 3 μL of 200 μM of the compound working solution was added to 597 μL of mouse plasma to give a plasma sample with a final concentration of 1 μM.
5. Equilibrium dialysis procedures: a dialysis device was assembled according to the instructions. 120 μL of the plasma sample containing 1 μM of the compound of the present invention was added to one side of the dialysis membrane, and a dialysate (phosphate-buffered saline) in the same volume was added to the other side. The sample in the experiment was in duplicate. The dialysis plate was sealed, placed in an incubation device, and incubated at approximately 100 rpm at 37 °C with 5% $CO_2$ for 6 h. After the incubation was completed, the film was removed, and 50 μL was pipetted from the buffer side and the plasma side of each well to different wells of a new plate. 50 μL of blank plasma was added to the phosphate-buffered saline sample. A blank phosphate-buffered saline in the same volume was added to the plasma sample, and then 300 μL of acetonitrile containing an internal standard was added to precipitate the protein. The mixture was vortexed for 5 min and centrifuged at 3,220 g at 4 °C for 30 min. 100 μL of the supernatant was transferred to a feeding plate, and 100 μL of ultrapure water was added. The mixture was well mixed for LC-MS/MS analysis.

The peak areas of the compound on the buffer side and the plasma side were measured. The plasma protein binding rate of the compound was calculated by the following formula:

%Free rate = (ratio of compound peak area to internal standard peak area for buffer side/ratio of compound peak area to internal standard peak area for plasma side) * 100%

$$\%\text{Binding rate} = 1 - \%\text{Free rate}$$

All data were calculated by Microsoft Excel software. Plasma protein binding values calculated for the compounds of the present invention are shown in Table 6.

Table 6. Protein binding rate values of the compounds of the present invention in CD-1 mouse plasma

| Example | %Binding rate |
|---|---|
| Example **23** | 91.3 |
| Example **40** | 92.9 |
| Example **55** | 95.7 |

**Experimental Example 6: Determination of Membrane Permeability and Transport Properties of the Compound of the Present Disclosure**

The membrane permeability and transport properties of the compound of the present disclosure were determined using the following method.

I. Experimental materials and instruments

1. Caco-2 cells (ATCC)
2. HEPES (Solarbio 804D049), penicillin/streptomycin (Solarbio 20200109), Trypsin/EDTA (Solarbio), and PBS (Solarbio 20200620)
3. Fetal Bovine Serum (FBS) (Sigma WXBD0055V), lucifer yellow (Sigma MKCJ3738), and $NaHCO_3$ (Sigma SLBZ4647)
4. Hank's balanced salt solution (Gibco 2085528), non-essential amino acid (NEAA) (Gibco 2211548), and Trypsin/EDTA (Gibco 2120732)
5. High glucose DMEM (Corning 20319014)
6. HTS Transwell-96 Well Permeable (Corning, 3391)
7. Resistance detector (Millipore, Millicell® ERS-2)
8. Cellometer® Vision (Nexcelom Bioscience)
9. Infinite 200 PRO microplate reader (Tecan, Infinite M200PRO)
10. Positive control compound metoprolol (Sinopharm 100084-201403), erythromycin (MCE 84550), and cimetidine (Sinopharm 100158-201406)
11. ABI QTrap 5500 liquid chromatography-mass spectrometry system

II. Procedures

1. Caco-2 cell culture

1) Preparation of transport buffer (HBSS containing 25 mM HEPES, pH 7.4): 5.958 g HEPES and 0.35 g NaHCOs were accurately weighed and dissolved in 900 mL pure water. 100 mL of 10× HBSS was then added and stirred well. The pH was adjusted to 7.4, and the mixture was filtered.
2) Preparation of Caco-2 cell culture medium: FBS, penicillin, streptomycin, kanamycin, and NEAA were added to a high glucose DMEM (containing L-glutamine) culture medium to prepare a cell culture medium

containing 10% FBS, 0.1 mg/mL streptomycin, 100 units penicillin, 0.6 μg/mL kanamycin, and 1× NEAA.
3) Cells were cultured with a T-75 flask at 37 °C with 5% $CO_2$ in an incubator. The culture medium was discarded when cell density reached 80-90%. The cells were rinsed with 5 mL PBS. 1.5 mL Trypsin/EDTA was added. The mixture was incubated at 37 °C for 5-10 min in an incubator until the cells shed like quicksand. Finally the Trypsin/EDTA was neutralized with a FBS-containing culture medium.
4) The cell suspension was centrifuged at 120 × g for 10 min, and the supernatant was discarded.
5) The cells were resuspended in a cell culture medium. The cell suspension was adjusted to a density of $6.86 \times 10^5$ cells/mL.

2. Caco-2 cell inoculation

1) 50 μL of culture medium was added to the Transwell chamber per well. 25 mL of culture medium was added to the lower chamber, and the chamber was preheated at 37 °C for 1 h with 5% $CO_2$ in an incubator.
2) 50 μL of the cell suspension was added to the preheated Transwell chamber per well at a final density of $2.4 \times 10^5$ cells/$cm^2$
3) The cells were cultured for 14-18 days. The culture medium was replaced every other day, and the culture medium was replaced within 48 h after the initial inoculation. The culture medium had to be replaced the day before the experiment.

3. Evaluation of integrity of monolayer cell membrane

1) The cells fused and differentiated after 14 days of culture and were ready for a transport experiment.
2) The resistance of the monolayer membrane was measured using a resistance detector. The resistance of each well was recorded.
3) After measurement, the Transwell culture plate was re-incubated.
4) The TEER (resistance of the monolayer cell membrane) value was calculated:

$$\text{TEER value} = \text{TEER } (\Omega) \text{ measurement value} \times \text{membrane area } (\text{cm}^2)$$

The resistance of the monolayer cell membrane < 230 Ω $cm^2$ indicates that a monolayer cell membrane is poorly dense and cannot be used for experiment.

4. Transport experiment

1) A 10 mM stock solution of the compound of the present disclosure or the positive control compound was diluted with DMSO to give a 2 mM stock solution. The 2 mM stock solution was then diluted with the transport buffer to give a 10 μM working solution of the compound of the present disclosure or the positive control compound.
2) The Caco-2 cell plate was taken out from the incubator, washed twice with the pre-heated transport buffer, and incubated at 37 °C in an incubator for 30 min.
3) To determine the transport rate of the compound from the apical end to the basal end (A → B), 108 μL of the working solution of the compound was added to the Transwell chamber (apical end), while 8 μL of the sample was immediately taken from the apical end to 72 μL of the transport buffer. 240 μL of a stop solution containing an internal standard was added to stop the transport to be an initial apical end sample. At the same time, 300 μL of the transport buffer was added to the receiving end (basal end). The sample in the experiment was in duplicate.
4) To determine the transport rate of the compound from the basal end to the apical end (B → A), 308 μL of the working solution of the compound was added to the receiving end (basal end), while 8 μL of the sample was immediately taken from the basal end to 72 μL of the transport buffer. 240 μL of a stop solution containing an internal standard was added to stop the transport to be an initial basal end sample. At the same time, 300 μL of the transport buffer was added to the Transwell chamber (apical end). The sample in the experiment was in duplicate.
5) The cell culture plate was incubated at 37 °C with $CO_2$ in an incubator for 2 h.
6) After the transport experiment was completed, 8 μL of the sample was taken from the administration end (i.e., the apical end in the A → B direction and the basal end in the B → A direction) to 72 μL of the transport buffer. 240 μL of a stop solution containing an internal standard was then added to stop the transport. 80 μL of the sample was taken from the receiving end (i.e., the basal end in the A → B direction

and the apical end in the B → A direction) to 240 μL of a stop solution containing an internal standard. The mixture was vortexed at 1000 rpm for 10 min and centrifuged at 3,220 g for 30 min. 100 μL of the supernatant was transferred to a feeding plate, and 100 μL of ultrapure water was added. The mixture was well mixed for LC-MS/MS analysis.

7) Fluorescence values were measured after the transport experiment was completed. A 10 mM lucifer yellow stock solution was prepared with water and then diluted to 100 μM with the transport buffer. 100 μL of the lucifer yellow solution was added to the Transwell chamber (apical end). 300 μL of the transport buffer was added to the basal end. The chamber was incubated at 37 °C with $CO_2$ in an incubator for 30 min. 80 μL solution was taken from the basal end to a 96-well plate, and the fluorescence value of the cells was measured by a microplate reader at an excitation wavelength of 485 nm and an emission wavelength of 530 nm (to detect integrity of the membrane).

The leakage rate (percent leakage (%) or LY (%)) was calculated by the following formula:

$$\text{Percentage leakage (\%)} = \{I_{\text{acceptor}} \times 0.3 / (I_{\text{acceptor}} \times 0.3 + I_{\text{donor}} \times 0.1)\} \times 100\%$$

$I_{acceptor}$ referred to the fluorescence density of the receiving end (0.3 mL), and $I_{donor}$ referred to the fluorescence density of the administration end (0.1 mL). LY > 1.0% indicates that a monolayer cell membrane is poorly dense.

The peak areas of the compound on the administration end and the receiving end were measured. The apparent permeability coefficient ($P_{app}$, in cm/s) and the Efflux ratio (ER) of the compound were calculated:

$$P_{\text{app}} = \{V_{\text{A}} \times [drug]_{acceptor} / (Area \times incubation\ time \times [drug]_{initial\ dono}\}$$

$V_A$ was the volume of the receiving end solution (A → B was 0.3 mL, and B → A was 0.1 mL). Area was the area of the membrane of the Transwell 96-well plate (0.143 $cm^2$). Incubation time was the time for incubation (in s). $[drug]_{acceptor}$ was the concentration of the drug at the receiving end. $[drug]_{initial\ donor}$ was the initial concentration of the drug at the administration end.

$$Efflux\ Ratio = \frac{P_{app\ (B-A)}}{P_{app\ (A-B)}}$$

$P_{app\ (B-A)}$ represented the apparent permeability coefficient from the basal end to the apical end; $P_{app\ (A-B)}$ represented the apparent permeability coefficient from the apical end to the basal end.

The calculated apparent permeability coefficient and efflux ratio of the compound of the present invention are shown in Table 7. The compound of the present invention has good permeability.

Table 7. Apparent permeability coefficients and efflux ratios of the compounds of the present invention

| Example | $P_{app}$ (A-B) ($10^{-6}$, cm/s) | $P_{app}$ (B-A) ($10^{-6}$, cm/s) | Efflux ratio |
|---|---|---|---|
| Example 40 | 2.10 | 27.58 | 13.14 |
| Example 55 | 5.09 | 24.30 | 4.77 |

**Experimental Example 7: Inhibitory Effect of the Compound of the Present Invention on Enzymatic Activity of CYP2C9, CYP2D6, and CYP3A4**

The inhibition of CYP2C9, CYP2D6, and CYP3A4 enzymatic activity by the compound of the present invention was determined using the following method.

I. Experimental materials and instruments

1. Human liver microsome (Corning 452117)
2. $Na_2HPO_4$ (Sigma SLBZ6180)

3. $KH_2PO_4$ (Sigma SLBT6559)
4. NADPH (Solarbio 705Y021)
5. NADPH (Solarbio 705Y021)
6. Positive substrates diclofenac (Sigma SLBV3438), dextromethorphan (TRC 3-EDO-175-1), and midazolam (Cerilliant FE01161704)
7. Positive inhibitors sulfaphenazole (D. Ehrenstorfer GmbH 109012), quinidine (TCI WEODL-RE), and ketoconazole (Sigma 100M1091V)
7. AB Sciex Triple Quad 5500 liquid chromatography-mass spectrometry system

II. Procedures

1. Preparation of 100 mM phosphate-buffered saline (PBS): 7.098 g $Na_2HPO_4$ was weighed. 500 mL pure water was added. The mixture was dissolved by sonication to give solution A. 3.400 g $KH_2PO_4$ was weighed. 250 mL pure water was added. The mixture was dissolved by sonication to give solution B. The solution A was placed in a stirrer, and the solution B was slowly added until the pH reached 7.4 to prepare the 100 mM PBS buffer.
2. A 10 mM NADPH solution was prepared with a 100 mM PBS buffer. A 10 mM stock solution of the compound of the present invention was diluted with DMSO to give a compound working solution at a concentration of 200× (6000, 2000, 600, 200, 60, 20, and 0 μM). The positive inhibitor stock solution was diluted with DMSO to give a positive inhibitor working solution at a concentration of 200× (sulfaphenazole, 1000, 300, 100, 30, 10, 3, and 0 μM; quinidine/ketoconazole, 100, 30, 10, 3, 1, 0.3, and 0 μM). Substrate working solutions (120 μM diclofenac, 400 μM dextromethorphan, and 200 μM midazolam) at a concentration of 200× were prepared with water, acetonitrile, or acetonitrile/methanol.
3. 2 μL of 20 mg/mL liver microsome solution, 1 μL of substrate working solution, 1 μL of compound working solution, and 176 μL of PBS buffer were taken, mixed well, and placed in a 37 °C water bath for pre-incubation for 15 min. 1 μL of sulfaphenazole, quinidine, or ketoconazole working solution was added to the positive control group instead of the compound working solution. At the same time, 10 mM NADPH solution was pre-incubated together in the 37 °C water bath for 15 min. After 15 min, 20 μL of NADPH was added to each well to initiate the reaction. The mixture was incubated at 37 °C for 5 min (CYP2C9), 20 min (CYP2D6), or 5 min (CYP3A4). All incubated samples were in duplicate. After incubation for the corresponding period of time, 400 μL of icy methanol containing internal standard was added to all samples to stop the reaction. The mixture was vortexed, mixed well, and centrifuged for 40 min at 4 °C at 3220 g. 100 μL of the supernatant was transferred to a feeding plate after the centrifugation was completed, and 100 μL ultrapure water was added. The mixture was well mixed for LC-MS/MS analysis.

The values were calculated by Excel XLfit 5.3.1.3 to obtain $IC_{50}$ values for the compounds of the present invention against CYP2C9, CYP2D6, and CYP3A4 as shown in Table 8. The results in Table 8 show that the compound of the present invention has a weak inhibitory effect on CYP450, indicating that the compound of the present invention has a lower potential risk of developing drug-drug interaction (DDI).

Table 8. $IC_{50}$ values of the compounds of the present invention against CYP2C9, CYP2D6, and CYP3A4

| Example | CYP2C9 (μM) | CYP2D6 (μM) | CYP3A4 (μM) |
|---|---|---|---|
| Example **23** | >30 | >30 | >30 |
| Example **40** | >30 | >30 | >30 |
| Example **55** | >30 | >30 | >30 |

**Experimental Example 8: Determination of Inhibitory Activity of Examples of the Present Invention on H358-SOS2 KO Cell Proliferation**

The effect of the compounds of the present invention on the proliferation of H358 cells with knockout of SOS2 (H358-SOS2 KO, Kyinno Biotechnology Co., Ltd.) was assessed by CellTiter-Glo luminescent cell viability assay kit method.

Procedures:

The H358-SOS2 KO cell strain was cultured in complete medium at 37 °C with 5% $CO_2$.

A sterilized 1% methylcellulose 3D medium was prepared in advance.

Cells in the logarithmic growth phase were harvested and counted using a platelet counter. The cell viability was determined by the trypan blue exclusion method, and the cell viability was kept 90% or more.

The cell concentration was adjusted. The cells were uniformly mixed and left to stand. After no visible gas exists in the cell suspension, 180 μL of the cell suspension was added into a 96-well plate at 180 μL/well, and the total number of cells was 2000.

The cells in the 96-well plate were incubated at 37 °C with 5% $CO_2$.

A diluted test compound was added to each well using a multi-channel pipette. The cells in the 96-well plate added with the compound were incubated at 37 °C with 5% $CO_2$ for 8 days, followed by the CTG analysis. The CTG reagent was thawed and the cell plate was equilibrated to room temperature for 30 minutes. An equal volume of CTG solution was added to each well. The cell plate was shaken on an orbital shaker for 5 min to lyse the cells. The cell plate was placed at room temperature for 20 min to stabilize the luminescence signal. The luminescence value was read, and the data were collected.

Data analysis:

The data were analyzed using the GraphPad Prism 7.0 software. Dose-response curves were fit to the data using nonlinear S-curve regression, and the $IC_{50}$ value was calculated from the curves.

Results: under the condition of the experiment, the compounds of the examples of the present invention have good proliferation inhibitory activity on H358-SOS2 KO cells. The corresponding activity test results for the test compounds were specified in Table 9.

Table 9. Results of H358-SOS2 KO cell proliferation inhibitory activity test by the compounds of the present invention

| Test compound | $IC_{50}$ (nM) |
|---|---|
| Example 55 | 79 |

**Experimental Example 9: Growth Inhibition Experiment of the Compound of the Present Invention on DLD-1 Cell Subcutaneous Tumor Model**

**Experimental reagent:**

Human colorectal cancer DLD-1 cell: ATCC, CCL-221
RPMI-1640 culture medium: Gibco, REF: 61870-036
Fetal bovine serum: Gibco, REF: 10091-148
0.25% trypsin-EDTA: Gibco, REF: 25200-072
D-PBS (phosphate-buffered saline without calcium and magnesium ions): Hyclone, Cat. No.: SH30256.01
Matrigel: Corning, Cat. No.: 356237

**Procedures:**

Animal information: NU/NU mice, female, aged 6-7 weeks, 24-30 g, purchased from Beijing Vital River Biotechnology Co., Ltd.

Cell culture: human colon cancer cell strain DLD-1 was cultured *in vitro* under the conditions of RPMI-1640 (cell culture medium) added with 10% fetal bovine serum and 1% Pen Strep (Gibco penicillin-streptomycin diabody), and an incubator at 37 °C with 5% $CO_2$. The cells were digested with a 0.25% trypsin-EDTA digestive solution twice a week for passaging as per conventional practice. When cell confluence was 80%-90% and a required amount was

achieved, the cells were collected and counted. Cell inoculation: 0.1 mL/(containing $5\times10^6$) DLD-1 cell suspension (RPMI-1640: Matrigel, volume ratio 1: 1) was inoculated subcutaneously into the right lumbar dorsal part of each mouse. On day 6 after inoculation of the cells, the mice were randomly grouped and administrated based on tumor volume. The day when random grouping was performed was day 0.

**Tumor measurement and experimental indices:**

Tumor diameters were measured twice weekly using a vernier caliper. The tumor volume was calculated using the following formula: $V = 0.5a \times b^2$, where a and b represent the long diameter and short diameter of the tumor, respectively. Mouse body weight was measured twice weekly.

The anti-tumor therapeutic effect of the compound was evaluated by tumor growth inhibition rate TGI (%). TGI (%) = [(1 - (average tumor volume at the end of administration in a treatment group - average tumor volume at the start of administration of the treatment group)) / (average tumor volume at the end of treatment of the solvent control group - average tumor volume at the start of treatment of the solvent control group)] $\times$ 100%.

**Experimental conclusion:**

The compounds of Example **40** and Example **55** of the present disclosure have significant inhibitory effect on tumor growth when administered at 15 mg/kg orally twice a day (P < 0.0001). The results are specified in Table 10 and FIG. 1. The body weights of the mice were measured and there was no significant change in each group compared to the vehicle group.

Table 10. *In vivo* efficacy data for the compounds of the present invention

| Test compound | Dose mg/kg | Administration method | Frequency of administration | TGI(%) |
|---|---|---|---|---|
| Vehicle | / | PO | BID | / |
| Example 40 | 15 | PO | BID | 59.0 |
| Example 55 | 15 | PO | BID | 65.4 |

**Claims**

**1.** A compound of formula (I) or a stereoisomer thereof or a pharmaceutically acceptable salt thereof,

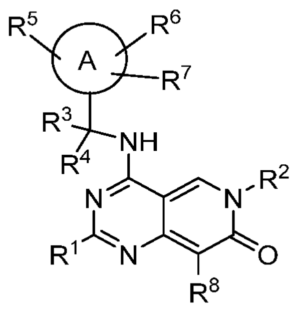

(I)

wherein

ring A is selected from $C_{6-10}$ aryl, 5-10 membered heterocyclyl, or 5-10 membered heteroaryl;
$R^1$ is selected from H, halogen, hydroxy, cyano, amino, $C_{1-6}$ alkyl, $C_{1-6}$ haloalkyl, $C_{1-6}$ deuterated alkyl, -O-$C_{1-6}$ alkyl, or $C_{3-6}$ cycloalkyl;
$R^2$ is selected from $C_{1-6}$ alkyl, $C_{2-6}$ alkenyl, $C_{2-6}$ alkynyl, $C_{3-10}$ cycloalkyl, $C_{4-10}$ cycloalkenyl, $C_{6-10}$ aryl, 3-10 membered heterocyclyl, or 5-10 membered heteroaryl, wherein the $C_{1-6}$ alkyl, $C_{2-6}$ alkenyl, $C_{2-6}$ alkynyl, $C_{3-10}$ cycloalkyl, $C_{4-10}$ cycloalkenyl, $C_{6-10}$ aryl, 3-10 membered heterocyclyl, or 5-10 membered heteroaryl is optionally substituted with $R^{2b}$ and/or $R^{2c}$;

$R^{2b}$ is independently selected from halogen, hydroxy, cyano, amino, $-OR^{2c}$, $-N(R^{2c})_2$, $-NHR^{2c}$, - $C(O)R^{2c}$, $-C(O)N(R^{2c})_2$, $-C(O)NHR^{2c}$, $-C(O)OR^{2c}$, $-S(O)_2R^{2c}$, $-S(O)_2N(R^{2c})_2$, $-S(O)_2NHR^{2c}$,$-NHC(O)R^{2c}$, $-C(S)R^{2c}$, or $-N(C_{1-4}$ alkyl)$C(O)R^{2c}$;

$R^{2c}$ is independently selected from $C_{1-6}$ alkyl, $C_{2-6}$ alkenyl, $C_{2-6}$ alkynyl, $C_{3-10}$ cycloalkyl, $C_{6-10}$ aryl, 3-10 membered heterocyclyl, or 5-10 membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3-10 membered heterocyclyl, or 5-10 membered heteroaryl is optionally substituted with $R^{2d}$ and/or $R^{2f}$;

$R^{2d}$ is independently selected from halogen, cyano, $-OR^{2f}$, $-N(R^{2f})_2$, $-C(O)R^{2f}$, $-C(O)N(R^{2f})_2$, - $C(O)OR^{2f}$, $-S(O)_2R^{2f}$, $-S(O)_2N(R^{2f})_2$, $-NC_{1-4}$ alkyl)$R^{2f}$, $-NHC(O)R^{2f}$, or $-N(C_{1-4}$ alkyl)$C(O)R^{2f}$;

$R^{2f}$ is independently selected from H, $C_{1-6}$ alkyl, $C_{2-6}$ alkenyl, $C_{2-6}$ alkynyl, $C_{3-10}$ cycloalkyl, $C_{6-10}$ aryl, 3-10 membered heterocyclyl, or 5-10 membered heteroaryl;

$R^3$ and $R^4$ are independently selected from H, deuterium, $C_{1-3}$ deuterated alkyl, $C_{1-6}$ alkyl, or $C_{1-6}$ haloalkyl;

$R^5$, $R^6$, and $R^7$ are independently selected from H, nitro, halogen, hydroxy, cyano, amino, $C_{1-6}$ alkyl, $C_{3-6}$ cycloalkyl, $-O-C_{1-6}$ alkyl, $-O-C_{3-6}$ cycloalkyl, -O-(3-8 membered heterocyclyl), $-S(O)_2-C_{1-4}$ alkyl, $-P(O)(R^{x2})_2$, $-C(O)R^{x2}$, $-C(O)N(R^{x2})_2$, $-C(O)OR^{x2}$,

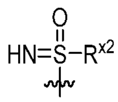
,

$-SF_5$, 3-8 membered heterocyclyl, or 5-10 membered heteroaryl, wherein the $C_{1-6}$ alkyl, $C_{3-6}$ cycloalkyl, $-S(O)_2-C_{1-4}$ alkyl, 3-8 membered heterocyclyl, or 5-10 membered heteroaryl is optionally substituted with $R^{x1}$;

$R^{x1}$ is independently selected from halogen, hydroxy, cyano, amino, or $-S(O)_2-C_{1-4}$ alkyl;

$R^{x2}$ is independently selected from H or $C_{1-6}$ alkyl;

$R^8$ is independently selected from H, halogen, hydroxy, cyano, amino, nitro,

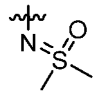
, 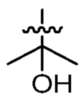
, -

$C(O)R^{8a}$, $-C(O)NHR^{8a}$, $-OC(O)R^{8a}$, $-NHC(O)R^{8a}$, $C_{1-6}$ alkyl, $C_{2-6}$ alkenyl, $C_{2-6}$ alkynyl, $C_{1-6}$ haloalkyl, $C_{3-6}$ cycloalkyl, $-S(O)_2-C_{1-6}$ alkyl, $-O-C_{1-6}$ alkyl, $-O-C_{1-6}$ deuterated alkyl, $-O-C_{3-6}$ cycloalkyl, -O-(3-10 membered heterocyclyl), -O-(5-10 membered heteroaryl), $-O-(C_{6-10}$ aryl), 3-10 membered heterocyclyl, 5-10 membered heteroaryl, or $C_{6-10}$ aryl, wherein the $C_{1-6}$ alkyl, amino, $C_{2-6}$ alkenyl, $C_{2-6}$ alkynyl, $C_{3-6}$ cycloalkyl, $-S(O)_2-C_{1-6}$ alkyl, $-O-C_{1-6}$ alkyl, $-O-C_{1-6}$ deuterated alkyl, $-O-C_{3-6}$ cycloalkyl, -O-(3-10 membered heterocyclyl), -O-(5-10 membered heteroaryl), $-O-(C_{6-10}$ aryl), 3-10 membered heterocyclyl, 5-10 membered heteroaryl, or $C_{6-10}$ aryl is optionally substituted with $R^{8a}$;

$R^{8a}$ is independently selected from deuterium, halogen, hydroxy, cyano, amino, $C_{1-6}$ alkyl, $C_{1-6}$ deuterated alkyl, $-O-C_{1-6}$ alkyl, $C_{1-6}$ haloalkyl, $-C(O)-C_{1-6}$ alkyl, $-S(O)_2R^{8b}$, $C_{3-6}$ cycloalkyl, 3-8 membered heterocyclyl, or 5-6 membered heteroaryl, wherein the amino, $-O-C_{1-6}$ alkyl, $C_{1-6}$ alkyl, $C_{3-6}$ cycloalkyl, 3-8 membered heterocyclyl, or 5-6 membered heteroaryl is optionally substituted with $R^{8b}$;

$R^{8b}$ is independently selected from halogen, hydroxy, cyano, amino, $C_{1-6}$ alkyl, 3-8 membered heterocyclyl, $-C(O)-C_{1-6}$ alkyl, or $C_{3-6}$ cycloalkyl, wherein the 3-8 membered heterocyclyl is optionally substituted with $C_{1-6}$ alkyl;

provided that when $R^8$ is selected from H, halogen, $C_{1-6}$ alkyl, or $C_{3-6}$ cycloalkyl, then $R^5$ is selected from nitro, hydroxy, cyano, $-O-C_{1-6}$ alkyl, $-O-C_{3-6}$ cycloalkyl, -O-(3-8 membered heterocyclyl), - $P(O)(R^{x2})_2$, $-C(O)R^{x2}$, $-C(O)N(R^{x2})_2$, $-C(O)OR^{x2}$,

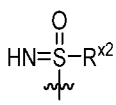
, $-SF_5$, 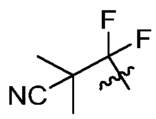
, 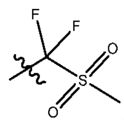
,

or 5-10 membered heteroaryl, wherein the 5-10 membered heteroaryl is optionally substituted with $R^{x1}$.

2. The compound of formula (I) or the stereoisomer thereof or the pharmaceutically acceptable salt thereof according to claim 1, wherein

$R^1$ is selected from H, halogen, $C_{1-6}$ alkyl, or $C_{1-6}$ deuterated alkyl; or
$R^1$ is selected from $C_{1-6}$ alkyl.

3. The compound of formula (I) or the stereoisomer thereof or the pharmaceutically acceptable salt thereof according to claim 1 or 2, wherein:

$R^2$ is selected from $C_{3-10}$ cycloalkyl, $C_{4-10}$ cycloalkenyl, $C_{6-10}$ aryl, 3-10 membered heterocyclyl, or 5-10 membered heteroaryl, wherein the $C_{3-10}$ cycloalkyl, $C_{4-10}$ cycloalkenyl, $C_{6-10}$ aryl, 3-10 membered heterocyclyl, or 5-10 membered heteroaryl is optionally substituted with $R^{2b}$ and/or $R^{2c}$; or
in the compound or the stereoisomer thereof or the pharmaceutically acceptable salt thereof, $R^2$ is selected from $C_{3-10}$ cycloalkyl or 3-10 membered heterocyclyl, wherein the $C_{3-10}$ cycloalkyl or 3-10 membered heterocyclyl is optionally substituted with $R^{2b}$ and/or $R^{2c}$; or
in the compound or the stereoisomer thereof or the pharmaceutically acceptable salt thereof, $R^2$ is selected from $C_{3-6}$ cycloalkyl or 4-9 membered heterocyclyl, wherein the $C_{3-6}$ cycloalkyl or 4-9 membered heterocyclyl is optionally substituted with $R^{2b}$ and/or $R^{2c}$.

4. The compound of formula (I) or the stereoisomer thereof or the pharmaceutically acceptable salt thereof according to any one of claims 1 to 3, wherein:

$R^{2b}$ is independently selected from $-OR^{2c}$, $-N(R^{2c})_2$, halogen, hydroxy, cyano, amino, $-C(O)R^{2c}$, or - $C(S)R^{2c}$; or
$R^{2b}$ is independently selected from cyano, $-C(O)R^{2c}$, or $-C(S)R^{2c}$.

5. The compound of formula (I) or the stereoisomer thereof or the pharmaceutically acceptable salt thereof according to any one of claims 1 to 4, wherein:

$R^{2c}$ is independently selected from $C_{1-6}$ alkyl, $C_{3-10}$ cycloalkyl, $C_{6-10}$ aryl, 3-10 membered heterocyclyl, or 5-10 membered heteroaryl, wherein the $C_{1-6}$ alkyl, $C_{3-10}$ cycloalkyl, $C_{6-10}$ aryl, 3-10 membered heterocyclyl, or 5-10 membered heteroaryl is optionally substituted with $R^{2d}$ and/or $R^{2f}$; or
in the compound or the stereoisomer thereof or the pharmaceutically acceptable salt thereof, $R^{2c}$ is independently selected from $C_{1-6}$ alkyl, 4-9 membered heterocyclyl, or 5-6 membered heteroaryl, wherein the $C_{1-6}$ alkyl, 4-9 membered heterocyclyl, or 5-6 membered heteroaryl is optionally substituted with $R^{2d}$ and/or $R^{2f}$; or
in the compound or the stereoisomer thereof or the pharmaceutically acceptable salt thereof, $R^{2c}$ is independently selected from $C_{1-6}$ alkyl, wherein the $C_{1-6}$ alkyl is optionally substituted with $R^{2d}$ and/or $R^{2f}$.

6. The compound of formula (I) or the stereoisomer thereof or the pharmaceutically acceptable salt thereof according to any one of claims 1 to 5, wherein:

$R^{2d}$ is selected from halogen; or
$R^{2d}$ is selected from F.

7. The compound of formula (I) or the stereoisomer thereof or the pharmaceutically acceptable salt thereof according to any one of claims 1 to 6, wherein:

$R^3$ and $R^4$ are independently selected from H, deuterium, $C_{1-3}$ deuterated alkyl, or $C_{1-6}$ alkyl; or
in the compound or the stereoisomer thereof or the pharmaceutically acceptable salt thereof, $R^3$ is selected from H or deuterium, and $R^4$ is selected from $CH_3$ or $CD_3$.

8. The compound of formula (I) or the stereoisomer thereof or the pharmaceutically acceptable salt thereof according to any one of claims 1 to 7, wherein:

$R^5$, $R^6$, and $R^7$ are independently selected from H, nitro, halogen, cyano, hydroxy, amino, $C_{1-6}$ alkyl, $C_{3-6}$ cycloalkyl, $-O-C_{1-6}$ alkyl, $-S(O)_2-C_{1-4}$ alkyl, $-C(O)R^{x2}$, $-SF_5$, 4-7 membered heterocyclyl, or 5-6 membered heteroaryl, wherein the $C_{1-6}$ alkyl, $C_{3-6}$ cycloalkyl, $-S(O)_2-C_{1-4}$ alkyl, 4-7 membered heterocyclyl, or 5-6 membered heteroaryl is optionally substituted with $R^{x1}$; or
in the compound or the stereoisomer thereof or the pharmaceutically acceptable salt thereof, $R^5$, $R^6$, and $R^7$

are independently selected from H, nitro, halogen, cyano, $-SF_5$, or $C_{1-6}$ alkyl, wherein the $C_{1-6}$ alkyl is optionally substituted with $R^{x1}$.

**9.** The compound of formula (I) or the stereoisomer thereof or the pharmaceutically acceptable salt thereof according to any one of claims 1 to 8, wherein:
$R^{x1}$ is independently selected from halogen or cyano.

**10.** The compound of formula (I) or the stereoisomer thereof or the pharmaceutically acceptable salt thereof according to any one of claims 1 to 9, wherein:

$R^8$ is independently selected from H, hydroxy, cyano, amino,

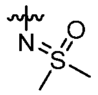, 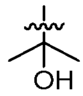,

$-C(O)R^{8a}$, $-C(O)NHR^{8a}$, $-OC(O)R^{8a}$, $-NHC(O)R^{8a}$, $C_{1-6}$ alkyl, $C_{2-6}$ alkenyl, $-O-C_{1-6}$ deuterated alkyl, $C_{1-6}$ haloalkyl, $-S(O)_2-C_{1-6}$ alkyl, $-O-C_{1-6}$ alkyl, $-O-C_{3-6}$ cycloalkyl, -O-(3-10 membered heterocyclyl), -O-(5-10 membered heteroaryl), $-O-(C_{6-10}$ aryl), 3-10 membered heterocyclyl, 5-10 membered heteroaryl, or $C_{6-10}$ aryl, wherein the amino, $C_{1-6}$ alkyl, $C_{2-6}$ alkenyl, $-O-C_{1-6}$ deuterated alkyl, $-S(O)_2-C_{1-6}$ alkyl, $-O-C_{1-6}$ alkyl, $-O-C_{3-6}$ cycloalkyl, -O-(3-10 membered heterocyclyl), -O-(5-10 membered heteroaryl), $-O-(C_{6-10}$ aryl), $R^{8a}$; or membered heterocyclyl, 5-10 membered heteroaryl, or $C_{6-10}$ aryl is optionally substituted with $R^{8a}$; or
$R^8$ is independently selected from H, hydroxy, cyano, amino,

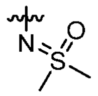, 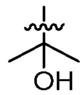,

$-C(O)R^{8a}$, $-C(O)NHR^{8a}$, $-OC(O)R^{a,}$ $-NHC(O)R^a$, $C_{1-3}$ alkyl, $C_{2-3}$ alkenyl, $-O-C_{1-3}$ deuterated alkyl, $C_{1-3}$ haloalkyl, $-S(O)_2-C_{1-3}$ alkyl, $-O-C_{1-3}$ alkyl, $-O-C_{3-6}$ cycloalkyl, -O-(4-7 membered heterocyclyl), -O-(5-6 membered heteroaryl), $-O-(C_{6-10}$ aryl), 4-9 membered heterocyclyl, 5-9 membered heteroaryl, or $C_{6-10}$ aryl, wherein the amino, $C_{1-3}$ alkyl, $C_{2-3}$ alkenyl, $-O-C_{1-3}$ deuterated alkyl, $-S(O)_2-C_{1-3}$ alkyl, $-O-C_{1-3}$ alkyl, $-O-C_{3-6}$ cycloalkyl, -O-(4-7 membered heterocyclyl), -O-(5-6 membered heteroaryl), $-O-(C_{6-10}$ aryl), 4-9 membered heterocyclyl, 5-9 membered heteroaryl, or $C_{6-10}$ aryl is optionally substituted with $R^{8a}$; or
$R^8$ is independently selected from H, hydroxy, cyano, amino,

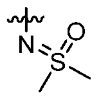, 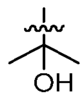,

$-C(O)R^{8a}$, $-C(O)NHR^{8a}$, $-OC(O)R^{8a}$, $-NHC(O)R^{8a}$, $C_{1-3}$ alkyl, $C_{2-3}$ alkenyl, $-O-C_{1-3}$ deuterated alkyl, $C_{1-3}$ haloalkyl, $-S(O)_2-C_{1-3}$ alkyl, $-O-C_{1-3}$ alkyl, $-O-C_{3-6}$ cycloalkyl, -O-(4-6 membered heterocyclyl), -O-(5-6 membered heteroaryl), -O-phenyl, 4-9 membered heterocyclyl, 5-9 membered heteroaryl, or $C_{6-10}$ aryl, wherein the amino, $C_{1-3}$ alkyl, $C_{2-3}$ alkenyl, $-O-C_{1-3}$ deuterated alkyl, $-S(O)_2-C_{1-3}$ alkyl, $-O-C_{1-3}$ alkyl, $-O-C_{3-6}$ cycloalkyl, -O-(4-6 membered heterocyclyl), -O-(5-6 membered heteroaryl), -O-phenyl, 4-9 membered heterocyclyl, 5-9 membered heteroaryl, or $C_{6-10}$ aryl is optionally substituted with $R^{8a}$.

**11.** The compound of formula (I) or the stereoisomer thereof or the pharmaceutically acceptable salt thereof according to any one of claims 1 to 10, wherein:
ring A is selected from $C_{6-10}$ aryl.

**12.** The compound of formula (I) or the stereoisomer thereof or the pharmaceutically acceptable salt thereof according to any one of claims 1 to 10, wherein the compound of formula (I) or the stereoisomer thereof or the pharmaceutically acceptable salt thereof is selected from a compound of formula (II) or a stereoisomer thereof or a pharmaceutically acceptable salt thereof,

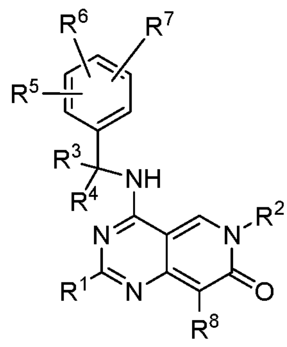

(II)

wherein $R^1$, $R^2$, $R^3$, $R^4$, $R^5$, $R^6$, $R^7$, and $R^8$ are as defined in claims 1 to 10.

**13.** The compound of formula (I) or the stereoisomer thereof or the pharmaceutically acceptable salt thereof according to any one of claims 1 to 10, wherein the compound of formula (I) or the stereoisomer thereof or the pharmaceutically acceptable salt thereof is selected from a compound of formula (III) or a stereoisomer thereof or a pharmaceutically acceptable salt thereof,

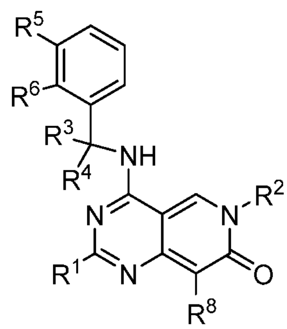

(III)

wherein $R^1$, $R^2$, $R^3$, $R^4$, $R^5$, $R^6$, and $R^8$ are as defined in claims 1 to 10.

**14.** A compound or a stereoisomer thereof or a pharmaceutically acceptable salt thereof, wherein the compound is selected from one of the following structures:

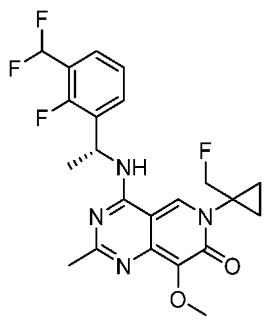

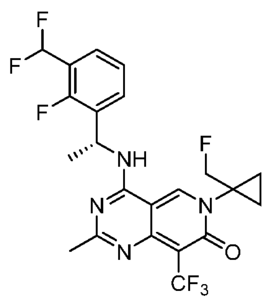

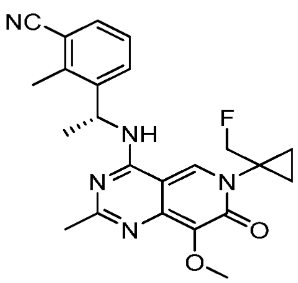

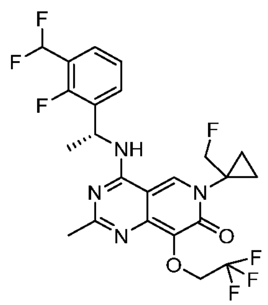

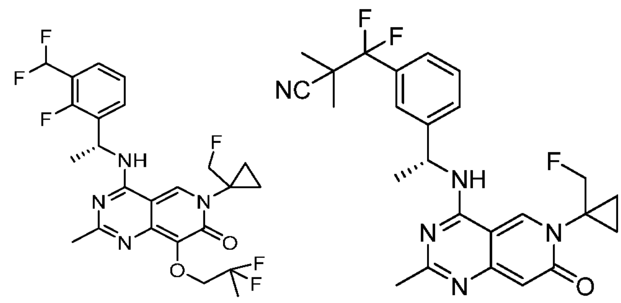

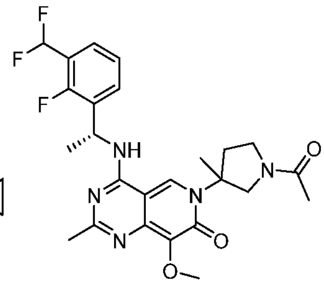

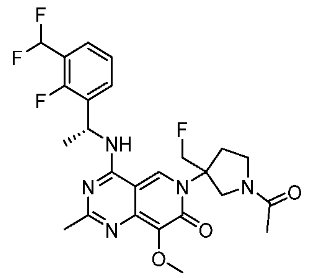

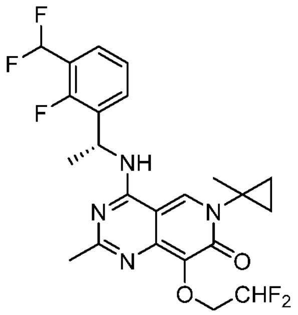

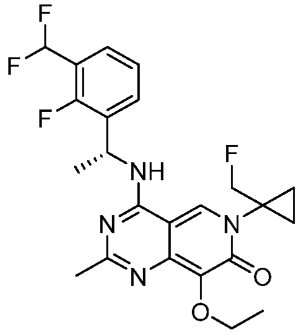

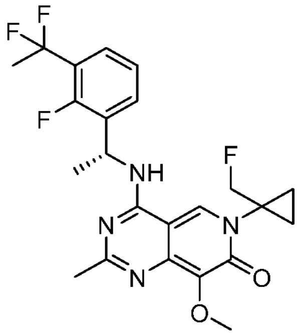

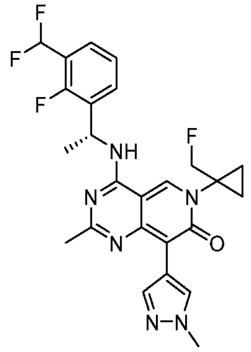

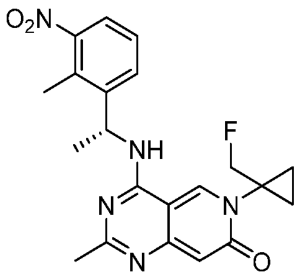

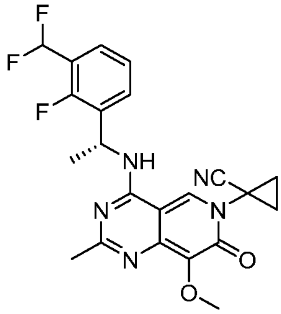

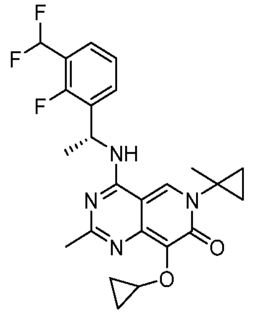

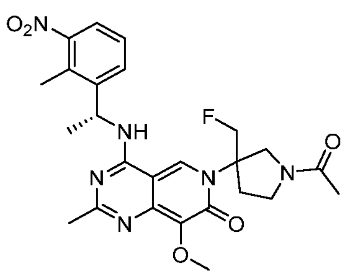

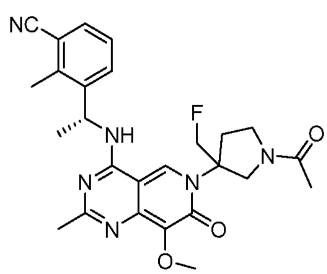

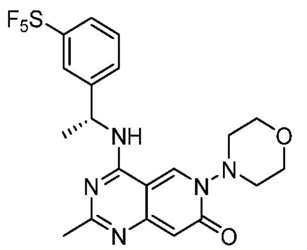

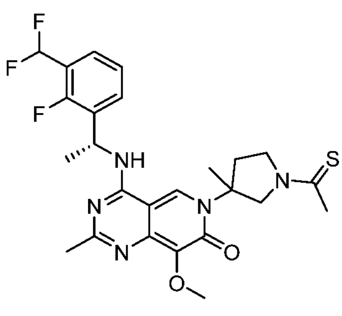

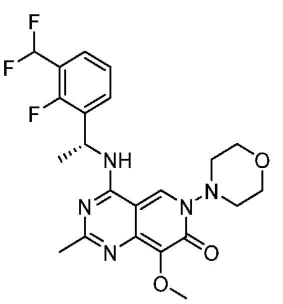

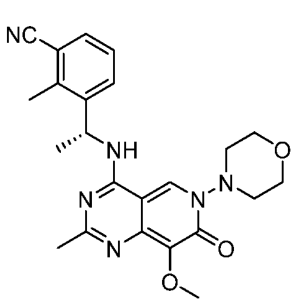

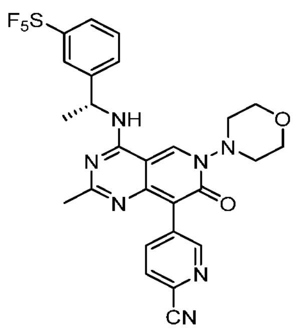

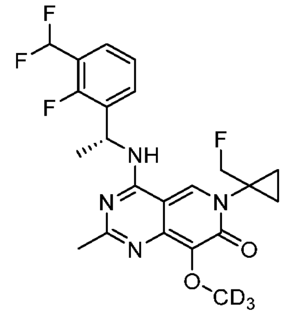

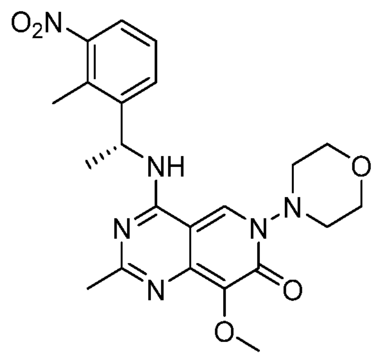

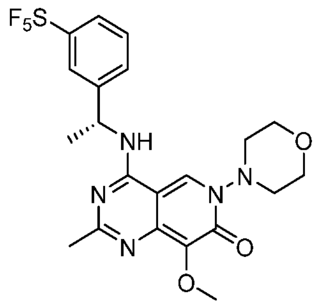

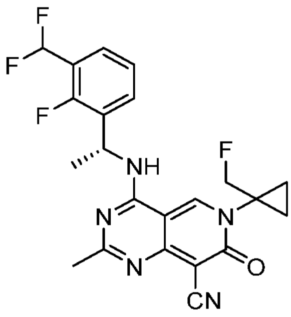

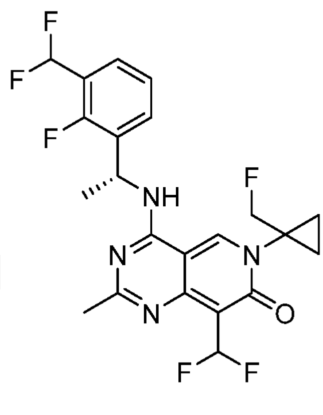

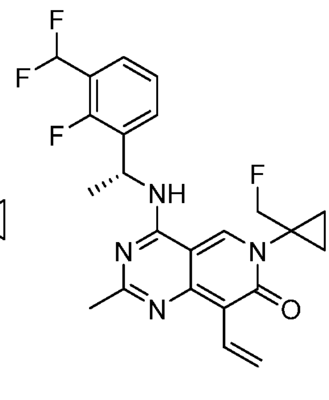

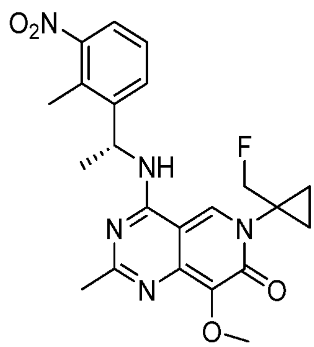

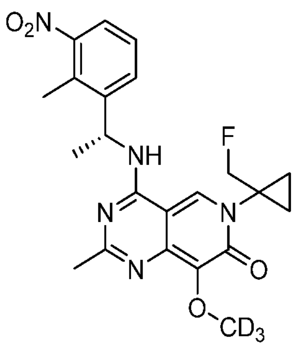

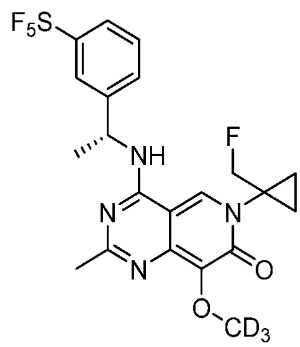

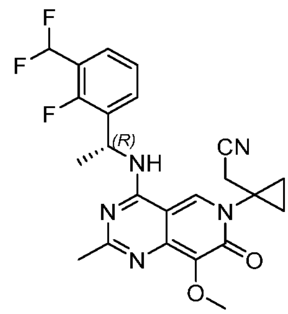

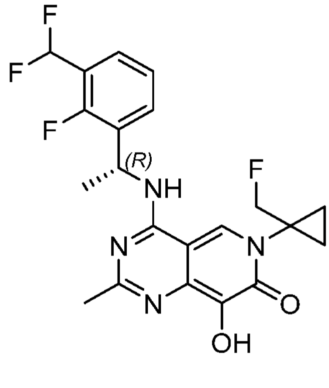

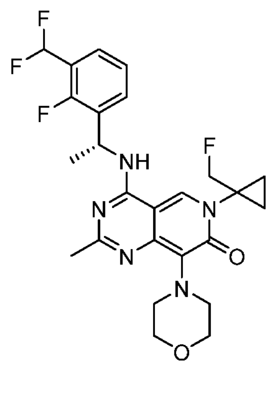

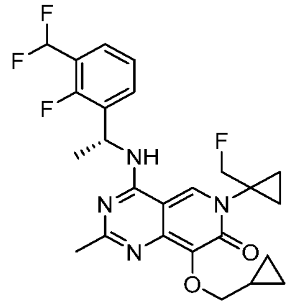

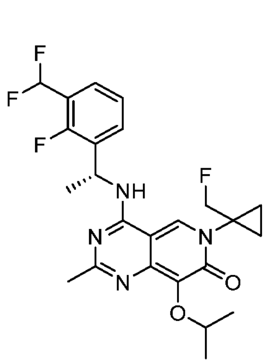
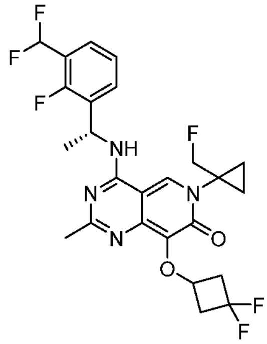
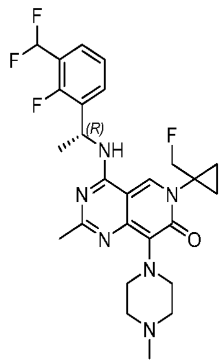
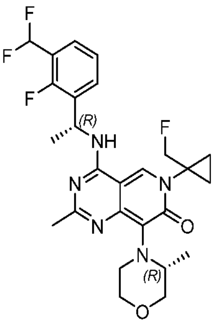

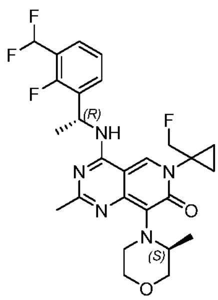
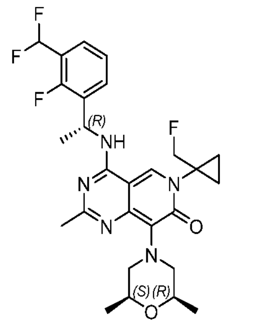
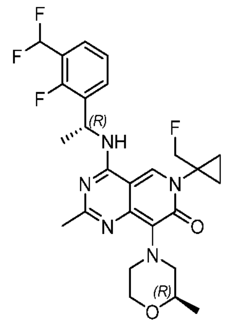
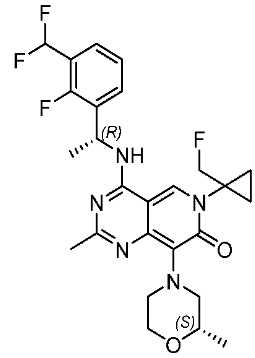

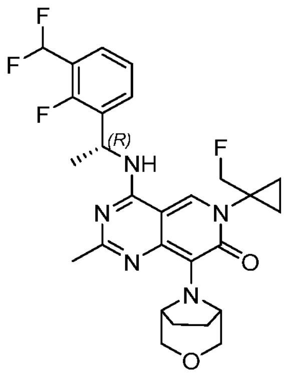
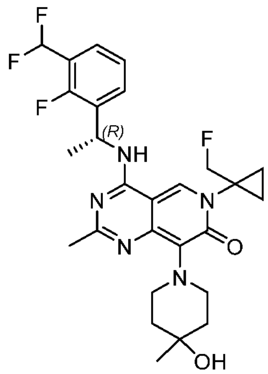
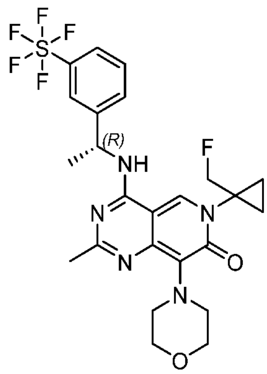
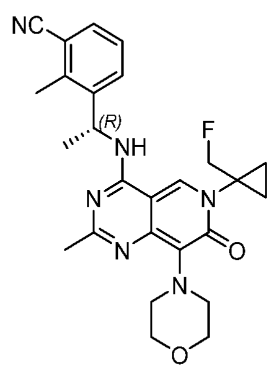

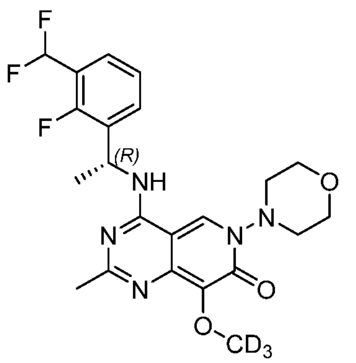
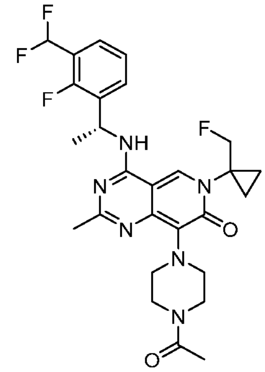
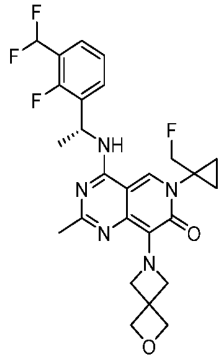
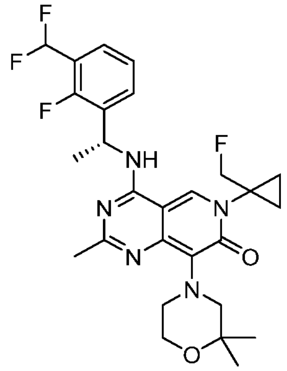

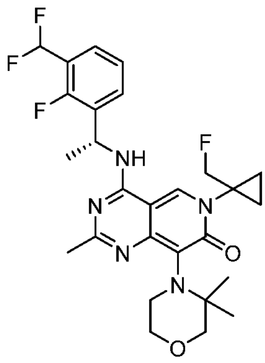
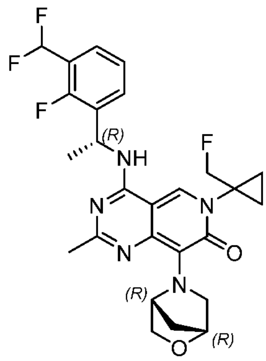
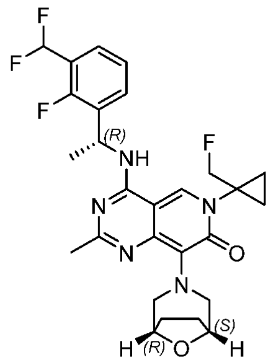
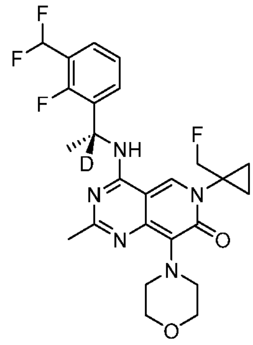

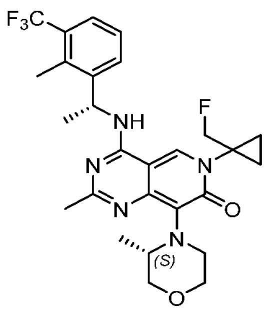
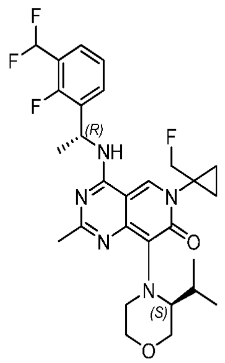
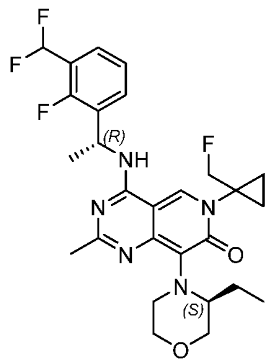
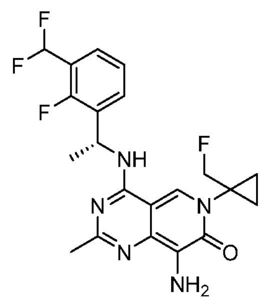

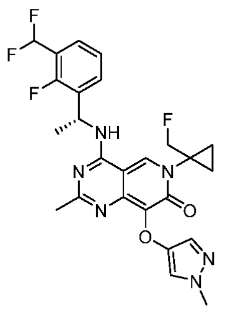
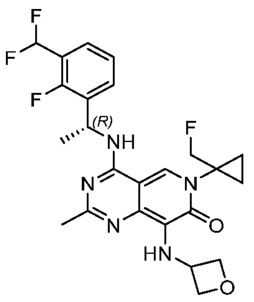
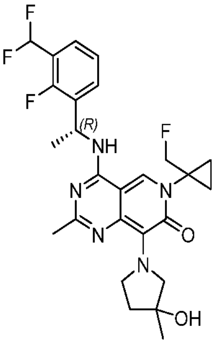
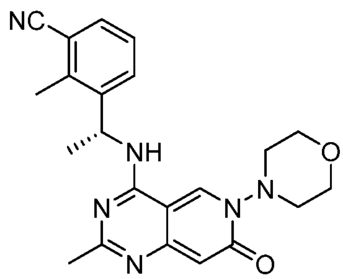

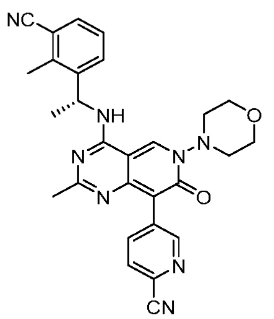
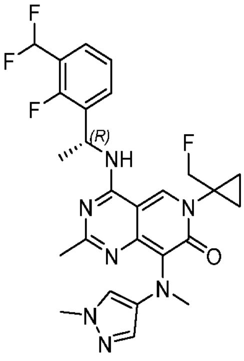
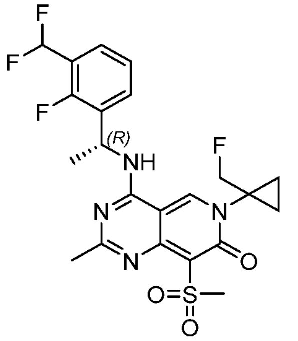
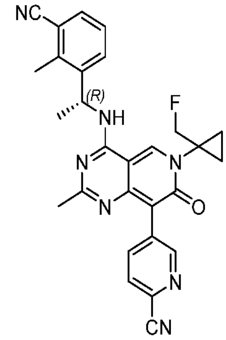

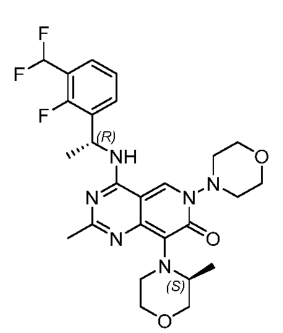

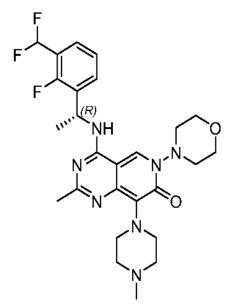

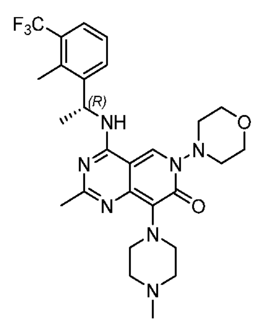

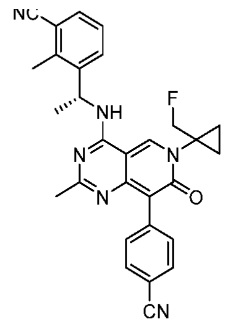

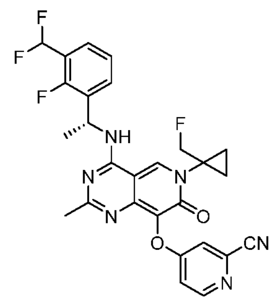

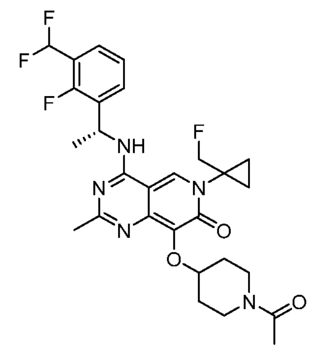

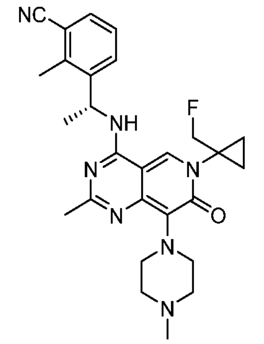

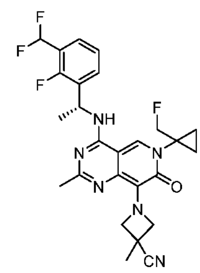

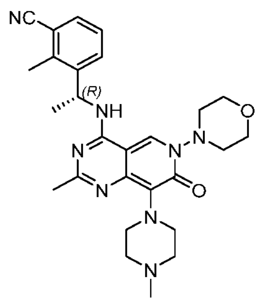

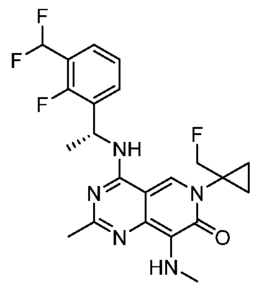

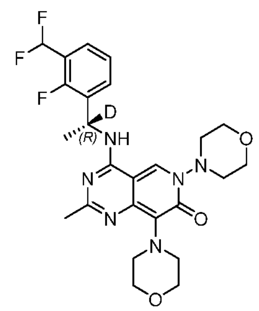

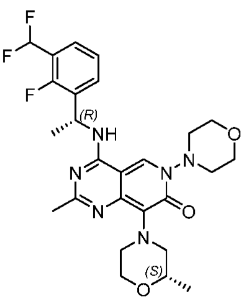

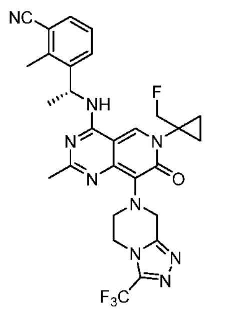

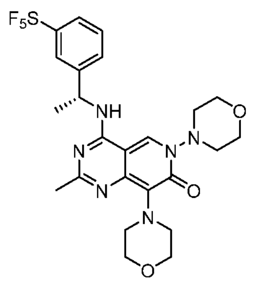

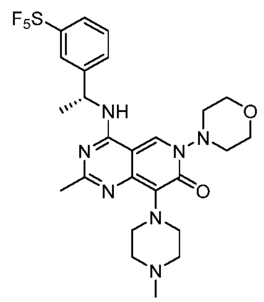

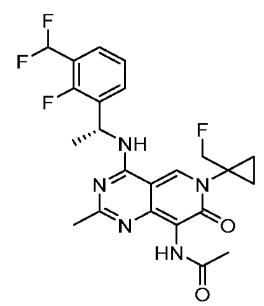

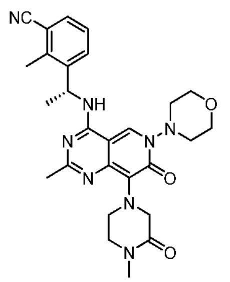

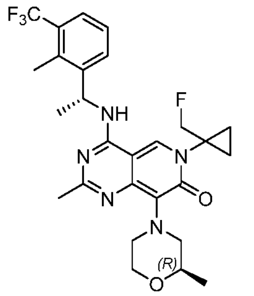

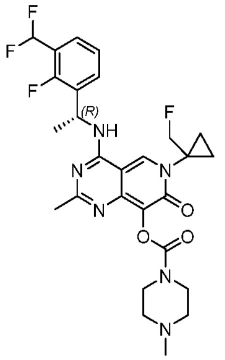

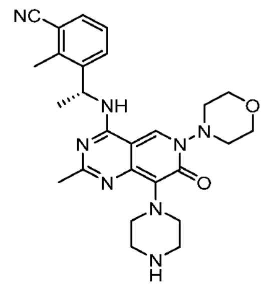

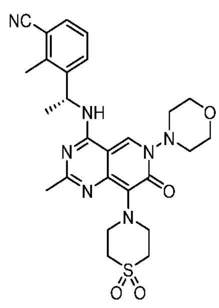

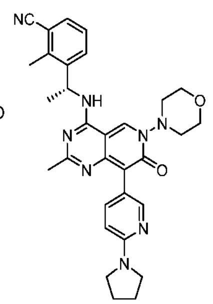

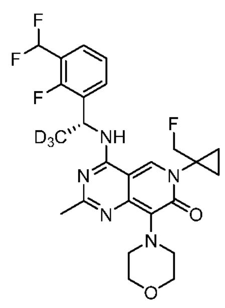

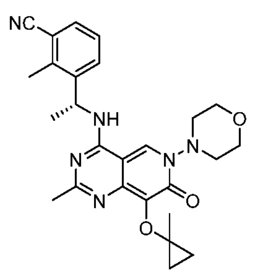

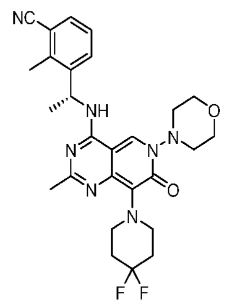

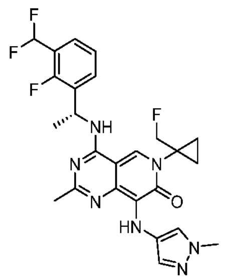

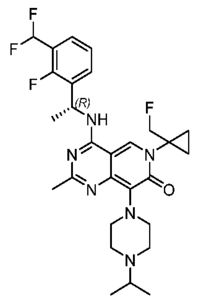

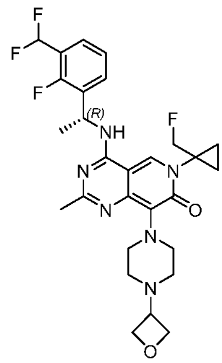

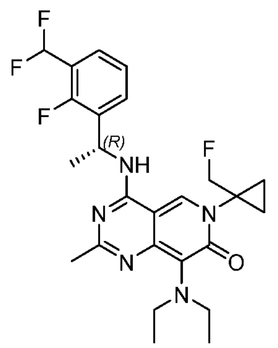

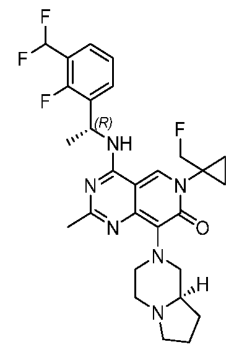

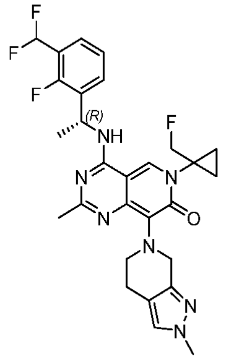

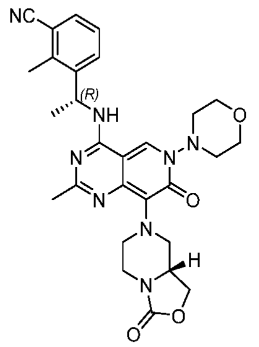

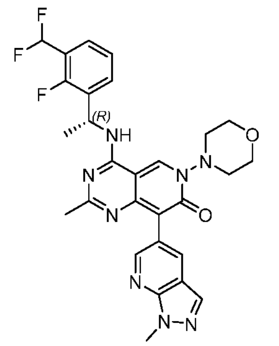

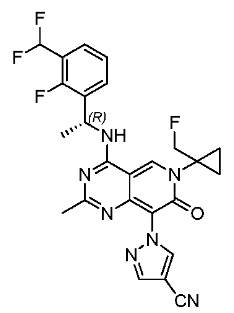

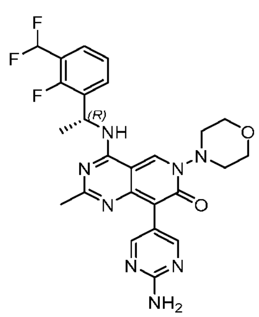

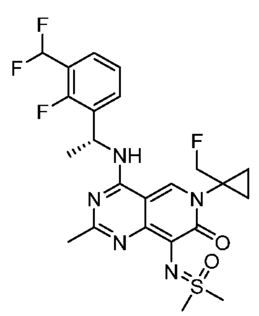

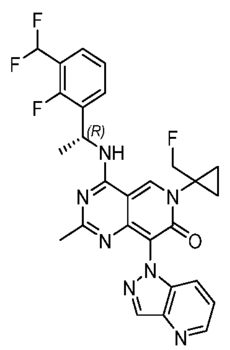

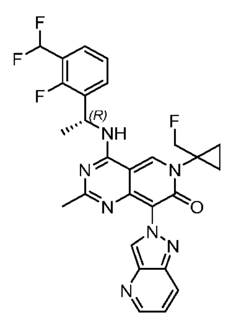

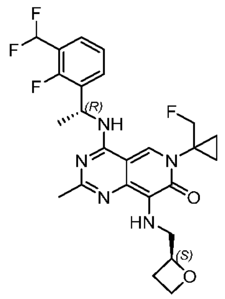
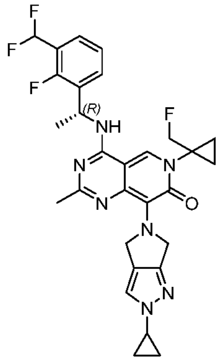
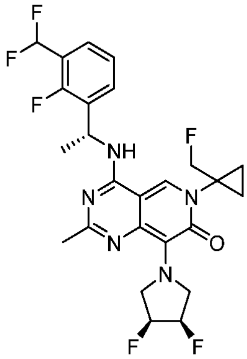
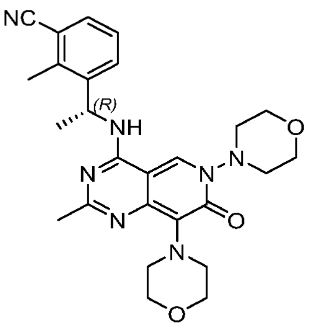

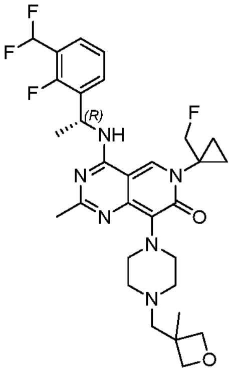
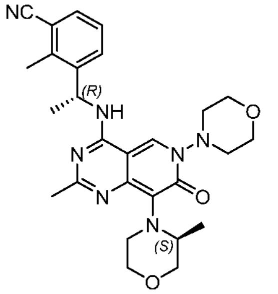
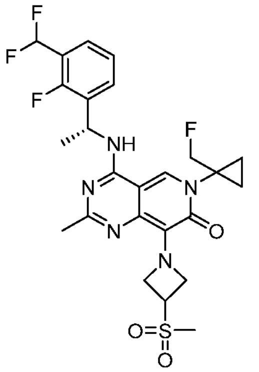
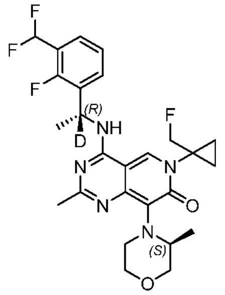

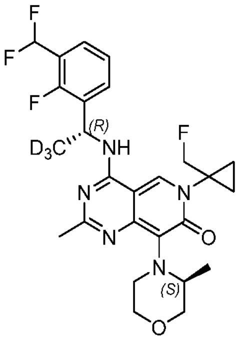
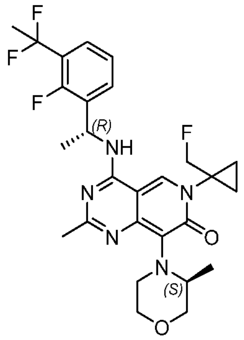
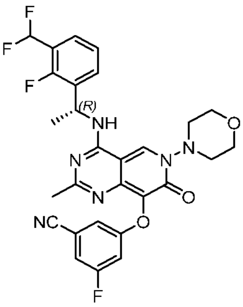
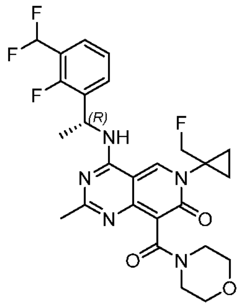

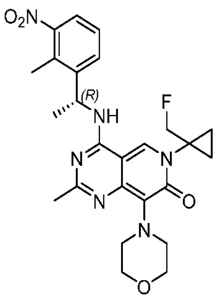
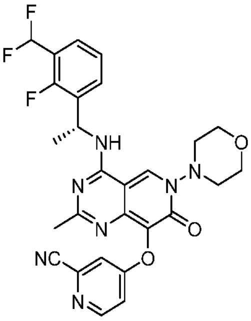
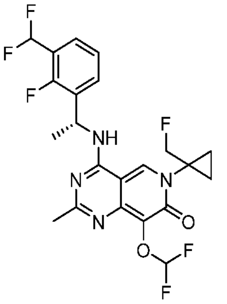
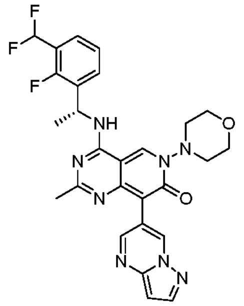

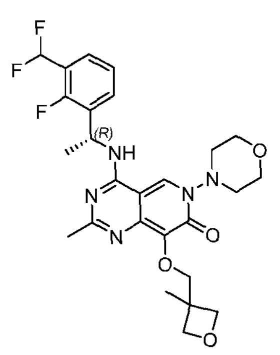
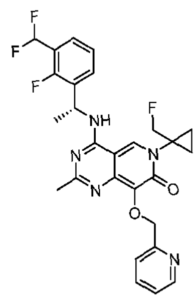
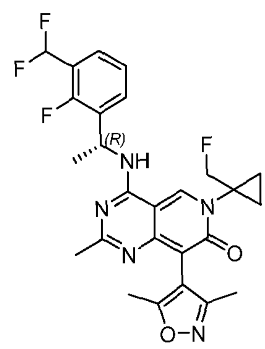
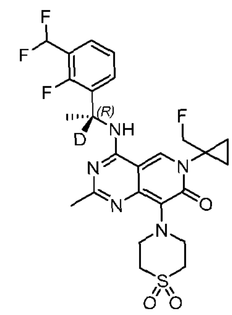
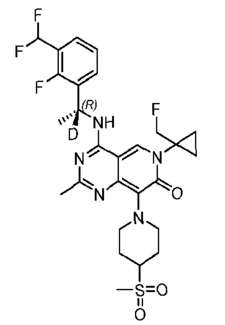
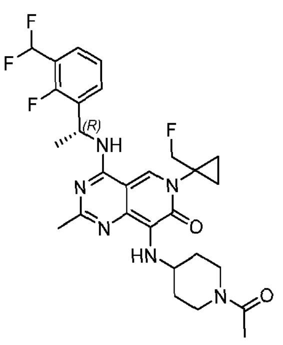
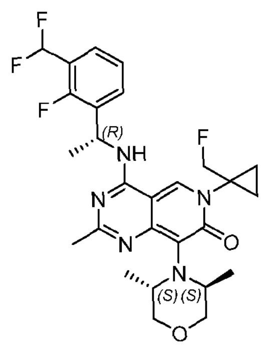
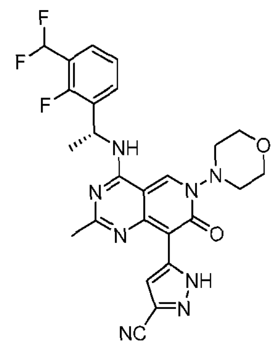
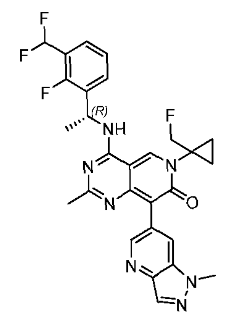
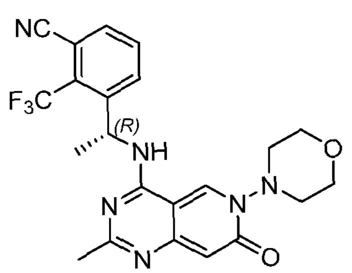
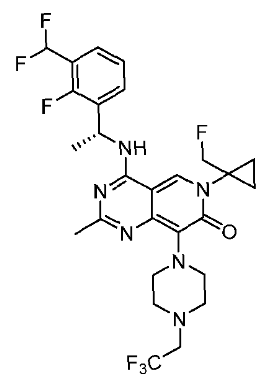
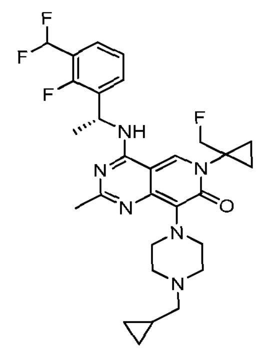
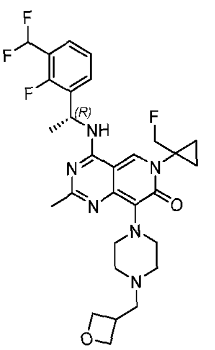
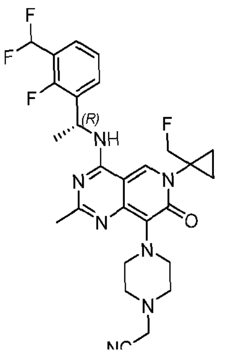
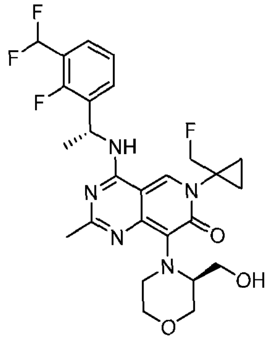
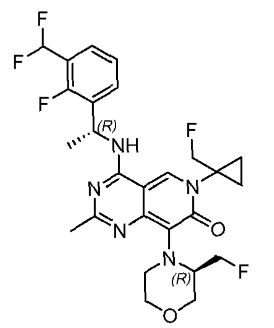
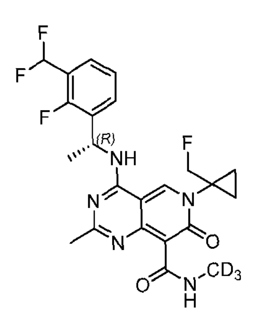

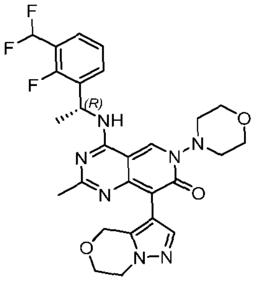
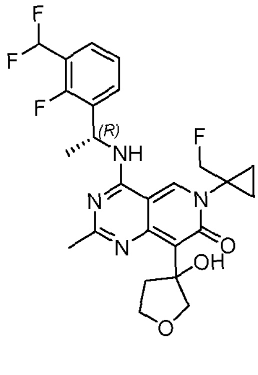
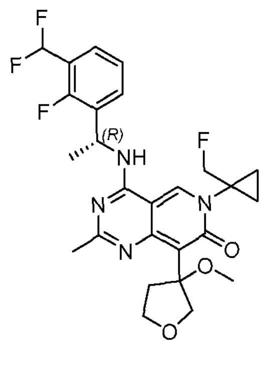
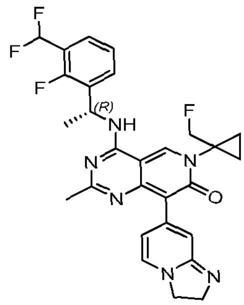
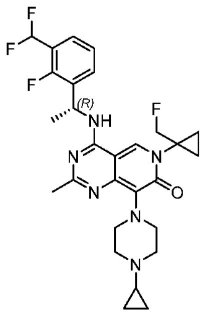
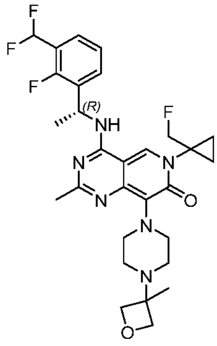
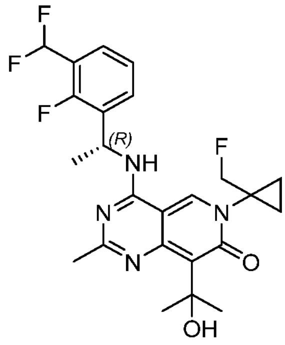
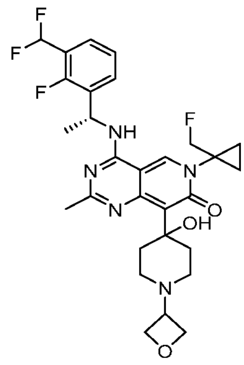
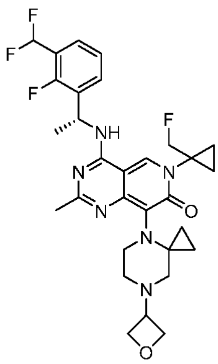
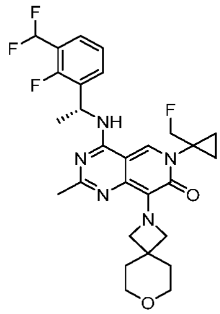
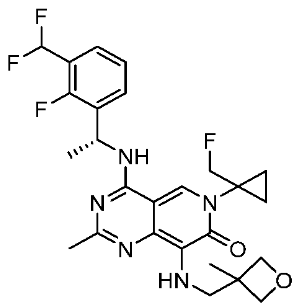
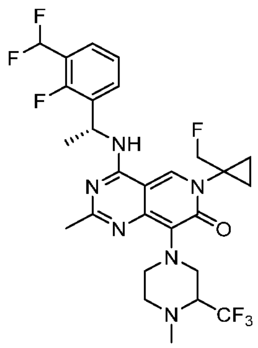
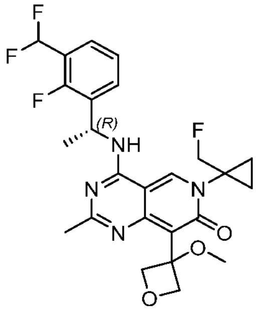
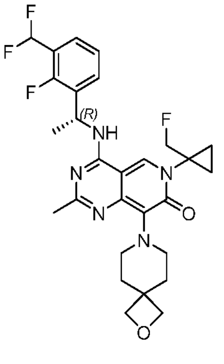
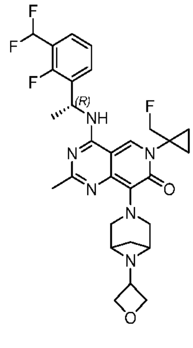
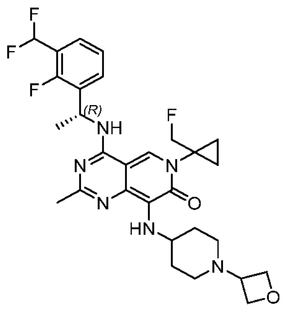

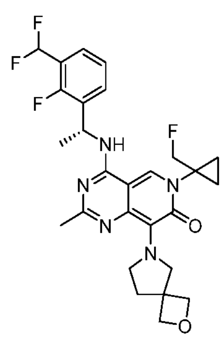
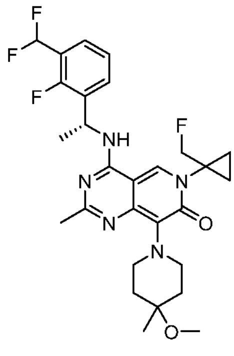
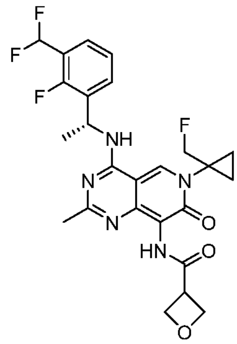
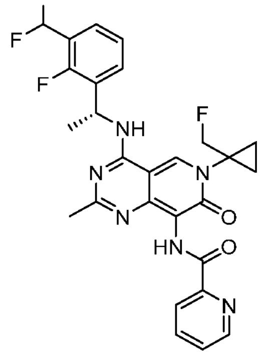

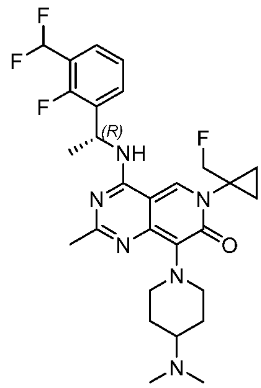
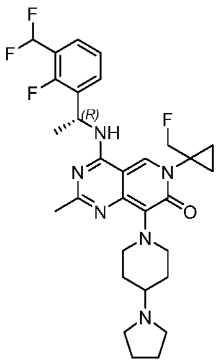
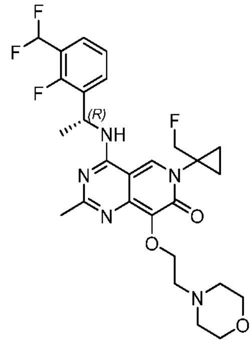
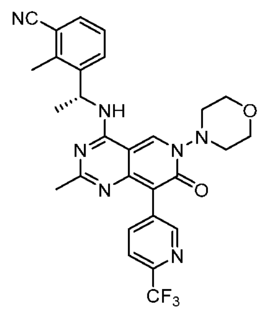

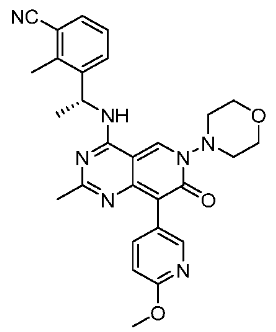
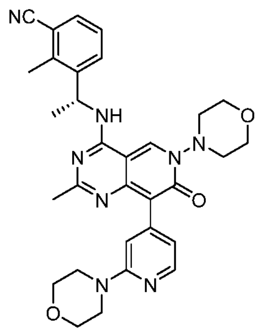
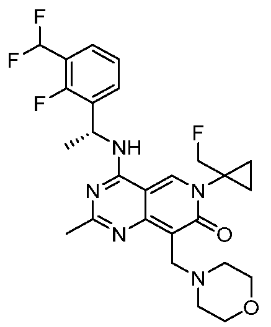
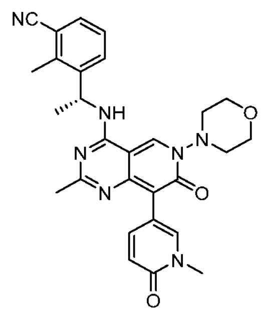

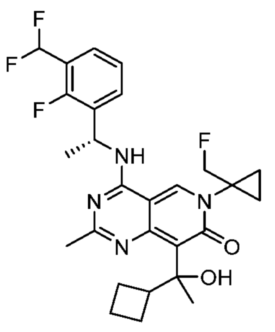
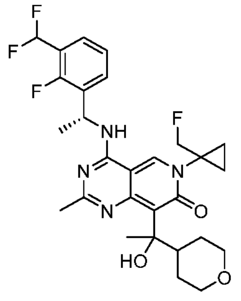
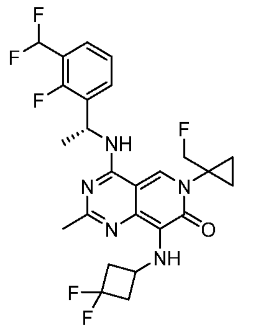
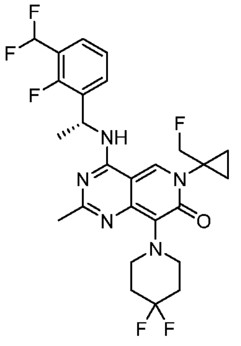

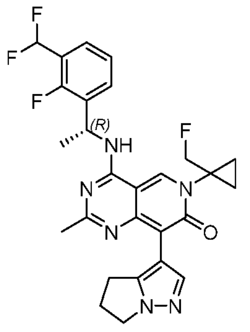 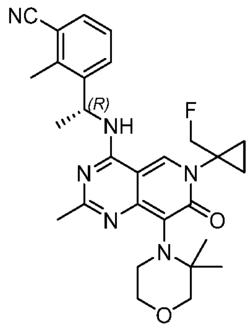 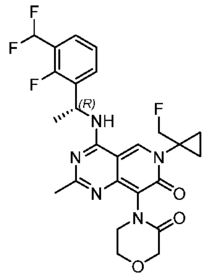 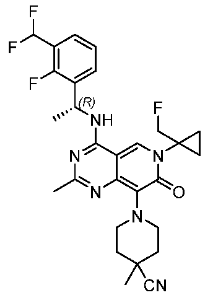 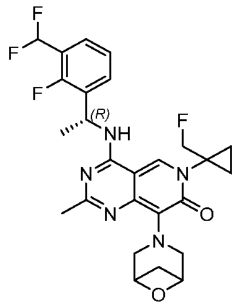

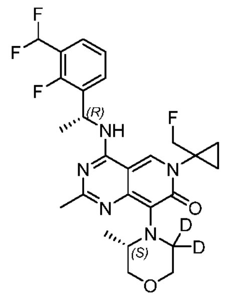 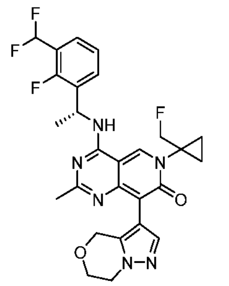 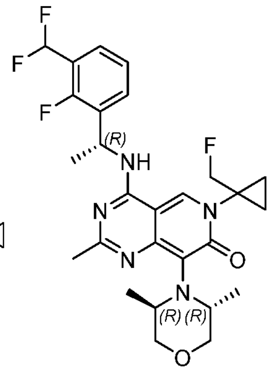 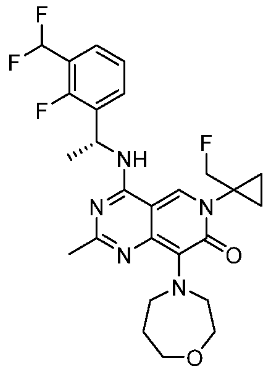 or

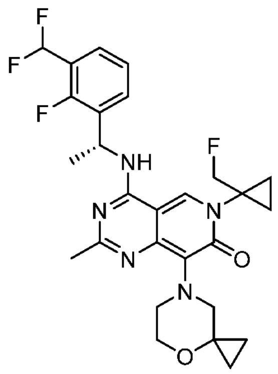.

**15.** A pharmaceutical composition, comprising the compound of formula (I) or the stereoisomer thereof or the pharmaceutically acceptable salt thereof according to any one of claims 1-14 and a pharmaceutically acceptable excipient.

**16.** Use of the compound of formula (I) or the stereoisomer thereof or the pharmaceutically acceptable salt thereof according to any one of claims 1-14 or the pharmaceutical composition according to claim 15 in preparing a medicament for use in preventing or treating an SOS1-related disease.

**17.** The use according to claim 16, wherein the SOS 1-related disease is selected from cancer.

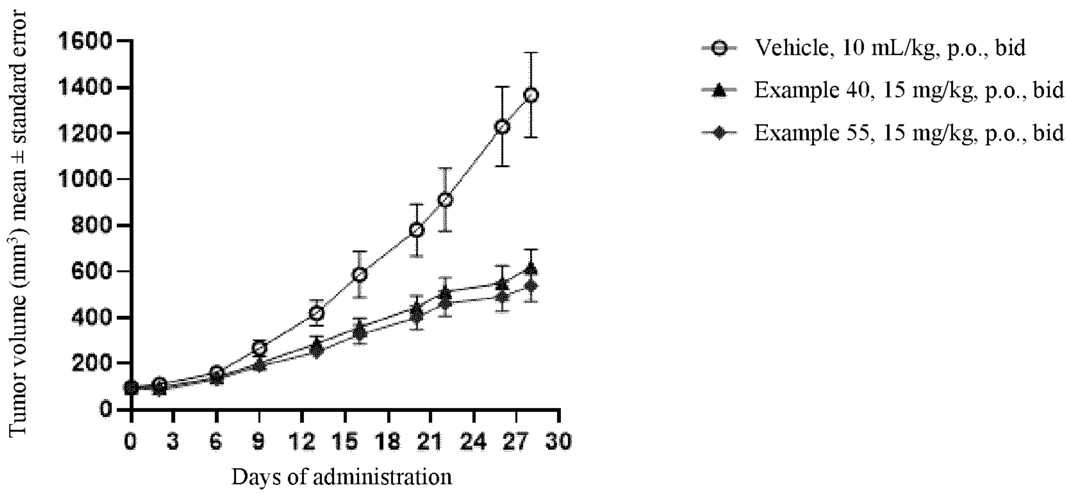
Tumor volume (mm3) mean ± standard error
1600
1400
1200
1000
800
600
400
200
0
0 3 6 9 12 15 18 21 24 27 30
Days of administration
Vehicle, 10 mL/kg, p.o., bid
Example 40, 15 mg/kg, p.o., bid
Example 55, 15 mg/kg, p.o., bid

FIG. 1

**INTERNATIONAL SEARCH REPORT**

International application No.
**PCT/CN2022/119376**

**A. CLASSIFICATION OF SUBJECT MATTER**

C07D 471/04(2006.01)i; A61K 31/519(2006.01)i; A61P 35/00(2006.01)i

According to International Patent Classification (IPC) or to both national classification and IPC

**B. FIELDS SEARCHED**

Minimum documentation searched (classification system followed by classification symbols)

C07，A61K，A61P

Documentation searched other than minimum documentation to the extent that such documents are included in the fields searched

Electronic data base consulted during the international search (name of data base and, where practicable, search terms used)

WPI, CNPAT, REGISTRY(STN), MARPAT(STN), CAPLUS(STN), CNKI, 万方, WANFANG: SOS1, 抑制剂, 吡啶并嘧啶酮, 杂环, 杂芳环, 稠合, 苄氨基, 癌, 肿瘤, inhibitor?, pyridopyrimidinone, fused, heterocycle, heterocyclic, benzylamino+, cancer?, tumor?, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| X | CN 111372932 A (BOEHRINGER INGELHEIM INTERNATIONAL GMBH) 03 July 2020 (2020-07-03)<br>claims 1, 21, and 25-26, and description, tables 20-24 | 1-17 |
| X | CN 113200981 A (HANGZHOU INNOGATE PHARMA CO., LTD.) 03 August 2021 (2021-08-03)<br>claims 1-12 and 14-17 | 1-17 |
| X | WO 2020254451 A1 (BOEHRINGER INGELHEIM INTERNATIONAL GMBH) 24 December 2020 (2020-12-24)<br>claim 1, and description, tables 20-24 | 1-17 |
| PX | WO 2021259972 A1 (BOEHRINGER INGELHEIM INTERNATIONAL GMBH) 30 December 2021 (2021-12-30)<br>claims 1, 3, and 6 | 1-17 |
| PX | WO 2022140427 A1 (QILU REGOR THERAPEUTICS INC et al.) 30 June 2022 (2022-06-30)<br>claim 1, and embodiments 1-77 | 1-17 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **24 November 2022** | **13 December 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2022/119376**

**C. DOCUMENTS CONSIDERED TO BE RELEVANT**

| Category* | Citation of document, with indication, where appropriate, of the relevant passages | Relevant to claim No. |
|---|---|---|
| PX | WO 2022166974 A1 (WUHAN HUMANWELL INNOVATIVE DRUG RESEARCH AND DEVELOPMENT CENTER LIMITED COMPANY) 11 August 2022 (2022-08-11)<br>claims 1, 18, and 28-33 | 1-17 |

Form PCT/ISA/210 (second sheet) (January 2015)

**INTERNATIONAL SEARCH REPORT**

International application No.
**PCT/CN2022/119376**

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☑ Claims Nos.: **1-11,15-17**
because they relate to parts of the international application that do not comply with the prescribed requirements to such an extent that no meaningful international search can be carried out, specifically:

[1] The structural range defined in claims 1-11 is very broad, and the compounds involved in said claims cover a large number of known compounds in the prior art. Claims 15-17 substantially refer to any one of claims 1-11. It is difficult for the examiner to exhaust searches of the prior art for the current claim scope. Regarding the described claims, the present search report was formed on the basis of the technical solution of the compound having the structure of formula (II) as defined in claim 12 involved in the compounds of the embodiments of the present application.

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2022/119376**

| Patent document cited in search report | | | Publication date (day/month/year) | Patent family member(s) | | | Publication date (day/month/year) |
|---|---|---|---|---|---|---|---|
| CN | 111372932 | A | 03 July 2020 | AU | 2018390927 | A1 | 28 May 2020 |
| | | | | IL | 275379 | A | 30 July 2020 |
| | | | | US | 2021009588 | A1 | 14 January 2021 |
| | | | | WO | 2019122129 | A1 | 27 June 2019 |
| | | | | US | 2019194192 | A1 | 27 June 2019 |
| | | | | PE | 20210163 | A1 | 26 January 2021 |
| | | | | CR | 20210307 | A | 27 July 2021 |
| | | | | EA | 202091491 | A1 | 13 November 2020 |
| | | | | CO | 2020007218 | A2 | 19 June 2020 |
| | | | | KR | 20200111163 | A | 28 September 2020 |
| | | | | TW | 201938557 | A | 01 October 2019 |
| | | | | CL | 2021000907 | A1 | 29 October 2021 |
| | | | | CA | 3085835 | A1 | 27 June 2019 |
| | | | | CL | 2020001501 | A1 | 13 November 2020 |
| | | | | JP | 2021506864 | A | 22 February 2021 |
| | | | | EC | SP20040257 | A | 31 August 2020 |
| | | | | CR | 20200312 | A | 11 September 2020 |
| | | | | SG | 11202005881 Y | A | 29 July 2020 |
| | | | | JO | P20200154 | A1 | 21 June 2019 |
| | | | | MA | 51290 | A | 31 March 2021 |
| | | | | EP | 3728254 | A1 | 28 October 2020 |
| | | | | BR | 112020010123 | A2 | 10 November 2020 |
| | | | | AR | 114164 | A1 | 29 July 2020 |
| | | | | UA | 126173 | C2 | 25 August 2022 |
| | | | | SA | 520412278 | B1 | 17 August 2022 |
| CN | 113200981 | A | 03 August 2021 | | None | | |
| WO | 2020254451 | A1 | 24 December 2020 | CN | 114375202 | A | 19 April 2022 |
| | | | | JP | 2022537044 | A | 23 August 2022 |
| | | | | CA | 3142239 | A1 | 24 December 2020 |
| | | | | US | 2022249492 | A1 | 11 August 2022 |
| | | | | BR | 112021024532 | A2 | 24 May 2022 |
| | | | | CL | 2021003353 | A1 | 30 September 2022 |
| | | | | TW | 202114683 | A | 16 April 2021 |
| | | | | KR | 20220024191 | A | 03 March 2022 |
| | | | | AU | 2020296914 | A1 | 23 December 2021 |
| | | | | EP | 3986408 | A1 | 27 April 2022 |
| WO | 2021259972 | A1 | 30 December 2021 | | None | | |
| WO | 2022140427 | A1 | 30 June 2022 | | None | | |
| WO | 2022166974 | A1 | 11 August 2022 | CN | 114907341 | A | 16 August 2022 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202111094943
- CN 202111236113
- CN 202111333282
- CN 202111431104
- CN 202111683730
- CN 202210185443
- CN 202210343652X
- WO 2018115380 A1
- WO 2018172250 A1
- CN 111372932 A


### Non-patent literature cited in the description


- **MCCORMICK et al.** *J.Mol.Med. (Berl).,* 2016, vol. 94 (3), 253-8
- **NIMNUAL et al.** *Sci. STKE.,* 2002, vol. 2002 (145), pe36
- **MALUMBRES ; BARBACID.** *Nature Reviews Cancer,* 2002
- **PYLAYEVA-GUPTA et al.** *Nature Reviews Cancer,* 2011
- **FREEDMAN et al.** *Proc. Natl. Acad. Sci. USA.,* 2006, vol. 103 (45), 16692-7
- **PIERRE et al.** *Biochem. Pharmacol.,* 2011, vol. 82 (9), 1049-56
- **JENG et al.** *Nat. Commun.,* 2012, vol. 3, 1168
- **BURNS et al.** *Proc. Natl. Acad. Sci.,* 2014, vol. 111 (9), 3401-6
- **MAEHR.** *J. Chem. Ed.,* 1985, vol. 62, 114-120